# EUROPEAN PATENT APPLICATION

(11) **EP 4 778 514 A2**
(43) Date of publication of application: **22.07.2026**
(21) Application number: 26183102.8
(22) Date of filing: 22.11.2023
(51) Int. Cl.: A61K 9/1272

(54) **NOVEL LIPIDS USED FOR NUCLEIC ACID DELIVERY AND LIPID NANOPARTICLE COMPOSITION**

(30) Priority: 24.11.2022 CN 202211480456
(62) Divisional of application: 23866704.2
(71) Applicant: Themedium Therapeutics Co., Ltd, Suzhou, Jiangsu 215011 (CN)
(72) Inventor: ZHANG, Lei, Suzhou, Jiangsu, 215011 (CN); CHEN, Jianxin, Suzhou, Jiangsu, 215011 (CN); YAN, Lu, Suzhou, Jiangsu, 215011 (CN)
(74) Representative: LLR

(57) **Abstract**

The present application provides a novel cationic lipid compound, which can be applied to a lipid nanoparticle, and the lipid nanoparticle is capable of targeting different tissues and organs for drug delivery. Further, the lipid nanoparticle may further comprise at least one helper lipid. The novel cationic lipid compound or a lipid nanoparticle composition comprising the same is capable of specifically delivering a prophylactic/therapeutic agent, particularly a nucleic acid component, to a target organ.

## Description

### TECHNICAL FIELD

The present disclosure relates generally to the field of molecular biology. More particularly, it relates to use of a novel lipid and a lipid nanoparticle composition comprising the same in organ-specific delivery of substances such as nucleic acids.

### BACKGROUND

Therapeutic or prophylactic nucleic acids have the potential to revolutionize vaccination, gene therapy, protein replacement therapy, and other therapies for genetic diseases. Since the first clinical study on therapeutic nucleic acids in 2000, significant advances have been made in the research on the design of nucleic acid molecules and the method for delivering them. However, nucleic acid drugs (including therapeutic and prophylactic drugs) still face several challenges, including that most liposome formulations accumulate through biological processes in the liver, thereby reducing the efficacy of compositions delivered into target organs. Similarly, other therapeutic agents such as proteins and small-molecule drugs can benefit from organ-specific delivery. Many different types of compounds such as chemical drugs exhibit significant cytotoxicity. If these compounds could be better directionally delivered to the desired organ, fewer off-target effects and side effects would be seen.

Currently, most lipid nanoparticle delivery systems passively target the liver. A common strategy to alter the organ targeted by a lipid nanoparticle is to adjust the composition of the lipid nanoparticle. According to some reports, lipid nanoparticles composed of 1-2 types of lipids can achieve the purpose of targeting the spleen by adjusting the ratio in which the nucleic acid and the nanoparticles are mixed (Stephan Grabbe et al., Translating Nanoparticulate-personalized Cancer Vaccines into Clinical Applications: Case Study with RNA-Lipoplexes for the Treatment of Melanoma, 2016); however, this strategy needs to be improved in terms of formulation stability, encapsulation efficiency, etc. In addition, according to some reports (Cheng Qiang et al., Selective organ targeting (SORT) nanoparticles for tissue-specific mRNA delivery and CRISPR-Cas gene editing, 2020), the addition of permanently anionic lipids to LNPs consisting of four components-a cationizable lipid, a steroid, a phospholipid, and a PEG lipid-also enables the specific targeting of the spleen, but at a price of an increase in the composition and an increase in the complexity of the formulation process. Therefore, a lipid nanoparticle delivery system with high delivery capacity, high stability and low complexity remains to be developed.

### SUMMARY

The present application aims to provide use of a cationic lipid compound in a nucleic acid drug delivery system, wherein the nucleic acid drug delivery system is capable of targeting nucleic acid drug molecules to different tissues and organs for drug delivery, and realizing efficient expression of nucleic acids, with low expression level of nucleic acids in liver, thereby reducing toxicity.

The present application provides a novel cationic lipid compound, which can be applied to a lipid nanoparticle, and the lipid nanoparticle is capable of targeting different tissues and organs for drug delivery. The cationic lipid compound is one or more of compounds represented by Formula (I) or pharmaceutically acceptable salts, prodrugs, stereoisomers or deuterides thereof: 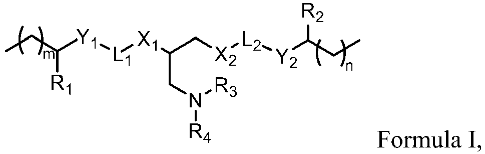
wherein X₁ and X₂ are each independently -OC(=O)-, -C(=O)O-, -NHC(=O)-, or -C(=O)NH-;
Y₁ and Y₂ are each independently -OC(=O)-, -C(=O)O-, -NR₅C(=O)-, -C(=O)NR₅-bond, optionally substituted C1-C8 alkylene, or optionally substituted C2-C8 alkenylene, wherein R₅ is selected from hydrogen or linear or branched C1-C8 hydrocarbyl;
L₁ and L₂ are each independently a bond, or optionally substituted C1-C10 alkylene;
R₃ and R₄ are each independently methyl, ethyl, propyl, hydroxymethyl, hydroxyethyl, hydroxypropyl, hydroxybutyl, 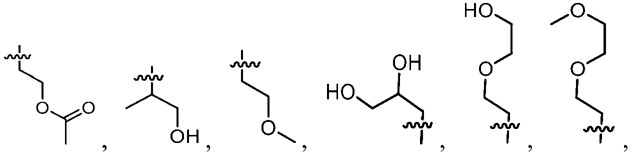 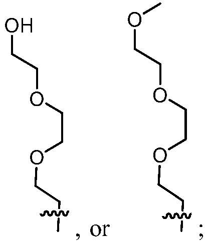 or R₃ and R₄, together with the nitrogen attached thereto, form a cyclic moiety, wherein R₃ or R₄ are selected from a group containing hydrogen or deuterium, and the cyclic moiety is 4- to 8-membered heterocycloalkyl selected from azetidin-1-yl, pyrrolidinyl, piperidin-1-yl, 4-hydroxypiperidin-1-yl, azepan-1-yl, morpholinyl, and 4-acetylpiperazin-1-yl; R₁ and R₂ are each independently H or deuterium or linear or branched C1-C8 hydrocarbyl;
m is an integer of 1-13, for example, m is 1, 2, 3, 4, 5, 6, 7, 8, 9, 10, 11, 12, or 13;
n is an integer of 1-13, for example, n is 1, 2, 3, 4, 5, 6, 7, 8, 9, 10, 11, 12, or 13.

According to some specific and preferred embodiments, the cationic lipid compound is one or more of the compounds represented by the following structural formulas:

**Table 1. Representative compounds**

| No. | Structural formula |
|---|---|
| 1 | 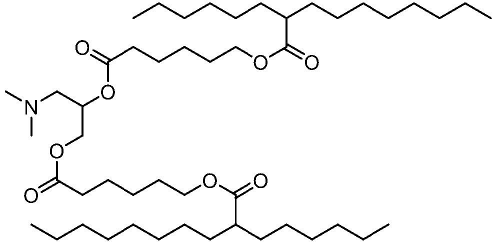 |
| 2 | 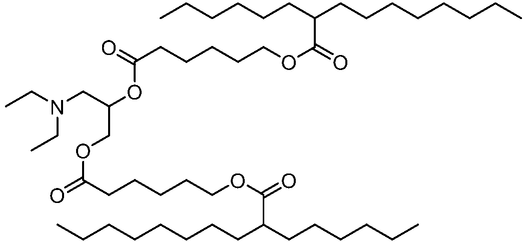 |
| 3 | 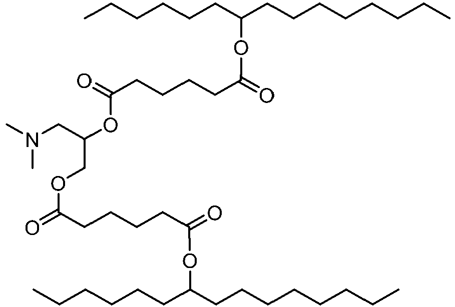 |
| 4 | 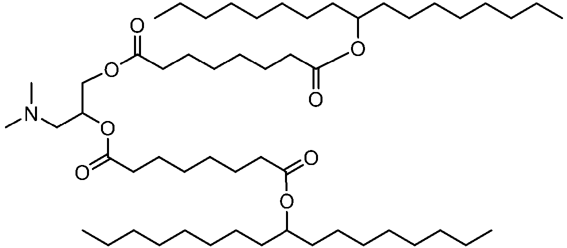 |
| 5 | 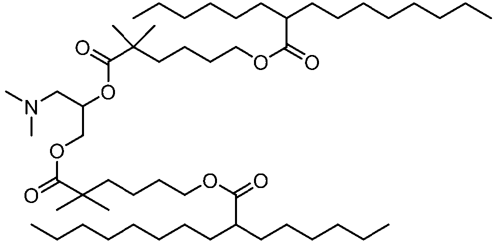 |
| 6 | 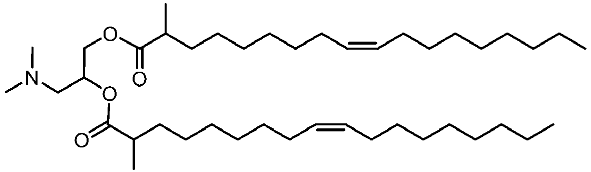 |
| 7 | 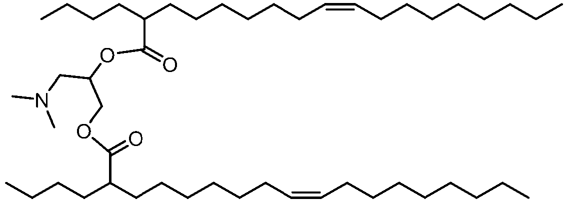 |
| 8 | 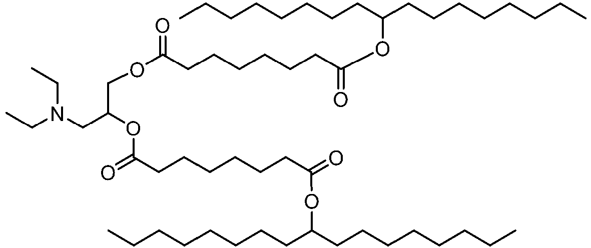 |
| 9 | 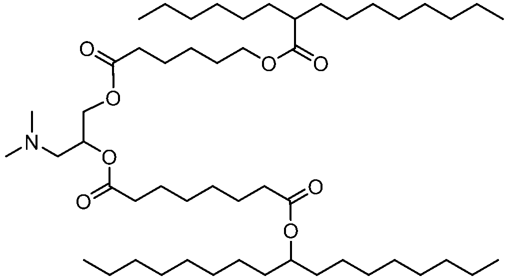 |
| 10 | 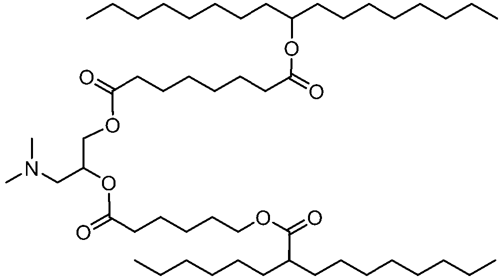 |
| 11 | 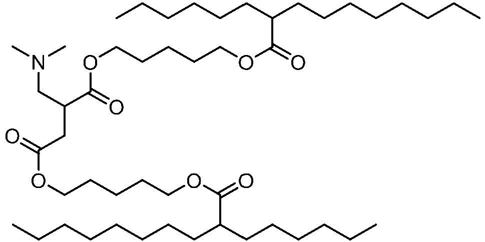 |
| 12 | 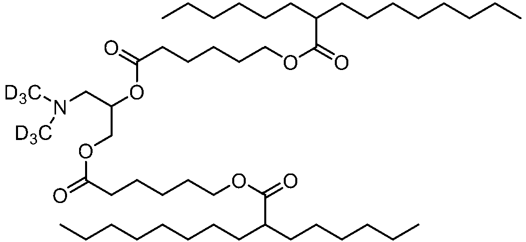 |
| 13 | 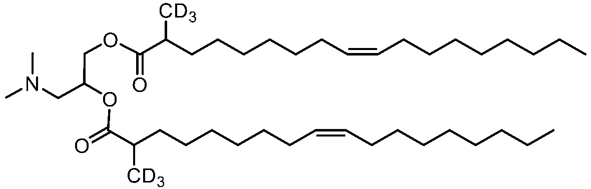 |
| 14 | 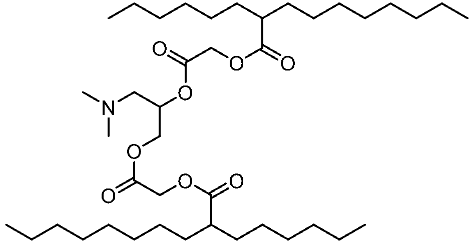 |
| 15 | 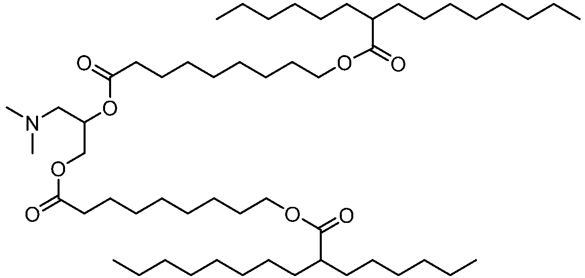 |
| 16 | 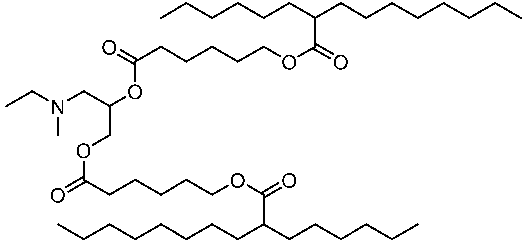 |
| 17 | 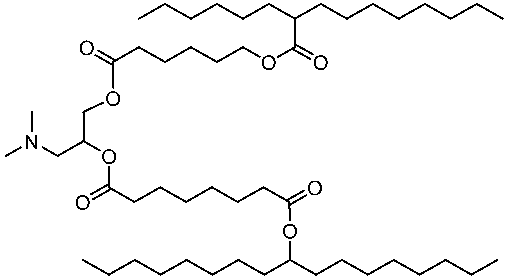 |
| 18 | 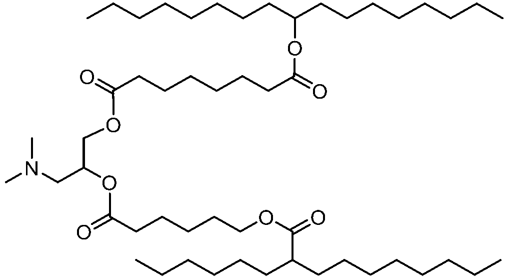 |
| 19 | 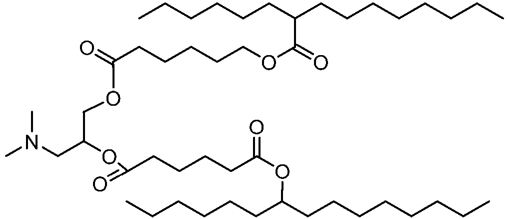 |
| 20 | 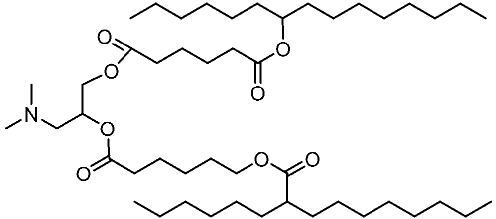 |
| 21 | 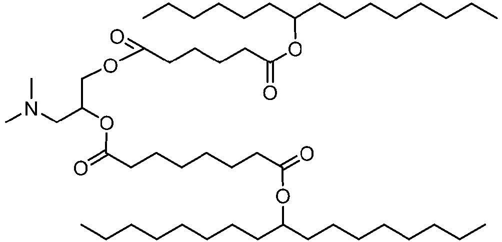 |
| 22 | 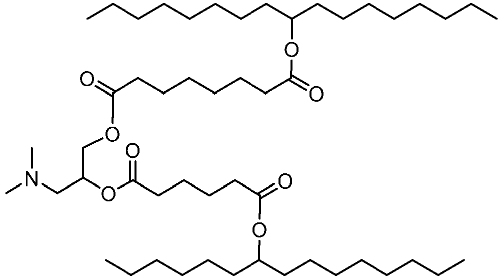 |
| 23 | 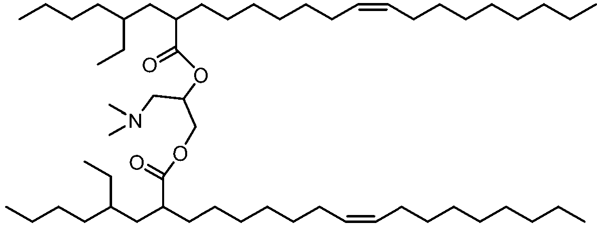 |
| 24 | 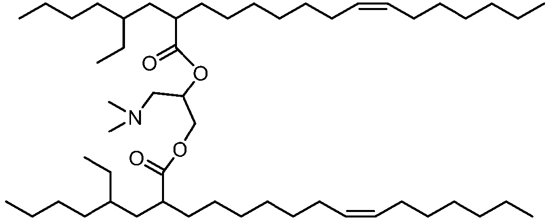 |
| 25 | 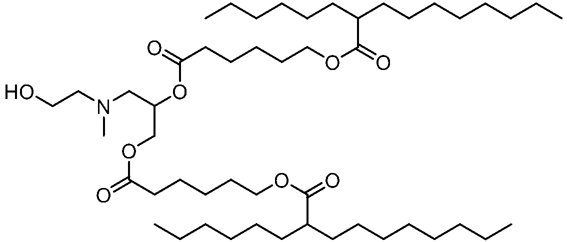 |
| 26 | 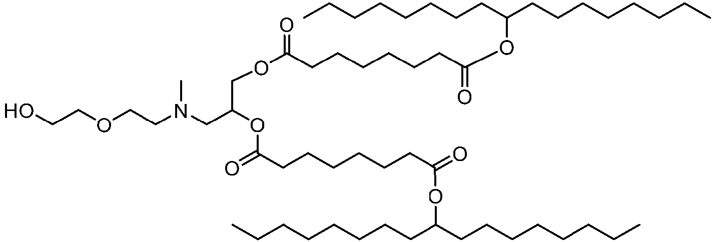 |
| 27 | 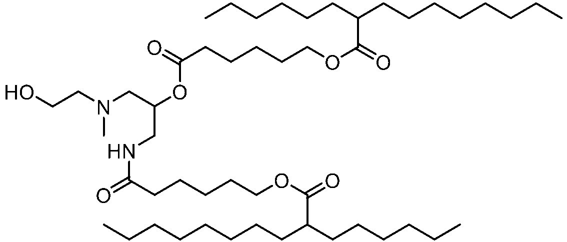 |
| 28 | 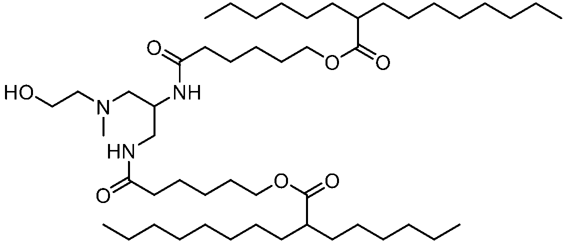 |
| 29 | 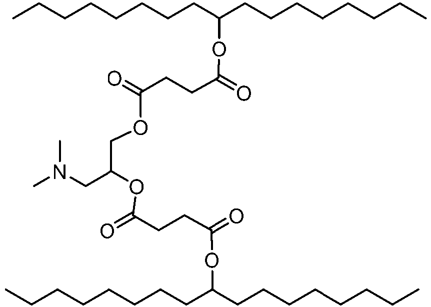 |
| 30 | 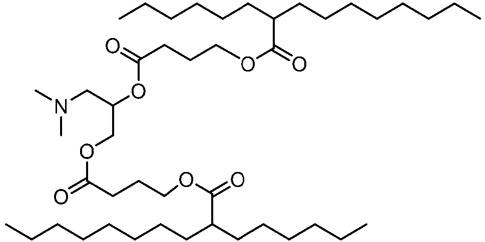 |
| 31 | 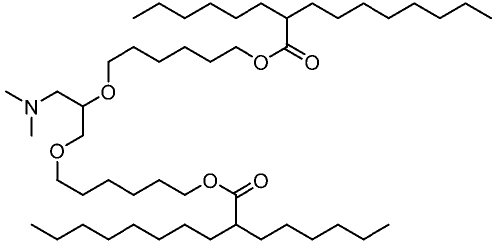 |
| 32 | 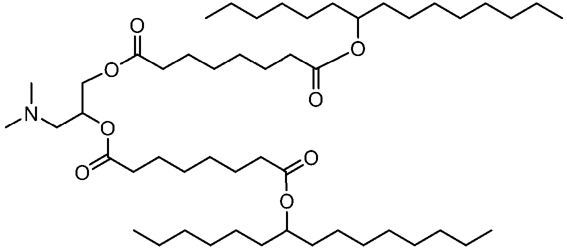 |
| 33 | 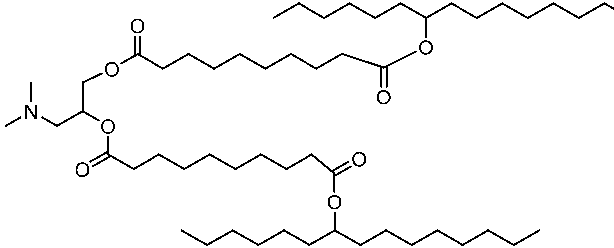 |
| 34 | 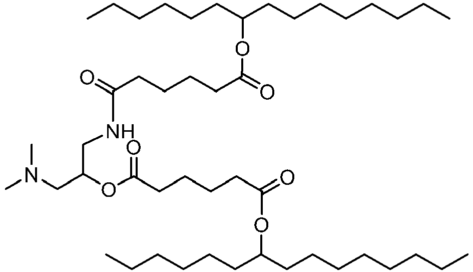 |
| 35 | 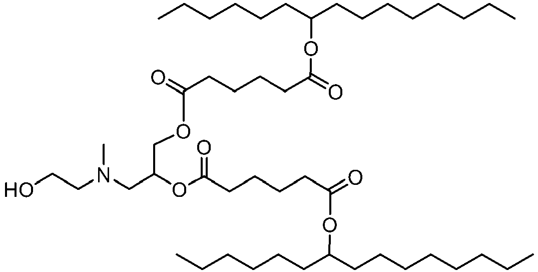 |
| 36 | 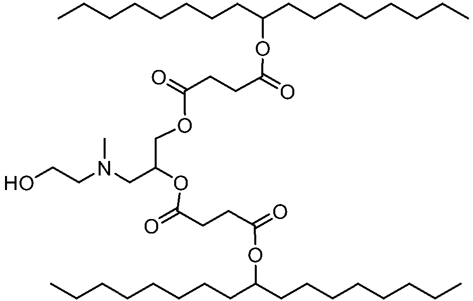 |
| 37 | 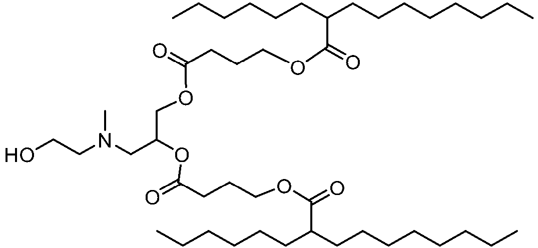 |
| 38 | 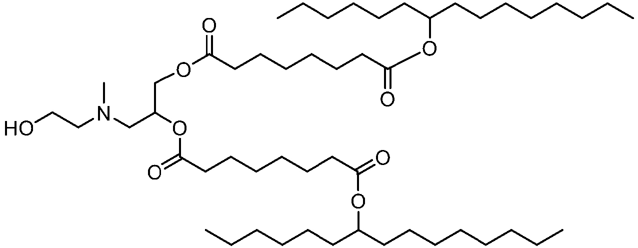 |
| 39 | 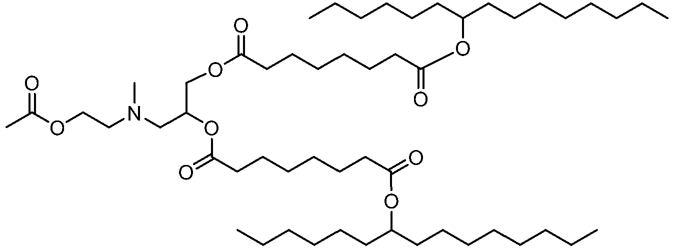 |
| 40 | 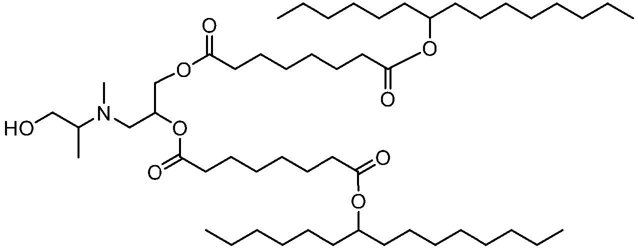 |
| 41 | 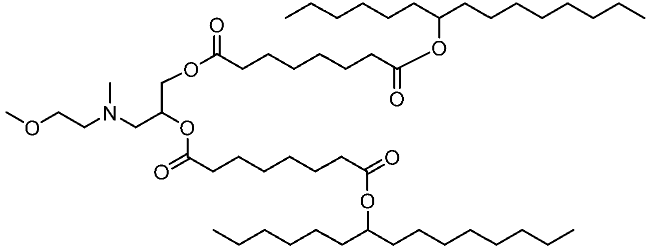 |
| 42 | 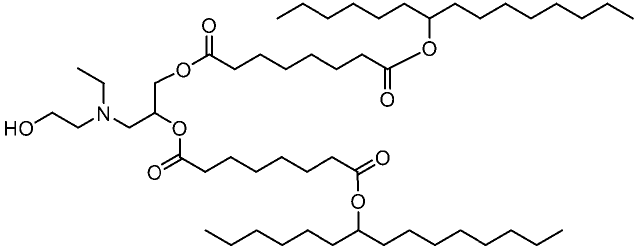 |
| 43 | 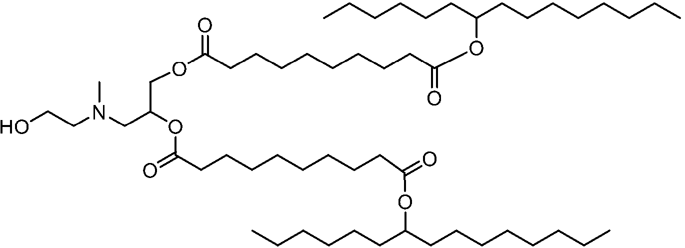 |
| 44 | 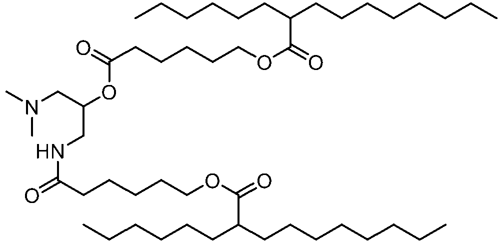 |
| 45 | 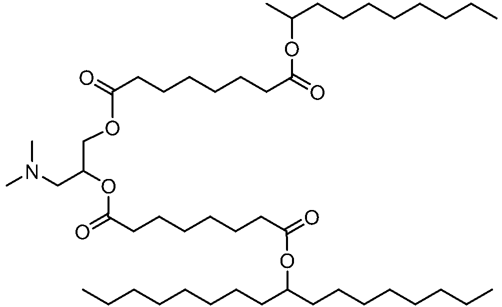 |
| 46 | 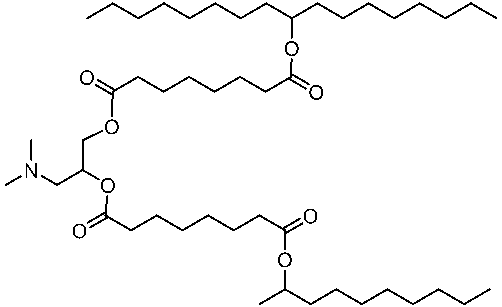 |
| 47 | 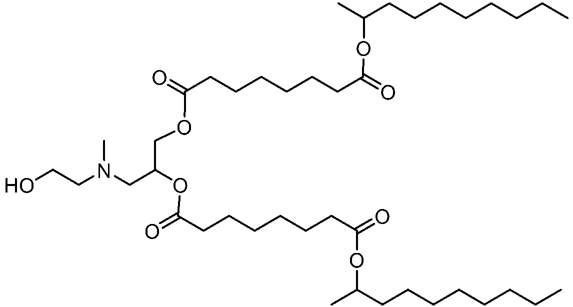 |
| 48 | 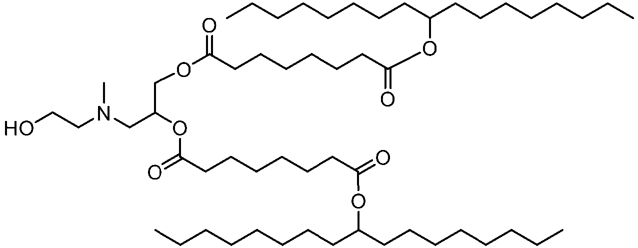 |
| 49 | 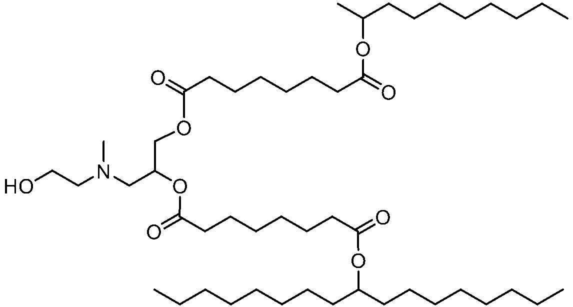 |
| 50 | 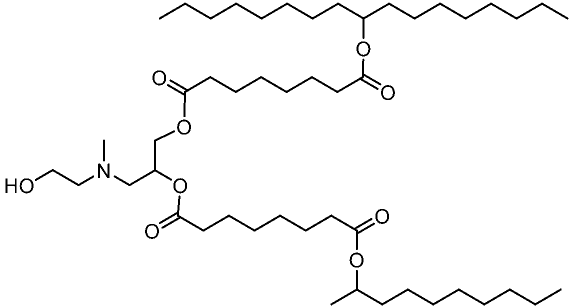 |
| 51 | 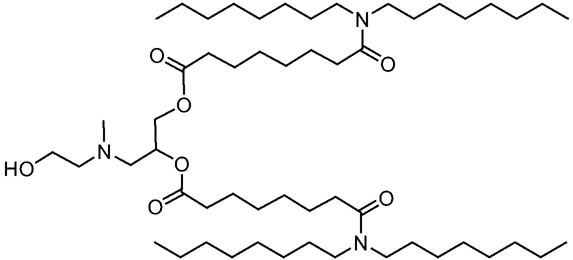 |
| 52 | 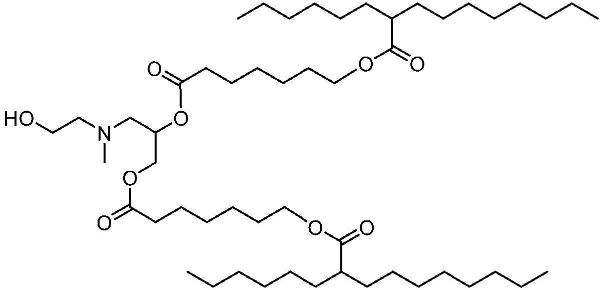 |
| 53 | 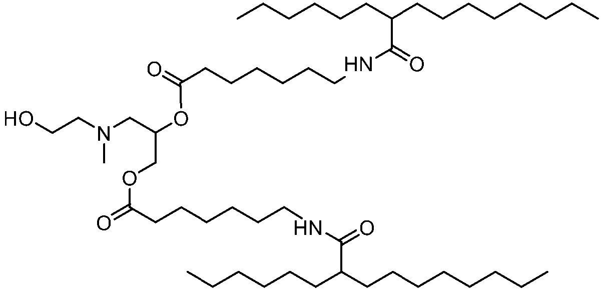 |
| 54 | 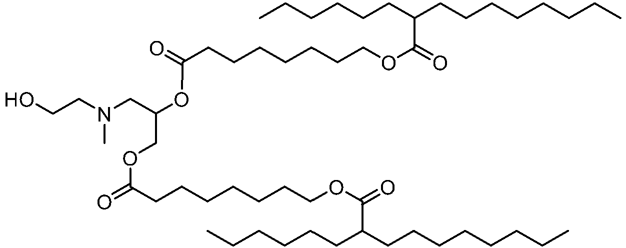 |
| 55 | 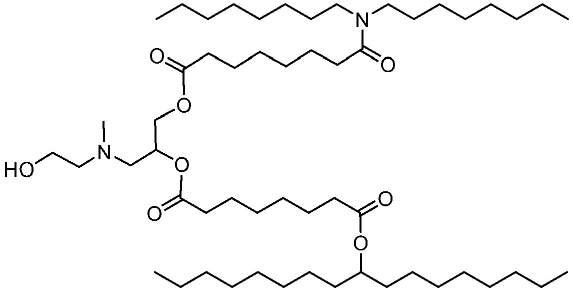 |
| 56 | 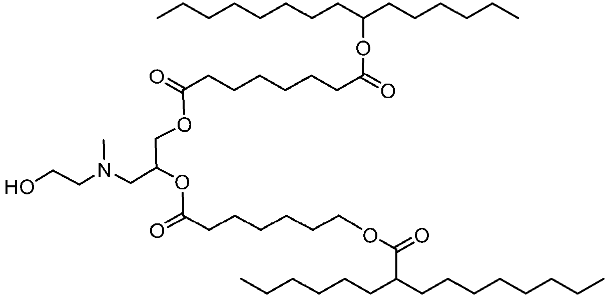 |
| 57 | 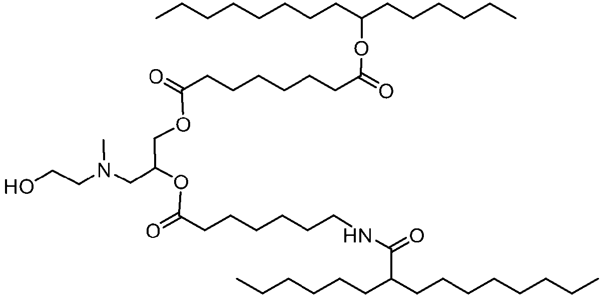 |
| 58 | 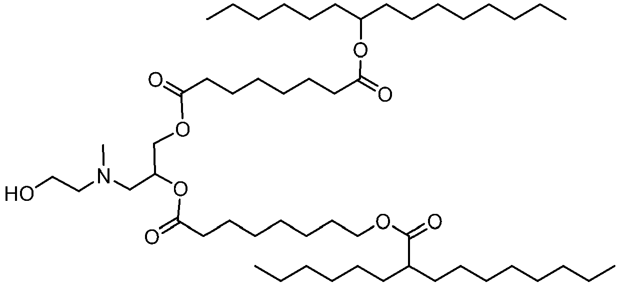 |
| 59 | 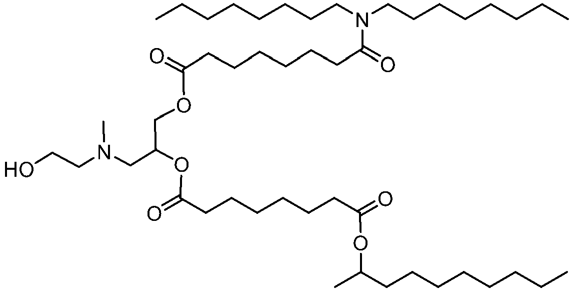 |
| 60 | 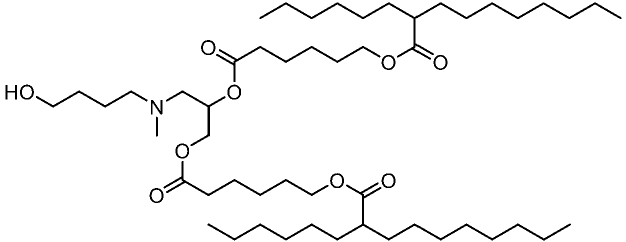 |
| 61 | 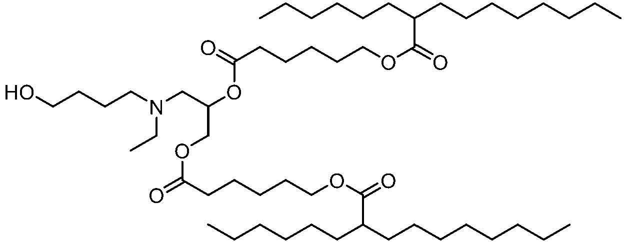 |
| 62 | 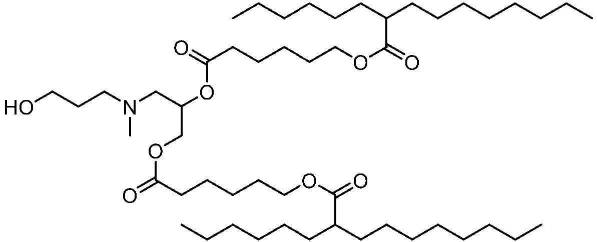 |
| 63 | 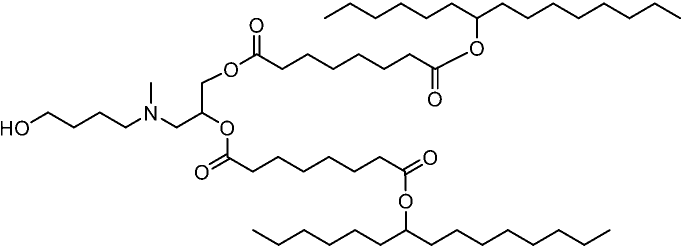 |
| 64 | 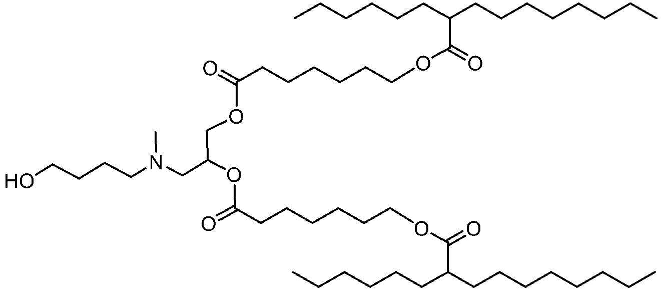 |
| 65 | 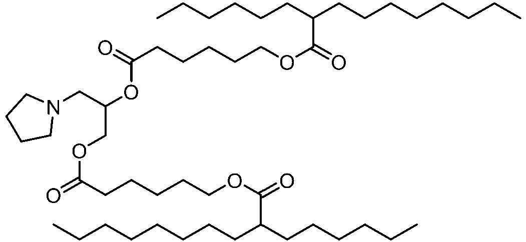 |
| 66 | 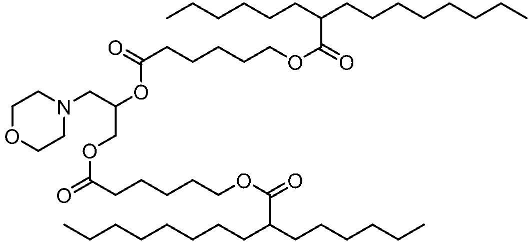 |
| 67 | 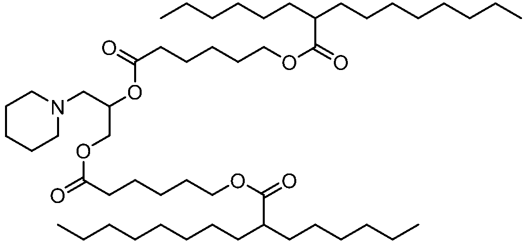 |
| 68 | 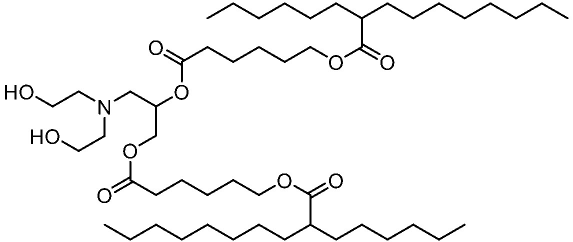 |
| 69 | 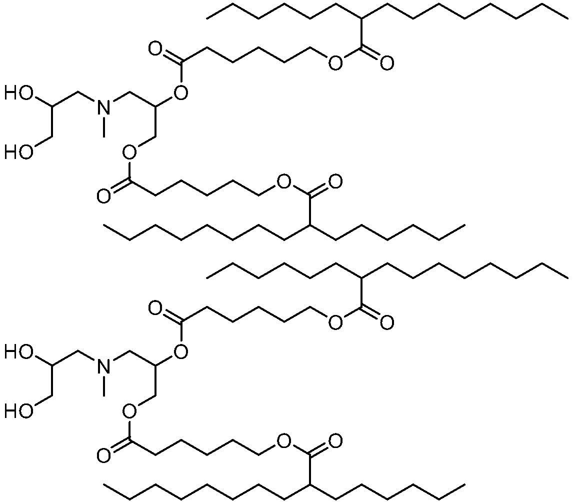 |
| 70 | 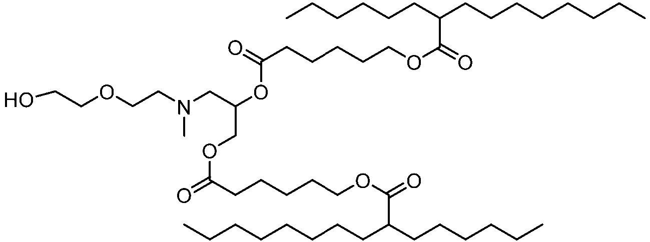 |
| 71 | 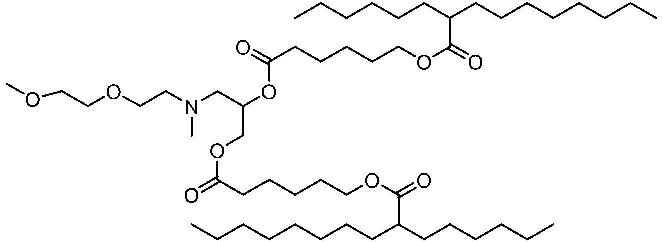 |
| 72 | 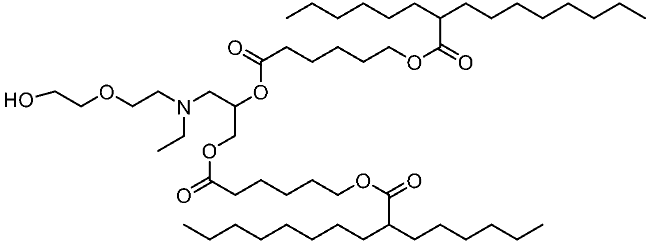 |
| 73 | 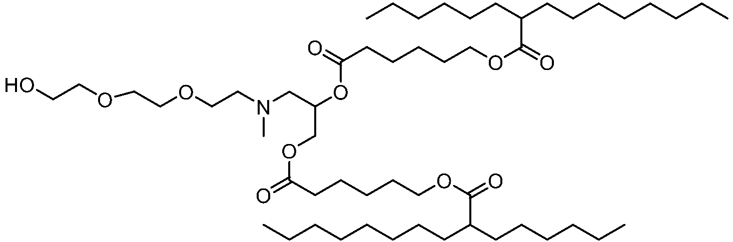 |
| 74 | 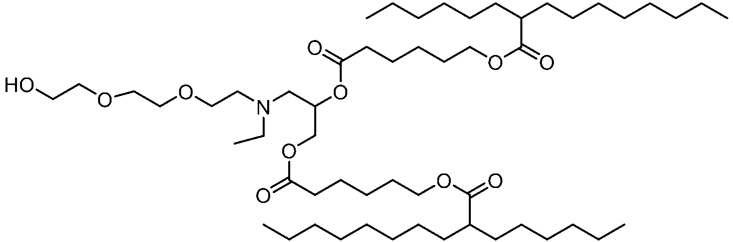 |
| 75 | 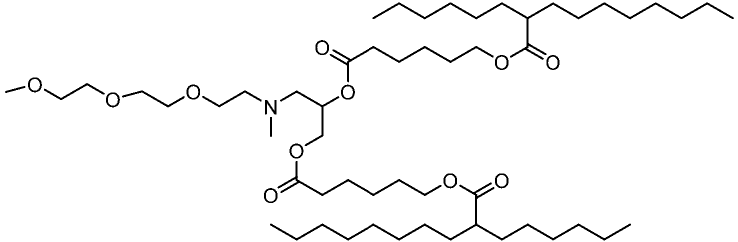 |
| 76 | 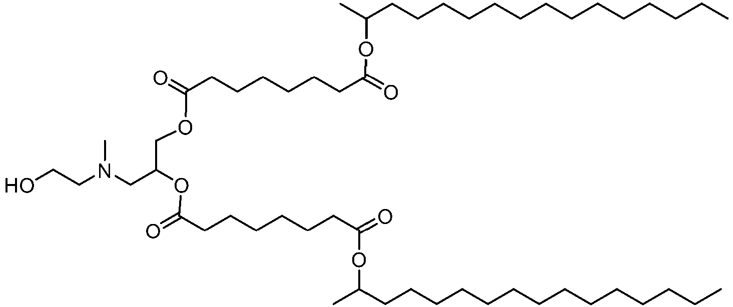 |
| 77 | 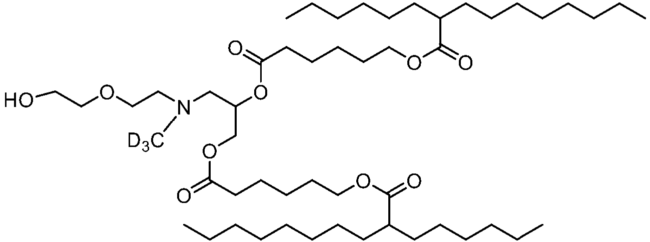 |
| 78 | 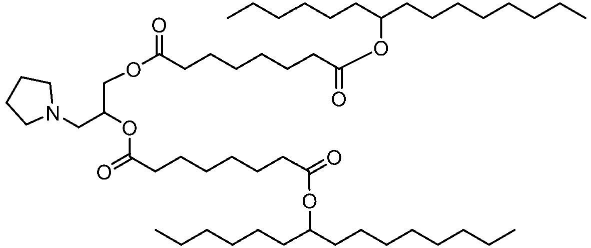 |
| 79 | 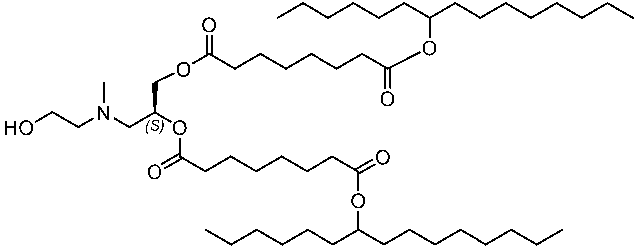 |
| 80 | 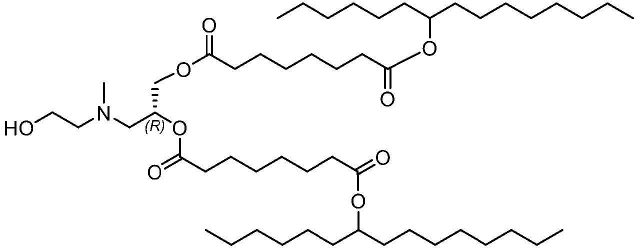 |
| 81 | 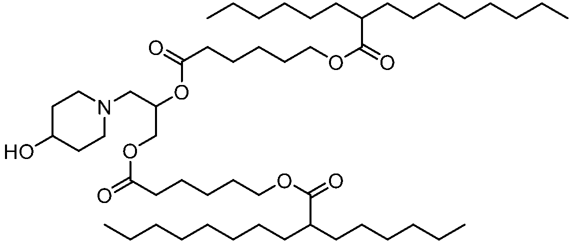 |
| 82 | 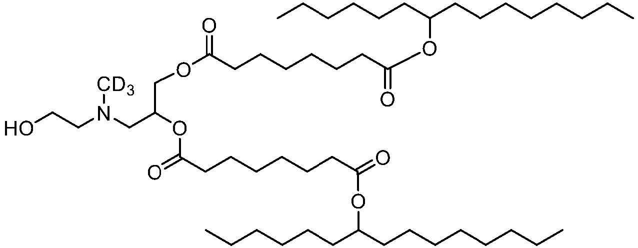 |

Particularly, the "pharmaceutically acceptable salt" refers to salts of the compound of the present disclosure that are pharmaceutically acceptable as defined above and that have desired pharmacological activity. Such salts include acid addition salts formed with the following acids: inorganic acids such as hydrochloric acid, hydrobromic acid, sulfuric acid, nitric acid, phosphoric acid, and the like; or organic acids such as 1,2-ethanedisulfonic acid, 2-hydroxyethanesulfonic acid, 2-naphthalenesulfonic acid, 3-phenylpropionic acid, 4,4'-methylene-bis(3-hydroxy-2-ene-1-carboxylic acid), 4-methylbicyclo[2.2.2]oct-2-ene-1-carboxylic acid, acetic acid, aliphatic monocarboxylic and dicarboxylic acids, aliphatic sulfuric acid, aromatic sulfuric acid, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, lauryl sulfuric acid, maleic acid, malic acid, malonic acid, mandelic acid, methanesulfonic acid, muconic acid, o-(4-hydroxybenzoyl)benzoic acid, oxalic acid, p-chlorobenzenesulfonic acid, phenyl-substituted alkanoic acid, propionic acid, p-toluenesulfonic acid, pyruvic acid, salicylic acid, stearic acid, succinic acid, tartaric acid, *tert-*butylacetic acid, trimethylacetic acid, and the like. The pharmaceutically acceptable salts further include base addition salts which may be formed when acidic protons present are capable of reacting with inorganic or organic bases. The acceptable inorganic bases include, but are not limited to, sodium hydroxide, sodium carbonate, potassium hydroxide, aluminum hydroxide, and calcium hydroxide. The acceptable organic bases include, but are not limited to, ethanolamine, diethanolamine, triethanolamine, tromethamine, N-methylglucamine, and the like. It should be recognized that the particular anion or cation forming part of any salt of the present application is not critical so long as the salt as a whole is pharmacologically acceptable. Particularly, the "prodrug" refers to a compound, such as a therapeutic agent that can be converted into a biologically active compound under physiological conditions or by dissolution. The prodrug is generally rapidly converted *in vivo* to yield a parent compound, for example, by hydrolysis in blood. The prodrug compound generally has the advantage of solubility, histocompatibility, or delayed release in mammalian organisms. The term "prodrug" is also meant to include any covalently bonded carrier that, when administered to a mammalian subject, releases the active compound *in vivo.* The prodrug includes a compound in which hydroxyl, amino or sulfhydryl is bonded to any group that, when the prodrug is administered to the mammalian subject, is cleaved to form free hydroxyl, free amino, or free sulfhydryl. Examples of the prodrug include, but are not limited to, acetate, formate and benzoate derivatives, or amide derivatives of amine functional groups, etc.

Particularly, the "stereoisomers" or "optical isomers" are isomers of a given compound in which the same atoms are bonded to the same other atoms, but with a different three-dimensional configuration of those atoms. "Enantiomers" are stereoisomers of a given compound that are mirror images of each other as left and right hands. "Diastereoisomers" are stereoisomers of a given compound that are not enantiomers. Chiral molecules contain a chiral center (also referred to as a stereocenter or a stereogenic center), which is any point (although not necessarily an atom) in the molecule that carries multiple groups, such that the interchange of any 2 groups produces a stereoisomer. In organic compounds, the chiral center is typically a carbon, phosphorus or sulfur atom, although other atoms may also be stereocenters in organic and inorganic compounds. A molecule may have multiple stereocenters, giving rise to many of its stereoisomers. In compounds whose stereoisomerism is due to tetrahedral stereogenic centers (e.g., tetrahedral carbon), it is assumed that the total number of possible stereoisomers does not exceed 2n, wherein n is the number of tetrahedral stereocenters. Molecules with symmetry often have fewer stereoisomers than the maximum possible number of stereoisomers. A 50:50 mixture of enantiomers is referred to as a racemic mixture. Alternatively, a mixture of enantiomers may be enriched such that one enantiomer is present in an amount greater than 50%. Generally, enantiomers and/or diastereoisomers may be resolved or separated using techniques known in the art. It is contemplated that for any stereocenter or chiral axis for which stereochemistry has not been defined, the stereocenter or chiral axis may exist in its R form, S form, or as a mixture of the R form and the S form (including racemic and non-racemic mixtures). As used herein, the phrase "substantially free of other stereoisomers" means that the composition contains 15% or less, more preferably 10% or less, even more preferably 5% or less, or most preferably 1% or less of another stereoisomer or multiple other stereoisomers.

Deuterium (²H or D) is a stable and nonradioactive isotope of hydrogen, with a mass approximately twice that of hydrogen (H), and is the most common isotope of hydrogen.

The novel cationic lipid provided by the present application also has a targeted delivery function, and is a guided on-target lipid delivery (GOLD) lipid.

The present application provides a lipid nanoparticle comprising the cationic lipid described above. The cationic lipid compound is one or more of compounds represented by general formula (I) or pharmaceutically acceptable salts, prodrugs or stereoisomers thereof. Further, the lipid nanoparticle may further comprise at least one helper lipid, which is mixed with a drug or a molecule/agent with pharmaceutical activity to achieve encapsulation and specific delivery to a specific tissue and organ. The specificity for the tissue and organ is achieved by the cationic lipid, i.e., the guided on-target lipid delivery (GOLD) lipid, in the lipid nanoparticle. The helper lipid in the lipid nanoparticle is optionally one or more of a phospholipid, a steroid, a polymer-conjugated lipid, and a modifiable lipid. Preferably, the phospholipid is selected from any one of DOPE, DSPC, DPPC, DMPC, DOPC, POPC, and SM, or a combination thereof. Preferably, the steroid is selected from one or more of cholesterol, sitosterol, stigmasterol, and ergosterol, further preferably, the steroid is cholesterol and sitosterol. Preferably, the polymer in the polymer-conjugated lipid is a high-molecular-weight compound formed by covalent bonding of one or more small-molecule repeating units; the polymer may be selected from polyethylene glycol, polylactic acid, polyamide, cationic polymer, polysarcosine (pSar), poly lactic-co-glycolic acid (PLGA), polyamino acid, polypeptide, polypeptoid, etc.; preferably, the polymer-conjugated lipid is selected from a polyethylene glycol-conjugated lipid; further, the polyethylene glycol-conjugated lipid is selected from one or more of ALC-0159, PEG1000-DMG, PEG5000-DMG, PEG2000-DMG, and PEG2000-DSPE. Preferably, the modifiable lipid includes lipids modified by small-molecule compounds, vitamins, carbohydrates, peptides, proteins, nucleic acids, lipopolysaccharides, inorganic molecules or particles, metal ions or particles, and combinations of the substances described above. In the lipid nanoparticle, the molar ratio of the cationic lipid (guided on-target delivery lipid) to the helper lipid is 1:(0.5-2), preferably 1:(0.6-1.5), further preferably 1:(0.8-1.2).

The lipid nanoparticle is capable of delivering a therapeutic/prophylactic agent to the following target organ: lung, heart, brain, spleen, lymph node, bone, skeletal muscle, stomach, small intestine, large intestine/colorectum, kidney, bladder, breast, testis, ovary, uterus, spleen, thymus, brainstem, cerebellum, spinal cord, eye, ear, tongue, or skin. Preferably, the target organ is spleen.

In other aspects, the present application provides a composition comprising a therapeutic or prophylactic agent and the lipid nanoparticle described above. Preferably, the mass ratio of the lipid nanoparticle to the prophylactic or therapeutic agent in the composition is 10:1 to 100:1, preferably 20:1 to 50:1, further preferably 20:1 to 30:1.

The composition has an average particle size of 90 nm to 600 nm, preferably 200 nm to 400 nm, further preferably 200 nm to 300 nm.

The composition has a polydispersity index (PDI) of 0.001 to 0.5, preferably 0.001 to 0.45, further preferably 0.001 to 0.4.

In a preferred instance, the therapeutic/prophylactic agent is a nucleic acid. The nucleic acid includes any and all forms of nucleic acid molecules, including but not limited to, single-stranded DNA, double-stranded DNA, single-stranded RNA, double-stranded RNA, short isomers, plasmid DNA, complementary DNA/cDNA, antisense oligonucleotide/ASO, small interference nucleic acid/siRNA, small activating nucleic acid/saRNA, asymmetric interfering nucleic acid/aiRNA, micro nucleic acid/miRNA, miRNA agonist/antagonist (agomir/antagomir), Dicer-substrate nucleic acid, small hairpin nucleic acid/shRNA, transfer RNA (tRNA), messenger RNA/mRNA, circular RNA/circRNA, self-amplifying mRNA/samRNA, aptamers, and other forms of nucleic acid molecules known in the art or likely to be discovered/prepared in the future.

The nucleic acid molecules described above may include natural nucleotides, or may include nucleotide mimics or functional analogs, or may include chemically modified forms of nucleotides.

Functional nucleotide analogs include, but are not limited to, one of or a combination of more than one of a locked nucleic acid (LNA), a peptide nucleic acid (PNA), and a morpholine ring oligonucleotide nucleic acid mimic or functional analog.

The chemical modification of nucleotides may be located on a backbone bond of the nucleic acid molecule. The backbone bond may be modified by replacement of one or more oxygen atoms. The modification to the backbone bond may include replacing at least one phosphodiester bond with a phosphorothioate bond.

The chemical modification of nucleotides may be located on nucleosides. The modification on the nucleoside may be located on the sugar and base of the nucleoside. The modification of the sugar on the nucleoside may be selected from one or more of 2'-fluororibose, ribose, 2'-deoxyribose, arabinose, and hexose.

The form of the chemical modification of nucleotides may be selected from one or more of: 5-methylcytosine, pseudouridine, 1-methylpseudouridine, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyluridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. These modifications may be random or at specific sites.

In a preferred instance, the nucleic acid is an mRNA capable of encoding at least one antigen or a fragment thereof or an epitope thereof, or encoding a certain therapeutic protein, the antigen being selected from a pathogenic antigen, such as a tumor-associated antigen or a pathogenic microbial antigen. The mRNA may be a monocistronic mRNA or a polycistronic mRNA.

The present application further provides use of the composition described above in the preparation of medicaments.

The present application further provides a drug comprising the composition described above and a pharmaceutically acceptable auxiliary material. Pharmaceutical auxiliary materials refer to excipients and additives used in the manufacture of a pharmaceutical product and in the formulation of a pharmaceutical formula, and are substances, other than the active ingredient, which have been rationally evaluated for safety and are contained in a pharmaceutical formulation. In addition to shaping, serving as carriers, and enhancing stability, pharmaceutical auxiliary materials also have such important functions as solubilization, aiding dissolution, sustaining or controlling release, etc. They are important ingredients which possibly affect the quality, safety and effectiveness of drugs. According to the effect and the purpose, pharmaceutical auxiliary materials may be classified into solvents, propellants, solubilizers, cosolvents, emulsifiers, colorants, binders, disintegrants, fillers, lubricants, wetting agents, osmotic pressure regulators, stabilizers, glidants, flavoring agents, preservatives, suspending agents, coating materials, fragrances, anti-adhesives, integrating agents, permeation promotors, pH regulators, buffers, plasticizers, surfactants, foaming agents, defoaming agents, thickeners, inclusion agents, humectants, absorbents, diluents, flocculants, deflocculants, filter aids, release retardants, etc.

### Beneficial Effects of the Present Application:

The present application provides a novel cationic lipid and a lipid nanoparticle comprising the same, which can deliver a therapeutic/prophylactic agent, particularly a nucleic acid component, to a specific organ, particularly to organs other than the liver preferentially, so as to provide more options for the delivery of nucleic acid drugs, genetic vaccines and the like, and particularly have important significance for the development and application of nucleic acid prophylactic and therapeutic agents.

Conventional lipid nanoparticles generally target the liver for delivery. However, by using the novel cationic lipid molecule of the present application, the targeting characteristics of the lipid nanoparticle comprising the novel cationic lipid molecule can be altered. In lipid nanoparticles, such as TMF2 to TMF53, comprising the novel cationic lipid of the present application, the ratio of the distribution (fluorescence intensity) of nucleic acid drugs in the spleen to that in the liver is 1-70 (Table 5). However, in conventional lipid nanoparticles without the addition of the cationic lipid of the present application, there is generally a higher delivery efficiency in the liver (Ansell, S. M.; Du, X. Novel Lipids and Lipid Nanoparticle Formulations for Delivery of Nucleic Acids. WO2017075531 A1).

### BRIEF DESCRIPTION OF THE DRAWINGS

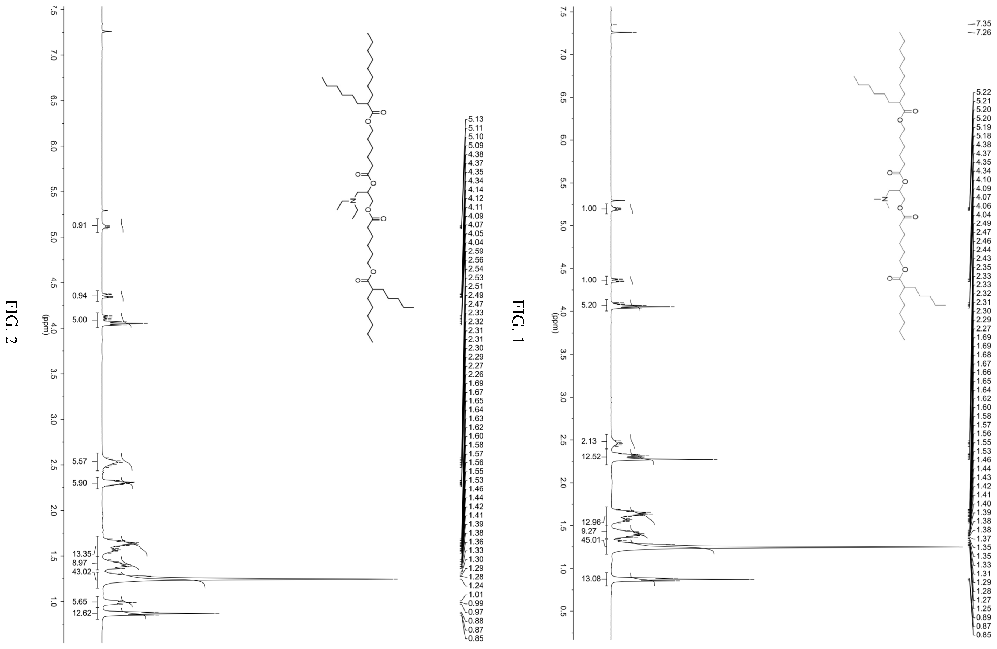

FIG. 1 is ¹H NMR of compound 1.
FIG. 2 is ¹H NMR of compound 2.

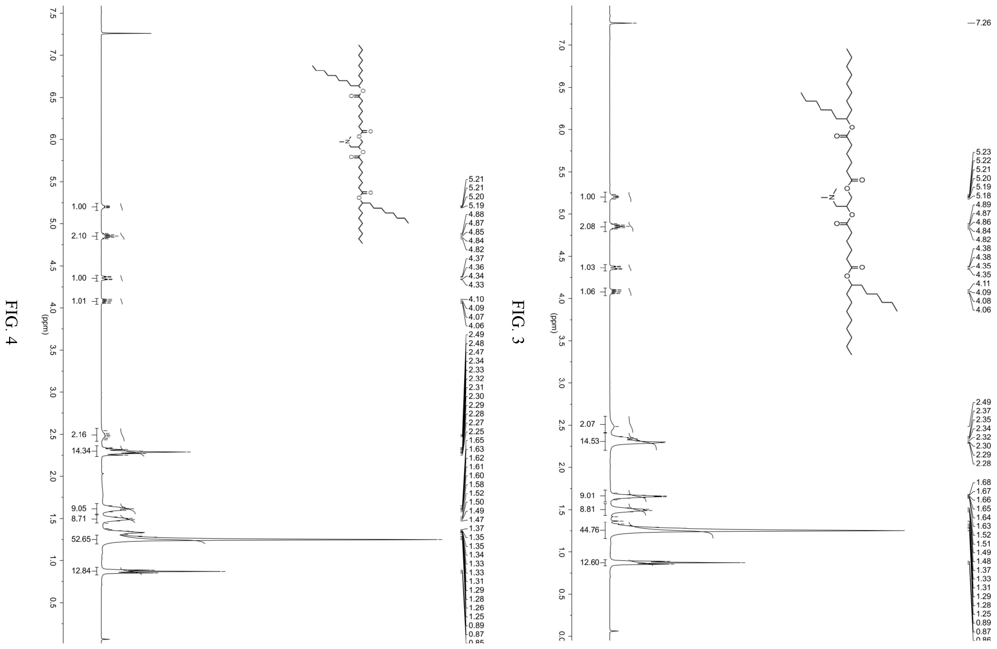

FIG. 3 is ¹H NMR of compound 3.
FIG. 4 is ¹H NMR of compound 4.

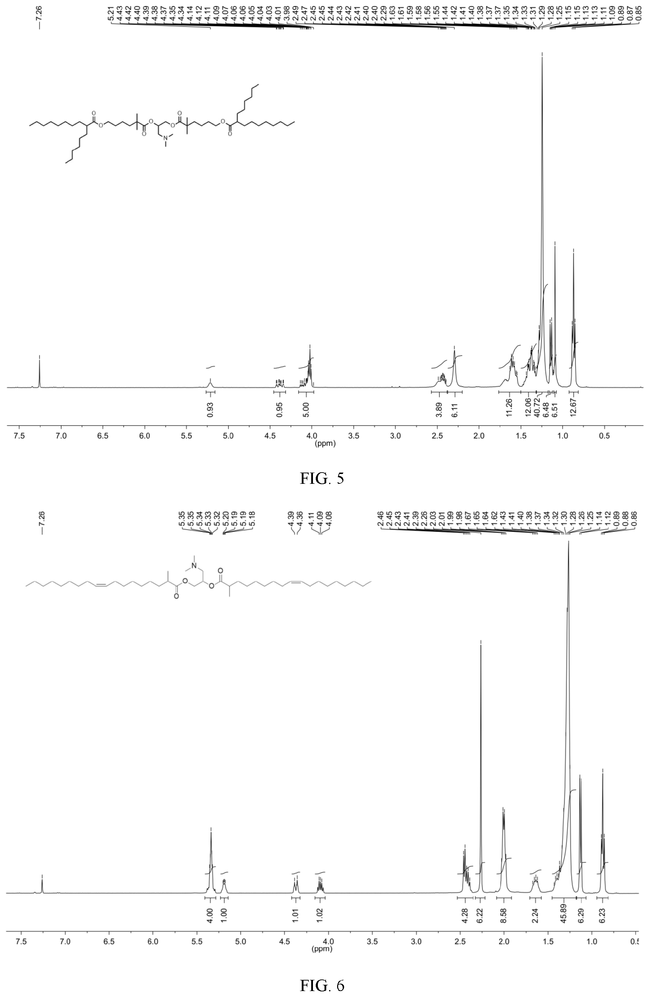

FIG. 5 is ¹H NMR of compound 5.
FIG. 6 is ¹H NMR of compound 6.

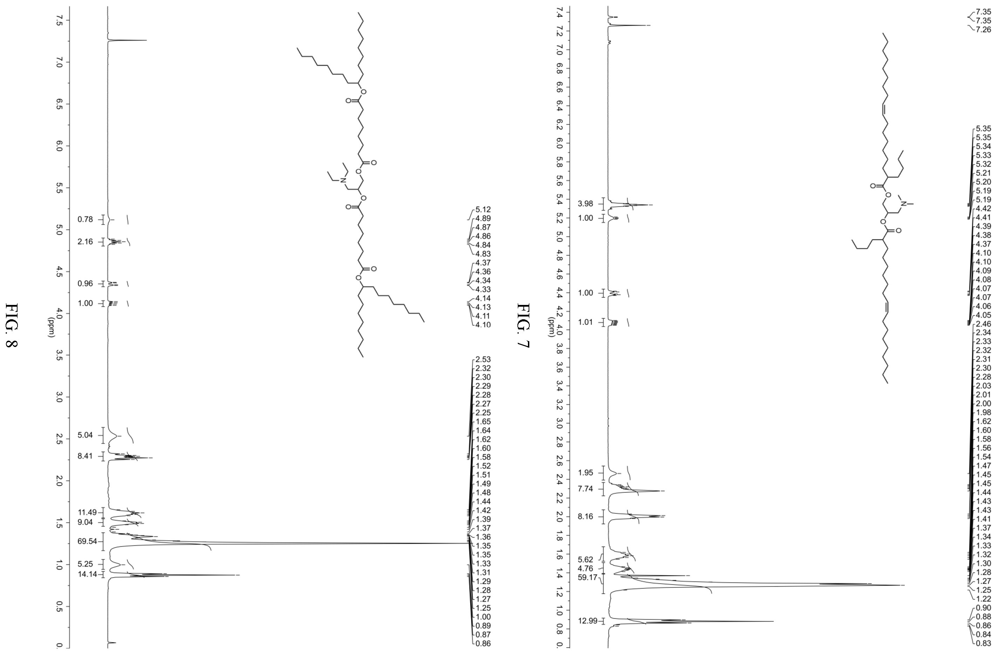

FIG. 7 is ¹H NMR of compound 7.
FIG. 8 is ¹H NMR of compound 8.

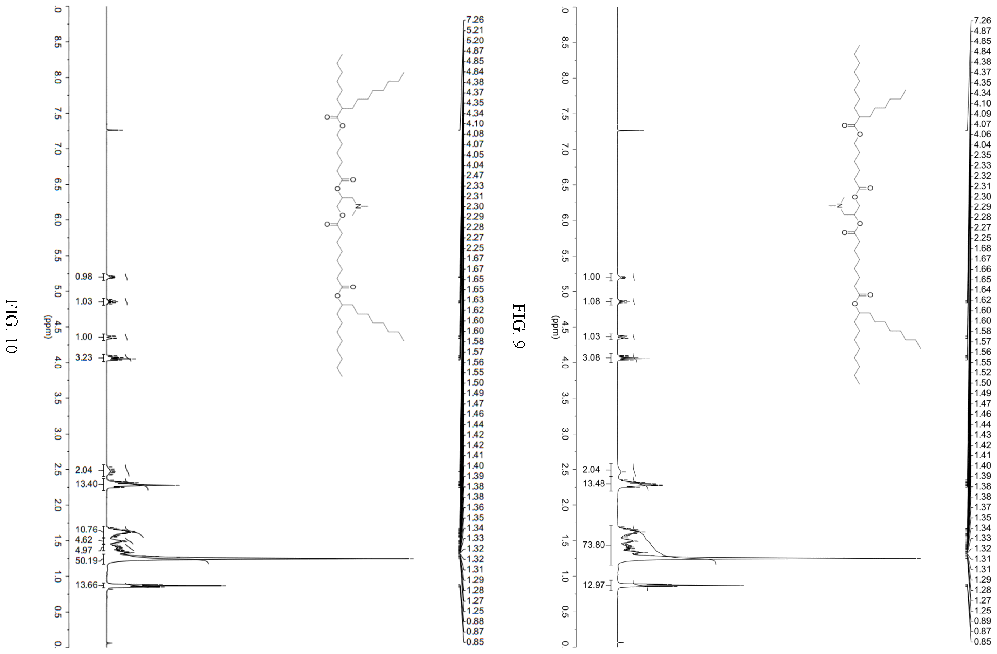

FIG. 9 is ¹H NMR of compound 17.
FIG. 10 is ¹H NMR of compound 18.

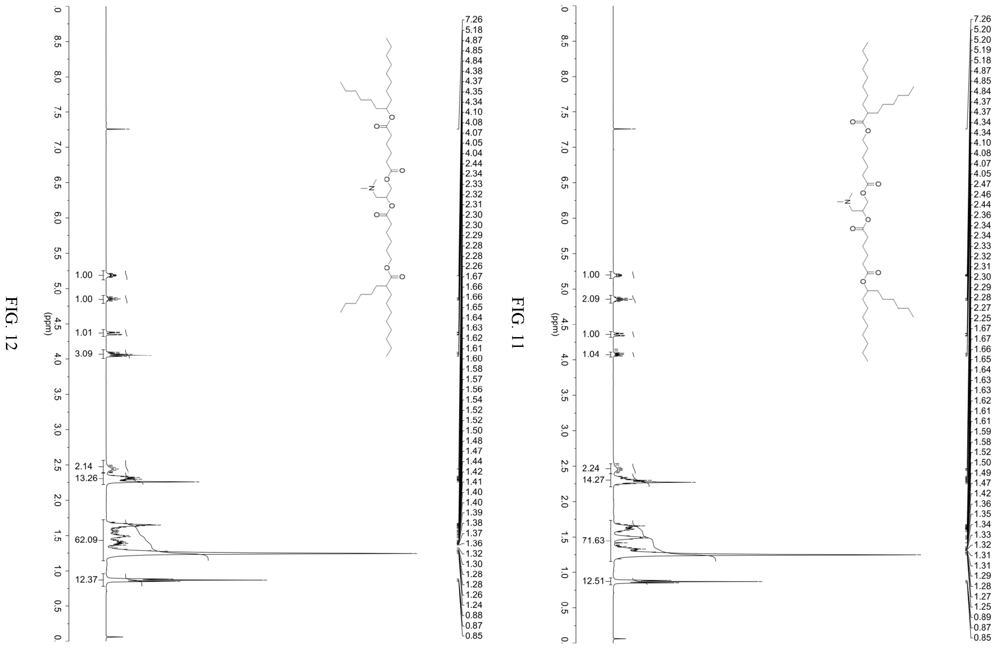

FIG. 11 is ¹H NMR of compound 19.
FIG. 12 is ¹H NMR of compound 20.

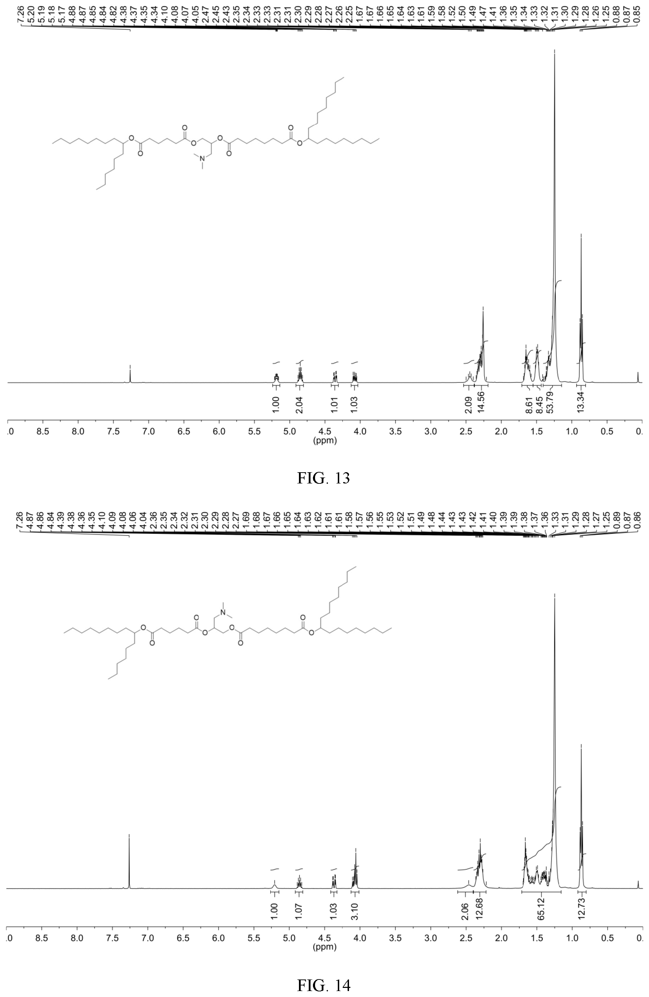

FIG. 13 is ¹H NMR of compound 21.
FIG. 14 is ¹H NMR of compound 22.

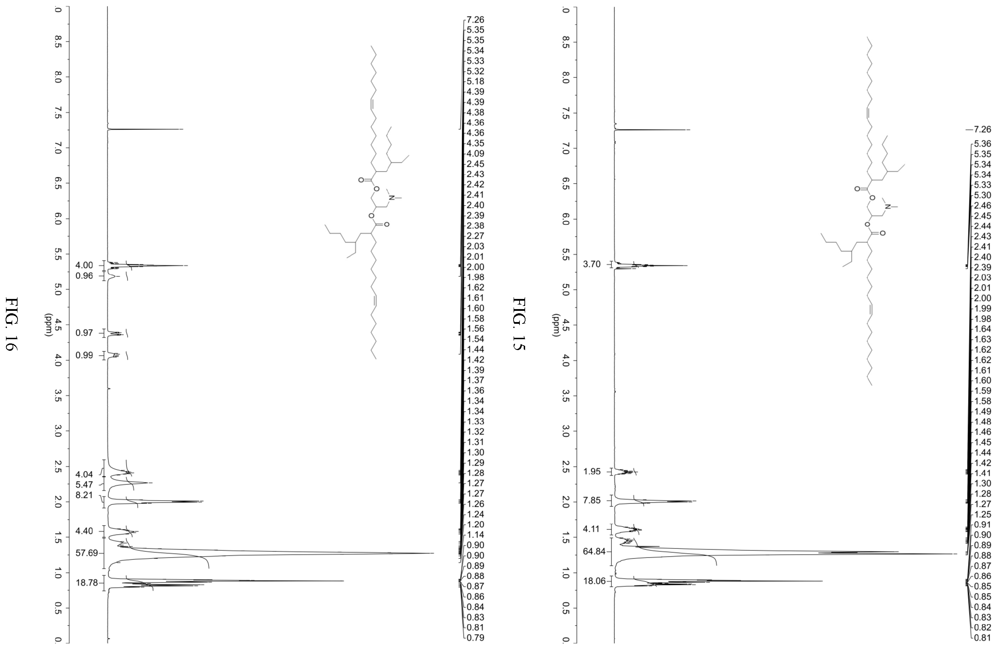

FIG. 15 is ¹H NMR of compound 23.
FIG. 16 is ¹H NMR of compound 24.

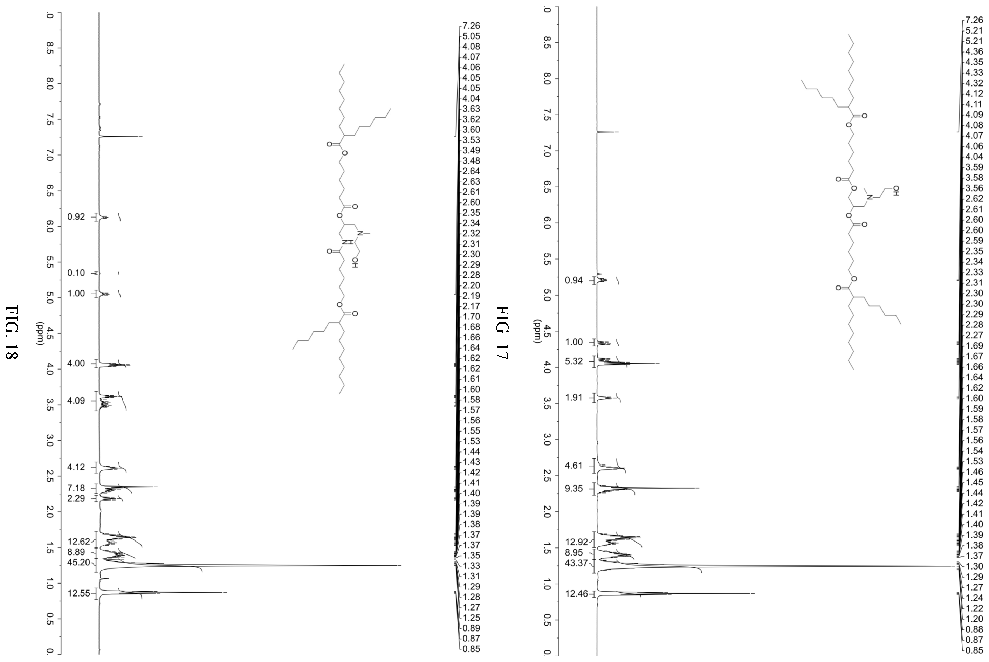

FIG. 17 is ¹H NMR of compound 25.
FIG. 18 is ¹H NMR of compound 27.

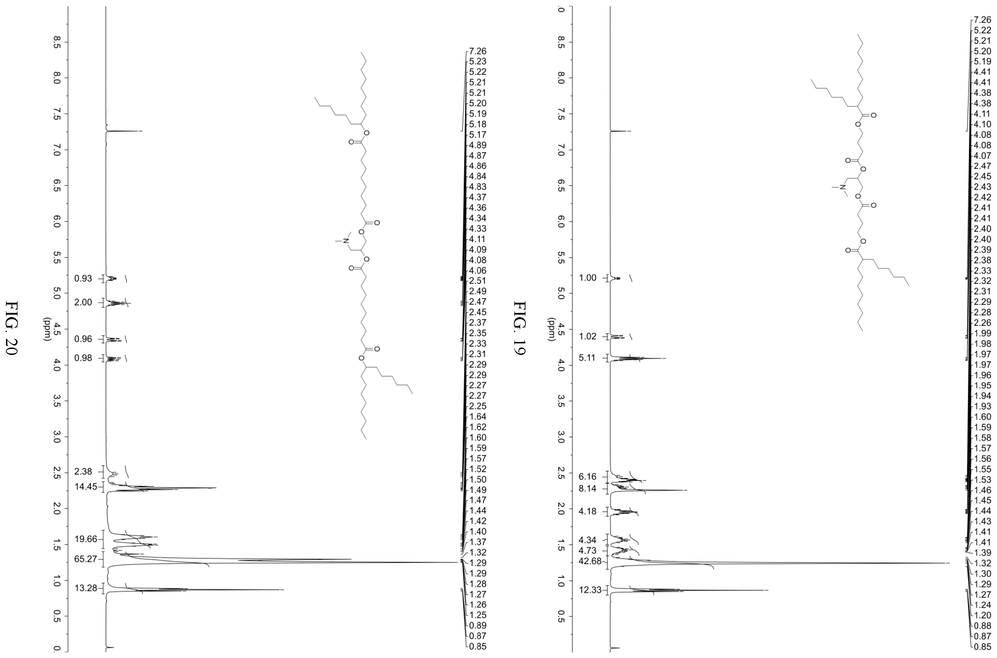

FIG. 19 is ¹H NMR of compound 30.
FIG. 20 is ¹H NMR of compound 33.

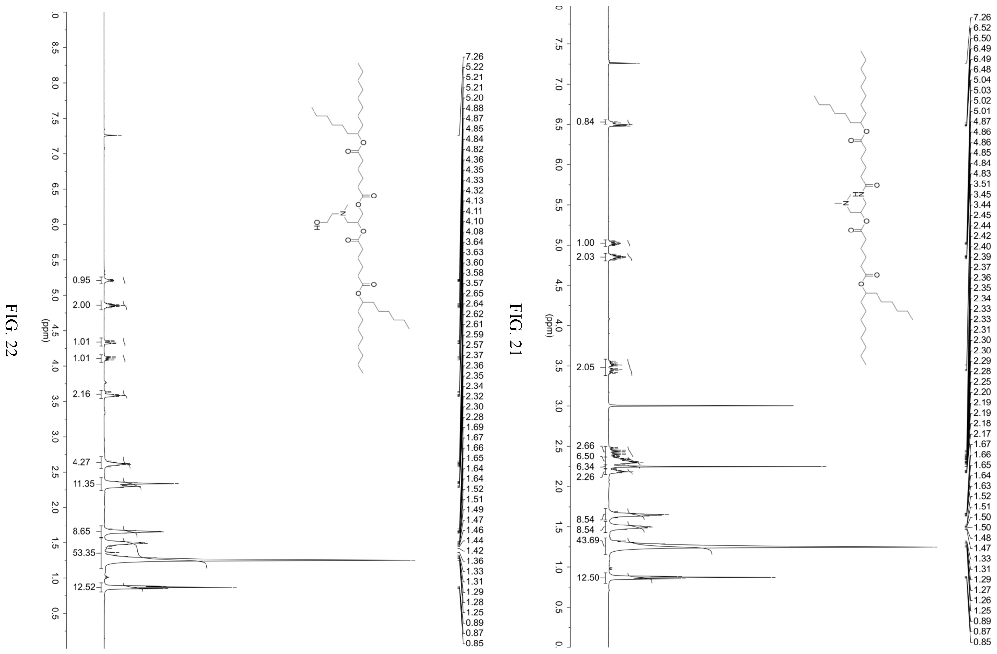

FIG. 21 is ¹H NMR of compound 34.
FIG. 22 is ¹H NMR of compound 35.

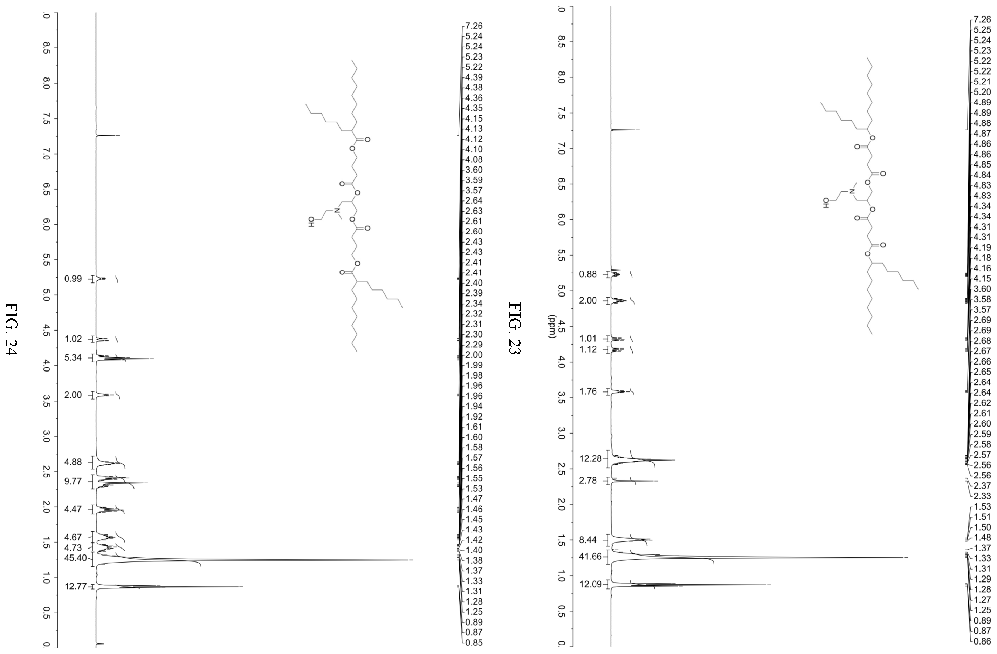

FIG. 23 is ¹H NMR of compound 36.
FIG. 24 is ¹H NMR of compound 37.

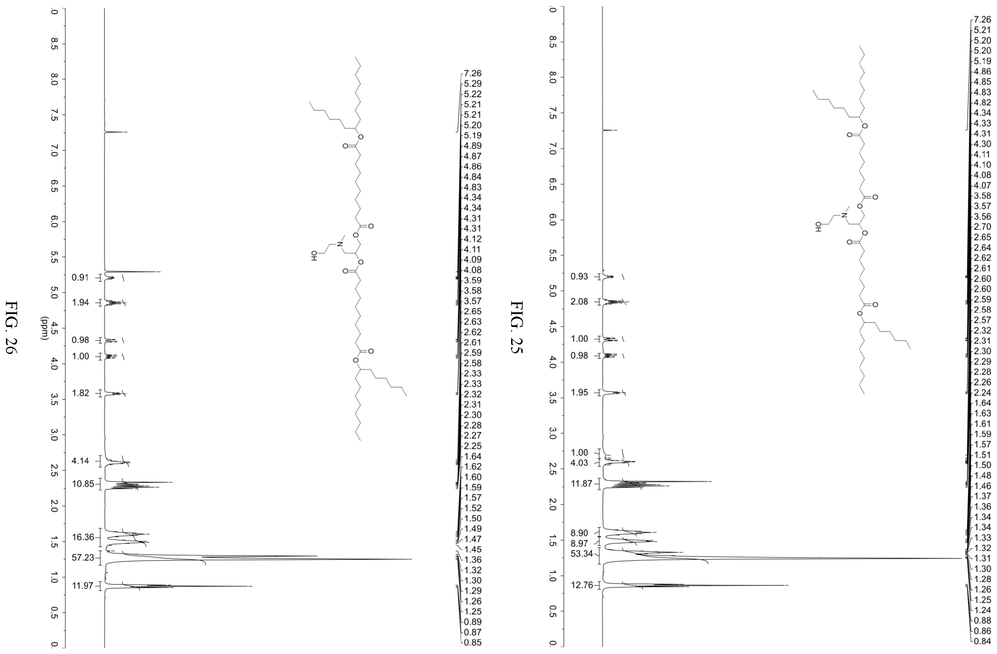

FIG. 25 is ¹H NMR of compound 38.
FIG. 26 is ¹H NMR of compound 43.

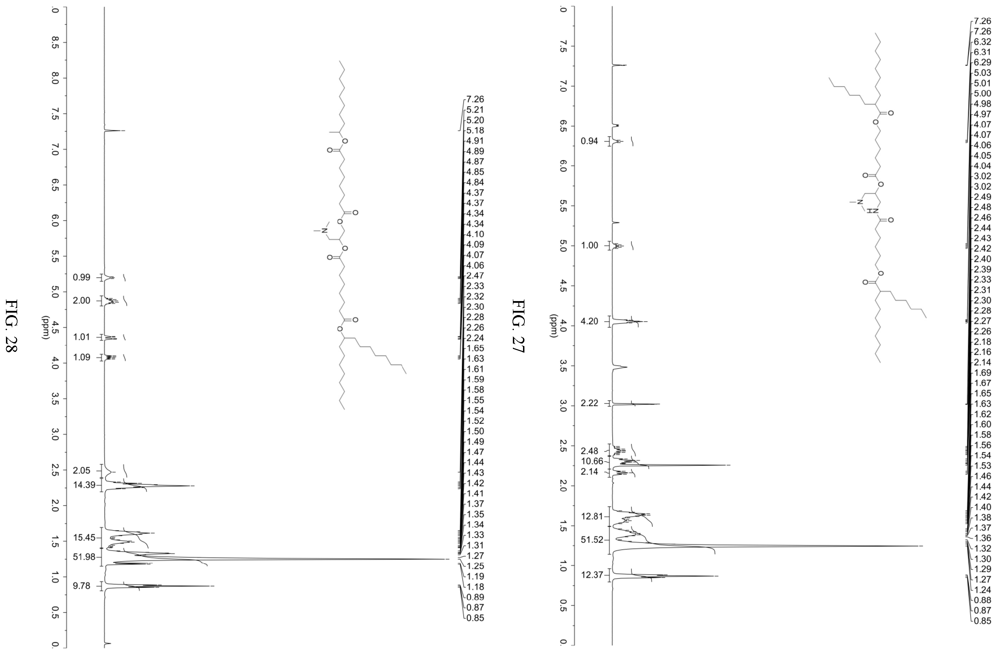

FIG. 27 is ¹H NMR of compound 44.
FIG. 28 is ¹H NMR of compound 45.

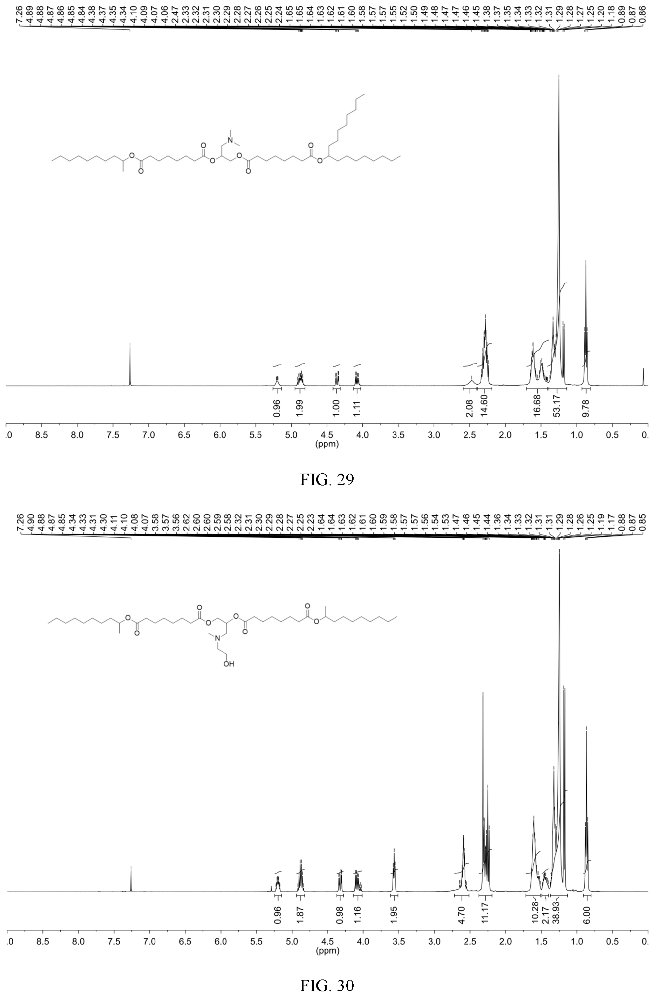

FIG. 29 is ¹H NMR of compound 46.
FIG. 30 is ¹H NMR of compound 47.

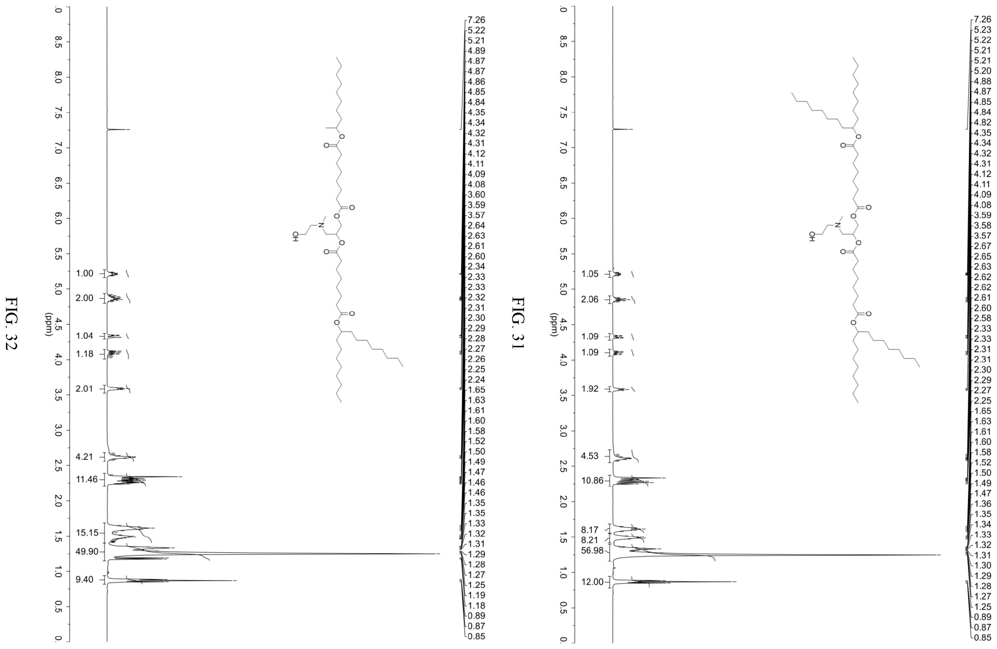

FIG. 31 is ¹H NMR of compound 48.
FIG. 32 is ¹H NMR of compound 49.

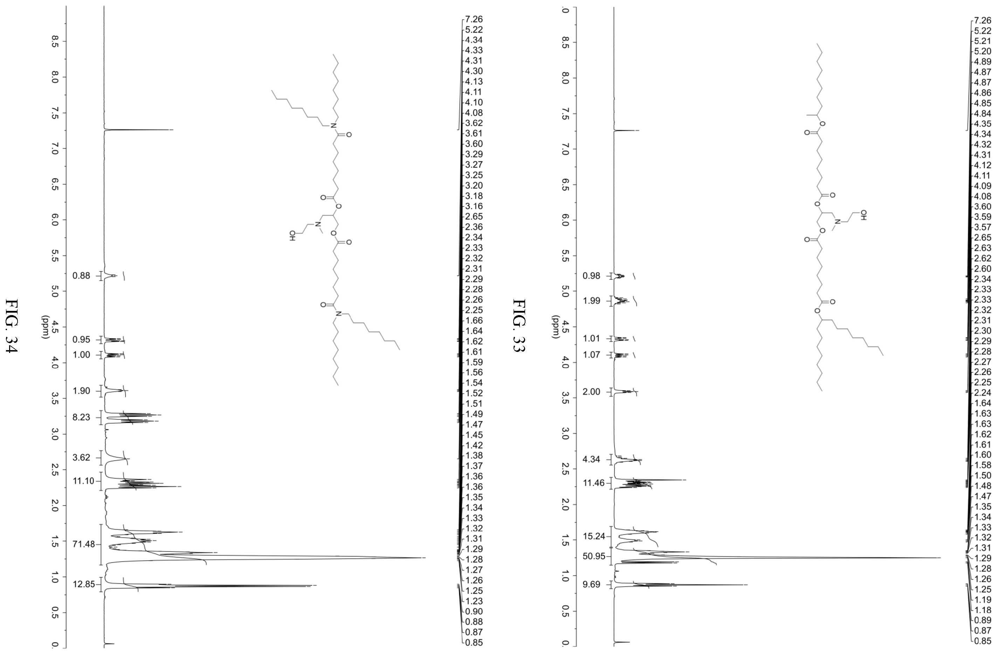

FIG. 33 is ¹H NMR of compound 50.
FIG. 34 is ¹H NMR of compound 51.

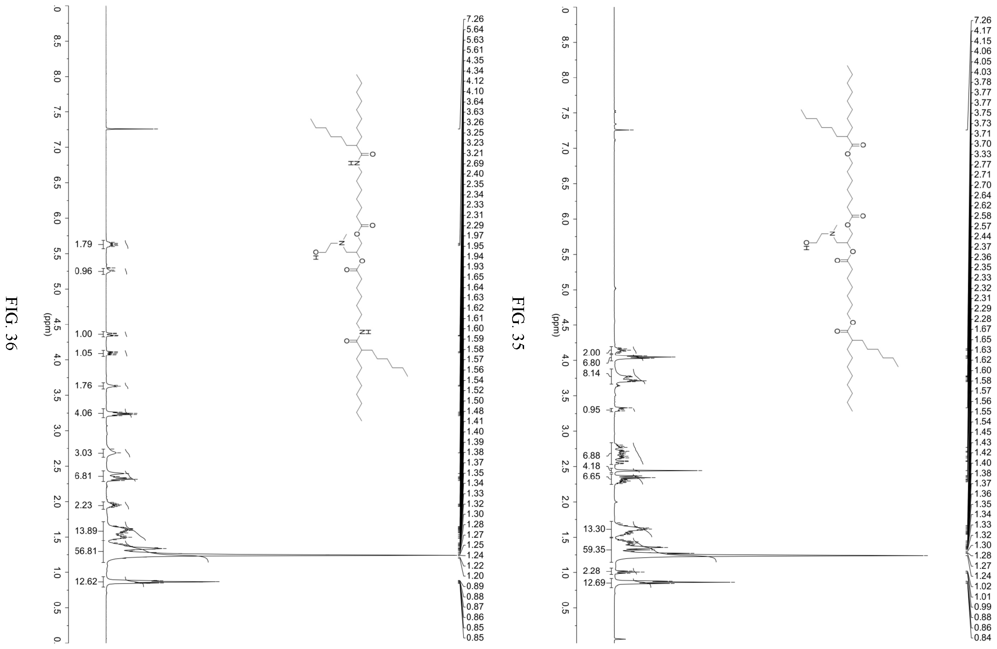

FIG. 35 is ¹H NMR of compound 52.
FIG. 36 is ¹H NMR of compound 53.

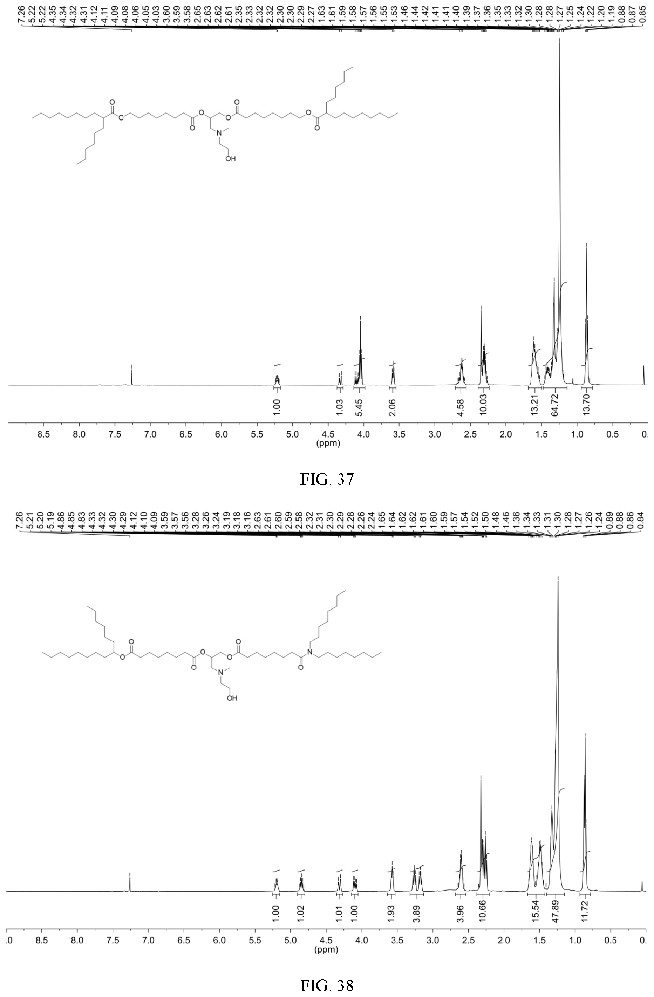

FIG. 37 is ¹H NMR of compound 54.
FIG. 38 is ¹H NMR of compound 55.

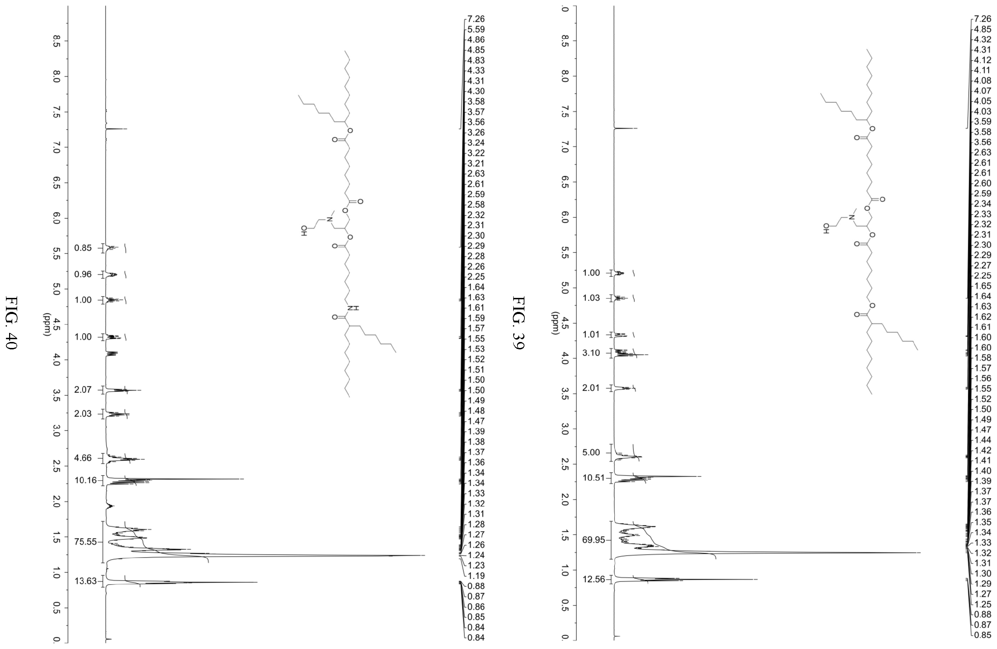

FIG. 39 is ¹H NMR of compound 56.
FIG. 40 is ¹H NMR of compound 57.

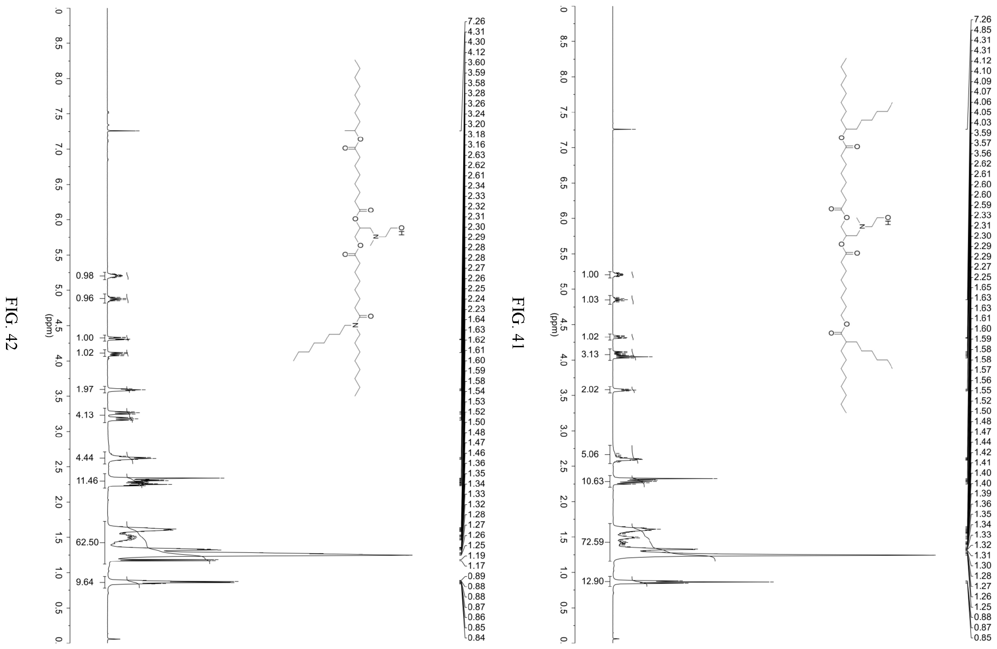

FIG. 41 is ¹H NMR of compound 58.
FIG. 42 is ¹H NMR of compound 59.

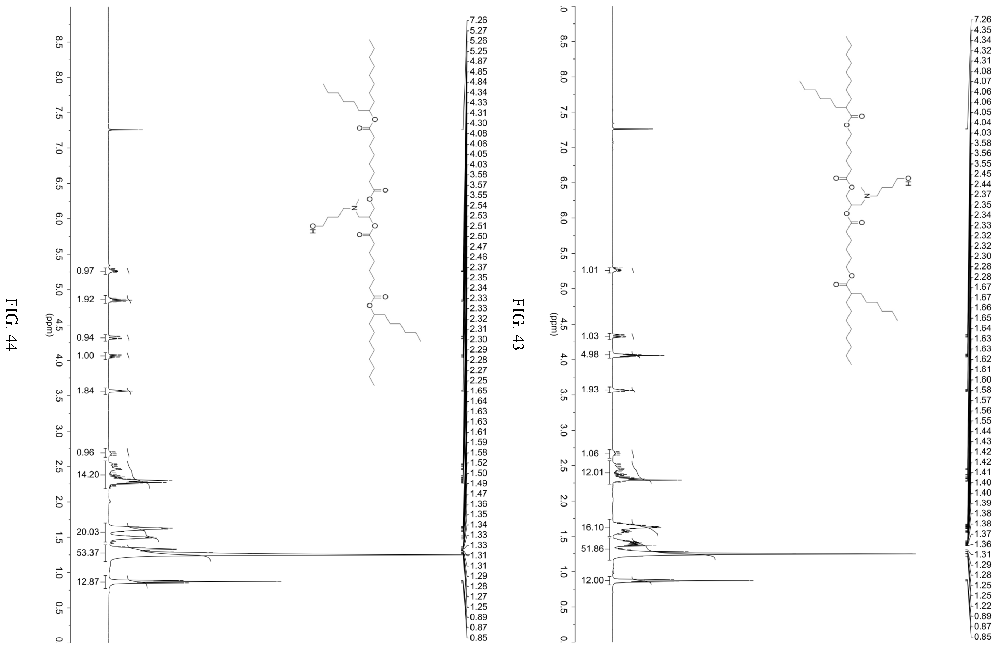

FIG. 43 is ¹H NMR of compound 60.
FIG. 44 is ¹H NMR of compound 63.

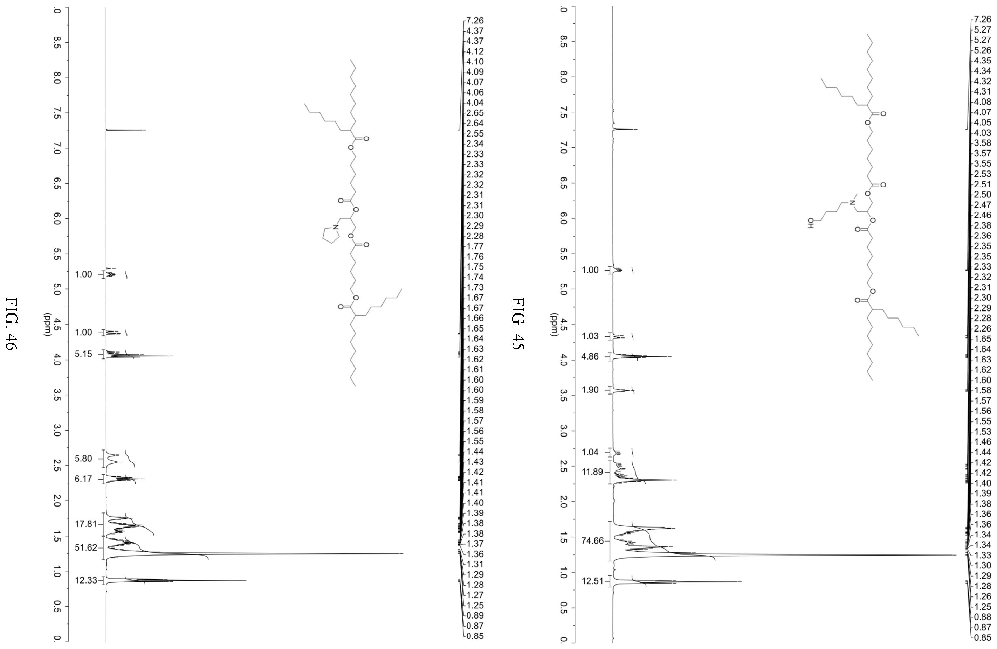

FIG. 45 is ¹H NMR of compound 64.
FIG. 46 is ¹H NMR of compound 65.

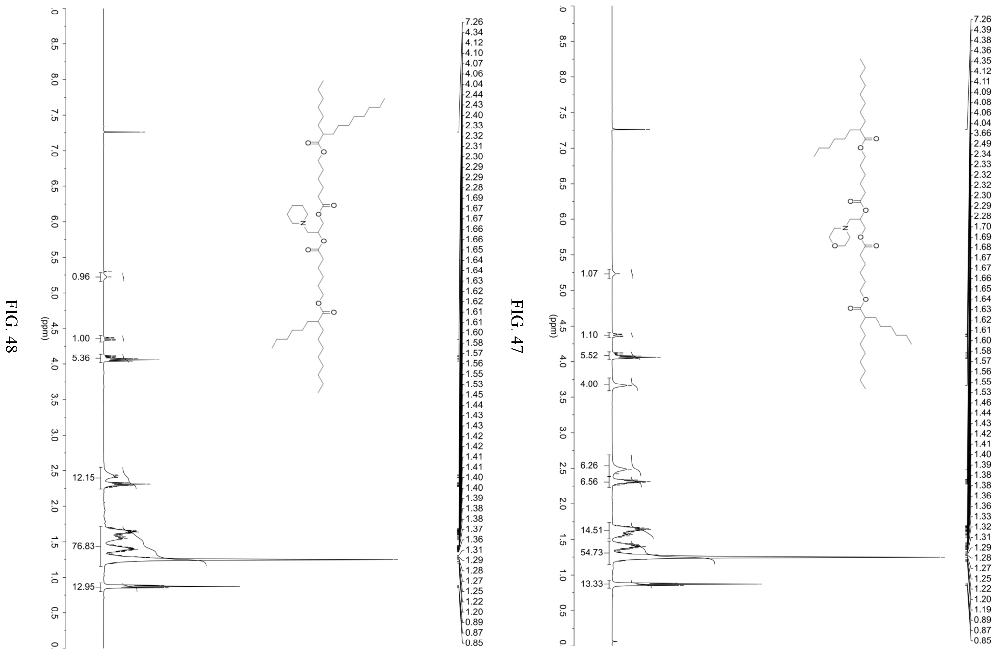

FIG. 47 is ¹H NMR of compound 66.
FIG. 48 is ¹H NMR of compound 67.

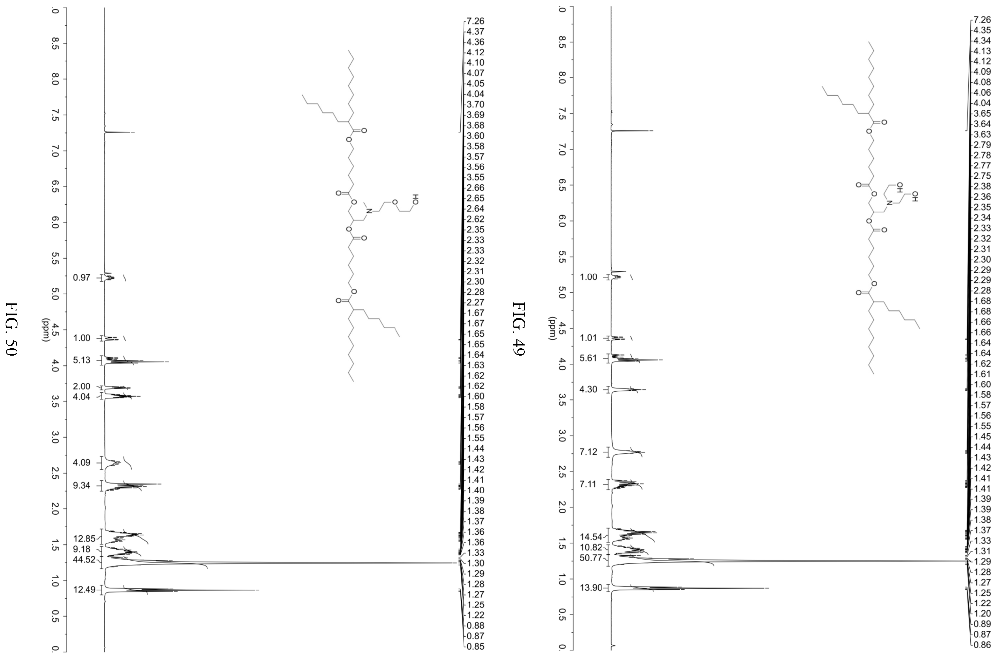

FIG. 49 is ¹H NMR of compound 68.
FIG. 50 is ¹H NMR of compound 70.

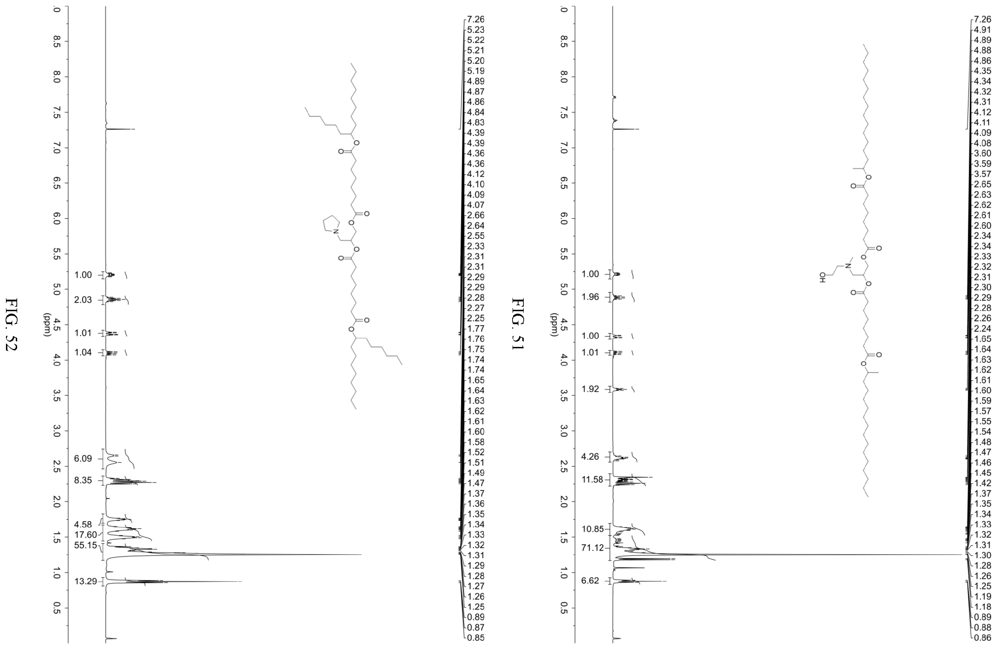

FIG. 51 is ¹H NMR of compound 76.
FIG. 52 is ¹H NMR of compound 78.

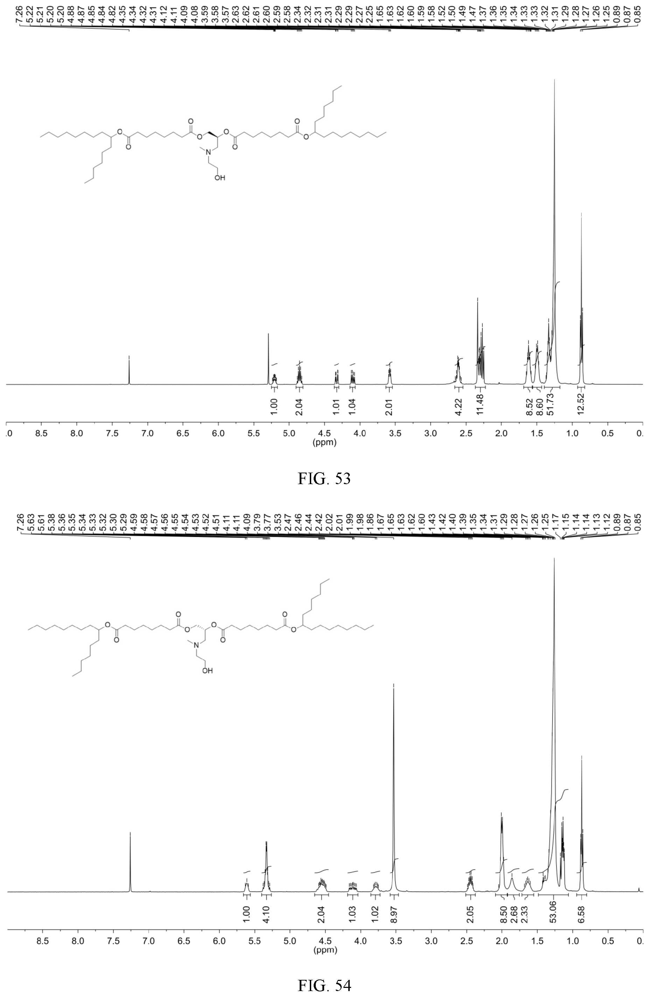

FIG. 53 is ¹H NMR of compound 79.
FIG. 54 is ¹H NMR of compound 80.

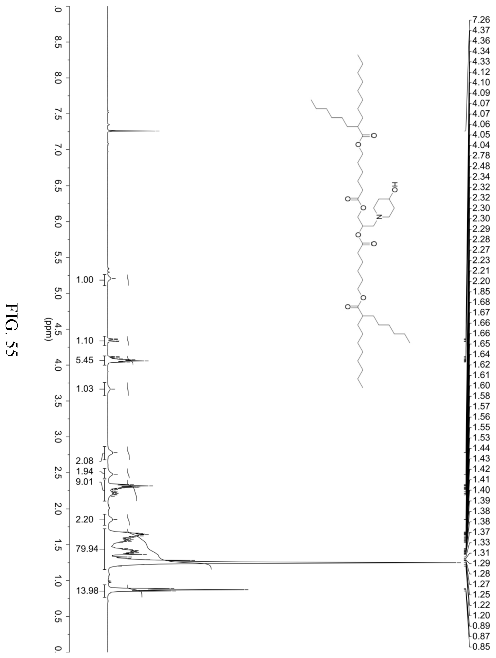

FIG. 55 is ¹H NMR of compound 81.

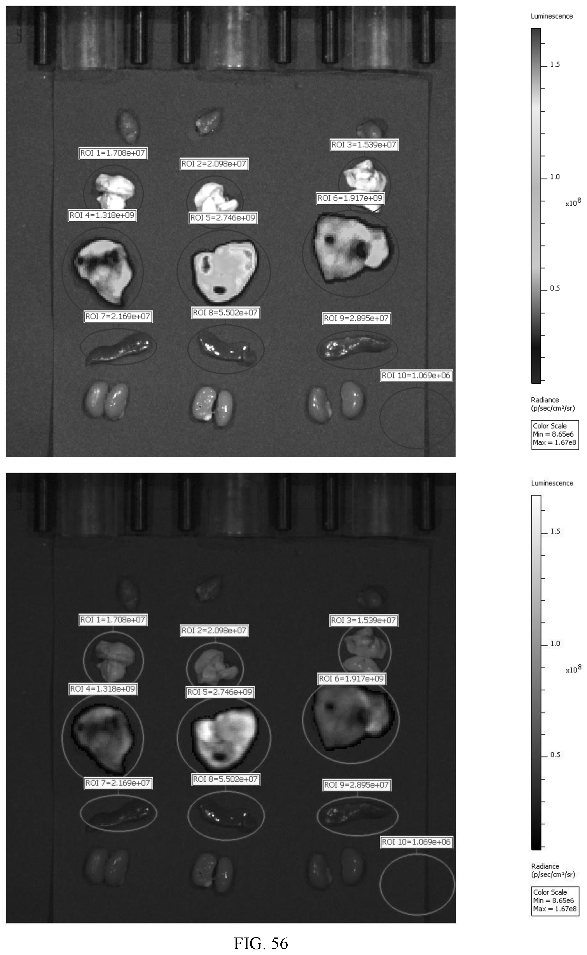

FIG. 56 is a graph showing fluorescence signals of different organs in mice 4 h after administration by intravenous injection of a sample of lipid nanoparticle TMF1 loaded with Luciferase mRNA. As shown in the graph, the fluorescence signal is strongest in the liver of the mice. It is indicated that the Luciferase mRNA loaded on TMF1 is mainly expressed in the liver.

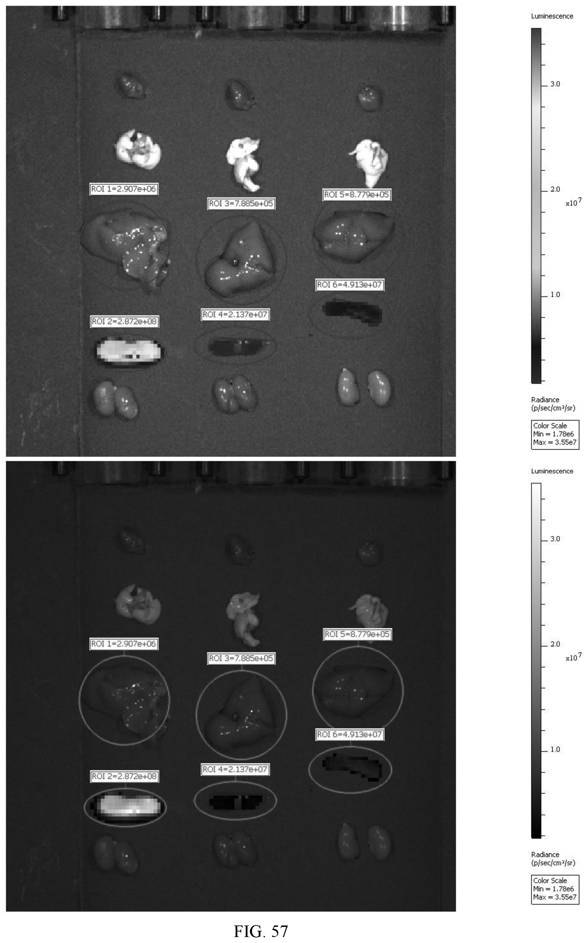

FIG. 57 is a graph showing fluorescence signals of different organs in mice 4 h after administration by intravenous injection of a sample of lipid nanoparticle TMF10 loaded with Luciferase mRNA. As shown in the graph, the fluorescence signal is strongest in the spleen of the mice, and very weak in the liver. It is indicated that the expression of the Luciferase mRNA loaded on TMF 10 in the spleen is significantly higher than that in the liver.

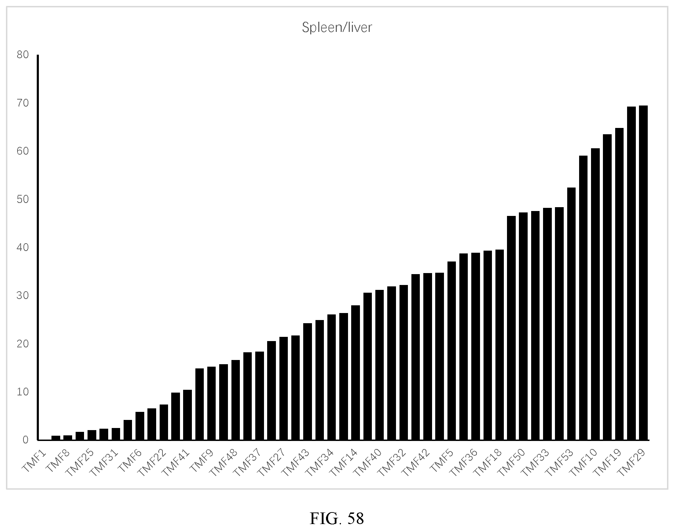

FIG. 58 shows summary data of fluorescence intensity ratios for different lipid nanoparticles delivering Luciferase mRNA in the spleen and liver.

### DETAILED DESCRIPTION

### A. Chemical and Formulation Definitions

When used in the context of a chemical group, "hydrogen" refers to -H; "deuterium" refers to ²H or D; "hydroxyl" refers to -OH; "oxo" refers to =O; "carbonyl" refers to -C(=O)-; "carboxyl" refers to -C(=O)OH (also written as -COOH or -CO₂H); "halo" independently refers to -F, -Cl, -Br, or -I; "amino" refers to -NH₂; "hydroxyamino" refers to -NHOH; "nitro" refers to -NO₂; imino refers to =NH; "cyano" refers to -CN; "isocyanate" refers to -N=C=O; "azido" refers to -N₃; in the context of a monovalent group, "phosphate" refers to -OP(O)(OH)₂ or a deprotonated form thereof; in the context of a divalent group, "phosphate" refers to -OP(O)(OH)O- or a deprotonated form thereof; "sulfhydryl" refers to -SH; and "thio" refers to =S; "sulfonyl" refers to -S(O)₂-; "hydroxysulfonyl" refers to -S(O)₂OH; "sulfonamide" refers to -S(O)₂NH₂; and "sulfinyl" refers to -S(O)-. In the context of a chemical formula, the symbol "-" refers to a single bond, "=" refers to a double bond, and "≡" refers to a triple bond. The symbol "----" represents an optional bond, which is a single or double bond, if present. When drawn perpendicularly across a bond, the symbol " 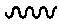 " indicates the point of attachment of the group. It should be noted that the point of attachment is generally identified in this manner only for larger groups to aid the reader in unequivocally identifying the point of attachment. The symbol " 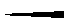 " refers to a single bond, wherein the group attached to the butt end of the wedge "emerges from the plane of the paper". The symbol " 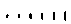 " refers to a single bond, wherein the group attached to the butt end of the wedge "goes into the plane of the paper". The symbol " 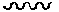 " refers to a single bond, wherein the geometry (e.g., E or Z) around the double bond is undefined. Thus, both options and combinations thereof are contemplated. Any undefined valence on an atom of a structure shown in the present application implicitly represents a hydrogen atom bonded to the atom. Bold dots on a carbon atom indicate that the hydrogen attached to the carbon is oriented out of the plane of the paper.

The term "alkyl" as used without the modifier "substituted" represents a monovalent saturated aliphatic group having a carbon atom as the point of attachment, having a linear or branched acyclic structure, and no atoms other than carbon and hydrogen. The groups -CH₃(Me), - CH₂CH₃(Et), -CH₂CH₂CH₃ (*n*-Pr or propyl), -CH(CH₃)₂ (*i*-Pr, iPr or isopropyl), - CH₂CH₂CH₂CH₃ (*n*-Bu), -CH(CH₃)CH₂CH₃ (*sec*-butyl), -CH₂CH(CH₃)₂ (isobutyl), -C(CH₃)₃ (*tert-*butyl*, t*-butyl, *t*-Bu or tBu), and -CH₂C(CH₃)₃ (neopentyl) are non-limiting examples of alkyl. The term "dialkyl" as used without the modifier "substituted" represents a divalent saturated aliphatic group having 1 or 2 saturated carbon atoms as the point of attachment, having a linear or branched acyclic structure, no carbon-carbon double or triple bonds, and no atoms other than carbon and hydrogen. The groups -CH₂-(methylene), -CH₂CH₂-, -CH₂C(CH₃)₂CH₂-, and -CH₂CH₂CH₂- are non-limiting examples of dialkyl. "Alkane" represents the class of compounds having formula H-R, wherein R is alkyl, which is as defined above. When any of these terms is used together with the modifier "substituted", one or more hydrogen atoms have been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, -NHCH₃, -NHCH₂CH₃, -N(CH₃)₂, -C(O)NH₂, -C(O)NHCH₃, - C(O)N(CH₃)₂, -OC(O)CH₃, -NHC(O)CH₃, -S(O)₂OH, or -S(O)₂NH₂. The following groups are non-limiting examples of substituted alkyl: -CH₂OH, -CH₂Cl, -CF₃, -CH₂CN, -CH₂C(O)OH, - CH₂C(O)OCH₃, -CH₂C(O)NH₂, -CH₂C(O)CH₃, -CH2OCH₃, -CH₂OC(O)CH₃, -CH₂NH₂, - CH₂N(CH₃)₂, and -CH₂CH₂Cl. The term "haloalkyl" is a subset of substituted alkyl, wherein hydrogen atom substitution is limited to halo (i.e., -F, -Cl, -Br, or -I), such that no atoms other than carbon, hydrogen and halogen are present. The group -CH₂Cl is one non-limiting example of haloalkyl. The term "fluoroalkyl" is a subset of substituted alkyl, wherein hydrogen atom substitution is limited to fluoro, such that no atoms other than carbon, hydrogen and fluorine are present. The groups -CH₂F, -CF₃, and -CH₂CF₃ are non-limiting examples of fluoroalkyl.

The term "alkenyl" as used without the modifier "substituted" represents a monovalent unsaturated aliphatic group having a carbon atom as the point of attachment, having a linear or branched acyclic structure, at least one non-aromatic carbon-carbon double bond, no carbon-carbon triple bonds, and no atoms other than carbon and hydrogen. Non-limiting examples include: -CH=CH₂ (vinyl), -CH=CHCH₃, -CH=CHCH₂CH₃, -CH₂CH=CH₂ (allyl), - CH₂CH=CHCH₃, and -CH=CHCH=CH₂. The term "alkenediyl" as used without the modifier "substituted" represents a divalent unsaturated aliphatic group having 2 carbon atoms as the point of attachment, having a linear or branched, linear or branched acyclic structure, at least one non-aromatic carbon-carbon double bond, no carbon-carbon triple bonds, and no atoms other than carbon and hydrogen. The groups -CH=CH-, -CH=C(CH₃)CH₂-, -CH=CHCH₂-, and -CH₂CH=CHCH₂- are non-limiting examples of an alkenediyl group. It should be noted that although the alkenediyl group is aliphatic, once it is attached at both ends, it is not excluded that the group forms part of an aromatic structure. The terms "olefin" and "chain olefin" are synonymous and represent the class of compounds having formula H-R, wherein R is alkenyl, which is as defined above. Similarly, the terms "terminal olefin" and "α-olefin" are synonymous and represent olefin having exactly one carbon-carbon double bond, wherein the bond is part of vinyl at the terminus of the molecule. When any of these terms is used together with the modifier "substituted", one or more hydrogen atoms have been independently replaced by -OH, -F, -Cl, -Br, -I, -NH₂, -NO₂, -CO₂H, -CO₂CH₃, -CN, -SH, -OCH₃, -OCH₂CH₃, -C(O)CH₃, - NHCH₃, -NHCH₂CH₃, -N(CH₃)₂, -C(O)NH₂, -C(O)NHCH₃, -C(O)N(CH₃)₂, -OC(O)CH₃, - NHC(O)CH₃, -S(O)₂OH, or -S(O)₂NH₂. The groups -CH=CHF, -CH=CHCl, and -CH=CHBr are non-limiting examples of substituted alkenyl.

The term "alkynyl" as used without the modifier "substituted" represents a monovalent unsaturated aliphatic group having a carbon atom as the point of attachment, having a linear or branched acyclic structure, at least one carbon-carbon triple bond, and no atoms other than carbon and hydrogen. The term alkynyl as used herein does not preclude the presence of one or more non-aromatic carbon-carbon double bonds. The groups -C≡CH, -C≡CCH₃, and - CH₂C≡CCH₃ are non-limiting examples of alkynyl. "Alkyne" represents the class of compounds having formula H-R, wherein R is alkynyl. When any of these terms is used together with the modifier "substituted", one or more hydrogen atoms have been independently replaced by -OH, -F, -Cl, -Br, -I, -NH₂, -NO₂, -CO₂H, -CO₂CH₃, -CN, -SH, -OCH₃, -OCH₂CH₃, - C(O)CH₃, -NHCH₃, -NHCH₂CH₃, -N(CH₃)₂, -C(O)NH₂, -C(O)NHCH₃, -C(O)N(CH₃)₂, - OC(O)CH₃, -NHC(O)CH₃, -S(O)₂OH, or -S(O)₂NH₂.

"Prevention" or "preventing" includes: (1) inhibiting the onset of a disease in a subject or patient who may be at risk of and/or susceptible to the disease, but who has not yet experienced or exhibited any or all of the conditions or symptoms of the disease; and/or (2) slowing the onset of the conditions or symptoms of a disease in a subject or patient who may be at risk of and/or susceptible to the disease, but who has not experienced or exhibited any or all of the conditions or symptoms of the disease.

"Treatment" or "treating" includes: (1) inhibiting a disease (e.g., arresting the further development of the conditions and/or symptoms) in a subject or patient experiencing or exhibiting the conditions or symptoms of the disease, (2) ameliorating a disease (e.g., reversing the conditions and/or symptoms) in a subject or patient experiencing or exhibiting the conditions or symptoms of the disease, and/or (3) effecting any measurable mitigation of a disease in a subject or patient experiencing or exhibiting the conditions or symptoms of the disease.

"Protein", "polypeptide" or "peptide" refers to a polymer of amino acid residues, including a wide range of protein molecules such as cytokines, chemokines, interleukins, interferons, growth factors, coagulation factors, anticoagulants, blood factors, bone morphogenic proteins, immunoglobulins, and enzymes. Some non-limiting examples of therapeutic proteins include the following therapeutic proteins or fragments, variants or derivatives thereof: therapeutic proteins for treating metabolic or endocrine disorders, including acid sphingomyelinase, adipotide, agalsidase-β, alglucosidase, α-galactosidase A, α-glucosidase, α-L-iduronidase, α-N-acetylglucosaminidase, amphiregulin, angiopoietins (Ang1, Ang2, Ang3, Ang4, ANGPTL2, ANGPTL3, ANGPTL4, ANGPTL5, ANGPTL6, ANGPTL7), β cellulin, β-glucuronidase, bone morphogenetic proteins (BMPs (BMP1, BMP2, BMP3, BMP4, BMP5, BMP6, BMP7, BMP8a, BMP8b, BMP10, BMP15)), CLN6 protein, epidermal growth factor (EGF), epigen, epiregulin, fibroblast growth factors (FGFs (FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23)), galsulphase, ghrelin, glucocerebrosidase, GM-CSF, heparin-binding EGF-like growth factor (HB-EGF), hepatocyte growth factor (HGF), hepcidin, human albumin, increased loss of albumin, idursulfase (iduronate-2-sulfatase), integrins αVβ3, αVβ5 and α5β1, iduronate sulfatase, laronidase, *N*-acetylgalactosamine-4-sulfatase (rhASB; galsulfase, arylsulfatase A (ARSA), arylsulfatase B (ARSB)), *N-*acetylglucosamine-6-sulfatase, nerve growth factor (NGF) (brain-derived neurotrophic factor (BDNF)), neurotrophin-3 (NT-3) and neurotrophin 4/5 (NT-4/5), neuregulins (NRG1, NRG2, NRG3, NRG4), neuropilins (NRP-1, NRP-2), obestatin, platelet-derived growth factors (PDGFs (PDFF-A, PDGF-B, PDGF-C, PDGF-D)), TGFβ receptors (endoglin, TGF-β1 receptor, TGF-β2 receptor, TGF-β3 receptor), thrombopoietin (THPO) (megakaryocyte growth and development factor (MGDF)), transforming growth factors (TGFs (TGF-a, TGF-β (TGFβ1, TGFβ2, and TGFβ3))), VEGF (VEGF-A, VEGF-B, VEGF-C, VEGF-D, VEGF-E, VEGF-F, and PIGF), nesiritide, trypsin, adrenocorticotrophic hormone (ACTH), atrial-natriuretic peptide (ANP), cholecystokinin, gastrin, leptin, oxytocin, somatostatin, vasopressin (antidiuretic hormone), calcitonin, exenatide, growth hormone (GH), somatotropin, insulin, insulin-like growth factor 1 (IGF-1), mecasermin rinfabate, IGF-1 analog, mecasermin, IGF-1 analog, pegvisomant, pramlintide, teriparatide (human parathyroid hormone residues 1-34), becaplermin, diboternnin-α (bone morphogenetic protein 2), histrelin acetate (gonadotropin releasing hormone; GnRH), octreotide, and palifermin (keratinocyte growth factor; KGF); therapeutic proteins for treating blood disorders, diseases of the circulatory system, diseases of the respiratory system, cancer or tumour diseases, infectious diseases, or immunedeficiencies, including alteplase (tissue plasminogen activator; tPA), anistreplase, antithrombin III (AT-III), bivalirudin, darbepoetin-α, drotrecogin-α (activated protein C), erythropoietin, epoetin-α, erythropoetin, hematopoietin, factor IX, factor VIIa, factor VIII, lepirudin, protein C concentrate, reteplase (deletion mutein of tPA), streptokinase, tenecteplase, urokinase, angiostatin, anti-CD22 immunotoxin, denileukin diftitox, immunocyanin, MPS (metallopanstimulin), aflibercept, endostatin, collagenase, human deoxy-ribonuclease I, dornase, hyaluronidase, papain, L-asparaginase, PEG-asparaginase, rasburicase, human chorionic gonadotropin (HCG), human follicle-stimulating hormone (FSH), lutropin-α, prolactin, α-1-proteinase inhibitor, lactase, pancreatic enzymes (lipase, amylase, protease), adenosine deaminase (pegademase bovine, PEG-ADA), abatacept, alefacept, anakinra, etanercept, interleukin-1 (IL-1) receptor antagonist, anakinra, thymosin, TNF-α antagonist, enfuvirtide, and thymosin α1; therapeutic proteins selected from adjuvant or immunostimulatory proteins, including: human adjuvant proteins, particularly pattern recognition receptors TLR1, TLR2, TLR3, TLR4, TLR5, TLR6, TLR7, TLR8, TLR9, TLR10, TLR11; NOD1, NOD2, NOD3, NOD4, NOD5, NALP1, NALP2, NALP3, NALP4, NALP5, NALP6, NALP6, NALP7, NALP7, NALP8, NALP9, NALP10, NALP11, NALP12, NALP13, NALP14, 1IPAF, NAIP, CIITA, RIG-I, MDA5, and LGP2, signal transducers of TLR signaling, including adaptor proteins, including, e.g., Trif and Cardif; components of small-GTPase signaling (RhoA, Ras, Rac1, Cdc42, Rab, etc.), components of PIP signaling (PI3K, Src-kinases, etc.), components of MyD88-dependent signaling (MyD88, IRAK1, IRAK2, IRAK4, TIRAP, TRAF6, etc.), components of MyD88-independent signaling (TICAM1, TICAM2, TRAF6, TBK1, IRF3, TAK1, IRAK1, etc.); activated kinases, including, e.g., Akt, MEKK1, MKK1, MKK3, MKK4, MKK6, MKK7, ERK1, ERK2, GSK3, PKC kinases, PKD kinases, GSK3 kinases, JNK, p38MAPK, TAK1, IKK, and TAK1; activated transcription factors, including, e.g., NF-κB, c-Fos, c-Jun, c-Myc, CREB, AP-1, Elk-1, ATF2, IRF-3, IRF-7, heat shock proteins, such as HSP10, HSP60, HSP65, HSP70, HSP75, and HSP90, gp96, fibrinogen, TypIII repeat extra domain A of fibronectin; or components of the complement system, including C1q, MBL, C1r, C1s, C2b, Bb, D, MASP-1, MASP-2, C4b, C3b, C5a, C3a, C4a, C5b, C6, C7, C8, C9, CR1, CR2, CR3, CR4, C1qR, C1INH, C4bp, MCP, DAF, H, I, P, and CD59, or induced target genes, including, e.g., β-defensin, cell surface proteins; or human adjuvant proteins, including trif, flt-3 ligand, Gp96, or fibronectin, cytokines that induce or enhance an innate immune response, including IL-1α, IL1β, IL-2, IL-6, IL-7, IL-8, IL-9, IL-12, IL-13, IL-15, IL-16, IL-17, IL-18, IL-21, IL-23, TNFα, IFNα, IFNβ, IFNγ, GM-CSF, G-CSF, M-CSF; chemokines, including IL-8, IP-10, MCP-1, MIP-1α, RANTES, eotaxin, CCL21; cytokines released by macrophages, including IL-1, IL-6, IL-8, IL-12, and TNF-α; as well as IL-1R1 and IL-1α; bacterial (adjuvant) proteins, particularly bacterial heat shock proteins or chaperones, including Hsp60, Hsp70, Hsp90, Hsp100; OmpA (outer membrane protein) from gram-negative bacteria; bacterial porins, including OmpF; bacterial toxins, including pertussis toxin (PT) from Bordetella pertussis, pertussis adenylate cyclase toxins CyaA and CyaC from Bordetella pertussis, PT-9K/129G mutant from pertussis toxin, pertussis adenylate cyclase toxins CyaA and CyaC from Bordetella pertussis, tetanus toxin, cholera toxin (CT), cholera toxin B-subunit, CTK63 mutant from cholera toxin, CTE112K mutant from CT, Escherichia coli heat-labile enterotoxin (LT), B subunit from heat-labile enterotoxin (LTB), Escherichia coli heat-labile enterotoxin mutants with reduced toxicity, including LTK63, LTR72; phenol-soluble modulin; neutrophil-activating protein (HP-NAP) from Helicobacter pylori; surfactant protein D; outer surface protein A lipoprotein from Borrelia burgdorferi, Ag38 (38 kDa antigen) from Mycobacterium tuberculosis; proteins from bacterial fimbriae; enterotoxin CT of Vibrio cholerae, pilin from the pili of gram-negative bacteria, and surfactant protein A and bacterial flagellins, protozoan (adjuvant) proteins, particularly Tc52 from Trypanosoma cruzi, PFTG from Trypanosoma gondii, protozoan heat shock proteins, LeIF from Leishmania spp., profilin-like protein from Toxoplasma gondii, viral (adjuvant) proteins, particularly respiratory syncytial virus fusion glycoprotein (F-protein), envelope protein from MMT virus, mouse leukemia virus protein, hemagglutinin protein of wild-type measles virus, fungal (adjuvant) proteins, particularly fungal immunomodulatory protein (FIP; LZ-8); and keyhole limpet hemocyanin (KLH), OspA; therapeutic proteins for hormone replacement therapy, particularly oestrogen, progesterone or progestin, and testosterone; therapeutic proteins for reprogramming somatic cells into pluripotent or totipotent stem cells, particularly Oct-3/4, Sox gene family (Sox1, Sox2, Sox3, and Sox15), Klf family (Klf1, Klf2, Klf4, and Klf5), Myc family (c-myc, L-myc, and N-myc), Nanog, and LIN28; therapeutic antibodies selected from antibodies for treating cancer or tumor diseases, particularly 131T-tositumomab, 3F8, 8H9, abagovomab, adecatumumab, afutuzumab, alacizumab pegol, alemtuzumab, amatuximab, AME-133v, AMG102, anatumomab mafenatox, apolizumab, bavituximab, bectumomab, belinnumab, bevacizumab, bivatuzumab-DM1, blinatumomab, brentuximab vedotin, cantuzumab, cantuzumab mertansine, cantuzumab ravtansine, capromab pendetide, carlumab, catumaxomab, cetuximab, citatuzumab bogatox, cixutumumab, clivatuzumab tetraxetan, CNTO328, CNTO 95, conatumumab, dacetuzumab, dalotuzumab, denosumab, detumomab, drozitumab, ecromeximab, edrecolomab, elotuzumab, elsilimomab, enavatuzumab, ensituximab, epratuzumab, ertumaxomab, ertumaxomab, etaracizumab, farletuzumab, FBTA05, ficlatuzumab, figitumumab, flanvotumab, galiximab, galiximab, ganitumab, GC1008, gemtuzumab, gemtuzumab ozogamicin, girentuximab, glembatumumab vedotin, GS6624, HuC242-DM4, HuHMFG1, HuN901-DM1, ibritumomab, icrucumab, ID09C3, indatuximab ravtansine, inotuzumab ozogamicin, intetumumab, ipilimumab, iratumumab, labetuzumab, lexatumumab, lintuzumab, lorvotuzumab mertansine, lucatumumab, lumiliximab, mapatumumab, matuzumab, MDX-060, MEDI 522, mitumomab, mogamulizumab, MORab-003, MORab-009, moxetumomab pasudotox, MT103, nacolomab tafenatox, naptumomab estafenatox, narnatumab, necitumumab, nimotuzumab, nimotuzumab, olaratumab, onartuzumab, oportuzumab monatox, oregovomab, oregovomab, PAM4, panitumumab, patritumab, pemtumomab, pertuzumab, priliximab, racotumomab, radretumab, ramucirumab, rilotumumab, rituximab, robatumumab, samalizumab, SGN-30, SGN-40, sibrotuzumab, siltuximab, tabalumab, tacatuzumab tetraxetan, taplitumomab paptox, tenatumomab, teprotumumab, TGN1412, ticilimumab (=tremelimumab), tigatuzumab, TNX-650, tositumomab, trastuzumab, TRBS07, tremelimumab, TRU-016, TRU-016, tucotuzumab celmoleukin, ublituximab, urelumab, veltuzumab, veltuzumab (IMMU-106), volociximab, votumumab, WX-G250, zalutumumab, and natalizumab; antibodies for treating immune disorders, particularly efalizumab, epratuzumab, etrolizumab, fontolizumab, ixekizumab, mepolizumab, milatuzumab, pooled immunoglobulins, priliximab, rituximab, rontalizumab, ruplizumab, sarilumab, vedolizumab, visilizumab, reslizumab, adalimumab, aselizumab, atinumab, atlizumab, belimumab, besilesomab, BMS-945429, briakinumab, brodalumab, canakinumab, canakinumab, certolizumab pegol, erlizumab, fezakinumab, golimumab, gomiliximab, infliximab, mavrilimumab, natalizumab, ocrelizumab, odulimomab, ofatumumab, ozoralizumab, pexelizumab, rovelizumab, SBI-087, SBI-087, secukinumab, sirukumab, talizumab, tocilizumab, toralizumab, TRU-015, TRU-016, ustekinumab, ustekinumab, vepalimomab, zolimomab aritox, sifalimumab, lumiliximab, and Rho(D) immunoglobulin; antibodies for treating infectious diseases, particularly afelimomab, CR6261, edobacomab, efungumab, exbivirumab, felvizumab, foravirumab, ibalizumab, libivirumab, motavizumab, nebacumab, tuvirumab, urtoxazumab, bavituximab, pagibaximab, palivizumab, panobacumab, PRO140, rafivirumab, raxibacumab, regavirumab, sevirumab, suvizumab, and tefibazumab; antibodies for treating blood disorders, particularly abciximab, atorolimumab, eculizumab, mepolizumab, and milatuzumab; antibodies for immunoregulation, particularly antithymocyte globulin, basilixinnab, cedelizumab, daclizumab, gavilimomab, inolinnomab, muromonab-CD3, muromonab-CD3, odulimomab, and siplizumab; antibodies for treating diabetes, particularly gevokizumab, otelixizumab, and teplizumab; antibodies for treating alzheimer's disease, particularly bapineuzumab, crenezumab, gantenerumab, ponezumab, R1450, and solanezumab; antibodies for treating asthma, particularly benralizumab, enokizumab, keliximab, lebrikizumab, omalizumab, oxelumab, pascolizumab, and tralokinumab; antibodies for treating a variety of disorders, particularly blosozumab, CaroRx, fresolimumab, fulranumab, romosozumab, stamulumab, tanezumab, and ranibizumab; erythropoietin (EPO), granulocyte colony-stimulating factor (G-CSF), α-galactosidase A, α-L-iduronidase, thyrotropin α, *N*-acetylgalactosamine-4-sulfatase (rhASB), dornase alfa, tissue plasminogen activator (TPA) activase, glucocerebrosidase, interferon (IF) β-1α, interferon β-1b, interferon γ, interferon α, TNF-α, IL-1 to IL-36, human growth hormone (rHGH), human insulin (BHI), human chorionic gonadotropin α, darbepoetin α, follicle-stimulating hormone (FSH) and factor VIII, antibodies and antibody derivatives such as bispecific antibodies, multispecific antibodies, ADCs, etc. for treating a variety of disorders. A peptide is a compound formed by linking α-amino acids by peptide bonds, and it is also an intermediate product of protein hydrolysis. Generally, the number of amino acids contained in a peptide is two to nine, and peptides are variously referred to depending on the number of amino acids in the peptides: dipeptide, tripeptide, tetrapeptide, pentapeptide, etc. Peptides consisting of three or more amino acid molecules are known as polypeptides, which have a molecular weight of less than 10,000 Da, being capable of passing through a semipermeable membrane without being precipitated by trichloroacetic acid and ammonium sulfate. In some literature, peptides consisting of 2-10 amino acids are referred to as oligopeptides (small-molecule peptides); peptides consisting of 10-50 amino acids are referred to as polypeptides; peptides consisting of 50 or more amino acids are referred to as proteins. In other words, proteins are sometimes referred to as polypeptides.

"Small-molecule compounds" include 7-methoxypteridine, 7-methylpteridine, abacavir, abafungin, abarelix, acebutolol, acenaphthene, acetaminophen, acetanilide, acetazolamide, acetohexamide, etretinate, acrivastine, adenine, adenosine, alatrafloxacin, albendazole, albuterol, alclofenac, aldesleukin, alemtuzumab, alfuzosin, alitretinoin, allobarbital, allopurinol, all-trans retinoic acid (ATRA), aloxiprin, alprazolam, alprenolol, altretamine, amifostine, amiloride, aminoglutethimide, aminophenazone, amiodarone hydrochloride, amitriptyline, amlodipine, amobarbital, amodiaquine, amoxapine, amphetamine, amphotericin, amphotericin B, ampicillin, amprenavir, amsacrine, amyl nitrate, amobarbital, anastrozole, anrinone, anthracene, anthracycline antibiotics, aprobarbital, arsenic trioxide, asparaginase, aspirin, astemizole, atenolol, atorvastatin, atovaquone, atrazine, atropine, atropine azathioprine, auranofin, azacitidine, azapropazone, azathioprine, azintamide, azithromycin, aztreonam, baclofen, barbital, live BCG vaccine, beclamide, beclomethasone, bendroflumethiazide, benezepril, benidipine, benorilate, benperidol, bentazepam, benzamide, benzanthracene, benzathine penicillin, benzhexol hydrochloride, benznidazole, benzodiazepines, benzoic acid, bephenium hydroxynaphthoate, betamethasone, bevacizumab (atorvastatin), bexarotene, bezafibrate, bicalutamide, bifonazole, biperiden, bisacodyl, bisantrene, bleomycin, bleomycin, bortezomib, brinzolamide, bromazepam, bromocriptine mesylate, bromperidol, brotizolam, budesonide, bumetanide, bupropion, busulfan, butalbital, butamben, butenafine hydrochloride, butobarbital, butobarbital (butethal), butoconazole, butoconazole nitrate, butyl p-hydroxybenzoate, caffeine, calcidiol, calciprotriene, calcitriol, calusterone, cambendazole, camphor, camptothecin, camptothecin analogue, candesartan, capecitabine, capsaicin, captopril, carbamazepine, carbimazole, carbofuran, carboplatin, carbromal, carimazole, carmustine, cefamandole, cephazolin, cefixime, ceftazidime, cefuroxime axetil, celecoxib, cefradine, cerivastatin, cetirizine, cetuximab, chlorambucil, chloramphenicol, chlordiazepoxide, chlormethiazole, chloroquine, chlorothiazide, chlorpheniramine, chlorproguanil hydrochloride, chlorpromazine, chlorpropamide, chlorprothixene, chlorpyrifos, chlortetracycline, chlorthalidone, chlorzoxazone, cholecalciferol, cilostazol, cimetidine, cinnarizine, cinoxacin, ciprofibrate, ciprofloxacin hydrochloride, cisapride, cisplatin, citalopram, cladribine, clarithromycin, clemastine fumarate, clioquinol, clobazam, clofarabine, clofazimine, clofibrate, clomifene citrate, clomipramine, clonazepam, clopidogrel, clotiazepam, clotrimazole, clotrimazole, cloxacillin, clozapine, cocaine, codeine, colchicine, colistin, conjugated estrogen, corticosterone, cortisone, cortisone acetate, cyclizine, cyclobarbital, cyclobenzaprine, cyclobutane-spirobarbiturate, cycloethane-spirobarbiturate, cycloheptane-spirobarbiturate, cyclohexane-spirobarbiturate, cyclopentane-spirobarbiturate, cyclophosphamide, cyclopropane-spirobarbiturate, cycloserine, ciclosporin, cyproheptadine, cyproheptadine hydrochloride, cytarabine, cytosine, dacarbazine, dactinomycin, danazol, danthron, dantrolene sodium, dapsone, darbepoetin alfa, darodipine, daunorubicin, decoquinate, dehydroepiandrosterone, delavirdine, demeclocycline, denileukin, deoxycorticosterone, desoximetasone, dexamethasone, dextroamphetamine, dextrochlorpheniramine, dexfenfluramine, dexrazoxane, dextropropoxyphene, heroin, amidotrizoic acid, diazepam, diazoxide, dichlorophene, dichlorprop, diclofenac, dicoumarin, didanosine, diflunisal, digitoxin, digoxin, dihydrocodeine, dihydroequilin, dihydroergotamine mesylate, diiodohydroxyquinoline, diltiazem hydrochloride, diloxanide furoate, dimenhydrinate, dimorpholamine, dinitolmide, diosgenin, diphenoxylate hydrochloride, biphenyl, dipyridamole, dirithromycin, disopyramide, disulfiram, diuron, docetaxel, domperidone, donepezil, doxazosin, doxazosin hydrochloride, doxorubicin (neutral), doxorubicin hydrochloride, doxycycline, dromostanolone propionate, droperidol, dyphylline, echinocandin, econazole, econazole nitrate, efavirenz, ellipticine, enalapril, enlimomab, enoximone, epinephrine, epipodophyllotoxin derivatives, epirubicin, epoetin alfa, eposartan, equilenin, equilin, ergocalciferol, ergotamine tartrate, erlotinib, erythromycin, estradiol, estramustine, estriol, estrone, ethacrvnic acid, ethambutol, ethinamate, ethionamide, ethopropazine hydrochloride, ethyl 4-aminobenzoate (benzocaine), ethyl *p*-hydroxybenzoate, ethinyl estradiol, etodolac, etomidate, etoposide, etretinate, exemestane, felbamate, felodipine, fenbendazole, fenbuconazole, fenbufen, fenchlorphos, fenclofenac, fenfluramine, fenofibrate, fenoldepam, fenoprofen calcium, fenoxycarb, fenpiclonil, fentanyl, fenticonazole, fexofenadine, filgrastim, finasteride, flecainide acetate, floxuridine, fludarabine, fluconazole, fluconazole, flucytosine, fludioxonil, fludrocortisone, fludrocortisone acetate, flufenamic acid, flunanisone, flunarizine hydrochloride, flunisolide, flunitrazepam, fluocortolone, fluometuron, fluorene, fluorouracil, fluoxetine hydrochloride, fluoxymesterone, flupentixol decanoate, fluphenthixol decanoate, flurazepam, flurbiprofen, fluticasone propionate, fluvastatin, folic acid, fosinopril, fosphenytoin sodium, frovatriptan, furosemide, fulvestrant, furazolidone, gabapentin, G-BHC (lindane), gefitinib, gemcitabine, gemfibrozil, gemtuzumab, glafenine, glibenclamide, gliclazide, glimepiride, glipizide, glutethimide, glibenclamide, glyceryl trinitrate (nitroglycerin), goserelin acetate, grepafloxacin, griseofulvin, guaifenesin, guanabenz acetate, guanine, halofantrine hydrochloride, haloperidol, hydrochlorothiazide, heptabarbital, heroin, hesperetin, hexachlorobenzene, hexethal, histrelin acetate, hydrocortisone, hydroflumethiazide, hydroxyurea, scopolamine, hypoxanthine, ibritumomab, ibuprofen, idarubicin, allylbutylbarbituric acid, ifosfamide, ihydroequilenin, imatinib mesylate, imipenem, indapamide, indinavir, indomethacin, indoprofen, interferon α-2a, interferon α-2b, iodamide, iopanoic acid, iprodione, irbesartan, irinotecan, isavuconazole, isocarboxazid, isoconazole, isoguanine, isoniazid, isopropyl barbiturate, isoproturon, isosorbide dinitrate, isosorbide mononitrate, isradipine, itraconazole, itraconazole, itraconazole (Itra), ivermectin, ketoconazole, ketoprofen, ketorolac, khellin, labetalol, lamivudine, lamotrigine, lanatoside C, lanosprazole, L-DOPA, leflunomide, lenalidomide, letrozole, folinic acid, leuprolide acetate, levamisole, levofloxacin, lidocaine, linuron, lisinopril, lomefloxacin, lomustine, loperamide, loratadine, lorazepam, lorefloxacin, lormetazepam, losartan mesylate, lovastatin, lisuride maleate, maprotiline hydrochloride, mazindol, mebendazole, meclozine hydrochloride, meclofenamic acid, medazepam, metildigoxin, medroxyprogesterone acetate, mefenamic acid, mefloquine hydrochloride, megestrol acetate, melphalan, mepenzolate bromide, meprobamate, meptazinol, purinethol, mesalazine, mesna, mesoridazine, mestranol, methadone, methaqualone, methocarbamol, mephenytoin, methotrexate, methoxsalen, methsuximide, methyclothiazide, methylphenidate, mephobarbital, methylparaben, methylprednisolone, methyltestosterone, methyprylon, methysergide maleate, metoclopramide, metolazone, metoprolol, metronidazole, mianserin hydrochloride, miconazole, midazolam, mifepristone, migltol, minocycline, minoxidil, mitomycin C, mitotane, mitoxantrone, mycophenolate mofetil, molindone, montelukast, morphine, moxifloxacin hydrochloride, nabumetone, nadolol, nalbuphine, nalidixic acid, nandrolone, tetracene, naphthalene, naproxen, naratriptan hydrochloride, natamycin, nelarabine, nelfinavir, nevirapine, nicardipine hydrochloride, niacinamide, niacin, acenocoumarol, nifedipine, nilutamide, nimodipine, nimorazole, nisodipine, nitrazepam, furantoin, furacillin, nizatidine, nofetumomab, norethindrone, norfloxacin, norgestrel, nortriptyline hydrochloride, nystatin, estradiol, ofloxacin, olanzapine, omeprazole, omoconazole, ondansetron hydrochloride, oprelvekin, ornidazole, oxaliplatin, oxamniquine, oxantel pamoate, oxaprozin, oxatomide, oxazepam, oxcarbazepine, oxfendazole, oxiconazole, oxprenolol, oxyphenbutazone, oxyphencyclimine hydrochloride, paclitaxel, palifermin, pamidronate, *p*-aminosalicylic acid, pantoprazole, paramethadione, paroxetine hydrochloride, pegademase, pegaspargase, pegfilgrastim, pemetrexed disodium, penicillamine, pentaerythritol tetranitrate, pentazocin, pentazocin, pentobarbital, pentobarbitone, pentostatin, pentoxifylline, perphenazine, perphenazine pimozide, perylene, phenacemide, phenacetin, phenanthrene, phenindione, phenobarbitone, phenobarbital, phenolphthalein, phenoxybenzamine, phenoxybenzamine hydrochloride, phenoxymethylpenicillin, phensuximide, phenylbutazone, phenytoin, pindolol, pioglitazone, pipobroman, piroxicam, pizotifen maleate, platinum compounds, mithramycin, polyenoid, polymyxin B, porfimer sodium, posaconazole (Posa), pramipexole, prasterone, pravastatin, praziquantel, prazosin, prazosin hydrochloride, prednisolone, prednisone, primidone, probarbital, probenecid, probucol, procarbazine, prochlorperazine, progesterone, guanatol hydrochloride, promethazine, propofol, propoxur, propranolol, propyl paraben, propylthiouracil, prostaglandin, pseudoephedrine, pteridine-2-methyl-thiol, pteridine-2-thiol, pteridine-4-methyl-thiol, pteridine-4-thiol, pteridine-7-methyl-thiol, pteridine-7-thiol, pyrantel pamoate, pyrazinamide, pyrene, pyridostigmine, pyrimethamine, quetiapine, mepacrine, quinapril, quinidine, quinidine sulfate, quinine, quinine sulfate, rabeprazole sodium, ranitidine hydrochloride, rasburicase, ravuconazole, repaglinide, reposal, reserpine, retinoids, rifabutin, rifampin, rifapentine, rimexolone, risperidone, ritonavir, rituximab, rizatriptan benzoate, rofecoxib, ropinirole hydrochloride, rosiglitazone, saccharin, salbutamol, salicylamide, salicylic acid, saquinavir, sargramostim, butabarbital, seco-barbital, sertaconazole, sertindole, sertraline hydrochloride, simvastatin, sirolimus, sorafenib, sparfloxacin, spiramycin, spironolactone, dihydrotestosterone, stanozolol, stavudine, diethylstilbestrol, streptozocin, strychnine, sulconazole, sulconazole nitrate, sulfacetamide, sulfadiazine, sulfamerazine, sulfamethazine, sulfamethoxazole, sulfanilamide, sulfathiazole, sulindac, sulphabenzamide, sulphacetamide, sulphadiazine, sulphadoxine, sulfisoxazole, sulphamerazine, sulpha-methoxazole, sulphapyridine, sulfasalazine, sulfinpyrazone, sulpiride, sulthiame, sumatriptan succinate, sunitinib maleate, tacrine, tacrolimus, talbutal, tamoxifen citrate, tamulosin, targretin, taxane, tazarotene, telmisartan, temazepam, temozolomide, teniposide, tenoxicam, terazosin, terazosin hydrochloride, terbinafine hydrochloride, terbutaline sulfate, terconazole, terfenadine, testolactone, testosterone, tetracycline, tetrahydrocannabinol, tetroxoprim, thalidomide, thebaine, theobromine, theophylline, thiabendazole, thiamphenicol, thioguanine, thioridazine, thiotepa, thotoin, thymine, tiagabine hydrochloride, tibolone, ticlopidine, tinidazole, tioconazole, tirofiban, tizanidine hydrochloride, tolazamide, tolbutamide, tolcapone, topiramate, topotecan, toremifene, tositumomab, tramadol, trastuzumab, trazodone hydrochloride, tretinoin, triamcinolone, triamterene, triazolam, triazoles, triflupromazine, trimethoprim, trimipramine maleate, triphenylene, troglitazone, tromethamine, tropicamide, trovafloxacin, tybamate, ubidecarenone (coenzyme Q10), undecylenic acid, uracil, uramustine, uric acid, valproic acid, valrubicin, valsartan, vancomycin, venlafaxine hydrochloride, vigabatrin, vinbarbital, vinblastine, vincristine, vinorelbine, voriconazole, xanthine, zafirlukast, zidovudine, zileuton, zoledronate, zoledronic acid, zolmitriptan, zolpidem, and zopiclone.

An antigen (abbreviated as Ag) refers to a substance capable of causing antibody production and is any substance capable of inducing an immune response. Foreign molecules may be recognized by immunoglobulin on B cells, or treated by antigen-presenting cells, combined with a major histocompatibility complex to obtain a complex to reactivate T cells and trigger a continuous immune response.

An antigenic epitope, also known as an antigenic determinant, may be a special chemical group which consists of a continuous sequence (a protein primary structure) or a discontinuous protein three-dimensional structure and determines antigenicity. Most of antigenic epitopes are present on the surface of antigenic substances, while some of the antigenic epitopes are present inside the antigenic substances and must be treated with enzymes or other means before being exposed. One natural antigenic substance may have a variety of and a plurality of determinants. The larger the molecule of an antigen, the greater the number of the epitopes.

Tumor-associated antigens (TAAs) refer to antigen molecules present on tumor cells or normal cells, including: embryonic proteins, glycoprotein antigens, squamous cell antigens, and the like, and are commonly used for clinical diagnosis of tumors. The tumor-associated antigens are not specific to the tumor cells, may be synthesized in minute amounts on the normal cells, are highly expressed when the tumor cells proliferate, and are therefore referred to as "associated antigens". Tumors derived from the same tissue type have the same tumor-associated antigens in different individuals.

Pathogenic microbes, also known as pathogens, refer to microbes that can invade human bodies and cause infections and even infectious diseases. Bacteria and viruses are the most harmful of pathogens. Pathogenic microbes refer to prions, fungi, bacteria, spirochetes, mycoplasmas, rickettsias, chlamydias, and viruses. Pathogenic microbial antigens refer to substances that are derived from pathogens and have the function of triggering an immune response.

"Lipid encapsulation" refers to lipid nanoparticles which provide an active or a therapeutic agent (e.g., a nucleic acid (e.g., mRNA)), and comprise full encapsulation and partial encapsulation, or both. In some embodiments, the nucleic acid (e.g., mRNA) is completely encapsulated in the lipid nanoparticle.

In various embodiments, the lipid nanoparticle has an average diameter of: about 90 nm to about 600 nm, about 100 nm to about 550 nm, about 150 nm to about 500 nm, about 200 nm to about 400 nm, about 250 nm to about 300 nm, or about 200 nm to about 300 nm, and is substantially non-toxic.

### B. Helper lipid

In some aspects of the present disclosure, a composition containing one or more helper lipids is mixed with the cationic lipid disclosed herein to produce a lipid nanoparticle. In some embodiments, the cationic lipid is mixed with 1, 2, 3, 4, or 5 different types of helper lipids. It is contemplated that the cationic lipid may be mixed with a single type of a variety of different helper lipids. In some embodiments, the helper lipid comprises, but is not limited to, one or more of a phospholipid, a steroid or a steroid derivative, a polymer-conjugated lipid, and a modified lipid.

"Phospholipid" is any lipid comprising a phosphoester group. In some embodiments, the phospholipid is a structure containing one or two long chain C6-C24 alkyl or alkenyl groups, glycerol or sphingosine, one or two phosphoester groups, and optionally a small organic molecule. In some embodiments, the small organic molecule is an amino acid, sugar, or an amino-substituted alkoxy group, such as choline or ethanolamine. In some embodiments, the phospholipid is phosphatidylcholine. In some embodiments, the phospholipid is DOPE, DSPC, DPPC, DMPC, DOPC, POPC, or SM. In some embodiments, the phospholipid is distearoyl phosphatidylcholine or dioleoyl phosphatidylethanolamine.

"Steroid and the steroid derivative" comprises any steroid or steroid derivative. As used herein, in some embodiments, the term "steroid" is a class of compounds having a tetracyclic 17-carbon cyclic structure, which may further contain one or more substitutions, wherein the substitutions include alkyl, alkoxy, hydroxyl, oxo and acyl or double bonds between two or more carbon atoms. In one aspect, the ring structure of the steroid comprises three fused cyclohexyl rings and a fused cyclopentyl ring. In some embodiments, the steroid derivative comprises the above ring structure with one or more non-alkyl substitutions. In some embodiments, the steroid or the steroid derivative is a sterol. In some embodiments of the present disclosure, the steroid or the steroid derivative is cholestane or a cholestane derivative. As described above, the cholestane derivative comprises one or more non-alkyl substitutions of the above ring system. In some embodiments, the cholestane or the cholestane derivative is a cholestene or a cholestene derivative, or a sterol or a sterol derivative. In other embodiments, the cholestane or the cholestane derivative is a cholestene and a sterol or a derivative thereof.

"Polymer-conjugated lipid" refers to a lipid that inhibits aggregation of lipid nanoparticles or improves the stability of lipid nanoparticles or alters the immune response or alters the circulation time *in vivo.* The polymer-conjugated lipid includes, but is not limited to, a polyethylene glycol-conjugated lipid, a polylactic acid-conjugated lipid, a polyamide-conjugated lipid, a cationic polymer-conjugated lipid, a polysarcosine (pSar)-conjugated lipid, a poly lactic-co-glycolic acid (PLGA)-conjugated lipid, a polyamino acid-conjugated lipid, a polypeptide-conjugated lipid, and a polypeptoid-conjugated lipid, or a mixture thereof. In some embodiments, the "polyethylene glycol-conjugated lipid" refers to any lipid to which a PEG group has been connected. In some embodiments, the PEG lipid is a diglyceride, also comprising a PEG chain connected to a glycerol group. In other embodiments, the PEG lipid is a compound containing one or more C6-C24 long chain alkyl or alkenyl groups or C6-C24 fatty acid groups connected to a linker group via a PEG chain. Some non-limiting examples of the PEG lipid include PEG-modified phosphatidylethanolamine and phosphatidic acid, PEG-conjugated ceramide, PEG-modified dialkylamine, PEG-modified 1,2-diacyloxypropane-3-amine, and PEG-modified diacylglycerol and dialkylglycerol. In some embodiments, PEG-modified distearoyl phosphatidylethanolamine or PEG-modified dimyristoyl-sn-glycerol. In some embodiments, PEG modification is measured with the molecular weight of the PEG component of the lipid. In some embodiments, the PEG used for modification has a molecular weight of about 100 to about 15,000. In some embodiments, the molecular weight is about 200 to about 500, about 400 to about 5000, about 500 to about 3000, or about 1200 to about 3000. The molecular weight of the PEG used for modification is about 100, 200, 400, 500, 600, 800, 1000, 1250, 1500, 1750, 2000, 2250, 2500, 2750, 3000, 3500, 4000, 4500, 5000, 6000, 7000, 8000, 9000, 10000 or 12500 to about 15000. "Modified lipid" includes lipids modified by small-molecule compounds, vitamins, carbohydrates, peptides, proteins, nucleic acids, lipopolysaccharides, inorganic molecules or particles, metal ions or particles, and combinations of the substances described above.

### English abbreviation list

| Abbreviation | In English |
|---|---|
| siRNA | small interference RNA |
| saRNA | small activating RNA |
| samRNA | self-amplifying mRNA |
| miRNA | microRNA |
| aiRNA | asymmetric interfering RNA |
| dsRNA | Dicer-substrate RNA |
| shRNA | small hairpin RNA |
| DOTAP | 1,2-dioleoyloxy-3-trimethylammonium propane chloride |
| DOTMA | N-[1-(2,3-dioleoyloxy)propyl]-N, N,N-trimethylammonium chloride |
| DSTAP | 1,2-stearoyl-3- |
| | trimethylammonium-propane |
| DMTAP | 1,2-dimyristoyl-3-trimethylammonium-propane |
| DDA | dimethyldioctadecylammonium |
| DOBAQ | N-(4-carboxybenzyl)-N,Ndimethyl-2,3-bis (oleoyloxy) propan-1-aminium |
| MC3 | 4-(dimethylamino)-butanoic acid, (10Z,13Z)-1-(9Z,12Z)-9,12-octadecadien-1-yl-10,13-nonadecadien-1-yl ester |
| SM | sphingomyelin |
| SM-102 | 8-[(2-hydroxyethyl)[6-oxo-6-(undecyloxy)hexyl]amino]-octanoic acid, 1-octylnonyl ester |
| ALC-0315 | 2-hexyl-decanoic acid, 1,1'-[[(4-hydroxybutyl)imino]di-6,1-hexanediyl] ester |
| Lipid 5 | 8-[(2-hydroxyethyl)[8-(nonyloxy)-8-oxooctyl]amino]-octanoic acid, 1-octylnonyl ester |
| A6 | di(dec-3-yn-1-yl) 9-((4-(dimethylamino)butanoyl)oxy)heptadecanedioate |
| DC-chol | 3β-[N-(N',N'-dimethylaminoethane)-carbamoyl]cholesterol |
| C12-200 | 1,1'-((2-(4-(2-((2-(bis(2-hydroxydodecyl)amino)ethyl)(2-hydroxydodecyl)amino)ethyl)piperazin-1-yl)ethyl)azanediyl)bis(dodecan-2-ol) |
| CKK-E12 | 3,6-bis(4-(bis(2-hydroxydodecyl)amino)butyl)piperazine-2,5-dione |
| 5A2-SC8 | 1,19-Bis[2-[2-methyl-3-(octylthio)-1-oxopropoxy]ethyl] 4,10,16-tris[3-[2-[2-methyl-3-(octylthio)-1-oxopropoxy]ethoxy]-3-oxopropyl]-4,7,10,13,16-pentaazanonadecanedioate |
| G0-C14 | N-[2-[Bis(2-hydroxytetradecyl)amino]ethyl]-16-hydroxy-14-(2-hydroxytetradecyl)-4,7-bis[3-[[2-[(2-hydroxytetradecyl)amino]ethyl]amino]-3-oxopropyl]-10-oxo-4,7,11,14-tetraazaoctacosanamide |
| OF-2 | 3,6-Bis[4-[bis[(9Z,12Z)-2-hydroxy-9,12-octadecadien-1-yl]amino]butyl]-2,5-piperazinedione |
| OF-Deg-Lin | 9,12-Octadecadienoic acid (9Z,12Z)-, 1,1',1",1‴-[(3,6-dioxo-2,5-piperazinediyl)bis(4,1-butanediylnitrilodi-2,1-ethanediyl)] ester |
| 92-O17S | bis(2-(tetradecylthio)ethyl) 3,3'-((3-(1H-imidazol-1-yl)propyl)azanediyl)dipropionate |
| OF-C4-Deg-Lin | 2-[4-[5-[4-[bis[2-[(9Z,12Z)-octadeca-9,12-dienoyl]oxyethyl]amino]butyl]-3,6-dioxopiperazin-2-yl]butyl-[2-[(9Z,12Z)-octadeca-9,12-dienoyl]oxyethyl]amino]ethyl (9Z,12Z)-octadeca-9,12-dienoate |
| A18-iso5-2DC18 | ethyl 1-(3-(2-ethylpiperidin-1-yl) propyl)-5,5-di((Z)-heptadec-8-en-1-yl)-2,5-dihydro-1H-imidazole-2-carboxylate |
| TT3 | N1,N3,N5-tris(3-(didodecylamino)propyl)benzene-1,3,5-tricarboxamide |
| FTT5 | hexa(octan-4-yl) 9,9',9",9‴,9ʺʺ,9ʺ‴-((((benzene-1,3,5-tricarbonyl)tris(azanediyl))tris(propane-3,1-diyl))tris(azanetriyl))hexanonanoate |
| BAMEA-O16B | bis(2-(dodecyldisulfaneyl)ethyl) 3,3'-((3-methyl-9-oxo-10-oxa-13,14-dithia-3,6-diazahexacosyl)azanediyl)dipropionate |
| Vc-Lipid | (S)-2-((R)-3,4-bis(benzyloxy)-5-oxo-2,5-dihydrofuran-2-yl)-2-hydroxyethyl 6-((3-(didodecylamino)propyl) (dodecyl)amino)hexanoate |
| C14-4 | 1,1'-((2-(2-(4-(2-((2-(2-(bis(2-hydroxytetradecyl)amino)ethoxy)ethyl)(2-hydroxytetradecyl)amino)ethyl)piperazin-1-yl)ethoxy)ethyl)azanediyl)bis(tetradecan-2-ol) |
| Lipid 14 | ((2-((4-(dimethylamino)butanoyl)oxy)ethyl)azanediyl)bis(octane-8,1- |
| | diyl) bis(2-hexyldecanoate) |
| 4A3-Cit | 2-[3-[3-[3-[bis[3-[2-[3-(3,7-dimethyloct-6-enylsulfanyl)-2-methylpropanoyl]oxyethoxy]-3-oxopropyl]amino]propyl-methylamino]propyl-[3-[2-[3-(3,7-dimethyloct-6-enylsulfanyl)-2-methylpropanoyl]oxyethoxy]-3-oxopropyl]amino]propanoyloxy]ethyl 3-(3,7-dimethyloct-6-enylsulfanyl)-2-methylpropanoate |
| ssPalmO-Phe | [4-[2-[2-[1-[2-[2-[4-[2-[2-[4-[(Z)-octadec-9-enoyl]oxyphenyl]acetyl]oxyethyl]piperidin-1-yl]ethyldisulfanyl]ethyl]piperidin-4-yl]ethoxy]-2-oxoethyl]phenyl] (Z)-octadec-9-enoate |
| DOPE | 1,2-dioleoyl-sn-glycero-3-phosphoethanolamine |
| DSPC | 1,2-distearoyl-sn-glycero-3-phosphocholine |
| DPPC | 1,2-dipalmitoyl-sn-glycero-3-phosphocholine |
| DMPC | 1,2-dimyristoyl-sn-glycero-3-phosphocholine |
| DOPC | 1,2-dioleoyl-sn-glycero-3-phosphocholine |
| POPC | 1-palmitoyl-2-oleoyl-sn-glycero-3-phosphocholine |
| ALC-0159 | 2-[(Polyethylene glycol)-2000]-N,N-ditetradecylacetamide |
| PEG-DMG | 1,2-Dimyristoyl-rac-glycero-3-methoxypolyethylene glycol |
| PEG-DSPE | 1, 2-Distearoyl-sn-glycero-3-phosphoethanolamine-Poly (ethylene glycol) |
| GOLD | Guided On-Target Lipid Delivery |
| ASO | Antisense oligonucleotide |
| Agomir | Agonist microRNA |
| Antagomir | Antagonist microRNA |
| pSar | Polysarcosine |
| PLGA | Poly lactic-co-glycolic acid |
| circRNA | Circular RNA |
| tRNA | Transfer RNA |
| cDNA | Complementary DNA |
| pDNA | Plasmid DNA |

In order to more clearly describe the objects, features, and advantages of the present application, the present application will be described below in detail with reference to the drawings and specific embodiments, but the embodiments of the present application described below in detail are intended only to illustrate the content of the present application and do not constitute any limitation to the present application.

In the following examples, TMF1 to TMF54 refer to lipid nanoparticles formed using the compounds prepared in Example 1 as cationic lipids and other components, with no limitation on the mRNA encapsulated therein.

ALC-0315 was used as a control, and the existing standard formulation for ALC-0315 was used to encapsulate mRNA to form a lipid nanoparticle. DODAP was used as a control, and a lipid nanoparticle was prepared with reference to the preparation method for TMF2, wherein the lipid composition was replaced according to the GOLD lipid and helper lipids listed in Table 3.

### Example 1: Synthesis of Compounds

The synthesis method for compound 1 was as follows:

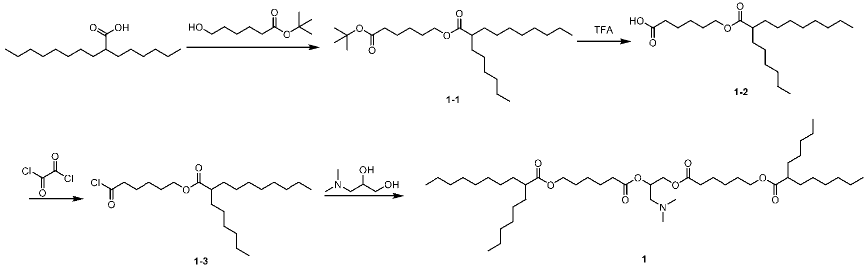

### Step 1: synthesis of compound 1-1

DMAP (2.5 g, 20.1 mmol), DCC (4.1 g, 20.1 mmol), and DCM (50 mL) were added into a 250 mL flask, and the mixture was stirred until it became clear. 2-Hexyldecanoic acid (5.13 g, 20.1 mmol) was added, and the mixture was stirred at room temperature for 30 min. *tert-*Butyl 6-hydroxyhexanoate (3.6 g, 19.1 mmol) was then added in one batch, and the mixture was stirred at room temperature overnight. The extent of the reaction was detected by TLC (30% *n-*heptane/DCM). After completion of the reaction, the reaction solution was filtered to remove solids, and the filtrate was washed with 15 mL of water and 15 mL of saturated brine, dried over anhydrous Na₂SO₄, and filtered. The organic phase was concentrated to give a crude product (about 9.3 g). The crude product was purified and separated using a Flash column (80 g of silica gel, loaded with 100 mL of n-heptane; the mobile phases were respectively: 300 mL of n-heptane; 300 mL of 10% DCM + 90% n-heptane; and 300 mL of 20% DCM + 60% *n-*heptane) to give a pure product (1-1, 7 g, 86% yield). ¹H NMR (400 MHz, CDCl₃): *δ* 4.05 (m, 2H), 2.31-2.26 (m, 3H), 1.67-1.18 (m, 39H), 0.88-0.85 (m, 6H).

### Step 2: synthesis of compound 1-2

1-1 (600 mg, 9.7 mmol) dissolved in DCM (12 mL) was added into a 50 mL flask, and TFA (4 mL) was added. After the mixture was stirred at room temperature for 2 h, the extent of the reaction was detected by TLC (0.5% MeOH/DCM). After completion of the reaction, the reaction solution was concentrated, followed by addition of MTBE (20 mL) for dissolution. The pH was adjusted to 8-9 with a 10% NaHCO₃ solution, and then the pH was adjusted to 1-2 with 1 M diluted hydrochloric acid. The mixture was stirred for 20 min, followed by layer separation. The aqueous phase was extracted with 20 mL of MTBE, and the organic phases were combined, dried over anhydrous Na₂SO₄, filtered, and concentrated to give a crude product (620 mg). The crude product was purified and separated using a Flash column (10 g of silica gel, loaded with 50 mL of DCM; the mobile phases were respectively: 100 mL of DCM; 100 mL of 1% MeOH + 99% DCM; 100 mL of 2% MeOH + 98% DCM; 100 mL of 3% MeOH + 97% DCM; and 100 mL of 4% MeOH + 96% DCM) to give a pure product (1-2, 410 mg, 78% yield). ¹H NMR (400 MHz, CDCl₃): *δ* 4.09-4.05 (m, 2H), 2.38-2.28 (m, 3H), 1.71-1.20 (m, 30H), 0.89-0.85 (m, 6H).

### Step 3: synthesis of compound 1-3

1-2 (240 mg, 0.65 mmol) dissolved in DCM (3 mL) was added into a 25 mL flask, and DMF (10 µL) was added. The reaction solution was cooled in an ice-salt bath to 0-5 °C. With the temperature of the reaction solution maintained at 0-5 °C, oxalyl chloride (98.6 mg, 0.78 mmol) was slowly added dropwise to the solution, and the mixture was stirred at this temperature for 3 h. The extent of the reaction was detected by TLC (20% EtOAc/n-heptane). After completion of the reaction, the reaction solution was concentrated to dryness to give a crude product (1-3, about 220 mg, 87% yield), which was used directly in the next step.

### Step 4: synthesis of compound 1

3-Dimethylamino-1,2-propanediol (30.6 mg, 0.26 mmol), N,N-diisopropylethylamine (99.7 mg, 0.77 mmol), and THF (2 mL) were added into a 25 mL flask, and the reaction solution was cooled in an ice bath to 0-5 °C. A solution of 1-3 (220 mg, 0.57 mmol) in THF (2 mL) was added dropwise to the reaction solution, and after the dropwise addition, the mixture was stirred at room temperature overnight. The extent of the reaction was detected by TLC (0.5% MeOH/DCM). After completion of the reaction, 2 mL of water was added to the reaction solution to quench the reaction, and the mixture was extracted twice with EtOAc (10 mL). The organic phases were combined, washed with 5 mL of saturated brine, dried over anhydrous Na₂SO₄, filtered, and concentrated to give a crude product (about 170 mg). The crude product was purified and separated using a Flash column (8 g of silica gel, loaded with 50 mL of *n-*heptane; the mobile phases were respectively: 100 mL of n-heptane; 50 mL of 1% EtOAc + 99% n-heptane; 50 mL of 2% EtOAc + 98% n-heptane; 50 mL of 3% EtOAc + 96% n-heptane; and 50 mL of 4% EtOAc + 96% n-heptane) to give a pure product (compound 1, 40 mg, 86% yield). ¹H NMR (400 MHz, CDCl₃): *δ* 5.22-5.18 (m, 1H), 4.38-4.34 (m, 1H), 4.10-4.04 (m, 5H), 2.49-2.43 (m, 2H), 2.35-2.27 (m, 12H), 1.69-1.25 (m, 54H), 0.89-0.85 (m, 12H).

The synthesis method for compound 2 was as follows:

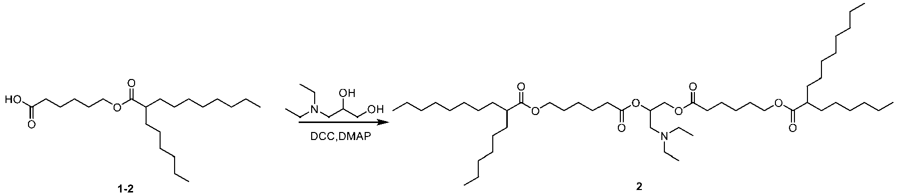

### Step 1: synthesis of compound 2

DMAP (278.4 mg, 1.35 mmol), DCC (37.5 mg, 0.31 mmol), and DCM (50 mL) were added into a 250 mL flask, and the mixture was stirred until it became clear. 1-2 (500 mg, 1.35 mmol) was added, and the mixture was stirred at room temperature for 30 min. 3-Diethylamino-1,2-propanediol (90.3 mg, 0.61 mmol) was then added, and the mixture was stirred at room temperature for 5 h. The extent of the reaction was detected by TLC (10% EtOAc/n-heptane). After completion of the reaction, the reaction solution was filtered to remove solids, and the filtrate was separately washed with 15 mL of water and 15 mL of saturated brine, followed by liquid separation. The organic phase was dried over anhydrous Na₂SO₄, filtered, and concentrated to give a crude product (about 530 mg). The crude product was purified and separated using a Flash column (8 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 3% EtOAc + 97% n-heptane; and 500 mL of 10% EtOAc + 90% *n-*heptane) to give a pure product (350 mg, 71% yield). ¹H NMR (400 MHz, CDCl₃): *δ* 5.13-5.09 (m, 1H), 4.38-4.34 (m, 1H), 4.14-4.04 (m, 5H), 2.59-2.47 (m, 6H), 2.33-2.26 (m, 6H), 1.69-1.53 (m, 13H), 1.46-1.36 (m, 9H), 1.33-1.24 (m, 43H), 0.99 (t, *J*=8Hz, 6H), 0.87(t, *J*=4Hz, 12H).

The synthesis method for compound 3 was as follows:

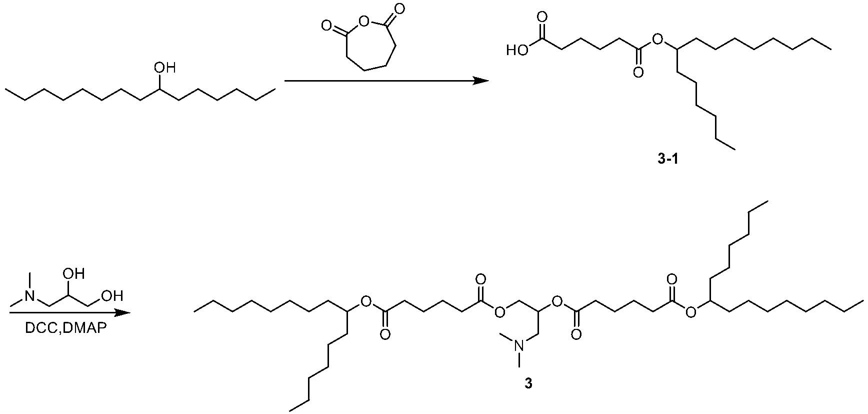

### Step 1: synthesis of compound 3-1

Pentadecan-7-ol (1.9 g, 8.32 mmol), DMAP (1.02 g, 8.32 mmol), and DCM (19 mL) were added into a 100 mL flask, and the mixture was stirred until it became clear. Adipic anhydride (1.6 g, 12.5 mmol) was added, and the mixture was stirred at room temperature for 2 h. The extent of the reaction was detected by TLC (30% EtOAc/n-heptane). After completion of the reaction, the reaction solution was separately washed with 5 mL of water and 5 mL of saturated brine, followed by liquid separation. The organic phase was dried over anhydrous Na₂SO₄, filtered, and concentrated to give a crude product (about 3.1 g). The crude product was purified and separated using a Flash column (60 g of silica gel, loaded with 200 mL of *n*-heptane; the mobile phases were respectively: 300 mL of 1% EtOAc + 99% *n*-heptane; 300 mL of 2% EtOAc + 98% *n*-heptane; 300 mL of 5% EtOAc + 95% n-heptane; and 1000 mL of 10% EtOAc + 90% *n*-heptane) to give a pure product (1.2 g, 40% yield). ¹H NMR (400 MHz, CDCl₃): *δ* 4.89-4.85 (m, 1H), 2.41-2.29 (m, 4H), 1.72-1.63 (m, 4H), 1.52-1.48 (m, 4H), 1.32-1.25 (m, 20H), 0.87 (t, *J*=4Hz, 6 H).

### Step 2: synthesis of compound 3

DMAP (39 mg, 0.32 mmol), DCC (289 mg, 1.4 mmol), and DCM (5 mL) were added into a 250 mL flask, and the mixture was stirred until it became clear. 3-1 (500 mg, 1.4 mmol) was added, and the mixture was stirred at room temperature for 30 min. 3-Dimethylamino-1,2-propanediol (76 mg, 0.64 mmol) was then added, and the mixture was stirred at room temperature overnight. The extent of the reaction was detected by TLC (30% EtOAc/*n-*heptane). After completion of the reaction, the reaction solution was filtered to remove solids, and the filtrate was separately washed with 5 mL of water and 5 mL of saturated brine, followed by liquid separation. The organic phase was dried over anhydrous Na₂SO₄, filtered, and concentrated to give a crude product (about 530 mg). The crude product was purified and separated using a Flash column (10 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 3% EtOAc + 97% *n*-heptane; 100 mL of 10% EtOAc + 90% *n-*heptane; and 500 mL of 20% EtOAc + 80% n-heptane) to give a pure product (320 mg, 63% yield). ¹H NMR (400 MHz, CDCl₃): *δ* 5.23-5.18 (m, 1H), 4.89-4.82 (m, 2H), 4.38-4.35 (m, 1H), 4.11-4.06 (m, 1H), 2.49 (s, 1H), 2.37-2.28 (m, 14H), 1.70-1.92 (m, 9H), 1.52-1.48 (m, 9H), 0.87 (t, J=4Hz, 12 H).

The synthesis method for compound 4 was as follows:

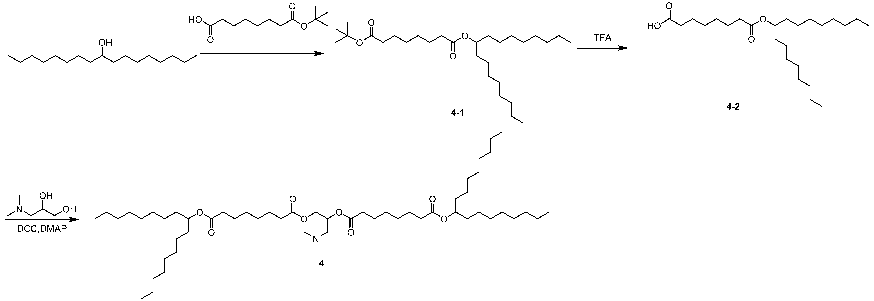

### Step 1: synthesis of compound 4-1

DMAP (500.2 mg, 4.1 mmol), DCC (1.69 g, 8.2 mmol), and DCM (20 mL) were added into a 50 mL flask, and the mixture was stirred until it became clear. 8-(*tert*-Butoxy)-8-oxooctanoic acid (1.9 g, 8.2 mmol) was added, and the mixture was stirred at room temperature for 30 min. 9-Heptadecanol (2.1 g, 8.2 mmol) was then added in one batch, and the mixture was stirred at room temperature overnight. The extent of the reaction was detected by TLC (10% EtOAc/*n-*heptane). After completion of the reaction, the reaction solution was filtered to remove solids, and the filtrate was washed with 5 mL of water and 5 mL of saturated brine, followed by liquid separation. The organic phase was dried over anhydrous Na₂SO₄, filtered, and concentrated to give a crude product (about 3.2 g). The crude product was purified and separated using a Flash column (30 g of silica gel, loaded with 200 mL of *n*-heptane; the mobile phases were respectively: 200 mL of 0.5% EtOAc + 99.5% *n*-heptane; 200 mL of 1% EtOAc + 99% *n-*heptane; and 1000 mL of 2% EtOAc + 98% *n*-heptane) to give a pure product (2.1 g, 55% yield). ¹H NMR (400 MHz, CDCl₃): *δ* 4.89-4.82 (m, 1H), 2.27 (t, *J*=8Hz, 2H), 2.19 (t, *J*=8Hz, 2H), 1.64-1.47 (m, 8H), 1.43 (s, 9H), 1.36-1.25 (m, 28H), 0.87 (t, *J*=4Hz, 6H).

### Step 2: synthesis of compound 4-2

4-1 (1.2 g, 2.56 mmol) dissolved in DCM (8 mL) was added into a 100 mL flask, and TFA (4 mL) was added. After the mixture was stirred at room temperature for 2 h, the extent of the reaction was detected by TLC (5% MeOH/DCM). After completion of the reaction, the reaction solution was concentrated, followed by addition of MTBE (20 mL) for dissolution. The pH was adjusted to 8-9 with a 10% NaHCO₃ solution, and then the pH was adjusted to 1-2 with 1 M diluted hydrochloric acid. The mixture was stirred for 20 min, followed by layer separation. The aqueous phase was extracted with 50 mL of MTBE, and the organic phases were combined, dried over anhydrous Na₂SO₄, filtered, and concentrated to give a crude product (1.5 g). The crude product was purified and separated using a Flash column (100 g of silica gel, loaded with 50 mL of *n*-heptane; the mobile phases were respectively: 300 mL of 1% EtOAc + 99% *n-*heptane; 200 mL of 1.5% EtOAc + 98.5% *n*-heptane; and 1000 mL of 2% EtOAc + 98% *n-*heptane) to give a pure product (800 mg, 80% yield). ¹H NMR (400 MHz, CDCl₃): *δ* 4.89-4.83 (m, 1H), 2.34 (t, *J*=8Hz, 2H), 2.28 (t, *J*=8Hz, 2H), 1.67-1.59 (m, 4H), 1.52-1.47 (m, 4H), 1.40-1.32 (m, 4H), 1.31-1.19 (m, 24H), 0.87 (t, J=8Hz, 6H).

### Step 3: synthesis of compound 4

DMAP (250 mg, 1.21 mmol), DCC (33.6 mg, 0.28 mmol), and DCM (5 mL) were added into a 25 mL flask, and the mixture was stirred until it became clear. 4-2 (500 mg, 1.21 mmol) was added, and the mixture was stirred at room temperature for 30 min. 3-Dimethylamino-1,2-propanediol (65.6 mg, 0.55 mmol) was then added in one batch, and the mixture was stirred at room temperature for 5 h. The extent of the reaction was detected by TLC (10% MeOH/DCM). After completion of the reaction, the reaction solution was filtered to remove solids, and the filtrate was washed with 10 mL of water and 10 mL of saturated brine, followed by liquid separation. The organic phase was dried over anhydrous Na₂SO₄, filtered, and concentrated to give a crude product (about 540 mg). The crude product was purified and separated using a Flash column (10 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 3% EtOAc + 97% *n*-heptane; 100 mL of 5% EtOAc + 95% *n*-heptane; 100 mL of 10% EtOAc + 90% *n*-heptane; and 300 mL of 20% EtOAc + 80% *n*-heptane) to give a pure product (200 mg, 40% yield). ¹H NMR (400 MHz, CDCl₃): *δ* 5.21-5.19 (m, 1H), 4.88-4.82 (m, 2H), 4.37-4.33 (m, 1H), 4.10-4.06 (m, 1H), 2.54-2.43 (m, 2H), 2.34-2.25 (m, 14H), 1.65-1.58 (m, 9H), 1.52-1.44 (m, 9H), 1.29-1.25 (m, 52H), 0.87 (t, *J*=8Hz, 12H).

The synthesis method for compound 5 was as follows:

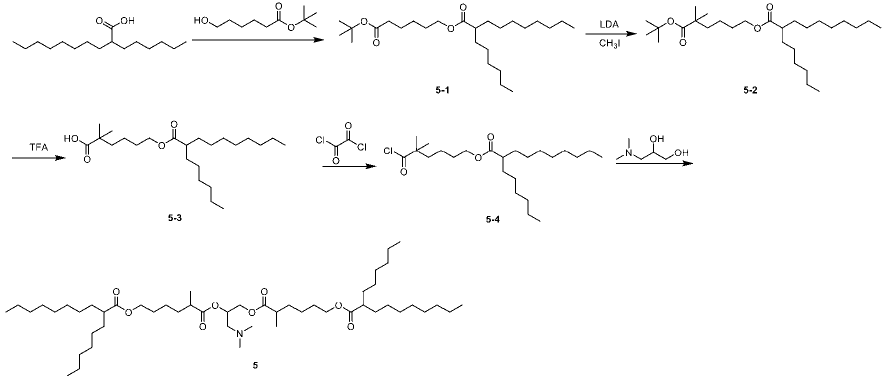

### Step 1: synthesis of compound 5-1

DMAP (1.6 g, 13.3 mmol), DCC (5.5 g, 26.6 mmol), and DCM (50 mL) were added into a 100 mL flask, and the mixture was stirred until it became clear. 2-Hexyldecanoic acid (6.8 g, 26.6 mmol) was added, and the mixture was stirred at room temperature for 30 min. *tert-*Butyl *6-*hydroxyhexanoate (5.0 g, 26.6 mmol) was then added in one batch, and the mixture was stirred at room temperature overnight. The extent of the reaction was detected by TLC (30% *n-*heptane/DCM). After completion of the reaction, the reaction solution was filtered to remove solids, and the filtrate was washed with 15 mL of water and 15 mL of saturated brine. The organic phase was dried over anhydrous Na₂SO₄, filtered, and concentrated to give a crude product (about 10.2 g). The crude product was purified and separated using a Flash column (120 g of silica gel, loaded with 200 mL of *n*-heptane; the mobile phases were respectively: 200 mL of 0.5% EtOAc + 99.5% *n*-heptane; and 1500 mL of 1% EtOAc + 99% *n*-heptane) to give a pure product (8.5 g, 75% yield).

### Step 2: synthesis of compound 5-2

1 mL of tetrahydrofuran was added into a 50 mL flask and cooled with liquid nitrogen to -60 °C. LDA (1.4 mL, 2.6 mmol) was slowly added dropwise, and after the dropwise addition, the mixture was stirred for 20 min. With the temperature controlled below -60 °C, a mixed solution of 5-1 (800 mg, 1.7 mmol) and tetrahydrofuran (6 mL) was added dropwise, and after the dropwise addition, the mixture was stirred for 2 h. With the temperature controlled below - 60 °C, a mixed solution of iodomethane (2.4 g, 17.1 mmol) and tetrahydrofuran (2 mL) was added dropwise, and after the dropwise addition, the mixture was stirred for 2 h. The extent of the reaction was detected by TLC (10% EtOAc/*n*-heptane). After completion of the reaction, 5 mL of a saturated aqueous ammonium chloride solution was added to the reaction solution to quench the reaction, followed by liquid separation. The mixture was extracted with EtOAc (20 mL × 2), and the organic phases were combined, then washed with 5 mL of saturated brine, dried over anhydrous Na₂SO₄, and concentrated to dryness to give a crude product (about 900 mg). The crude product was purified and separated using a Flash column (25 g of silica gel, loaded with 200 mL of *n*-heptane; the mobile phases were respectively: 200 mL of *n*-heptane; 200 mL of 1% EtOAc + 99% *n*-heptane; and 500 mL of 3% EtOAc + 97% *n*-heptane) to give a pure product (510 mg, 62% yield). ¹H NMR (400 MHz, CDCl₃): *δ* 4.07-4.01 (m, 2H), 2.33-2.26 (m, 1H), 1.69-1.49 (m, 6H), 1.44 (s, 9H), 1.42-1.34 (m, 5H), 1.29-1.25 (m, 20H), 1.11-1.09 (m, 6H), 0.89-0.85 (m, 6H).

### Step 3: synthesis of compound 5-3

5-2 (500 mg, 1.01 mmol) dissolved in DCM (4 mL) was added into a 25 mL flask, and TFA (2 mL) was added. After the mixture was stirred at room temperature for 2 h, the extent of the reaction was detected by TLC (5% MeOH/DCM). After completion of the reaction, the reaction solution was concentrated, followed by addition of MTBE (20 mL) for dissolution. The pH was adjusted to 8-9 with a 10% NaHCO₃ solution, and then the pH was adjusted to 1-2 with 1 M diluted hydrochloric acid. The mixture was stirred for 20 min, followed by layer separation. The aqueous phase was extracted with 50 mL of MTBE, and the organic phases were combined, dried over anhydrous Na₂SO₄, filtered, and concentrated to give a crude product (400 mg). The crude product was purified and separated using a Flash column (8 g of silica gel, loaded with 50 mL of *n*-heptane; the mobile phases were respectively: 100 mL of 1% EtOAc + 99% *n-*heptane; 100 mL of 2% EtOAc + 98% *n*-heptane; 100 mL of 5% EtOAc + 95% *n*-heptane; and 300 mL of 10% EtOAc + 90% *n*-heptane) to give a pure product (280 mg, 63% yield). ¹H NMR (400 MHz, CDCl₃): *δ* 4.13-4.03 (m, 5H), 2.50-2.42 (m, 2H), 2.04 (s, 1H), 1.77-1.55 (m, 12H), 1.51-1.33 (m, 12H), 1.30-1.24 (m, 40H), 1.19-1.18 (m, 6H), 1.09 (s, 6H), 0.87 (t, *J*=8Hz, 12H)

### Step 4: synthesis of compound 5-4

5-3 (280 mg, 0.7 mmol) dissolved in DCM (3 mL) was added into a 25 mL flask, and DMF (10 µL) was added. The reaction solution was cooled in an ice-salt bath to 0-5 °C, and oxalyl chloride (177 mg, 1.4 mmol) was slowly added dropwise to the solution. The mixture was stirred at this temperature for 2 h. The extent of the reaction was detected by TLC (10% EtOAc/*n*-heptane). After completion of the reaction, the reaction solution was concentrated to dryness to give a crude product (about 265 mg, 90% yield), which was used directly in the next step.

### Step 5: synthesis of compound 5

3-Dimethylamino-1,2-propanediol (34.4 mg, 0.29 mmol), *N*,*N-*diisopropylethylamine (112 mg, 0.87 mmol), and THF (3 mL) were added into a 25 mL flask, and the reaction solution was cooled in an ice bath to 0-5 °C. A solution of 5-4 (265 mg, 0.64 mmol) in THF (2 mL) was added dropwise to the reaction solution, and after the dropwise addition, the mixture was stirred at room temperature overnight. The extent of the reaction was detected by TLC (10% MeOH/DCM). After completion of the reaction, 2 mL of water was added to the reaction solution to quench the reaction, and the mixture was extracted with EtOAc (10 mL × 2). The organic phases were combined, washed with 5 mL of saturated brine, dried over anhydrous Na₂SO₄, filtered, and concentrated to give a crude product (about 130 mg). The crude product was purified and separated using a Flash column (8 g of silica gel, loaded with 50 mL of *n-*heptane; the mobile phases were respectively: 100 mL of *n*-heptane; 100 mL of 1% EtOAc + 99% *n*-heptane; 100 mL of 2% EtOAc + 98% *n*-heptane; 100 mL of 3% EtOAc + 97% *n-*heptane; 100 mL of 5% EtOAc + 95% *n*-heptane; 100 mL of 10% EtOAc + 90% *n*-heptane; and 100 mL of 20% EtOAc + 80% *n*-heptane) to give a pure product (5, 60 mg, 24% yield). ¹H NMR (400 MHz, CDCl₃): *δ* 5.21 (s, 1H), 4.43-4.34 (m, 1H), 4.14-3.98 (m, 5H), 2.49-2.40 (m, 4H), 2.29 (s, 6H), 1.63-1.55 (m, 12H), 1.44-1.33 (m, 12H), 1.31-1.25 (m, 40H), 1.15-1.13 (m, 6H), 0.87 (t, *J*=8Hz, 12H).

The synthesis method for compound 6 was as follows:

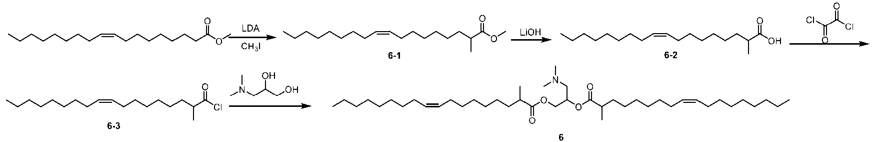

### Step 1: synthesis of compound 6-1

10 mL of tetrahydrofuran was added into a 500 mL flask and cooled with liquid nitrogen to - 60 °C. LDA (34 mL, 68 mmol) was slowly added dropwise, and after the dropwise addition, the mixture was stirred for 20 min. With the temperature controlled below -60 °C, a mixed solution of methyl oleate (10 g, 33.8 mmol) and tetrahydrofuran (90 mL) was added dropwise, and after the dropwise addition, the mixture was stirred for 2 h. With the temperature controlled below -60 °C, a mixed solution of iodomethane (48.0 g, 338 mmol) and tetrahydrofuran (10 mL) was added dropwise, and after the dropwise addition, the mixture was stirred for 2 h. The extent of the reaction was detected by TLC (*n*-heptane). After completion of the reaction, 50 mL of a saturated aqueous ammonium chloride solution was added to the reaction solution to quench the reaction, followed by liquid separation. The mixture was extracted twice with EtOAc (200 mL), and the organic phases were combined, then washed with 30 mL of saturated brine, dried over anhydrous Na₂SO₄, and concentrated to dryness to give a crude product (about 12 g). The crude product was purified and separated using a Flash column (100 g of silica gel, loaded with 200 mL of *n*-heptane; the mobile phase was: 2000 mL of 0.1% EtOAc + 99.9% *n-*heptane) to give a pure product (compound 2-1, 8.1 g, 76.1% yield). ¹H NMR (400 MHz, CDCl₃): *δ* 5.36-5.33 (m, 2H), 3.65 (d, *J*=4Hz, 3H), 2.46-2.40 (m, 1H), 2.04-1.98 (m, 4H), 1.68-1.17 (m, 22H), 1.15 (d, *J=*4Hz*,* 3H), 0.88(t, *J*=12Hz, 3H).

### Step 2: synthesis of compound 6-2

Lithium hydroxide monohydrate (10.8 g, 257.6 mmol) and water (80 mL) were added into a 500 mL flask, and a mixed solution of 6-1 (8 g, 25.7 mmol) and THF (240 mL) was added. The mixture was heated to 60 °C and stirred at this temperature for 6 h, and then stirred at room temperature overnight. The extent of the reaction was detected by TLC (30% EtOAc/*n-*heptane). After completion of the reaction, THF in the reaction solution was removed by concentration, and the pH was adjusted to 1-2 with diluted hydrochloric acid. The solution was then extracted twice with EtOAc (200 mL), and the organic phases were combined, washed with 30 mL of saturated brine, dried over anhydrous Na₂SO₄, filtered, and concentrated to give a crude product (about 7.2 g). The crude product was purified and separated using a Flash column (80 g of silica gel, loaded with 300 mL of n-heptane; the mobile phases were respectively: 500 mL of 1% EtOAc + 99% n-heptane; 300 mL of 1.5% EtOAc + 98.5% *n-*heptane; and 1000 mL of 2% EtOAc + 98% n-heptane) to give a pure product (compound 2-2, 4.7 g, 62% yield). ¹H NMR (400 MHz, CDCl₃): *δ* 5.39-5.30 (m, 2H), 4.15-4.09 (m, 1H), 2.48-2.43 (m, 1H), 2.04-1.98 (m, 4H), 1.69-1.22 (m, 22H), 1.18-1.17 (m, 3H), 0.88(t, J=12Hz, 3H).

### Step 3: synthesis of compound 6-3

The reaction product 6-2 (500 mg, 1.69 mmol) dissolved in DCM (3 mL) was added into a 50 mL flask, and DMF (0.7 mg, 0.01 mmol) was added. The mixture was cooled in an ice bath to 0 °C, and oxalyl chloride (256.8 mg, 2.02 mmol) was added dropwise with the temperature maintained at 0-10 °C. The extent of the reaction was detected by TLC (30% EtOAc/*n*-heptane). After completion of the reaction, the reaction solution was concentrated to dryness to give a crude product (2-3, about 530 mg), which was used directly in the next step.

### Step 4: synthesis of compound 6

3-Dimethylamino-1,2-propanediol (91.2 mg, 0.76 mmol), DIPEA (296.6 mg, 2.3 mmol), and THF (2 mL) were added into a 50 mL flask, and the reaction solution was cooled in an ice-salt bath to -5 °C. A solution of 6-3 (530 mg, 1.69 mmol) in THF (3 mL) was added dropwise. After the dropwise addition, the mixture was stirred at room temperature for 2 h, and then the extent of the reaction was detected by TLC (30% EtOAc/n-heptane). After completion of the reaction, methanol (1 mL) was added to quench the reaction, and then the mixture was diluted with 10 mL of water, followed by liquid separation. The aqueous phase was extracted twice with EtOAc (50 mL), and the organic phases were combined, then washed with 5 mL of saturated brine, dried over anhydrous Na₂SO₄, and concentrated to dryness to give a crude product (about 0.7 g). The crude product was purified and separated using a Flash column (10 g of silica gel, loaded with 100 mL of *n*-heptane; the mobile phase was: 500 mL of 0.1% EtOAc + 99.9% *n*-heptane) to give a pure product (compound 6, 300 mg, 26% two-step yield). ¹H NMR (400 MHz, CDCl₃): *δ* 5.35-5.32 (m, 4H), 5.20-5.18 (m, 1H), 4.39-4.36 (m, 1H), 4.11-4.08 (m, 1H), 2.46-2.39 (m, 4H), 2.26 (s, 6H), 2.03-1.98 (m, 8H), 1.67-1.62 (m, 2H), 1.43-1.25 (m, 30H), 1.13 (d, J=8Hz, 6H), 0.88(t, *J=*12Hz*,* 6H).

The synthesis method for compound 7 was as follows:

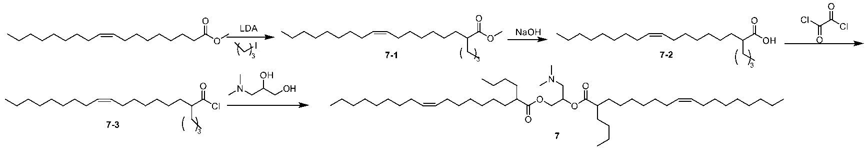

### Step 1: synthesis of compound 7-1

5 mL of tetrahydrofuran was added into a 50 mL flask and cooled with liquid nitrogen to -60 °C. LDA (5.1 mL, 10.1 mmol) was slowly added dropwise, and after the dropwise addition, the mixture was stirred for 20 min. With the temperature controlled below -60 °C, a mixed solution of methyl oleate (1.5 g, 5.06 mmol) and tetrahydrofuran (10 mL) was added dropwise, and after the dropwise addition, the mixture was stirred for 2 h. With the temperature controlled below - 60 °C, a mixed solution of iodobutane (9.3 g, 50.6 mmol) and tetrahydrofuran (5 mL) was added dropwise, and after the dropwise addition, the mixture was stirred for 2 h. The extent of the reaction was detected by TLC (5% EtOAc/n-heptane). After completion of the reaction, 5 mL of a saturated aqueous ammonium chloride solution was added to the reaction solution to quench the reaction, followed by liquid separation. The mixture was extracted with EtOAc (20 mL × 2), and the organic phases were combined, then washed with 5 mL of saturated brine, dried over anhydrous Na₂SO₄, and concentrated to dryness to give a crude product (about 1.2 g). The crude product was purified and separated using a Flash column (25 g of silica gel, loaded with 200 mL of *n*-heptane; the mobile phase was: 500 mL of *n*-heptane) to give a pure product (compound 7-1, 850 mg, 47.6% yield). ¹H NMR (400 MHz, CDCl₃): *δ* 5.38-5.32 (m, 2H), 3.66-3.64 (m, 3H), 2.36-2.27 (m, 1H), 2.03-1.98 (m, 4H), 1.65-1.08 (m, 38H), 0.91-0.86 (m, 9H).

### Step 2: synthesis of compound 7-2

3 mL of methanol, 1.5 mL of THF, and 1 mL of sodium hydroxide (8 M/L) were added into a 50 mL flask, and 7-1 (600 mg, 1.7 mmol) was added. The mixture was heated to 70 °C and stirred at this temperature for 16 h. The extent of the reaction was detected by TLC (20% EtOAc/*n*-heptane). After completion of the reaction, the solvent in the reaction solution was removed by concentration. The solution was diluted with water, and the pH was adjusted to 1-2 with diluted hydrochloric acid. The solution was then extracted with DCM (20 mL × 2), and the organic phases were combined, washed with 3 mL of saturated brine, dried over anhydrous Na₂SO₄, filtered, and concentrated to give a crude product (about 500 mg). The crude product was purified and separated using a Flash column (20 g of silica gel, loaded with 300 mL of *n-*heptane; the mobile phases were respectively: 100 mL of 1% EtOAc + 99% *n*-heptane; 100 mL of 1.5% EtOAc + 98.5% *n*-heptane; 100 mL of 2% EtOAc + 98% *n*-heptane; and 300 mL of 5% EtOAc + 95% *n*-heptane) to give a pure product (340 mg, 59% yield). ¹H NMR (400 MHz, CDCl₃): *δ* 5.37-5.33 (m, 2H), 2.37-2.30 (m, 1H), 2.03-1.99 (m, 4H), 1.65-1.58 (m, 2H), 1.52-1.42 (m, 3H), 1.37-1.22 (m, 26H), 0.91-0.86 (m, 6H).

### Step 3: synthesis of compound 7-3

7-2 (340 mg, 1.0 mmol) dissolved in DCM (5 mL) was added into a 25 mL flask, and DMF (10 µL) was added. The reaction solution was cooled in an ice-salt bath to 0-5 °C, and oxalyl chloride (152.9 mg, 1.2 mmol) was slowly added dropwise to the solution. The mixture was stirred at this temperature for 3 h. The extent of the reaction was detected by TLC (20% EtOAc/*n*-heptane). After completion of the reaction, the reaction solution was concentrated to dryness to give a crude product (about 357 mg, 100% yield), which was used directly in the next step.

### Step 4: synthesis of compound 7

3-Dimethylamino-1,2-propanediol (54 mg, 0.45 mmol), *N*,*N-*diisopropylethylamine (175.7 mg, 1.36 mmol), and THF (2 mL) were added into a 25 mL flask, and the reaction solution was cooled in an ice bath to 0-5 °C. A solution of 7-3 (357 mg, 1.0 mmol) in THF (2 mL) was added dropwise to the reaction solution, and after the dropwise addition, the mixture was stirred at room temperature overnight. The extent of the reaction was detected by TLC (0.5% MeOH/DCM). After completion of the reaction, 2 mL of water was added to the reaction solution to quench the reaction, and the mixture was extracted with EtOAc (10 mL × 2). The organic phases were combined, washed with 5 mL of saturated brine, dried over anhydrous Na₂SO₄, filtered, and concentrated to give a crude product (about 180 mg). The crude product was purified and separated using a Flash column (8 g of silica gel, loaded with 50 mL of *n-*heptane; the mobile phases were respectively: 100 mL of *n*-heptane; 50 mL of 1% EtOAc + 99% *n*-heptane; and 200 mL of 2% EtOAc + 98% *n*-heptane) to give a pure product (7, 100 mg, 29% yield). ¹H NMR (400 MHz, CDCl₃): *δ* 5.35-5.32 (m, 4H), 5.21-5.19 (m, 1H), 4.42-4.37 (m, 1H), 4.10-4.05 (m, 1H), 2.46 (s, 2H), 2.34-2.28 (m, 7H), 2.03-1.98 (m, 8H), 1.62-1.54 (m, 4H), 1.47-1.41 (m, 6H), 1.37-1.22 (m, 58H), 0.90-0.86 (t, *J*=8 Hz, 12H).

### Synthesis of compound 8

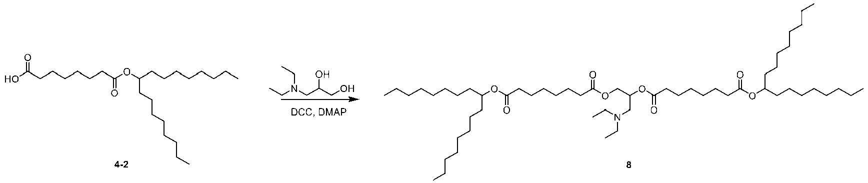

### Step 1: synthesis of compound 8

DMAP (250 mg, 1.21 mmol), DCC (33.6 mg, 0.28 mmol), and DCM (5 mL) were added into a 25 mL flask, and the mixture was stirred until it became clear. 4-2 (500 mg, 1.21 mmol) was added, and the mixture was stirred at room temperature for 30 min. 3-Diethylamino-1,2-propanediol (80.1 mg, 0.55 mmol) was then added in one batch, and the mixture was stirred at room temperature for 5 h. The extent of the reaction was detected by TLC (10% MeOH/DCM). After completion of the reaction, the reaction solution was filtered to remove solids, and the filtrate was washed with 10 mL of water and 10 mL of saturated brine, followed by liquid separation. The organic phase was dried over anhydrous Na₂SO₄, filtered, and concentrated to give a crude product (about 600 mg). The crude product was purified and separated using a Flash column (10 g of silica gel, loaded with 100 mL of n-heptane; the mobile phases were respectively: 100 mL of 1% EtOAc + 99% *n*-heptane; 100 mL of 2% EtOAc + 98% *n*-heptane; 100 mL of 3% EtOAc + 97% *n*-heptane; 100 mL of 5% EtOAc + 95% *n*-heptane; 100 mL of 10% EtOAc + 90% *n*-heptane; and 300 mL of 20% EtOAc + 80% *n*-heptane) to give a pure product (230 mg, 45% yield). ¹H NMR (400 MHz, CDCl₃): *δ* 5.12 (m, 1H), 4.89-4.83 (m, 2H), 4.37-4.33 (m, 1H), 4.14-4.10 (m, 1H), 2.53 (m, 2H), 2.32-2.25 (m, 8H), 1.65-1.58 (m, 11H), 1.52-1.48 (m, 9H), 1.39-1.25 (m, 69H), 1.00(m,5H), 0.87 (t, *J*=8Hz, 14H).

### Synthesis of compound 17

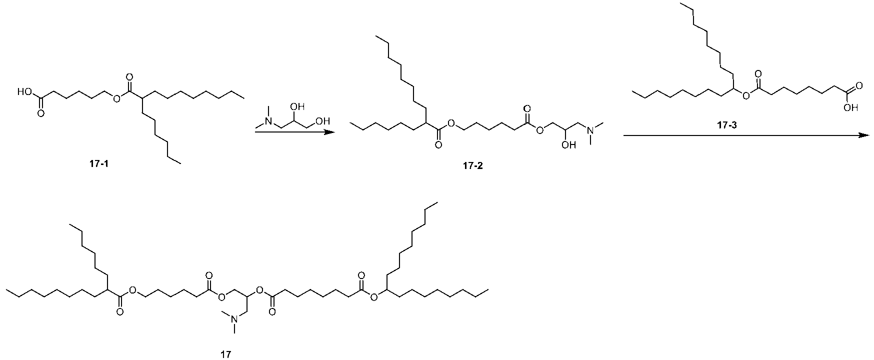

### Step 1: synthesis of compound 17-2

DMAP (74 mg, 0.61 mmol), DCC (250 mg, 1.21 mmol), and DCM (5 mL) were added into a 25 mL flask, and the mixture was stirred until it became clear. 17-1 (500 mg, 1.21 mmol) was added, and the mixture was stirred at room temperature for 30 min. 3-Dimethylamino-1,2-propanediol (144.4 mg, 1.21 mmol) was then added in one batch, and the mixture was stirred at room temperature for 16 h. The extent of the reaction was detected by TLC (5% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 580 mg). The crude product was purified and separated using a Flash column (50 g of silica gel, loaded with 100 mL of DCM; the mobile phases were respectively: 100 mL of 1% MeOH + 99% DCM; 100 mL of 2% MeOH + 98% DCM; 100 mL of 4% MeOH + 96% DCM; and 300 mL of 10% MeOH + 90% DCM) to give a pure product (300 mg, 48% yield). ¹H NMR (400 MHz, CDCl₃): *δ* 4.88-4.82 (m, 1H), 4.17-4.14 (m, 1H), 4.03-3.98 (m, 1H), 3.95-3.90 (m, 1H), 2.48-2.42 (m, 1H), 2.37-2.25 (m, 12H), 1.67-1.58 (m, 4H), 1.52-1.47 (m, 4H), 1.37-1.25 (m, 33 H), 0.87(t, *J*=8Hz, 6H).

### Step 2: synthesis of compound 17

DMAP (35.7 mg, 0.29 mmol), DCC (120.5 mg, 0.58 mmol), and DCM (5 mL) were added into a 25 mL flask, and the mixture was stirred until it became clear. 17-3 (216.4 mg, 0.58 mmol) was added, and the mixture was stirred at room temperature for 30 min. 17-2 (300 mg, 0.58 mmol) was then added in one batch, and the mixture was stirred at room temperature overnight. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 530 mg). The crude product was purified and separated using a Flash column (100 g of silica gel, loaded with 100 mL of *n-*heptane; the mobile phases were respectively: 100 mL of 1% EtOAc + 99% *n*-heptane; 100 mL of 2% EtOAc + 98% *n*-heptane; 100 mL of 5% EtOAc + 95% n-heptane; 100 mL of 10% EtOAc + 90% *n*-heptane; and 500 mL of 30% EtOAc + 70% *n*-heptane) to give a pure product (280 mg, 55% yield). ¹H NMR (400 MHz, CDCl₃): *δ* 5.21-5.19 (m, 1H), 4.87-4.84 (m, 1H), 4.38-4.34 (m, 1H), 4.10-4.04 (m, 3H), 2.46 (s, 2H), 2.35-2.25 (m, 13H), 1.69-1.25 (m, 73H), 0.87(t, *J*=8Hz, 12H).

### Synthesis of compound 18

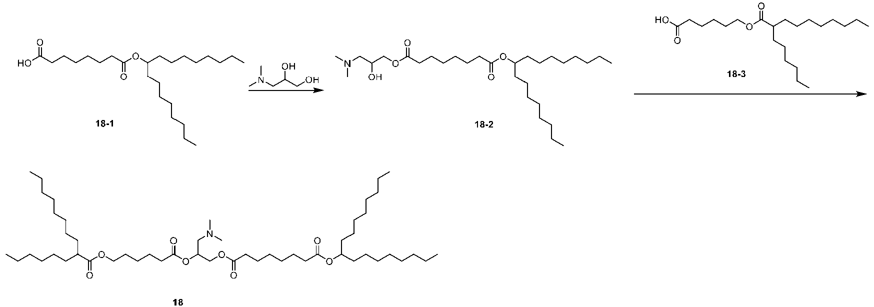

### Step 1: synthesis of compound 18-2

DMAP (82.4 mg, 0.67 mmol), DCC (278.4 mg, 1.35 mmol), and DCM (5 mL) were added into a 25 mL flask, and the mixture was stirred until it became clear. 18-1 (500 mg, 1.35 mmol) was added, and the mixture was stirred at room temperature for 30 min. 3-Dimethylamino-1,2-propanediol (160.8 mg, 1.35 mmol) was then added in one batch, and the mixture was stirred at room temperature for 16 h. The extent of the reaction was detected by TLC (5% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 540 mg). The crude product was purified and separated using a Flash column (50 g of silica gel, loaded with 100 mL of DCM; the mobile phases were respectively: 100 mL of 1% MeOH + 99% DCM; 100 mL of 2% MeOH + 98% DCM; 100 mL of 4% MeOH + 96% DCM; and 300 mL of 10% MeOH + 90% DCM) to give a pure product (250 mg, 39% yield). ¹H NMR (400 MHz, CDCl₃): *δ* 4.89-4.83 (m, 1H), 2.37-2.26 (m, 4H), 1.68-1.59 (m, 4H), 1.52-1.44 (m, 4H), 1.42-1.33 (m, 4H), 1.31-1.18 (m, 25 H), 0.87(t, *J*=8Hz, 6H).

### Step 2: synthesis of compound 18

DMAP (34.9 mg, 0.29 mmol), DCC (118.1 mg, 0.57 mmol), and DCM (5 mL) were added into a 25 mL flask, and the mixture was stirred until it became clear. 18-3 (236.2 mg, 0.57 mmol) was added, and the mixture was stirred at room temperature for 30 min. 18-2 (270 mg, 0.57 mmol) was then added in one batch, and the mixture was stirred at room temperature overnight. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered.

The organic phase was concentrated to give a crude product (about 550 mg). The crude product was purified and separated using a Flash column (100 g of silica gel, loaded with 100 mL of *n-*heptane; the mobile phases were respectively: 100 mL of 1% EtOAc + 99% *n*-heptane; 100 mL of 2% EtOAc + 98% *n*-heptane; 100 mL of 5% EtOAc + 95% *n*-heptane; 100 mL of 10% EtOAc + 90% *n*-heptane; and 500 mL of 30% EtOAc + 70% *n*-heptane) to give a pure product (200 mg, 40% yield). ¹H NMR (400 MHz, CDCl₃): *δ* 5.22-5.18 (m, 1H), 4.88-4.82 (m, 1H), 4.38-4.34 (m, 1H), 4.10-4.04 (m, 3H), 2.53-2.42 (m, 2H), 2.35-2.25 (m, 13H), 1.69-1.55 (m, 10H), 1.50-1.44 (m, 4H), 1.42-1.36 (m, 4H), 1.31-1.25 (m, 50 H), 0.87(t, *J*=8Hz, 12H).

### Synthesis of compound 19

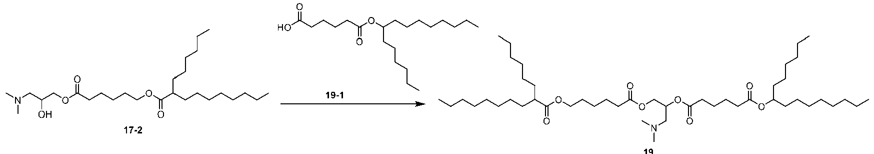

### Step 1: synthesis of compound 19

DMAP (51.4 mg, 0.42 mmol), DCC (173.6 mg, 0.84 mmol), and DCM (8 mL) were added into a 25 mL flask, and the mixture was stirred until it became clear. 19-1 (300 mg, 0.84 mmol) was added, and the mixture was stirred at room temperature for 30 min. 17-2 (432.3 mg, 0.84 mmol) was then added in one batch, and the mixture was stirred at room temperature overnight. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 530 mg). The crude product was purified and separated using a Flash column (100 g of silica gel, loaded with 100 mL of *n-*heptane; the mobile phases were respectively: 100 mL of 1% EtOAc + 99% n-heptane; 100 mL of 2% EtOAc + 98% n-heptane; 100 mL of 5% EtOAc + 95% n-heptane; 100 mL of 10% EtOAc + 90% *n*-heptane; and 500 mL of 30% EtOAc + 70% n-heptane) to give a pure product (400 mg, 56% yield). ¹H NMR (400 MHz, CDCl₃): *δ* 5.21-5.17 (m, 1H), 4.88-4.82 (m, 1H), 4.38-4.34 (m, 1H), 4.10-4.05 (m, 1H), 2.54-2.41 (m, 2H), 2.36-2.23 (m, 14H), 1.69-1.19 (m, 71H), 0.87(t, *J*=8Hz, 12H).

### Synthesis of compound 20

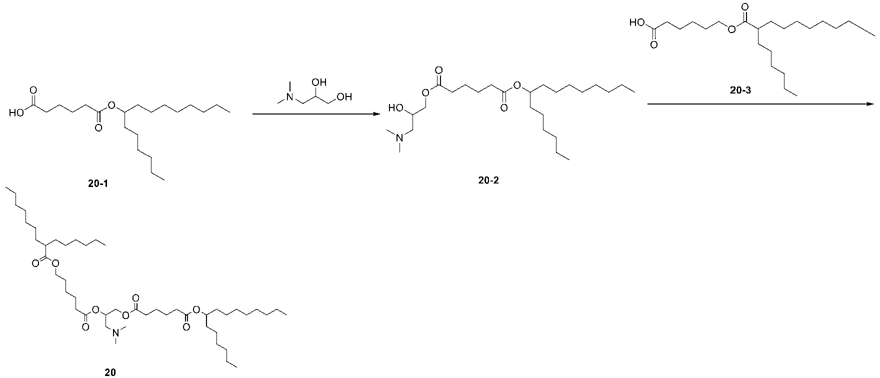

### Step 1: synthesis of compound 20-2

DMAP (137.1 mg, 1.12 mmol), DCC (463 mg, 2.24 mmol), and DCM (8 mL) were added into a 25 mL flask, and the mixture was stirred until it became clear. 20-1 (800 mg, 2.24 mmol) was added, and the mixture was stirred at room temperature for 30 min. 3-Dimethylamino-1,2-propanediol (267.4 mg, 2.24 mmol) was then added in one batch, and the mixture was stirred at room temperature overnight. The extent of the reaction was detected by TLC (30% EtOAc/n-heptane). After completion of the reaction, the reaction solution was filtered to remove solids, and the filtrate was washed with 3 mL of water and 3 mL of saturated brine, dried over anhydrous Na₂SO₄, and filtered. The organic phase was concentrated to give a crude product (about 950 mg). The crude product was purified and separated using a Flash column (100 g of silica gel, loaded with 100 mL of *n*-heptane; the mobile phases were respectively: 100 mL of 99% DCM + 1% MeOH; 100 mL of 98% DCM + 2% MeOH; 100 mL of 96% DCM + 4% MeOH; and 500 mL 92% DCM + 8% MeOH) to give a pure product (400 mg, 40% yield). ¹H NMR (400 MHz, CDCl₃): *δ* 4.88-4.82 (m, 1H), 4.18-3.75 (m, 3H), 2.42-2.22 (m, 12H), 1.68-1.63 (m, 4H), 1.51-1.47 (m, 4H), 1.31-1.24 (m, 20H), 0.86(t, *J*=8Hz, 6H).

### Step 2: synthesis of compound 20

DMAP (33.4 mg, 0.27 mmol), DCC (112.7 mg, 0.55 mmol), and DCM (5 mL) were added into a 25 mL flask, and the mixture was stirred until it became clear. 20-3 (202.4 mg, 0.55 mmol) was added, and the mixture was stirred at room temperature for 30 min. 20-2 (250 mg, 0.55 mmol) was then added in one batch, and the mixture was stirred at room temperature overnight. The extent of the reaction was detected by TLC (10% MeOH/DCM). After completion of the reaction, the reaction solution was filtered to remove solids, and the filtrate was washed with 3 mL of water and 3 mL of saturated brine, dried over anhydrous Na₂SO₄, and filtered. The organic phase was concentrated to give a crude product (about 550 mg). The crude product was purified and separated using a Flash column (100 g of silica gel, loaded with 100 mL of *n-*heptane; the mobile phases were respectively: 100 mL of 1% EtOAc + 99% *n*-heptane; 100 mL of 2% EtOAc + 98% *n*-heptane; 100 mL of 5% EtOAc + 95% *n*-heptane; 100 mL of 10% EtOAc + 90% *n*-heptane; 100 mL of 30% EtOAc + 70% *n*-heptane; and 300 mL of 50% EtOAc + 50% *n*-heptane) to give a pure product (230 mg, 53% yield). ¹H NMR (400 MHz, CDCl₃): *δ 5.21-*5.16 (m, 1H), 4.88-4.82 (m, 1H), 4.38-4.34 (m, 1H), 4.10-4.04 (m, 3H), 2.50-2.39 (m, 2H), 2.36-2.26 (m, 13H), 1.69-1.24 (m, 62H), 0.87(t, *J*=8Hz, 12H).

### Synthesis of compound 21

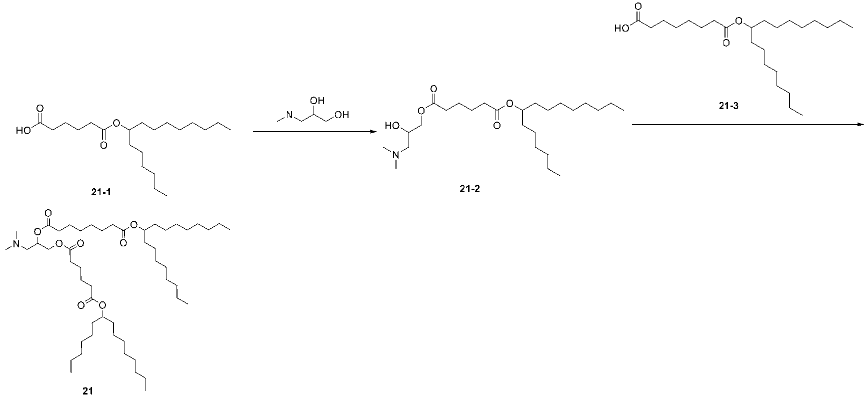

### Step 1: synthesis of compound 21-2

DMAP (137.1 mg, 1.12 mmol), DCC (463 mg, 2.24 mmol), and DCM (8 mL) were added into a 25 mL flask, and the mixture was stirred until it became clear. 21-1 (800 mg, 2.24 mmol) was added, and the mixture was stirred at room temperature for 30 min. 3-Dimethylamino-1,2-propanediol (267.4 mg, 2.24 mmol) was then added in one batch, and the mixture was stirred at room temperature overnight. The extent of the reaction was detected by TLC (30% EtOAc/n-heptane). After completion of the reaction, the reaction solution was filtered to remove solids, and the filtrate was washed with 3 mL of water and 3 mL of saturated brine, dried over anhydrous Na₂SO₄, and filtered. The organic phase was concentrated to give a crude product (about 950 mg). The crude product was purified and separated using a Flash column (100 g of silica gel, loaded with 100 mL of *n*-heptane; the mobile phases were respectively: 100 mL of 99% DCM + 1% MeOH; 100 mL of 98% DCM + 2% MeOH; 100 mL of 96% DCM + 4% MeOH; and 500 mL 92% DCM + 8% MeOH) to give a pure product (400 mg, 40% yield). ¹H NMR (400 MHz, CDCl₃): *δ* 4.88-4.82 (m, 1H), 4.18-3.75 (m, 3H), 2.42-2.22 (m, 12H), 1.68-1.63 (m, 4H), 1.51-1.47 (m, 4H), 1.31-1.24 (m, 20H), 0.86(t, J=8Hz, 6H).

### Step 2: synthesis of compound 21

DMAP (33.4 mg, 0.27 mmol), DCC (112.7 mg, 0.55 mmol), and DCM (5 mL) were added into a 25 mL flask, and the mixture was stirred until it became clear. 21-3 (225.9 mg, 0.55 mmol) was added, and the mixture was stirred at room temperature for 30 min. 21-2 (250 mg, 0.55 mmol) was then added in one batch, and the mixture was stirred at room temperature overnight. The extent of the reaction was detected by TLC (10% MeOH/DCM). After completion of the reaction, the reaction solution was filtered to remove solids, and the filtrate was washed with 3 mL of water and 3 mL of saturated brine, dried over anhydrous Na₂SO₄, and filtered. The organic phase was concentrated to give a crude product (about 550 mg). The crude product was purified and separated using a Flash column (100 g of silica gel, loaded with 100 mL of *n-*heptane; the mobile phases were respectively: 100 mL of 1% EtOAc + 99% *n*-heptane; 100 mL of 2% EtOAc + 98% *n*-heptane; 100 mL of 5% EtOAc + 95% *n*-heptane; 100 mL of 10% EtOAc + 90% *n*-heptane; 100 mL of 30% EtOAc + 70% *n*-heptane; and 400 mL of 50% EtOAc + 50% *n*-heptane) to give a pure product (340 mg, 73% yield). ¹H NMR (400 MHz, CDCl₃): *δ 5.21-*5.16 (m, 1H), 4.88-4.82 (m, 1H), 4.38-4.33 (m, 1H), 4.10-4.05 (m, 1H), 2.50-2.40 (m, 2H), 2.36-2.21 (m, 14H), 1.68-1.58 (m, 8H), 1.52-1.47 (m, 8H), 1.38-1.25 (m, 52H), 0.87(t, *J*=8Hz, 12H).

### Synthesis of compound 22

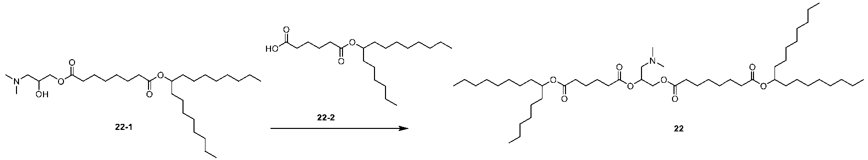

### Step 1: synthesis of compound 22

DMAP (51.4 mg, 0.42 mmol), DCC (173.6 mg, 0.84 mmol), and DCM (8 mL) were added into a 25 mL flask, and the mixture was stirred until it became clear. 22-2 (300 mg, 0.84 mmol) was added, and the mixture was stirred at room temperature for 30 min. 22-1 (396.9 mg, 0.84 mmol) was then added in one batch, and the mixture was stirred at room temperature overnight. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 530 mg). The crude product was purified and separated using a Flash column (100 g of silica gel, loaded with 100 mL of *n-*heptane; the mobile phases were respectively: 100 mL of 1% EtOAc + 99% *n*-heptane; 100 mL of 2% EtOAc + 98% *n*-heptane; 100 mL of 5% EtOAc + 95% *n*-heptane; 100 mL of 10% EtOAc + 90% *n*-heptane; and 500 mL of 30% EtOAc + 70% *n*-heptane) to give a pure product (430 mg, 63% yield). ¹H NMR (400 MHz, CDCl₃): *δ* 5.21 (s, 1H), 4.89-4.83 (m, 1H), 4.39-4.35 (m, 1H), 4.10-4.04 (m, 3H), 2.46 (s, 2H), 2.36-2.27 (m, 12H), 1.70-1.25 (m, 65H), 0.87(t, *J*=8Hz, 12H).

### Synthesis of compound 23

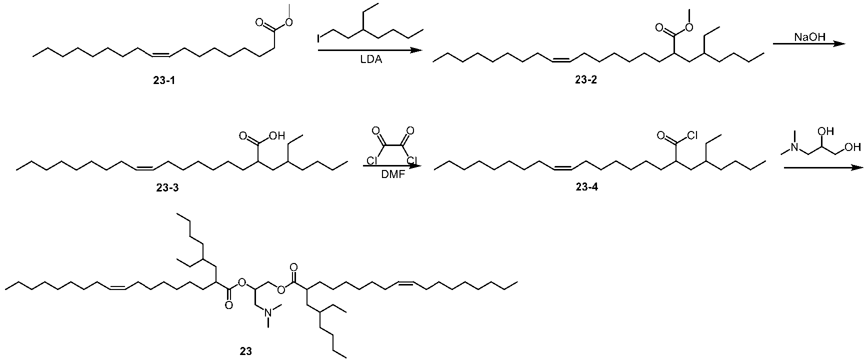

### Step 1: synthesis of compound 23-2

5 mL of tetrahydrofuran was added into a 50 mL flask and cooled with liquid nitrogen to -60 °C. LDA (16.86 mL, 33.7 mmol) was slowly added dropwise, and after the dropwise addition, the mixture was stirred for 20 min. With the temperature controlled below -60 °C, a mixed solution of methyl oleate (23-1, 5.0 g, 16.86 mmol) and tetrahydrofuran (20 mL) was added dropwise, and after the dropwise addition, the mixture was stirred for 2 h. With the temperature controlled below -60 °C, a mixed solution of 2-ethyl iodoethane (8.1 g, 33.7 mmol) and tetrahydrofuran (5 mL) was added dropwise, and after the dropwise addition, the mixture was slowly warmed to room temperature and stirred for 16 h. The extent of the reaction was detected by TLC (*n-*heptane). After completion of the reaction, 5 mL of a saturated aqueous ammonium chloride solution was added to the reaction solution to quench the reaction, followed by liquid separation. The mixture was extracted with EtOAc (20 mL × 2), and the organic phases were combined, then washed with 5 mL of saturated brine, dried over anhydrous Na₂SO₄, and concentrated to dryness to give a crude product (about 5.0 g). The crude product was purified and separated using a Flash column (50 g of silica gel, loaded with 200 mL of *n*-heptane; the mobile phases were respectively: 300 mL of *n*-heptane; 300 mL of 0.1% EtOAc + 99.9% *n-*heptane; and 500 mL of 0.2% EtOAc + 99.8% *n*-heptane) to give a pure product (3.0 g, 43.5% yield).

### Step 2: synthesis of compound 23-3

Methanol (15 mL), THF (12 mL), and 4.6 mL of sodium hydroxide (8 mM) were added into a 100 mL flask, and 23-2 (3.0 g, 7.34 mmol) was added. The mixture was heated to 65 °C and stirred for 16 h. The extent of the reaction was detected by TLC (20% EtOAc/*n*-heptane). After completion of the reaction, the solvent in the reaction solution was removed by concentration. The solution was diluted with water, and the pH was adjusted to 1-2 with diluted hydrochloric acid. The solution was then extracted with DCM (20 mL × 2), and the organic phases were combined, washed with 3 mL of saturated brine, dried over anhydrous Na₂SO₄, filtered, and concentrated to give a crude product (about 1.5 g). The crude product was purified and separated using a Flash column (60 g of silica gel, loaded with 300 mL of *n*-heptane; the mobile phases were respectively: 100 mL of 1% EtOAc + 99% *n*-heptane; 100 mL of 1.5% EtOAc + 98.5% *n*-heptane; 100 mL of 2% EtOAc + 98% *n*-heptane; 300 mL of 5% EtOAc + 95% *n*-heptane; and 300 mL of 10% EtOAc + 90% *n*-heptane) to give a pure product (650 mg, 22% yield).

### Step 3: synthesis of compound 23-4

23-3 (100 mg, 0.25 mmol) dissolved in DCM (1 mL) was added into a 25 mL flask, and DMF (10 µL) was added. The reaction solution was cooled in an ice-salt bath to 0-5 °C, and oxalyl chloride (51.5 mg, 0.41 mmol) was slowly added dropwise to the solution. The mixture was stirred at this temperature for 3 h. The extent of the reaction was detected by TLC (20% EtOAc/*n*-heptane). After completion of the reaction, the reaction solution was concentrated to dryness to give a crude product (about 104 mg, 100% yield), which was used directly in the next step.

### Step 4: synthesis of compound 23

3-Dimethylamino-1,2-propanediol (13.6 mg, 0.11 mmol), *N*,*N*-diisopropylethylamine (44.4 mg, 0.34 mmol), and THF (1 mL) were added into a 25 mL flask, and the reaction solution was cooled in an ice bath to 0-5 °C. A solution of 23-4 (104 mg, 0.25 mmol) in THF (2 mL) was added dropwise to the reaction solution, and after the dropwise addition, the mixture was stirred at room temperature overnight. The extent of the reaction was detected by TLC (0.5% MeOH/DCM). After completion of the reaction, 2 mL of water was added to the reaction solution to quench the reaction, and the mixture was extracted with EtOAc (10 mL × 2). The organic phases were combined, washed with 5 mL of saturated brine, dried over anhydrous Na₂SO₄, filtered, and concentrated to give a crude product (about 180 mg). The crude product was purified and separated using a Flash column (8 g of silica gel, loaded with 50 mL of *n-*heptane; the mobile phases were respectively: 100 mL of *n*-heptane; 100 mL of 1% EtOAc + 99% *n*-heptane; 100 mL of 2% EtOAc + 98% *n*-heptane; 100 mL of 3% EtOAc + 97% *n-*heptane; 100 mL of 5% EtOAc + 95% *n*-heptane; and 200 mL of 10% EtOAc + 90% *n*-heptane) to give a pure product (C021, 40 mg, 40% yield). ¹H NMR (400 MHz, CDCl₃): *δ* 5.36-5.33 (m, 4H), 2.46-2.39 (m, 2H), 2.03-1.98 (m, 8H), 1.64-1.58 (m, 4H), 1.49-1.19 (m, 66H), 0.91-0.81 (m, 18H).

### Synthesis of compound 24

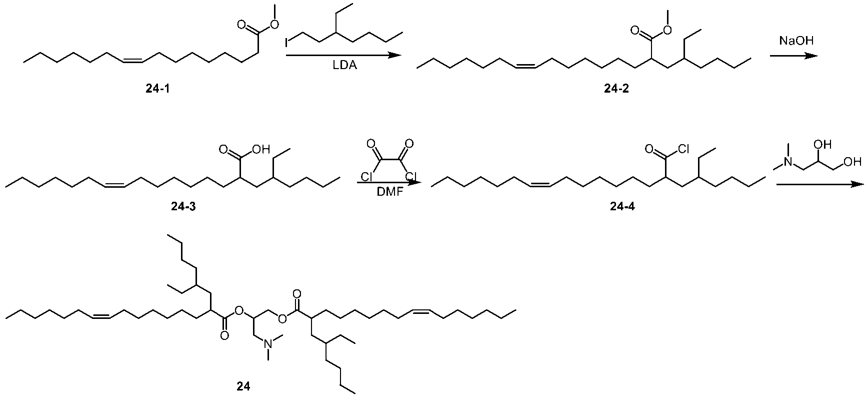

### Step 1: synthesis of compound 24-2

2 mL of tetrahydrofuran was added into a 50 mL flask and cooled with liquid nitrogen to -60 °C. LDA (7.5 mL, 14.9 mmol) was slowly added dropwise, and after the dropwise addition, the mixture was stirred for 20 min. With the temperature controlled below -60 °C, a mixed solution of methyl palmitoleate (24-1, 2.0 g, 7.5 mmol) and tetrahydrofuran (5 mL) was added dropwise, and after the dropwise addition, the mixture was stirred for 2 h. With the temperature controlled below -60 °C, a mixed solution of 2-ethyl iodoethane (4.8 g, 20.1 mmol) and tetrahydrofuran (1 mL) was added dropwise, and after the dropwise addition, the mixture was slowly warmed to room temperature and stirred for 16 h. The extent of the reaction was detected by TLC *(n-*heptane). After completion of the reaction, 5 mL of a saturated aqueous ammonium chloride solution was added to the reaction solution to quench the reaction, followed by liquid separation. The mixture was extracted with EtOAc (20 mL × 2), and the organic phases were combined, then washed with 5 mL of saturated brine, dried over anhydrous Na₂SO₄, and concentrated to dryness to give a crude product (about 1.6 g). The crude product was purified and separated using a Flash column (50 g of silica gel, loaded with 200 mL of *n*-heptane; the mobile phases were respectively: 200 mL of *n*-heptane; 200 mL of 0.2% EtOAc + 99.8% *n-*heptane; 200 mL of 0.3% EtOAc + 99.7% *n*-heptane; and 300 mL of 0.4% EtOAc + 99.6% *n-*heptane) to give a pure product (600 mg, 21.2% yield).

### Step 2: synthesis of compound 24-3

Methanol (4 mL), THF (6 mL), and sodium hydroxide (0.98 mL,8 mM) were added into a 100 mL flask, and 24-2 (600 mg, 1.58 mmol) was added. The mixture was heated to 65 °C and stirred for 16 h. The extent of the reaction was detected by TLC (20% EtOAc/*n*-heptane). After completion of the reaction, the solvent in the reaction solution was removed by concentration. The solution was diluted with water, and the pH was adjusted to 1-2 with diluted hydrochloric acid. The solution was then extracted with DCM (20 mL × 2), and the organic phases were combined, washed with 3 mL of saturated brine, dried over anhydrous Na₂SO₄, filtered, and concentrated to give a crude product (about 800 mg). The crude product was purified and separated using a Flash column (30 g of silica gel, loaded with 100 mL of *n*-heptane; the mobile phases were respectively: 100 mL of 1% EtOAc + 99% *n*-heptane; 100 mL of 1.5% EtOAc + 98.5% *n*-heptane; 100 mL of 2% EtOAc + 98% *n*-heptane; 100 mL of 5% EtOAc + 95% *n-*heptane; and 300 mL of 10% EtOAc + 90% *n*-heptane) to give a pure product (300 mg, 52% yield).

### Step 3: synthesis of compound 24-4

24-3 (300 mg, 0.82 mmol) dissolved in DCM (3 mL) was added into a 25 mL flask, and DMF (10 µL) was added. The reaction solution was cooled in an ice-salt bath to 0-5 °C, and oxalyl chloride (166.2 mg, 1.31 mmol) was slowly added dropwise to the solution. The mixture was stirred at this temperature for 3 h. The extent of the reaction was detected by TLC (20% EtOAc/*n*-heptane). After completion of the reaction, the reaction solution was concentrated to dryness to give a crude product (about 315 mg, 100% yield), which was used directly in the next step.

### Step 4: synthesis of compound 24

3-Dimethylamino-1,2-propanediol (44.3 mg, 0.37 mmol), *N*,*N*-diisopropylethylamine (114.2 mg, 1.2 mmol), and THF (1 mL) were added into a 25 mL flask, and the reaction solution was cooled in an ice bath to 0-5 °C. A solution of 24-4 (315 mg, 0.82 mmol) in THF (2 mL) was added dropwise to the reaction solution, and after the dropwise addition, the mixture was stirred at room temperature overnight. The extent of the reaction was detected by TLC (0.5% MeOH/DCM). After completion of the reaction, 2 mL of water was added to the reaction solution to quench the reaction, and the mixture was extracted with EtOAc (10 mL × 2). The organic phases were combined, washed with 5 mL of saturated brine, dried over anhydrous Na₂SO₄, filtered, and concentrated to give a crude product (about 180 mg). The crude product was purified and separated using a Flash column (8 g of silica gel, loaded with 50 mL of *n-*heptane; the mobile phases were respectively: 100 mL of *n*-heptane; 100 mL of 1% EtOAc + 99% *n*-heptane; 100 mL of 2% EtOAc + 98% *n*-heptane; 100 mL of 4% EtOAc + 96% *n-*heptane; and 300 mL of 6% EtOAc + 94% *n*-heptane) to give a pure product (C022, 150 mg, 50% yield). ¹H NMR (400 MHz, CDCl₃): *δ* 5.38-5.29 (m, 4H), 5.18 (s, 1H), 4.39-4.35 (m, 1H), 4.10-4.05 (m, 1H), 2.45-2.38 (m, 4H), 2.27 (s, 5H), 2.03-1.98 (m, 8H), 1.62-1.54 (m, 4H), 1.44-1.14 (m, 56H), 0.90-0.79 (m, 18H).

### Synthesis of compound 25

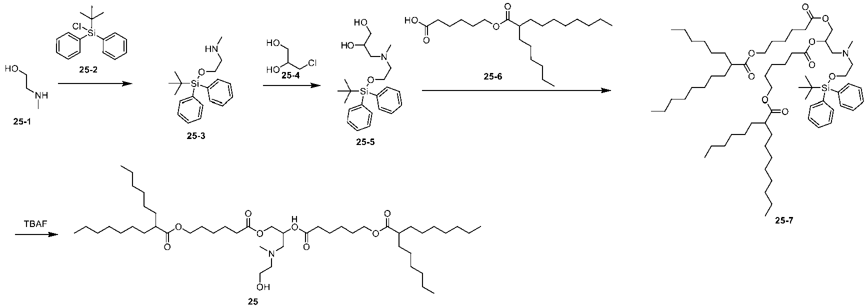

### Step 1: synthesis of compound 25-3

*N-*Methylethanolamine (25-1, 3.4 g, 45.3 mmol) and DCM (50 mL) were added into a 500 mL flask, and the solution was cooled in an ice-salt bath to 0-5 °C. A solution of TBDPSCl (25-2, 12.44 g, 45.3 mmol) in DCM (50 mL) was slowly added dropwise to the cooled solution, and after the dropwise addition, the mixture was stirred at room temperature for 2 h. The extent of the reaction was detected by TLC (10% MeOH/DCM). After completion of the reaction, 30 mL of water was added to quench the reaction, followed by liquid separation. The aqueous phase was extracted with DCM (50 mL × 3), and the organic phases were combined, washed with 50 mL of saturated brine, dried over anhydrous Na₂SO₄, filtered, and concentrated to give a crude product (about 12 g). The crude product was purified and separated using a Flash column (200 g of silica gel, loaded with 300 mL of *n*-heptane; the mobile phases were respectively: 300 mL of *n*-heptane; 300 mL of 0.1% EtOAc + 99.9% *n*-heptane; 300 mL of 0.3% EtOAc + 99.7% *n-*heptane; 300 mL of 1% EtOAc + 99% *n*-heptane; 300 mL of 1% EtOAc + 99% *n*-heptane; 300 mL of 0.2% MeOH + 99.8% DCM; and 500 mL of 0.3% MeOH + 99.7% DCM) to give a pure product (9 g, 63% yield).

### Step 2: synthesis of compound 25-5

25-3 (5.0 g, 16 mmol), potassium carbonate (5.5 g, 39.9 mmol) and 3-chloro-1,2-propanediol (25-4, 2.12 g, 19.1 mmol) dissolved in isopropanol (15 mL) were added into a 100 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 10.1 g). The crude product was purified and separated using a Flash column (100 g of silica gel; 200 mL of 0.1% MeOH + 99.9% DCM; 200 mL of 0.2% MeOH + 99.8% DCM; 200 mL of 0.3% MeOH + 99.7% DCM; 200 mL of 0.5% MeOH + 99.5% DCM; and 300 mL of 1% MeOH + 99% DCM) to give a pure product (3.6 g, 58% yield).

### Step 3: synthesis of compound 25-7

25-6 (1.0 g, 2.7 mmol), DCC (556.8 mg, 2.7 mmol), DMAP (82.4 mg, 0.67 mmol), and DCM (3 mL) were added into a 50 mL flask. The mixture was stirred at room temperature for 30 min, and then 25-5 (523 mg, 1.35 mmol) was added to the reaction solution. The extent of the reaction was detected by TLC (30% EtOAc/*n*-heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 1.5 g). The crude product was purified and separated using a Flash column (50 g of silica gel; the mobile phases were respectively: 200 mL of *n-*heptane; 150 mL of 1% EtOAc + 99% *n*-heptane; 150 mL of 2% EtOAc + 98% *n*-heptane; 200 mL of 5% EtOAc + 95% *n*-heptane; and 500 mL of 8% EtOAc + 92% *n*-heptane) to give a pure product (1.2 g, 81% yield).

### Step 4: synthesis of compound 25

25-7 (1.0 g, 0.92 mmol) dissolved in THF (5 mL) was added into a 50 mL flask and cooled in an ice-salt bath to -5 °C. TBAF (717.9 mg, 2.75 mmol) was then added, and the mixture was stirred at room temperature overnight. The extent of the reaction was detected by TLC (10% MeOH/DCM). After completion of the reaction, 5 mL of an aqueous ammonium chloride solution was added to quench the reaction, followed by liquid separation. The aqueous phase was extracted with EtOAc (10 mL × 3), and the organic phases were combined, washed with 5 mL of saturated brine, dried over anhydrous Na₂SO₄, filtered, and concentrated to give a crude product (about 700 mg). The crude product was purified and separated using a Flash column (12 g of silica gel; the mobile phases were respectively: 300 mL of *n*-heptane; 200 mL of 1% EtOAc + 99% n-heptane; 200 mL of 5% EtOAc + 95% n-heptane; 200 mL of 8% EtOAc + 92% *n*-heptane; 200 mL of 15% EtOAc + 85% *n*-heptane; and 400 mL of 30% EtOAc + 70% *n-*heptane) to give a pure product (350 mg, 45% yield). ¹H NMR (400 MHz, CDCl₃): *δ* 5.23-5.19 (m, 1H), 4.36-4.32 (m, 1H), 4.12-4.04 (m, 5H), 3.58 (t, *J*=4Hz, 2H), 2.66-2.56 (m, 4H), 2.36-2.26 (m, 9H), 1.69-1.53 (m, 13H), 1.46-1.35 (m, 8H), 1.32-1.19 (m, 43H), 0.88-0.85 (m, 12H).

### Synthesis of compound 27

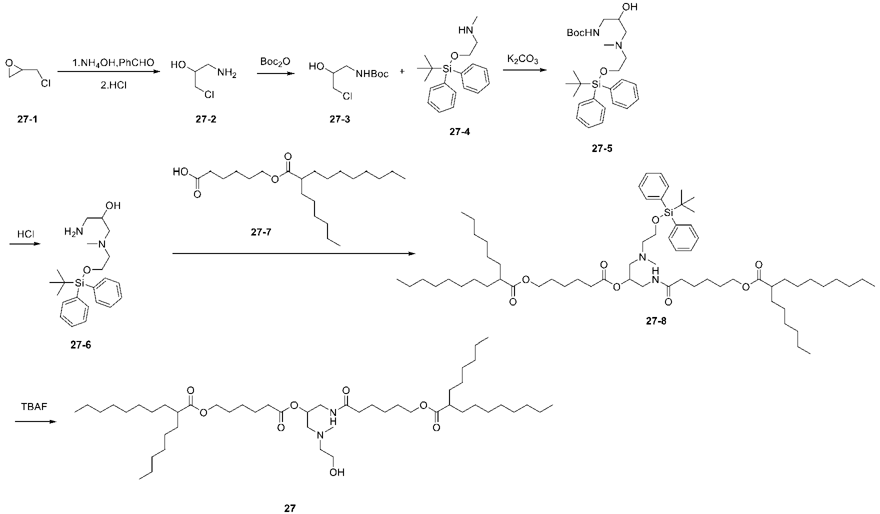

### Step 1: synthesis of compound 27-2

Benzaldehyde (5.91 g, 55.66 mmol) and ethanol (17 mL) were added into a 100 mL flask, and the solution was cooled to 18 °C. Ammonium hydroxide (11.97 g, 85.39 mmol) was slowly added dropwise to the cooled solution, and after the dropwise addition, the mixture was stirred at 18 °C for 2 h. Epichlorohydrin (5 g, 54.04 mmol) was then weighed out and added, and the mixture was stirred at 40-45 °C for 16 h. After completion of the reaction, the reaction solution was concentrated to dryness, and toluene (20 mL) and water (20 mL) were added to dissolve the crude product. Concentrated hydrochloric acid (8.32 g, 82.13 mmol) was then weighed out and added dropwise into the reaction system, and the mixture was stirred for 3 h with the temperature maintained at 35-40 °C, followed by liquid separation. The toluene phase was washed with water (10 mL). The aqueous phases were combined and concentrated to give a product (6 g).

### Step 2: synthesis of compound 27-3

27-2 (3 g, 20.55 mmol), di-*tert*-butyl dicarbonate (4.89 g, 22.40 mmol), methanol (12 mL), and water (7 mL) were added into a 100 mL flask Potassium bicarbonate (2.26 g, 11.60 mmol) was then weighed out and added to the reaction solution in three batches, and the mixture was stirred for 3 h. The extent of the reaction was detected by TLC (10% MeOH/DCM). After completion of the reaction, dichloromethane (15 mL) was added, and the mixture was extracted with water (6 mL), followed by liquid separation. The organic phase was concentrated to give a crude product (about 5 g). The crude product was purified and separated using a Flash column (20 g of silica gel; 100 mL of *n*-heptane; 100 mL of 1% EtOAc + 99% *n*-heptane; 100 mL of 5% EtOAc + 95% *n*-heptane; and 600 mL of 10% EtOAc + 90% *n*-heptane) to give a pure product (800 mg, 19% yield).

### Step 3: synthesis of compound 27-5

27-3 (700 mg, 3.34 mmol), 27-4 (1.05 g, 3.34 mmol), potassium carbonate (1.15 g, 8.35 mmol), and isopropanol (8 mL) were added into a 25 mL flask, and the mixture was heated to 80 °C and stirred for 16 h. The extent of the reaction was detected by TLC (10% MeOH/DCM). After completion of the reaction, the mixture was filtered, and the organic phase was concentrated to give a crude product (about 2 g). The crude product was purified and separated using a Flash column (12 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 15% EtOAc + 85% *n*-heptane; and 600 mL of 30% EtOAc + 70% *n*-heptane) to give a pure product (1 g, 62% yield). ¹H NMR (400 MHz, CDCl₃): *δ* 7.68-7.66 (m, 4H), 7.43-7.37 (m, 6H), 4.99 (m, 1H), 3.74-3,70 (m, 3H), 3.35-3.31 (m, 1H), 3.03-2.97 (m, 1H), 2.73-2.67 (m, 1H), 2.55-2.52 (m, 1H), 2.41-2.38 (m, 2H), 2.30 (m, 3H), 1.44 (s, 9H), 1.06-1.04 (m, 9H).

### Step 4: synthesis of compound 27-6

27-5 (300 mg, 0.62 mmol) and ethyl acetate (2 mL) were added into a 25 mL flask, and the mixture was cooled in an ice bath to 0-5 °C. HCl/EtOAc (0.6 mL, 4 M) was then weighed out and added, and the mixture was warmed to room temperature and stirred for 3 h. The extent of the reaction was detected by TLC (10% MeOH/DCM). After completion of the reaction, the organic phase was concentrated to give a product (about 300 mg).

### Step 5: synthesis of compound 27-8

27-7 (496.14 mg, 1.34 mmol), DCC (276.25 mg, 1.34 mmol), DMAP (122.68 mg, 1.0 mmol), and DCM (5 mL) were added into a 25 mL flask, and the mixture was stirred at room temperature for 30 min. 27-6 (330 mg, 0.66 mmol) was then added dropwise, and the mixture was stirred at room temperature for 16 h. The extent of the reaction was detected by TLC (30% EtOAc/*n*-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1 g). The crude product was purified and separated using a Flash column (12 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 200 mL of 20% EtOAc + 80% *n*-heptane) to give a pure product (330 mg, 45% yield). ¹H NMR (400 MHz, CDCl₃): *δ* 7.67-7.65 (m, 4H), 7.45-7.36 (m, 6H), 6.26 (m, 1H), 4.98 (m, 1H), 4.07-4.02 (m, 4H), 3.75 (m, 2H), 3.49-3.45 (m, 2H), 2.59 (m, 4H), 2.34-2.22 (m, 7H), 2.11-2.08 (m, 2H), 1.67-1.53 (m, 14H), 1.46-1.25 (m, 60H), 1.05 (s, 9H), 0.89-0.85 (m, 12H).

### Step 6: synthesis of compound 27

27-8 (330 mg, 0.31 mmol) and THF (3 mL) were added into a 25 mL flask, and the mixture was cooled in an ice bath to 0-5 °C. TBAF (240.19 mg, 0.92 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (4 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; 100 mL of 30% EtOAc + 70% *n*-heptane; and 300 mL of 50% EtOAc + 50% *n*-heptane) to give a pure product (100 mg, 39% yield). ¹H NMR (400 MHz, CDCl₃): *δ* 6.14-6.11 (m, 1H), 5.06-5.04 (m, 1H), 4.08-4.04 (m, 4H), 3.63-3.46 (m, 4H), 2.64-2.60 (m, 4H), 2.35-2.26 (m, 7H), 2.20-2.17 (m, 2H), 1.70-1.53 (m, 12H), 1.46-1.35 (m, 8H), 1.33-1.25 (m, 45H), 0.89-0.85 (m, 12H).

### Synthesis of compound 29

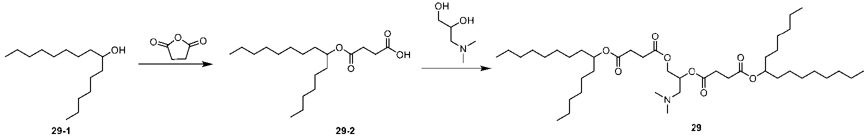

### Step 1: synthesis of compound 29-2

Pentadecan-7-ol (29-1, 1.0 g, 4.4 mmol), succinic anhydride (525.7 mg, 5.3 mmol), DMAP (267.4 mg, 2.2 mmol), and DCM (5 mL) were added into a 50 mL flask, and the mixture was stirred at room temperature overnight. The extent of the reaction was detected by TLC (30% EtOAc/*n*-heptane). After completion of the reaction, the mixture was washed with 5 mL of water and 5 mL of saturated brine, dried over anhydrous Na₂SO₄, and filtered. The organic phase was concentrated to give a crude product (about 800 mg). The crude product was purified and separated using a Flash column (50 g of silica gel; the mobile phases were respectively: 200 mL of *n*-heptane; 150 mL of 1% EtOAc + 99% *n*-heptane; 150 mL of 2% EtOAc + 98% *n-*heptane; 200 mL of 8% EtOAc + 92% *n*-heptane; 200 mL of 20% EtOAc + 80% *n*-heptane; and 500 mL of 35% EtOAc + 70% *n*-heptane) to give a pure product (540 mg, 38% yield).

### Step 2: synthesis of compound 29

DMAP (46.5 mg, 0.38 mmol), DCC (314.1 mg, 1.52 mmol), and DCM (5 mL) were added into a 25 mL flask, and the mixture was stirred until it became clear. 29-2 (500 mg, 1.52 mmol) was added, and the mixture was stirred at room temperature for 30 min. 3-Dimethylamino-1,2-propanediol (90.7 mg, 0.76 mmol) was then added in one batch, and the mixture was stirred at room temperature for 5 h. The extent of the reaction was detected by TLC (10% MeOH/DCM). After completion of the reaction, the reaction solution was filtered to remove solids, and the filtrate was washed with 10 mL of water and 10 mL of saturated brine, dried over anhydrous Na₂SO₄, and filtered. The organic phase was concentrated to give a crude product (about 510 mg). The crude product was purified and separated using a Flash column (20 g of silica gel, loaded with 100 mL of *n*-heptane; the mobile phases were respectively: 100 mL of 1% EtOAc + 99% *n*-heptane; 100 mL of 2% EtOAc + 98% *n-*heptane; 100 mL of 3% EtOAc + 97% *n-*heptane; 100 mL of 5% EtOAc + 95% *n*-heptane; 100 mL of 10% EtOAc + 90% *n*-heptane; 100 mL of 20% EtOAc + 80% *n*-heptane; and 300 mL of 30% EtOAc + 70% *n*-heptane) to give a pure product (260 mg, 46% yield).

### Synthesis of compound 30

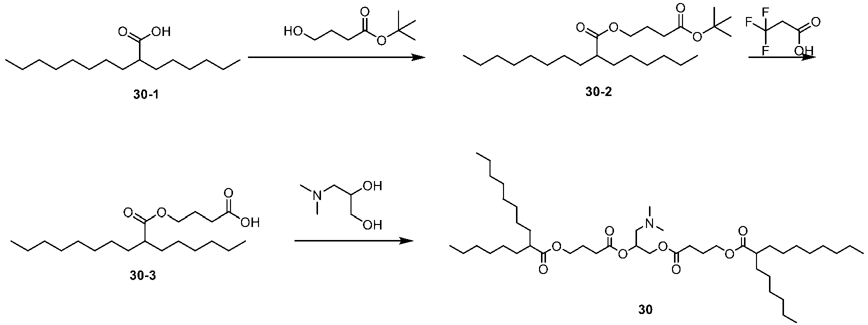

### Step 1: synthesis of compound 30-2

2-Hexyldecanoic acid (30-1, 1.0 g, 3.9 mmol), DMAP (238.2 mg, 1.95 mmol), DCC (804.6 mg, 3.9 mmol), and DCM (5 mL) were added into a 50 mL flask, and the mixture was stirred at room temperature for 30 min. *tert*-Butyl 4-hydroxybutyrate (624.8 mg, 3.9 mmol) was added, and the mixture was stirred at room temperature overnight. The extent of the reaction was detected by TLC (20% EtOAc/*n*-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 2.0 g). The crude product was purified and separated using a Flash column (50 g of silica gel; the mobile phases were respectively: 200 mL of *n*-heptane; 150 mL of 1% EtOAc + 99% *n*-heptane; 150 mL of 1.5% EtOAc + 98.5% *n*-heptane; 150 mL of 2% EtOAc + 98% *n*-heptane; and 500 mL of 3% EtOAc + 97% *n*-heptane) to give a pure product (1.5 g, 96% yield).

### Step 2: synthesis of compound 30-3

30-2 (1.5 g, 3.76 mmol) dissolved in DCM (6 mL) was added into a 100 mL flask, and TFA (3 mL) was added. After the mixture was stirred at room temperature for 2 h, the extent of the reaction was detected by TLC (5% MeOH/DCM). After completion of the reaction, the reaction solution was concentrated, followed by addition of MTBE (20 mL) for dissolution. The pH was adjusted to 8-9 with a 10% NaHCO₃ solution, and then the pH was adjusted to 1-2 with 1 M diluted hydrochloric acid. The mixture was stirred for 20 min, followed by layer separation. The aqueous phase was extracted with MTBE (50 mL), and the organic phases were combined, dried over anhydrous Na₂SO₄, filtered, and concentrated to give a crude product (1.5 g). The crude product was purified and separated using a Flash column (100 g of silica gel, loaded with 50 mL of *n*-heptane; the mobile phases were respectively: 200 mL of 1% EtOAc + 99% *n-*heptane; 200 mL of 2% EtOAc + 98% *n*-heptane; 200 mL of 5% EtOAc + 95% *n*-heptane; and 500 mL of 10% EtOAc + 90% n-heptane) to give a pure product (1 g, 77% yield). ¹H NMR (400 MHz, CDCl₃): *δ* 4.12 (t, *J*=4Hz, 2H), 2.46 (t, *J*=4Hz, 2H), 2.35-2.28 (m, 1H), 2.01-1.94 (m, 2H), 1.62-1.53 (m, 2H), 1.47-1.39 (m, 2H), 1.30-1.20 (m, 20H), 0.88-0.85 (m, 6H).

### Step 3: synthesis of compound 30

DMAP (44.6 mg, 0.36 mmol), DCC (301.2 mg, 1.46 mmol), and DCM (5 mL) were added into a 25 mL flask, and the mixture was stirred until it became clear. 30-3 (500 mg, 1.46 mmol) was added, and the mixture was stirred at room temperature for 30 min. 3-Dimethylamino-1,2-propanediol (86.9 mg, 0.73 mmol) was then added in one batch, and the mixture was stirred at room temperature for 5 h. The extent of the reaction was detected by TLC (30% EtOAc/*n-*heptane). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 510 mg). The crude product was purified and separated using a Flash column (20 g of silica gel, loaded with 100 mL of *n*-heptane; the mobile phases were respectively: 100 mL of 1% EtOAc + 99% *n*-heptane; 100 mL of 2% EtOAc + 98% n-heptane; 100 mL of 3% EtOAc + 97% *n*-heptane; 100 mL of 5% EtOAc + 95% n-heptane; 100 mL of 10% EtOAc + 90% *n-*heptane; 100 mL of 20% EtOAc + 80% *n*-heptane; and 300 mL of 30% EtOAc + 70% *n-*heptane) to give a pure product (330 mg, 59% yield). ¹H NMR (400 MHz, CDCl₃): *δ* 5.22-5.19 (m, 1H), 4.41-4.38 (m, 1H), 4.11-4.07 (m, 5H), 2.47-2.38 (m, 6H), 2.34-2.26 (m, 8H), 1.99-1.92 (m, 4H), 1.62-1.53 (m, 4H), 1.46-1.36 (m, 4H), 1.32-1.20 (m, 42H), 0.88-0.85 (m, 12H). Synthesis of compound 31

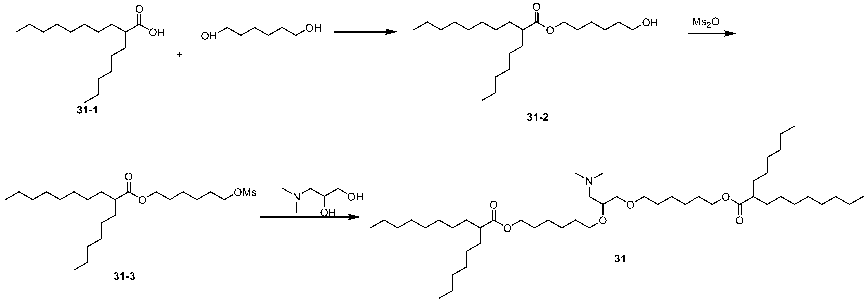

### Step 1: synthesis of compound 31-2

2-Hexyldecanoic acid (31-1, 3.6 g, 14 mmol), DCC (3.2 g, 15.4 mmol), DMAP (2.1 g, 16.9 mmol), and DCM (15 mL) were added into a 50 mL flask, and the mixture was stirred at room temperature for 30 min. 1,6-Hexanediol (4.98 g, 42.1 mmol) was then added dropwise, and the mixture was stirred at room temperature overnight. The extent of the reaction was detected by TLC (50% EtOAc/*n*-heptane). After completion of the reaction, the mixture was filtered, and the filtrate was washed with 10 mL of water and 10 mL of saturated brine, dried over anhydrous Na₂SO₄, and filtered. The organic phase was concentrated to give a crude product (about 6 g). The crude product was purified and separated using a Flash column (100 g of silica gel; the mobile phases were respectively: 100 mL of n-heptane; 200 mL of 1% EtOAc + 99% *n*-heptane; 200 mL of 2% EtOAc + 98% *n*-heptane; 200 mL of 8% EtOAc + 92% *n*-heptane; and 1000 mL of 20% EtOAc + 80% *n*-heptane) to give a pure product (3 g, 60% yield).

### Step 2: synthesis of compound 31-3

31-2 (1.0 g, 2.8 mmol), triethylamine (851.3 mg, 8.4 mmol), and DCM (10 mL) were added into a 50 mL flask, and the mixture was cooled in an ice bath to 0-5 °C. Methanesulfonic anhydride (732.7 mg, 4.2 mmol) was added dropwise with the temperature maintained at 0-5 °C, and the mixture was stirred at room temperature overnight. The extent of the reaction was detected by TLC (30% EtOAc/*n*-heptane). After completion of the reaction, the reaction was quenched with 2 mL of water, and the mixture was extracted with DCM (5 mL × 3). The organic phases were combined, dried over anhydrous Na₂SO₄, filtered, and concentrated to give a crude product (about 2 g). The crude product was purified and separated using a Flash column (60 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 5% EtOAc + 95% *n*-heptane; and 500 mL of 20% EtOAc + 80% *n*-heptane) to give a pure product (1.2 g, 98% yield).

### Step 3: synthesis of compound 31

DMF (1 mL) was added into a 10 mL flask, and sodium hydride (46 mg, 1.2 mmol) was added in batches. The mixture was cooled in an ice bath to 5-10 °C, and a mixed solution of 3-dimethylamino-1,2-propanediol (45.7 mg, 0.38 mmol) and DMF (1 mL) was added dropwise. After the dropwise addition, the mixture was heated to 50 °C for reaction for 10 min and then cooled to 5-10 °C again. A mixed solution of 31-3 (500 mg, 1.2 mmol) and DMF (1 mL) was then added dropwise, and the mixture was stirred at room temperature overnight. The extent of the reaction was detected by TLC (30% EtOAc/*n*-heptane). After completion of the reaction, the reaction was quenched with 1 mL of water, and the mixture was extracted with EtOAc (5 mL × 3). The organic phases were combined, dried over anhydrous Na₂SO₄, filtered, and concentrated to give a crude product (about 200 mg). The crude product was purified and separated using a Flash column (50 g of silica gel; the mobile phases were respectively: 100 mL of *n*-heptane; 100 mL of 1% EtOAc + 99% n-heptane; 100 mL of 2% EtOAc + 98% *n-*heptane; 100 mL of 5% EtOAc + 95% *n*-heptane; 100 mL of 10% EtOAc + 90% *n*-heptane; and 300 mL of 20% EtOAc + 80% *n*-heptane) to give a pure product (40 mg, 13% yield).

### Synthesis of compound 32

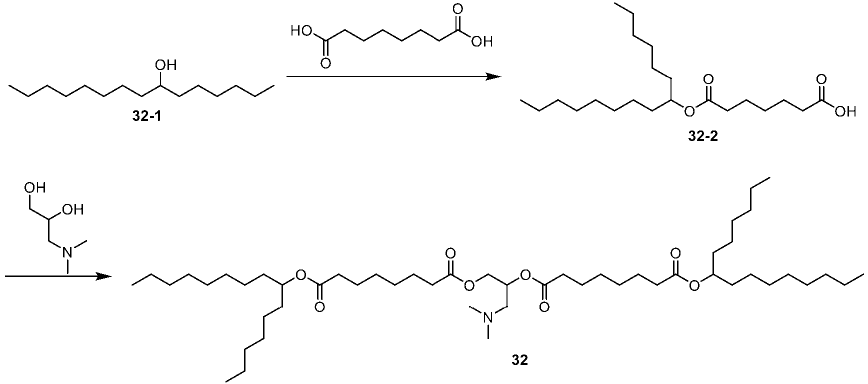

### Step 1: synthesis of compound 32-2

Suberic acid (5.72 g, 32.8 mmol), DCC (6.77 g, 32.8 mmol), DMAP (802.3 mg, 6.6 mmol), and DCM (80 mL) were added into a 250 mL flask, and the mixture was stirred at room temperature for 30 min. A solution of pentadecan-7-ol (32-1, 1.5 g, 6.6 mmol) in DCM (40 mL) was then added dropwise, and the mixture was stirred at room temperature for 5 h. The extent of the reaction was detected by TLC (30% EtOAc/*n*-heptane). After completion of the reaction, the mixture was filtered, and the filtrate was washed with 15 mL of water and 15 mL of saturated brine, dried over anhydrous Na₂SO₄, and filtered. The organic phase was concentrated to give a crude product (about 3.7 g). The crude product was purified and separated using a Flash column (50 g of silica gel; the mobile phases were respectively: 200 mL of *n*-heptane; 150 mL of 1% EtOAc + 99% *n*-heptane; 150 mL of 2% EtOAc + 98% *n*-heptane; 200 mL of 8% EtOAc + 92% *n*-heptane; and 1000 mL of 20% EtOAc + 80% *n*-heptane) to give a pure product (2.1 g, 83% yield).

### Step 2: synthesis of compound 32

DMAP (39.7 mg, 0.33 mmol), DCC (268.2 mg, 1.3 mmol), and DCM (1.5 mL) were added into a 25 mL flask, and the mixture was stirred until it became clear. 32-2 (500 mg, 1.3 mmol) was added, and the mixture was stirred at room temperature for 30 min. 3-Dimethylamino-1,2-propanediol (77.5 mg, 0.65 mmol) was then added in one batch, and the mixture was stirred at room temperature for 5 h. The extent of the reaction was detected by TLC (5% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 510 mg). The crude product was purified and separated using a Flash column (20 g of silica gel, loaded with 100 mL of *n*-heptane; the mobile phases were respectively: 100 mL of 1% EtOAc + 99% *n*-heptane; 100 mL of 2% EtOAc + 98% n-heptane; 100 mL of 3% EtOAc + 97% *n-*heptane; 100 mL of 5% EtOAc + 95% n-heptane; 100 mL of 10% EtOAc + 90% n-heptane; and 100 mL of 20% EtOAc + 80% *n*-heptane) to give a pure product (260 mg, 47% yield).

### Synthesis of compound 33

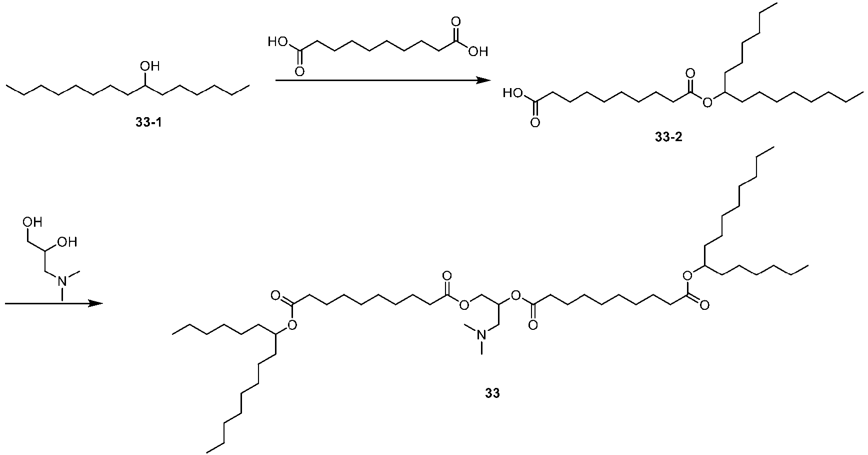

### Step 1: synthesis of compound 33-2

Sebacic acid (6.64 g, 32.8 mmol), DCC (6.77 g, 32.8 mmol), DMAP (802.3 mg, 6.6 mmol), and DCM (80 mL) were added into a 250 mL flask, and the mixture was stirred at room temperature for 30 min. A solution of pentadecan-7-ol (33-1, 1.5 g, 6.6 mmol) in DCM (40 mL) was then added dropwise, and the mixture was stirred at room temperature for 5 h. The extent of the reaction was detected by TLC (30% EtOAc/*n*-heptane). After completion of the reaction, the mixture was filtered, and the filtrate was washed with 15 mL of water and 15 mL of saturated brine, dried over anhydrous Na₂SO₄, and filtered. The organic phase was concentrated to give a crude product (about 3.7 g). The crude product was purified and separated using a Flash column (50 g of silica gel; the mobile phases were respectively: 200 mL of *n*-heptane; 150 mL of 1% EtOAc + 99% *n*-heptane; 150 mL of 2% EtOAc + 98% *n*-heptane; 200 mL of 8% EtOAc + 92% *n*-heptane; and 1000 mL of 20% EtOAc + 80% *n-*heptane) to give a pure product (2.1 g, 77% yield). ¹H NMR (400 MHz, CDCl₃): *δ* 4.89-4.85 (m, 1H), 2.36-2.25 (m, 4H), 1.66-1.47 (m, 8H), 1.37-1.25 (m, 30H), 0.89-0.85 (m, 6H).

### Step 2: synthesis of compound 33

DMAP (74 mg, 0.61 mmol), DCC (250 mg, 1.21 mmol), and DCM (1.5 mL) were added into a 25 mL flask, and the mixture was stirred until it became clear. 33-2 (500 mg, 1.21 mmol) was added, and the mixture was stirred at room temperature for 30 min. 3-Dimethylamino-1,2-propanediol (72.2 mg, 0.61 mmol) was then added in one batch, and the mixture was stirred at room temperature for 5 h. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 500 mg). The crude product was purified and separated using a Flash column (20 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 3% EtOAc + 97% *n-*heptane; 100 mL of 5% EtOAc + 95% *n*-heptane; 100 mL of 10% EtOAc + 90% *n*-heptane; 100 mL of 20% EtOAc + 80% *n*-heptane; and 300 mL of 30% EtOAc + 70% *n*-heptane) to give a pure product (230 mg, 42% yield). ¹H NMR (400 MHz, CDCl₃): *δ* 5.23-5.17 (m, 1H), 4.89-4.83 (m, 2H), 4.37-4.33 (m, 1H), 4.11-4.06 (m, 1H), 2.51-2.45 (m, 2H), 2.37-2.25 (m, 14H), 1.64-1.47 (m, 20H), 1.37-1.25 (m, 65H), 0.89-0.85 (m, 12H).

### Synthesis of compound 34

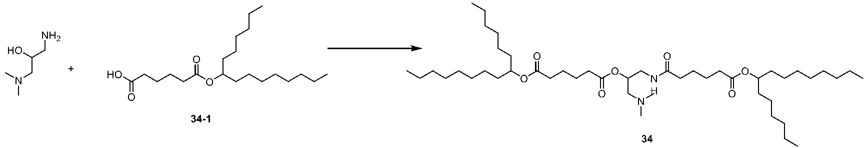

### Step 1: synthesis of compound 34

DMAP (34.3 mg, 0.28 mmol), DCC (115.7 mg, 0.56 mmol), and DCM (1.5 mL) were added into a 50 mL flask, and the mixture was stirred until it became clear. 34-1 (200 mg, 0.56 mmol) was added, and the mixture was stirred at room temperature for 30 min. 3-Dimethylamino-1,2-propanediol (72.2 mg, 0.61 mmol) was then added in one batch, and the mixture was stirred at room temperature for 5 h. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 300 mg). The crude product was purified and separated using a Flash column (20 g of silica gel, loaded with 100 mL of *n*-heptane; the mobile phases were respectively: 100 mL of 1% EtOAc + 99% *n*-heptane; 100 mL of 2% EtOAc + 98% n-heptane; 100 mL of 3% EtOAc + 97% *n-*heptane; 100 mL of 10% EtOAc + 90% *n*-heptane; 100 mL of 30% EtOAc + 70% *n*-heptane; 100 mL of 50% EtOAc + 50% *n*-heptane; and 300 mL of 80% EtOAc + 20% *n*-heptane) to give a pure product (60 mg, 27% yield). ¹H NMR (400 MHz, CDCl₃): *δ* 6.53-6.50 (m, 1H), 5.05-5.00 (m, 1H), 4.89-4.81 (m, 2H), 3.56-3.40 (m, 2H), 2.45-2.37 (m, 3H), 2.36-2.28 (m, 6H), 2.25 (s, 6H), 2.21-2.17 (m, 2H), 1.69-1.61 (m, 8H), 1.52-1.47 (m, 8H), 1.33-1.25 (m, 43H), 0.89-0.85 (m, 12H).

### Synthesis of compound 35

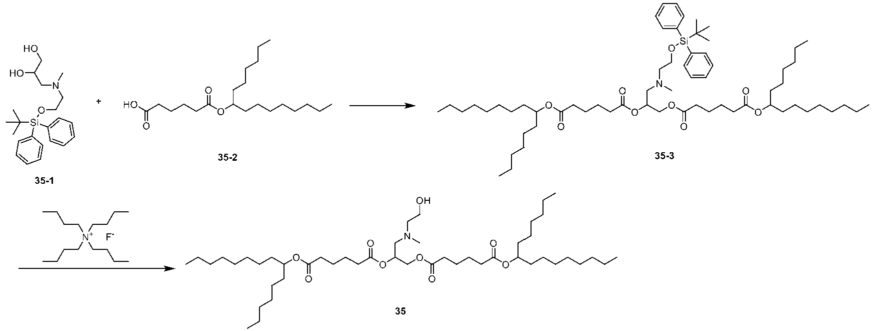

### Step 1: synthesis of compound 35-3

35-2 (300 mg, 0.84 mmol), DCC (173.6 mg, 0.84 mmol), DMAP (51.4 mg, 0.42 mmol), and DCM (1.5 mL) were added into a 25 mL flask, and the mixture was stirred at room temperature for 30 min. 35-1 (163.1 mg, 0.42 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 420 mg). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 300 mL of 20% EtOAc + 80% *n*-heptane) to give a pure product (150 mg, 33% yield). ¹H NMR (400 MHz, CDCl₃): *δ* 7.68-7.66 (m, 4H), 7.43-7.37 (m, 6H), 4.89-4.83 (m, 2H), 4.16-4.12 (m, 1H), 4.01-3.97 (m, 1H), 3.90-3.84 (m, 1H), 3.78-3.68 (m, 2H), 2.75-2.69 (m, 1H), 2.63-2.45 (m, 3H), 2.39-2.26 (m, 7H), 1.67-1.64 (m, 5H), 1.51-1.49 (m, 4H), 1.29-1.25 (m, 22H), 1.05-1.03 (m, 8H), 0.89-0.86 (m, 6H).

### Step 2: synthesis of compound 35

35-3 (140 mg, 0.13 mmol) and THF (2 mL) were added into a 25 mL flask, and the mixture was cooled in an ice bath to 0-5 °C. TBAF (103.2 mg, 0.39 mmol) was added dropwise with the temperature maintained at 0-5 °C, and the mixture was stirred at this temperature for 3 h.

The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 510 mg). The crude product was purified and separated using a Flash column (20 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 3% EtOAc + 97% *n*-heptane; 100 mL of 5% EtOAc + 95% n-heptane; 100 mL of 10% EtOAc + 90% *n*-heptane; and 300 mL of 30% EtOAc + 70% *n*-heptane) to give a pure product (80 mg, 74% yield). ¹H NMR (400 MHz, CDCl₃): *δ* 5.22-5.20 (m, 1H), 4.88-4.82 (m, 2H), 4.36-4.32 (m, 1H), 4.13-4.08 (m, 1H), 3.64-3.47 (m, 2H), 2.65-2.57 (m, 4H), 2.37-2.28 (m, 11H), 1.69-1.64 (m, 8H), 1.52-1.25 (m, 53H), 0.89-0.85 (m, 12H).

### Synthesis of compound 36

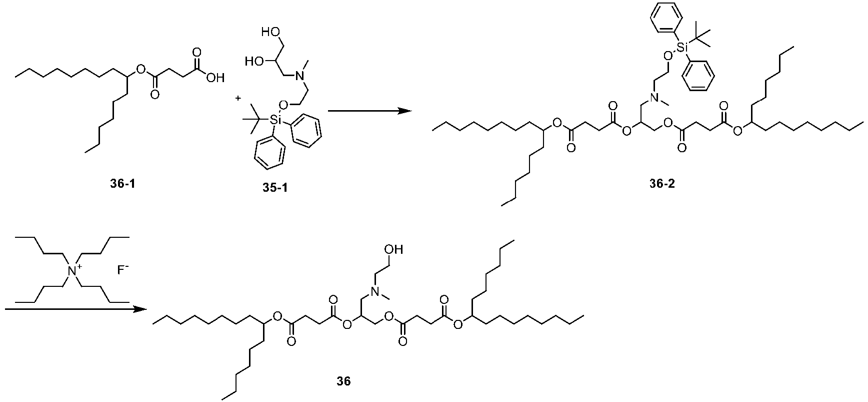

### Step 1: synthesis of compound 36-2

36-1 (480 mg, 1.46 mmol), DCC (301.5 mg, 1.46 mmol), DMAP (89.3 mg, 0.73 mmol), and DCM (3 mL) were added into a 25 mL flask, and the mixture was stirred at room temperature for 30 min. 35-1 (283.2 mg, 0.73 mmol) was then added dropwise, and the mixture was stirred at room temperature overnight. The extent of the reaction was detected by TLC (30% EtOAc/n-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 420 mg). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 500 mL of 12% EtOAc + 88% *n*-heptane) to give a pure product (450 mg, 31% yield). ¹H NMR (400 MHz, CDCl₃): *δ* 7.68-7.62 (m, 4H), 7.44-7.36 (m, 6H), 5.16-5.10 (m, 1H), 4.90-4.83 (m, 2H), 4.37-4.33 (m, 1H), 4.14-4.10 (m, 1H), 3.58 (t, *J*=4Hz, 2H), 2.63-2.55 (m, 11H), 2.25 (s, 3H), 1.53-1.48 (m, 8H), 1.37-1.19 (m, 43H), 1.04 (s, 9H), 0.89-0.86 (m, 12H).

### Step 2: synthesis of compound 36

36-2 (480 mg, 0.46 mmol) and THF (4 mL) were added into a 25 mL flask, and the mixture was cooled in an ice bath to 0-5 °C. TBAF (363.2 mg, 1.39 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (20 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 5% EtOAc + 95% *n*-heptane; 100 mL of 10% EtOAc + 90% *n*-heptane; 100 mL of 20% EtOAc + 80% *n*-heptane; and 300 mL of 50% EtOAc + 50% *n-*heptane) to give a pure product (210 mg, 57% yield). ¹H NMR (400 MHz, CDCl₃): *δ* 5.26-5.20 (m, 1H), 4.89-4.83 (m, 2H), 4.34-4.31 (m, 1H), 4.19-4.15 (m, 1H), 3.58 (t, *J*=4Hz, 2H), 2.69-2.56 (m, 12H), 2.33 (s, 3H), 1.53-1.48 (m, 8H), 1.37-1.25 (m, 41H), 0.89-0.86 (m, 12H).

### Synthesis of compound 37

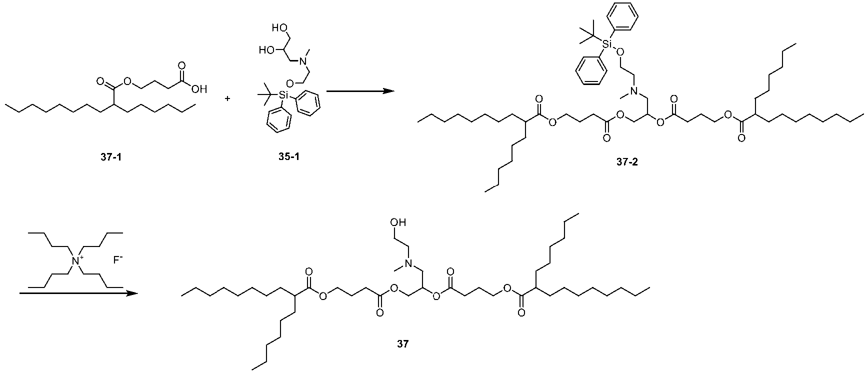

### Step 1: synthesis of compound 37-2

37-1 (500 mg, 1.46 mmol), DCC (301.5 mg, 1.46 mmol), DMAP (89.3 mg, 0.73 mmol), and DCM (3 mL) were added into a 25 mL flask, and the mixture was stirred at room temperature for 30 min. 35-1 (283 mg, 0.73 mmol) was then added dropwise, and the mixture was stirred at room temperature overnight. The extent of the reaction was detected by TLC (30% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 820 mg). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% n-heptane; and 300 mL of 10% EtOAc + 90% *n*-heptane) to give a pure product (480 mg, 63% yield). ¹H NMR (400 MHz, CDCl₃): *δ* 7.68-7.64 (m, 4H), 7.44-7.36 (m, 6H), 5.17-5.12 (m, 1H), 4.39-4.39 (m, 1H), 4.11-4.06 (m, 5H), 3.71 (t, *J*=4Hz, 2H), 2.65-2.55 (m, 4H), 2.38-2.26 (m, 9H), 1.97-1.90 (m, 4H), 1.62-1.53 (m, 5H), 1.47-1.40 (m, 4H), 1.31-1.30 (m, 43H), 1.04 (s, 9H), 0.89-0.86 (m, 12H).

### Step 2: synthesis of compound 37

37-2 (530 mg, 0.53 mmol) and THF (4 mL) were added into a 25 mL flask, and the mixture was cooled in an ice bath to 0-5 °C. TBAF (412.2 mg, 1.58 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (20 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 5% EtOAc + 95% *n*-heptane; 100 mL of 10% EtOAc + 90% n*-*heptane; and 300 mL of 20% EtOAc + 80% *n*-heptane) to give a pure product (190 mg, 47% yield). ¹H NMR (400 MHz, CDCl₃): *δ* 5.24-5.22 (m, 1H), 4.39-4.35 (m, 1H), 4.15-4.08 (m, 5H), 3.58 (t, *J*=4Hz, 2H), 2.64-2.58 (m, 5H), 2.43-2.27 (m, 10H), 2.00-1.92 (m, 4H), 1.61-1.53 (m, 5H), 1.47-1.37 (m, 5H), 1.33-1.19 (m, 45 H), 0.89-0.85 (m, 12H).

### Synthesis of compound 38

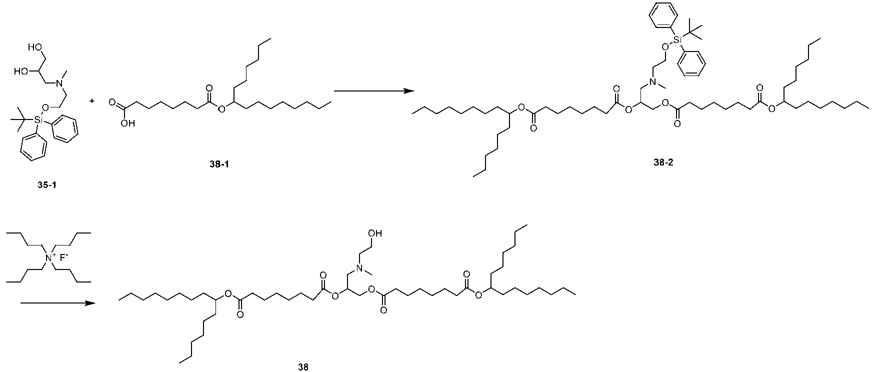

### Step 1: synthesis of compound 38-2

38-1 (500 mg, 1.3 mmol), DCC (268.2 mg, 1.3 mmol), DMAP (79.4 mg, 0.65 mmol), and DCM (2 mL) were added into a 25 mL flask, and the mixture was stirred at room temperature for 30 min. 35-1 (252 mg, 0.65 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.7 g). The crude product was purified and separated using a Flash column (50 g of silica gel; the mobile phases were respectively: 200 mL of *n-*heptane; 150 mL of 1% EtOAc + 99% *n*-heptane; 150 mL of 2% EtOAc + 98% *n*-heptane; 150 mL of 8% EtOAc + 92% *n*-heptane; 150 mL of 20% EtOAc + 80% n-heptane; and 500 mL of 30% EtOAc + 70% *n*-heptane) to give a pure product (700 mg, 96% yield). ¹H NMR (400 MHz, CDCl₃): *δ* 7.68-7.65 (m, 4H), 7.42-7.36 (m, 6H), 5.12 (s, 1H), 4.87-4.84 (m, 2H), 4.35-4.31 (m, 1H), 4.09-4.04 (m, 1H), 3.72-3.69 (m, 2H), 2.61-2.54 (m, 4H), 2.30-2.24 (m, 11H), 1.65-1.48 (m, 18H), 1.37-119 (m, 53H), 1.04 (s, 9H), 0.89-0.86 (m, 12H). Step 2: synthesis of compound 38
38-2 (700 mg, 0.63 mmol) and THF (5 mL) were added into a 50 mL flask, and the mixture was cooled in an ice bath to 0-5 °C. TBAF (496.1 mg, 1.9 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 510 mg). The crude product was purified and separated using a Flash column (20 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 3% EtOAc + 97% *n*-heptane; 100 mL of 5% EtOAc + 95% *n*-heptane; 100 mL of 10% EtOAc + 90% *n*-heptane; and 500 mL of 30% EtOAc + 70% *n*-heptane) to give a pure product (300 mg, 54% yield). ¹H NMR (400 MHz, CDCl₃): *δ* 5.21-5.19 (m, 1H), 4.86-4.83 (m, 2H), 4.34-4.30 (m, 1H), 4.11-4.07 (m, 1H), 3.57 (t, *J*=4Hz, 2H), 2.70-2.56 (m, 5H), 2.32-2.24 (m, 12H), 1.64-1.57 (m, 9H), 1.51-1.46 (m, 9H), 1.37-1.24 (m, 53 H), 0.88-0.84 (m, 12H).

### Synthesis of compound 43

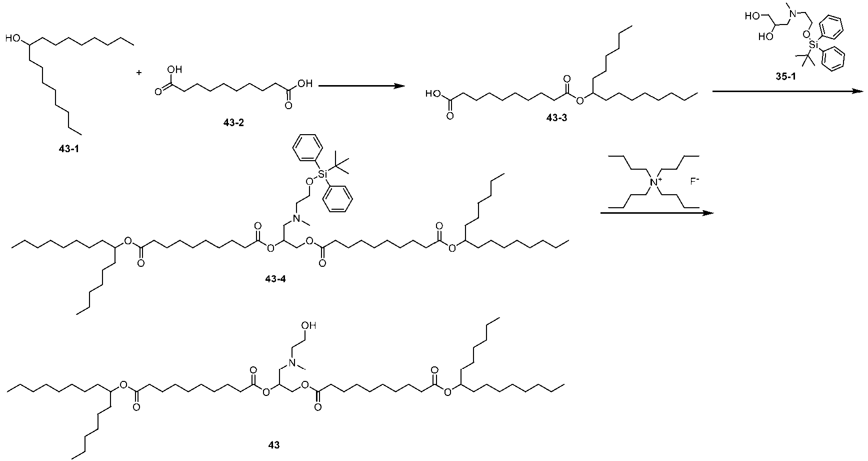

### Step 1: synthesis of compound 43-3

Sebacic acid (43-2, 6.79 g, 39 mmol), DCC (8.04 g, 39 mmol), DMAP (0.95 g, 7.8 mmol), and DCM (150 mL) were added into a 250 mL flask, and the mixture was stirred at room temperature for 30 min. 9-Heptadecanol (43-1, 2.0 g, 7.8 mmol) was then added dropwise, and the mixture was stirred at room temperature overnight. The extent of the reaction was detected by TLC (50% EtOAc/*n*-heptane). After completion of the reaction, the mixture was filtered, and the filtrate was washed with 30 mL of water and 30 mL of saturated brine, dried over anhydrous Na₂SO₄, and filtered. The organic phase was concentrated to give a crude product (about 6 g). The crude product was purified and separated using a Flash column (100 g of silica gel; the mobile phases were respectively: 100 mL of *n*-heptane; 200 mL of 1% EtOAc + 99% n-heptane; 200 mL of 2% EtOAc + 98% n-heptane; 200 mL of 8% EtOAc + 92% n-heptane; 200 mL of 20% EtOAc + 80% n-heptane; and 1000 mL of 50% EtOAc + 50% n-heptane) to give a pure product (2.6 g, 81% yield).

### Step 2: synthesis of compound 43-4

43-3 (500 mg, 1.2 mmol), DCC (250 mg, 1.2 mmol), DMAP (74 mg, 0.6 mmol), and DCM (3 mL) were added into a 25 mL flask, and the mixture was stirred at room temperature for 30 min. B048n-4 (234.8 mg, 0.6 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 900 mg). The crude product was purified and separated using a Flash column (50 g of silica gel; the mobile phases were respectively: 100 mL of *n*-heptane; 100 mL of 1% EtOAc + 99% n-heptane; 100 mL of 2% EtOAc + 98% *n*-heptane; and 300 mL of 5% EtOAc + 95% n-heptane) to give a pure product (430 mg, 60% yield). ¹H NMR (400 MHz, CDCl₃): *δ* 7.68-7.65 (m, 4H), 7.42-7.36 (m, 6H), 5.13-5.12 (m, 1H), 4.88-4.85 (m, 2H), 4.34-4.30 (m, 1H), 4.10-4.05 (m, 1H), 3.71 (t, *J*=4Hz, 2H), 2.63-2.55 (m, 4H), 2.30-2.24 (m, 11H), 1.64-1.48 (m, 18H), 1.31-1.19 (m, 60H), 1.04 (s, 9H), 0.89-0.86 (m, 12H).

### Step 3: synthesis of compound 43

43-4 (440 mg, 0.37 mmol) and THF (3 mL) were added into a 25 mL flask, and the mixture was cooled in an ice bath to 0-5 °C. TBAF (293.3 mg, 1.12 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (50 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 5% EtOAc + 95% *n*-heptane; 100 mL of 20% EtOAc + 80% *n*-heptane; and 400 mL of 50% EtOAc + 50% *n*-heptane) to give a pure product (280 mg, 80% yield). ¹H NMR (400 MHz, CDCl₃): *δ* 5.22-5.19 (m, 1H), 4.89-4.83 (m, 2H), 4.34-4.31 (m, 1H), 4.12-4.08 (m, 1H), 3.58 (t, *J*=4Hz, 2H), 2.65-2.58 (m, 4H), 2.33-2.25 (m, 11H), 1.64-1.45 (m, 16H), 1.36-1.25 (m, 57 H), 0.89-0.85 (m, 12H).

### Synthesis of compound 44

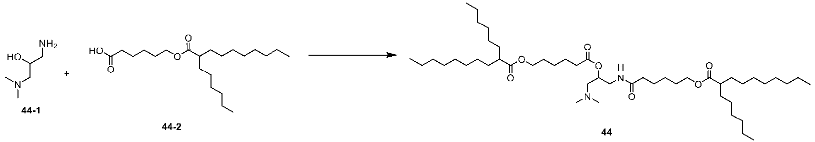

### Step 1: synthesis of compound 44

DMAP (65.9 mg, 0.54 mmol), DCC (222.7 mg, 1.1 mmol), and DCM (1.5 mL) were added into a 25 mL flask, and the mixture was stirred until it became clear. 44-2 (400 mg, 1.1 mmol) was added, and the mixture was stirred at room temperature for 30 min. 1-Amino-3-(dimethylamino)propanol (44-1, 63.8 mg, 0.55 mmol) was then added in one batch, and the mixture was stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 510 mg). The crude product was purified and separated using a Flash column (20 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 3% EtOAc + 97% *n*-heptane; 100 mL of 5% EtOAc + 95% *n*-heptane; 100 mL of 10% EtOAc + 90% *n*-heptane; 100 mL of 20% EtOAc + 80% *n*-heptane; and 300 mL of 50% EtOAc + 50% *n*-heptane) to give a pure product (300 mg, 67% yield). ¹H NMR (400 MHz, CDCl₃): *δ 6.32-*6.29 (m, 1H), 5.03-4.97 (m, 2H), 4.07-4.04 (m, 4H), 3.02 (m, 2H), 2.49-2.39 (m, 2H), 2.33-2.26 (m, 11H), 2.18-2.14 (m, 2H), 1.69-1.53 (m, 12H), 1.46-1.24 (m, 51H), 0.88-0.85 (m, 12H). Synthesis of compound 45

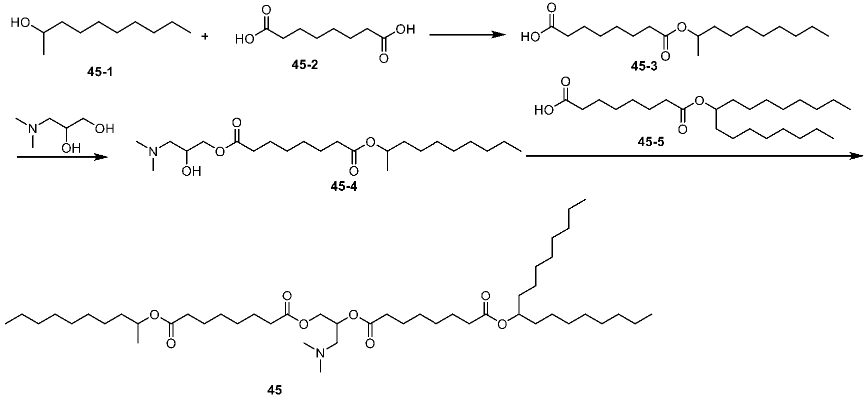

### Step 1: synthesis of compound 45-3

DMAP (1.54 g, 12.6 mmol), DCC (13.1 g, 63.2 mmol), and DCM (100 mL) were added into a 250 mL flask, and the mixture was stirred until it became clear. Suberic acid (45-2, 11 g, 63.2 mmol) was added, and the mixture was stirred at room temperature for 30 min. 2-Decanol (45-1, 2 g, 12.6 mmol) was then added in one batch, and the mixture was stirred at room temperature overnight. The extent of the reaction was detected by TLC (10% MeOH/DCM). After completion of the reaction, the reaction solution was filtered to remove solids, and the filtrate was washed with 50 mL of water and 50 mL of saturated brine, dried over anhydrous Na₂SO₄, and filtered. The organic phase was concentrated to give a crude product (about 5.3 g). The crude product was purified and separated using a Flash column (100 silica gel, loaded with 200 mL of *n*-heptane; the mobile phases were respectively: 200 mL of 1% EtOAc + 99% *n*-heptane; 200 mL of 2% EtOAc + 98% *n*-heptane; 200 mL of 3% EtOAc + 97% n-heptane; 200 mL of 5% EtOAc + 95% *n*-heptane; 200 mL of 10% EtOAc + 90% *n*-heptane; and 1000 mL of 20% EtOAc + 80% *n*-heptane) to give a pure product (3.4 g, 85% yield).

### Step 2: synthesis of compound 45-4

45-3 (500 mg, 1.6 mmol), DCC (328.1 mg, 1.6 mmol), DMAP (97.1 mg, 0.8 mmol), and DCM (5 mL) were added into a 25 mL flask, and the mixture was stirred at room temperature for 30 min. 3-Dimethylamino-1,2-propanediol (189.5 mg, 1.6 mmol) was then added, and the mixture was stirred at room temperature overnight. The extent of the reaction was detected by TLC (10% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 700 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane; and 300 mL of 10% MeOH + 90% DCM) to give a pure product (480 mg, 73% yield).

### Step 3: synthesis of compound 45

DMAP (29.4 mg, 0.24 mmol), DCC (99.3 mg, 0.48 mmol), and DCM (1 mL) were added into a 25 mL flask, and the mixture was stirred until it became clear. 45-5 (198.6 mg, 0.48 mmol) was added, and the mixture was stirred at room temperature for 30 min. 45-4 (200 mg, 0.48 mmol) was then added in one batch, and the mixture was stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 500 mg). The crude product was purified and separated using a Flash column (20 g of silica gel, loaded with 100 mL of *n-*heptane; the mobile phases were respectively: 100 mL of 1% EtOAc + 99% *n*-heptane; 100 mL of 2% EtOAc + 98% *n*-heptane; 100 mL of 3% EtOAc + 97% *n*-heptane; 100 mL of 5% EtOAc + 95% *n*-heptane; 100 mL of 10% EtOAc + 90% *n*-heptane; 100 mL of 20% EtOAc + 80% *n-*heptane; and 300 mL of 50% EtOAc + 50% *n*-heptane) to give a pure product (110 mg, 28% yield). ¹H NMR (400 MHz, CDCl₃): *δ* 5.21-5.18 (m, 1H), 4.91-4.84 (m, 2H), 4.37-4.34 (m, 1H), 4.10-4.06 (m, 1H), 2.47 (m, 2H), 2.33-2.24 (m, 14H), 2.33-2.26 (m, 11H), 1.65-1.41 (m, 15H), 1.37-1.18 (m, 51H), 0.89-0.85 (m, 12H).

### Synthesis of compound 46

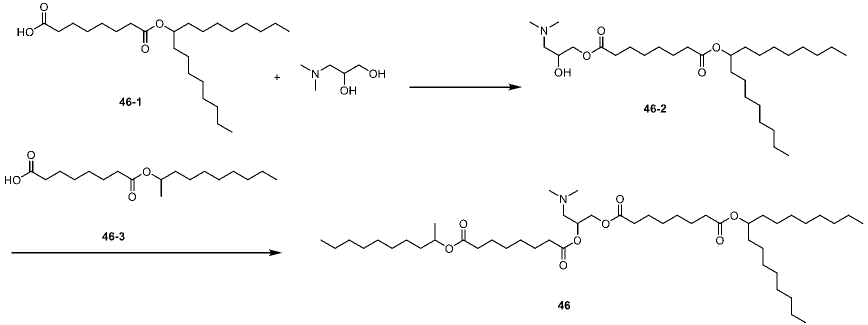

### Step 1: synthesis of compound 46-2

46-1 (500 mg, 1.2 mmol), DCC (250 mg, 1.2 mmol), DMAP (74 mg, 0.6 mmol), and DCM (5 mL) were added into a 25 mL flask, and the mixture was stirred at room temperature for 30 min. 3-Dimethylamino-1,2-propanediol (144.4 mg, 1.2 mmol) was then added, and the mixture was stirred at room temperature overnight. The extent of the reaction was detected by TLC (10% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 700 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n-*heptane; 100 mL of 20% EtOAc + 80% *n*-heptane; and 300 mL of 10% MeOH + 90% DCM) to give a pure product (380 mg, 61% yield).

### Step 2: synthesis of compound 46

DMAP (23.4 mg, 0.19 mmol), DCC (80.3 mg, 0.39 mmol), and DCM (1 mL) were added into a 25 mL flask, and the mixture was stirred until it became clear. 46-3 (122.4 mg, 0.39 mmol) was added, and the mixture was stirred at room temperature for 30 min. 46-2 (200 mg, 0.39 mmol) was then added in one batch, and the mixture was stirred at room temperature overnight. The extent of the reaction was detected by TLC (10% MeOH/DCM). After completion of the reaction, the reaction solution was filtered to remove solids, and the filtrate was washed with 5 mL of water and 5 mL of saturated brine, dried over anhydrous Na₂SO₄, and filtered. The organic phase was concentrated to give a crude product (about 500 mg). The crude product was purified and separated using a Flash column (20 g of silica gel, loaded with 100 mL of *n-*heptane; the mobile phases were respectively: 100 mL of 1% EtOAc + 99% *n*-heptane; 100 mL of 2% EtOAc + 98% *n*-heptane; 100 mL of 3% EtOAc + 97% *n*-heptane; 100 mL of 5% EtOAc + 95% *n*-heptane; 100 mL of 10% EtOAc + 90% *n*-heptane; 100 mL of 20% EtOAc + 80% *n-*heptane; and 300 mL of 50% EtOAc + 50% *n*-heptane) to give a pure product (150 mg, 48% yield). ¹H NMR (400 MHz, CDCl₃): *δ* 5.21-5.19 (m, 1H), 4.91-4.84 (m, 2H), 4.38-4.34 (m, 1H), 4.10-4.06 (m, 1H), 2.47 (m, 2H), 2.33-2.24 (m, 14H), 1.65-1.41 (m, 16H), 1.38-1.18 (m, 53H), 0.89-0.86 (m, 9H).

### Synthesis of compound 47

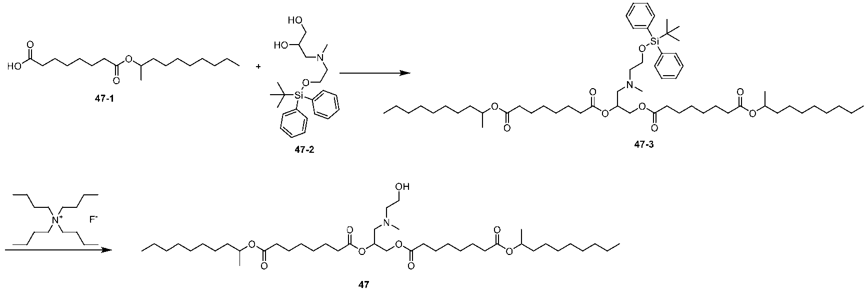

### Step 1: synthesis of compound 47-3

47-1 (405.7 mg, 1.3 mmol), DCC (266.2 mg, 1.3 mmol), DMAP (78.9 mg, 0.65 mmol), and DCM (5 mL) were added into a 25 mL flask, and the mixture was stirred at room temperature for 30 min. 47-2 (250 mg, 0.65 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 900 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% n-heptane; and 500 mL of 10% EtOAc + 90% *n*-heptane) to give a pure product (480 mg, 76% yield). ¹H NMR (400 MHz, CDCl₃): *δ* 7.68-5.65 (m, 4H), 7.42-7.38 (m, 6H), 5.12 (m, 1H), 4.91-4.86 (m, 2H), 4.35-4.31 (m, 1H), 4.09-4.05 (m, 1H), 3.72-3.69 (m, 2H), 2.61-2.54 (m, 5H), 2.28-2.23 (m, 11H), 1.62-1.45 (m, 14H), 1.33-1.18 (m, 42H), 1.04 (s, 9H), 0.89-0.86 (m, 6H).

### Step 2: synthesis of compound 47

47-3 (480 mg, 0.49 mmol) and THF (2 mL) were added into a 25 mL flask, and the mixture was cooled in an ice bath to 0-5 °C. TBAF (384 mg, 1.47 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (60 g of silica gel, loaded with 100 mL of *n*-heptane; the mobile phases were respectively: 100 mL of 1% EtOAc + 99% n-heptane; 100 mL of 2% EtOAc + 98% *n*-heptane; 100 mL of 5% EtOAc + 95% *n*-heptane; 100 mL of 10% EtOAc + 90% *n*-heptane; 100 mL of 40% EtOAc + 60% *n*-heptane; and 300 mL of 60% EtOAc + 40% *n*-heptane) to give a pure product (240 mg, 66% yield). ¹H NMR (400 MHz, CDCl₃): *δ* 5.23-5.17 (m, 1H), 4.92-4.84 (m, 2H), 4.34-4.30 (m, 1H), 4.11-4.02 (m, 1H), 3.58 (t, *J*=4Hz, 2H), 2.65-2.55 (m, 5H), 2.32-2.23 (m, 11H), 1.64-1.51 (m, 10H), 1.49-1.40 (m, 2H), 1.37-1.17 (m, 38H), 0.88-0.86 (m, 6H).

### Synthesis of compound 48

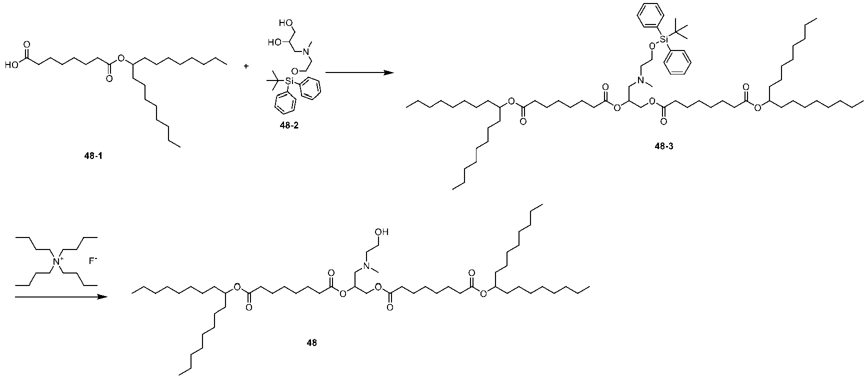

### Step 1: synthesis of compound 48-3

48-1 (425.9 mg, 1.03 mmol), DCC (212.9 mg, 1.03 mmol), DMAP (63 mg, 0.52 mmol), and DCM (2 mL) were added into a 25 mL flask, and the mixture was stirred at room temperature for 30 min. 48-2 (200 mg, 0.52 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 880 mg). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 500 mL of 10% EtOAc + 90% *n*-heptane) to give a pure product (500 mg, 82% yield). ¹H NMR (400 MHz, CDCl₃): *δ* 7.68-5.65 (m, 4H), 7.44-7.36 (m, 6H), 5.13-5.11 (m, 1H), 4.89-4.83 (m, 2H), 4.35-4.31 (m, 1H), 4.09-4.05 (m, 1H), 3.72-3.69 (m, 2H), 2.65-2.54 (m, 4H), 2.28-2.22 (m, 12H), 1.65-1.19 (m, 84H), 1.04 (s, 9H), 0.89-0.86 (m, 12H).

### Step 2: synthesis of compound 48

D020n-1 (500 mg, 0.42 mmol) and THF (2 mL) were added into a 25 mL flask, and the mixture was cooled in an ice bath to 0-5 °C. TBAF (333.3 mg, 1.27 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 600 mg). The crude product was purified and separated using a Flash column (20 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 5% EtOAc + 95% *n*-heptane; 100 mL of 10% EtOAc + 90% n-heptane; and 300 mL of 40% EtOAc + 60% n-heptane) to give a pure product (260 mg, 65% yield). ¹H NMR (400 MHz, CDCl₃): *δ* 5.24-5.19 (m, 1H), 4.88-4.82 (m, 2H), 4.35-4.31 (m, 1H), 4.12-4.08 (m, 1H), 3.58 (t, *J*=4Hz, 2H), 2.67-2.57 (m, 5H), 2.33-2.25 (m, 11H), 1.65-1.58 (m, 8H), 1.52-1.47 (m, 8H), 1.36-1.25 (m, 56H), 0.89-0.85 (m, 12H).

### Synthesis of compound 49

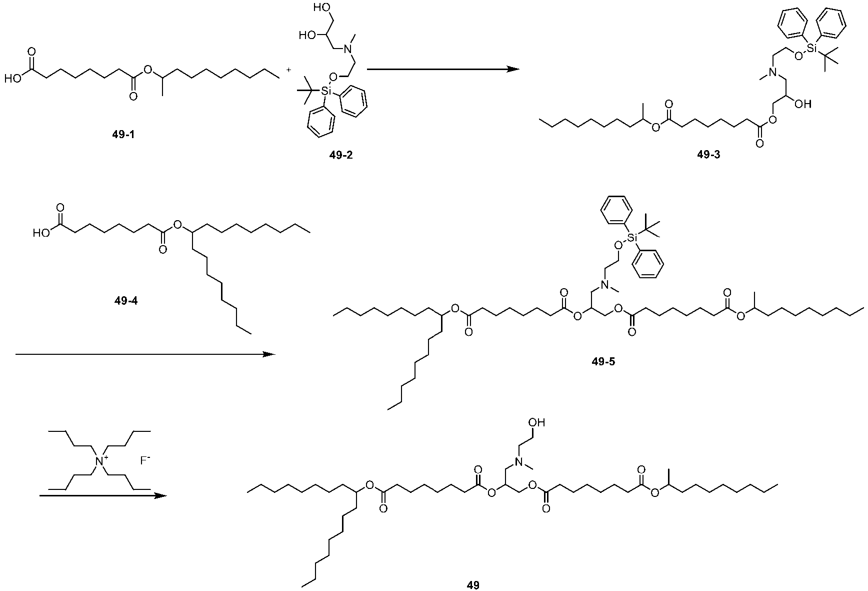

### Step 1: synthesis of compound 49-3

49-1 (500 mg, 1.6 mmol), DCC (328 mg, 1.6 mmol), DMAP (97 mg, 0.8 mmol), and DCM (5 mL) were added into a 25 mL flask, and the mixture was stirred at room temperature for 30 min. 49-2 (677.9 mg, 1.75 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 900 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 500 mL of 10% EtOAc + 90% *n*-heptane) to give a pure product (540 mg, 49% yield). ¹H NMR (400 MHz, CDCl₃): *δ* 7.68-7.65 (m, 4H), 7.45-7.37 (m, 6H), 4.93-4.85 (m, 1H), 4.16-4.12 (m, 1H), 4.01-3.97 (m, 1H), 3.90-3.84 (m, 1H), 2.79-2.69 (m, 2H), 2.75-2.69 (m, 1H), 2.63-2.55 (m, 1H), 2.48-2.46 (m, 1H), 2.36-2.32 (m, 5H), 2.28-2.24 (m, 2H), 1.65-1.58 (m, 5H), 1.48-1.41 (m, 1H), 1.35-1.26 (m, 20H), 1.20-1.18 (m, 3H), 1.05 (s, 9H), 0.90-0.86 (m, 6H).

### Step 2: synthesis of compound 49-5

49-4 (325.8 mg, 0.79 mmol), DCC (162.9 mg, 0.79 mmol), DMAP (96.4 mg, 0.79 mmol), and DCM (2 mL) were added into a 25 mL flask, and the mixture was stirred at room temperature for 30 min. 49-3 (540 mg, 0.79 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 900 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 5% EtOAc + 95% *n*-heptane; 100 mL of 10% EtOAc + 90% *n*-heptane; and 500 mL of 20% EtOAc + 80% *n*-heptane) to give a pure product (650 mg, 76% yield). ¹H NMR (400 MHz, CDCl₃): *δ* 7.68-7.65 (m, 4H), 7.42-7.36 (m, 6H), 5.14-5.11 (m, 1H), 4.91-4.84 (m, 2H), 4.35-4.31 (m, 1H), 4.09-4.05 (m, 1H), 3.72-3.69 (m, 2H), 2.63-2.55 (m, 4H), 2.30-2.24 (m, 12H), 1.64-1.42 (m, 16H), 1.37-1.18 (m, 56H), 1.04 (s, 9H), 0.90-0.86 (m, 12H).

### Step 3: synthesis of compound 49

49-5 (650 mg, 0.6 mmol) and THF (4 mL) were added into a 25 mL flask, and the mixture was cooled in an ice bath to 0-5 °C. TBAF (472.7 mg, 1.8 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (60 g of silica gel, loaded with 100 mL of *n*-heptane; the mobile phases were respectively: 100 mL of 1% EtOAc + 99% *n*-heptane; 100 mL of 2% EtOAc + 98% n-heptane; 100 mL of 5% EtOAc + 95% *n*-heptane; 100 mL of 10% EtOAc + 90% n-heptane; and 500 mL of 40% EtOAc + 60% n-heptane) to give a pure product (380 mg, 75% yield). ¹H NMR (400 MHz, CDCl₃): *δ* 5.24-5.18 (m, 1H), 4.92-4.82 (m, 2H), 4.35-4.31 (m, 1H), 4.12-4.03 (m, 1H), 3.59 (t, *J*=4Hz, 2H), 2.68-2.58 (m, 4H), 2.37-2.24 (m, 11H), 1.65-1.14 (m, 16H), 1.35-1.18 (m, 50H), 0.89-0.85 (m, 9H).

### Synthesis of compound 50

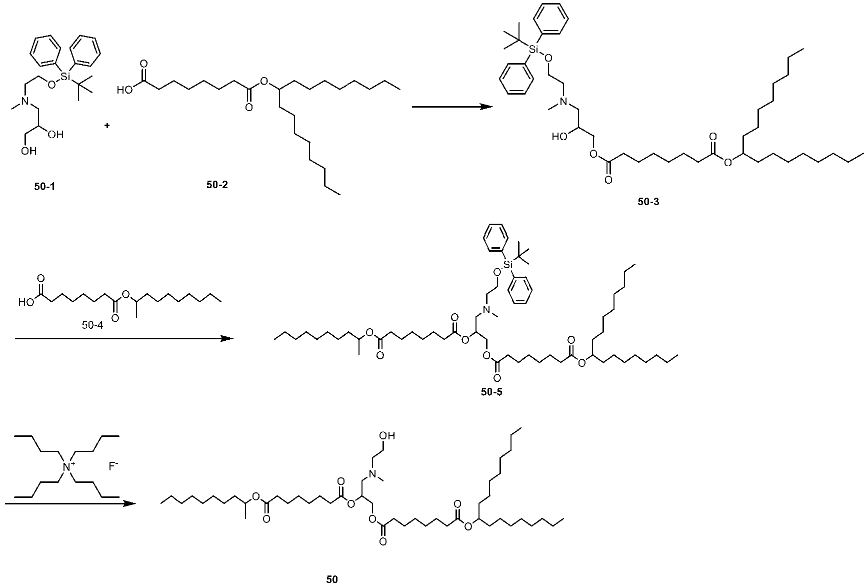

### Step 1: synthesis of compound 50-3

50-2 (532.3 mg, 1.3 mmol), DCC (266.2 mg, 1.3 mmol), DMAP (157.6 mg, 1.3 mmol), and DCM (5 mL) were added into a 25 mL flask, and the mixture was stirred at room temperature for 30 min. 50-1 (500 mg, 1.3 mmol) was then added dropwise, and the mixture was stirred at room temperature overnight. The extent of the reaction was detected by TLC (50% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 900 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane; and 400 mL of 50% EtOAc + 50% *n*-heptane) to give a pure product (550 mg, 54% yield).

### Step 2: synthesis of compound 50-5

50-4 (221.1 mg, 0.7 mmol), DCC (145.1 mg, 0.7 mmol), DMAP (43 mg, 0.35 mmol), and DCM (5 mL) were added into a 25 mL flask, and the mixture was stirred at room temperature for 30 min. 50-3 (550 mg, 0.7 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 900 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 5% EtOAc + 95% *n*-heptane; 100 mL of 10% EtOAc + 90% *n*-heptane; and 500 mL of 20% EtOAc + 80% *n*-heptane) to give a pure product (530 mg, 70% yield). ¹H NMR (400 MHz, CDCl₃): *δ* 7.68-7.65 (m, 4H), 7.44-7.36 (m, 6H), 5.13-5.11 (m, 1H), 4.91-4.84 (m, 2H), 4.35-4.31 (m, 1H), 4.09-4.05 (m, 1H), 3.72-3.69 (m, 2H), 2.67-2.55 (m, 4H), 2.29-2.23 (m, 12H), 1.65-1.42 (m, 16H), 1.37-1.18 (m, 50H), 1.04 (s, 9H), 0.89-0.86 (m, 9H).

### Step 3: synthesis of compound 50

50-5 (530 mg, 0.49 mmol) and THF (5 mL) were added into a 25 mL flask, and the mixture was cooled in an ice bath to 0-5 °C. TBAF (385.4 mg, 1.5 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 530 mg). The crude product was purified and separated using a Flash column (60 g of silica gel, loaded with 100 mL of n-heptane; the mobile phases were respectively: 100 mL of 1% EtOAc + 99% *n*-heptane; 100 mL of 2% EtOAc + 98% *n*-heptane; 100 mL of 5% EtOAc + 95% *n*-heptane; 100 mL of 10% EtOAc + 90% *n*-heptane; and 500 mL of 30% EtOAc + 70% *n*-heptane) to give a pure product (330 mg, 80% yield). ¹H NMR (400 MHz, CDCl₃): *δ* 5.24-5.19 (m, 1H), 4.91-4.82 (m, 2H), 4.35-4.31 (m, 1H), 4.12-4.08 (m, 1H), 3.59 (t, *J*=4Hz, 2H), 2.68-2.59 (m, 4H), 2.34-2.24 (m, 11H), 1.64-1.47 (m, 15H), 1.35-1.18 (m, 50H), 0.89-0.85 (m, 9H).

### Synthesis of compound 51

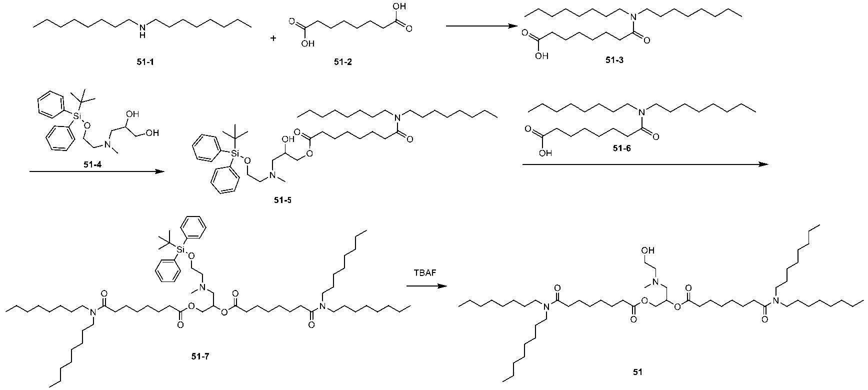

### Step 1: synthesis of compound 51-3

Suberic acid (51-2, 7.21 g, 41.4 mmol), DCC (8.55 g, 41.4 mmol), DMAP (1.01 g, 8.28 mmol), and DCM (100 mL) were added into a 250 mL flask, and the mixture was stirred at room temperature for 30 min. Di-n-octylamine (51-1, 2 g, 8.28 mmol) was then added dropwise, and the mixture was stirred at room temperature overnight. The extent of the reaction was detected by TLC (5% MeOH/DCM). After completion of the reaction, the mixture was filtered, and the filtrate was washed with 15 mL of water and 15 mL of saturated brine, dried over anhydrous Na₂SO₄, and filtered. The organic phase was concentrated to give a crude product (about 4 g). The crude product was purified and separated using a Flash column (120 g of silica gel; the mobile phases were respectively: 150 mL of *n*-heptane; 150 mL of 1% EtOAc + 99% *n*-heptane; 150 mL of 2% EtOAc + 98% *n*-heptane; 200 mL of 8% EtOAc + 92% *n*-heptane; 200 mL of 20% EtOAc + 80% *n*-heptane; and 1000 mL of 50% EtOAc + 50% *n*-heptane) to give a pure product (2.7 g, 82% yield).

### Step 2: synthesis of compound 51-5

51-3 (1.7 g, 4.3 mmol), DCC (882.1 mg, 4.3 mmol), DMAP (522.3 mg, 4.3 mmol), and DCM (10 mL) were added into a 25 mL flask, and the mixture was stirred at room temperature for 30 min. 51-4 (1.66 g, 4.3 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 2.8 g). The crude product was purified and separated using a Flash column (100 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 20% EtOAc + 80% *n*-heptane; and 500 mL of 50% EtOAc + 50% *n*-heptane) to give a pure product (1.6 g, 48% yield).

### Step 3: synthesis of compound 51-7

51-5 (259.1 mg, 0.65 mmol), DCC (134.5 mg, 0.65 mmol), DMAP (79.6 mg, 0.65 mmol), and DCM (3 mL) were added into a 25 mL flask, and the mixture was stirred at room temperature for 30 min. 51-6 (500 mg, 0.65 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 800 mg). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 5% EtOAc + 95% *n*-heptane; and 500 mL of 10% EtOAc + 90% *n*-heptane) to give a pure product (430 mg, 58% yield).

### Step 3: synthesis of compound 51

51-7 (430 mg, 0.37 mmol) and THF (3 mL) were added into a 25 mL flask, and the mixture was cooled in an ice bath to 0-5 °C. TBAF (294.1 mg, 1.12 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 530 mg). The crude product was purified and separated using a Flash column (50 g of silica gel, loaded with 100 mL of n-heptane; the mobile phases were respectively: 100 mL of 1% EtOAc + 99% *n-*heptane; 100 mL of 2% EtOAc + 98% *n*-heptane; 100 mL of 5% EtOAc + 95% *n*-heptane; 100 mL of 10% EtOAc + 90% *n*-heptane; and 400 mL of 30% EtOAc + 70% *n*-heptane) to give a pure product (200 mg, 80% yield). ¹H NMR (400 MHz, CDCl₃): *δ* 5.25-5.19 (m, 1H), 4.34-4.30 (m, 1H), 4.13-4.08 (m, 1H), 3.62-3.60 (m, 2H), 3.29-3.16 (m, 8H), 2.65 (m, 4H), 2.36-2.25 (m, 11H), 1.68-1.59 (m, 8H), 1.56-1.44 (m, 8H), 1.39-1.23 (m, 52H), 0.90-0.85 (m, 12H).

### Synthesis of compound 52

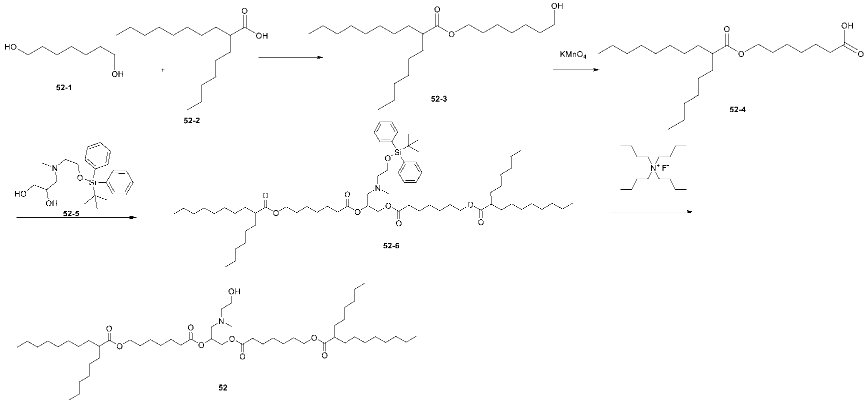

### Step 1: synthesis of compound 52-3

2-Hexyldecanoic acid (52-2, 5.8 g, 22.7 mmol), DCC (4.68 g, 22.7 mmol), DMAP (2.77 g, 22.7 mmol), and DCM (100 mL) were added into a 250 mL flask, and the mixture was stirred at room temperature for 30 min. 1,7-Heptanediol (52-1, 3.0 g, 22.7 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 100 mL of water and 100 mL of saturated brine, dried over anhydrous Na₂SO₄, and filtered. The organic phase was concentrated to give a crude product (about 9 g). The crude product was purified and separated using a Flash column (100 g of silica gel; the mobile phases were respectively: 100 mL of *n*-heptane; 200 mL of 1% EtOAc + 99% *n*-heptane; 200 mL of 2% EtOAc + 98% *n*-heptane; 200 mL of 8% EtOAc + 92% *n-*heptane; and 1500 mL of 15% EtOAc + 85% *n*-heptane) to give a pure product (7 g, 83% yield). Step 2: synthesis of compound 52-4
52-3 (3.0 g, 8.1 mmol), DCM (60 mL), acetic acid (18 mL), and tetrabutylammonium bromide (1.3 g, 4.05 mmol) were added into a 250 mL flask, and the mixture was cooled in an ice bath to 0-5 °C. A mixed solution of potassium permanganate (3.84 g, 24.3 mmol) and water (49 mL) was added dropwise with the temperature maintained at 0-5 °C, and after the dropwise addition, the mixture was stirred for 16 h. The extent of the reaction was detected by TLC (5% MeOH/DCM). After completion of the reaction, the reaction was quenched with a saturated aqueous sodium sulfite solution, and the mixture was extracted with DCM (50 mL × 2). The organic phases were combined, dried over anhydrous Na₂SO₄, filtered, and concentrated to give a crude product (about 3.2 g). The crude product was purified and separated using a Flash column (60 g of silica gel, loaded with 100 mL of *n*-heptane; the mobile phases were respectively: 100 mL of 1% EtOAc + 99% *n*-heptane; 100 mL of 2% EtOAc + 98% *n*-heptane; and 500 mL of 5% EtOAc + 95% *n*-heptane) to give a pure product (2 g, 65% yield).

### Step 3: synthesis of compound 52-6

52-4 (1.0 g, 2.6 mmol), DCC (536.5 mg, 2.6 mmol), DMAP (158.8 mg, 2.6 mmol), and DCM (5 mL) were added into a 25 mL flask, and the mixture was stirred at room temperature for 30 min. 52-5 (503.9 mg, 1.3 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 2 g). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; 100 mL of 15% EtOAc + 85% *n*-heptane; and 400 mL of 20% EtOAc + 80% *n*-heptane) to give a pure product (1 g, 68% yield). ¹H NMR (400 MHz, CDCl₃): *δ* 7.68-7.65 (m, 4H), 7.45-7.37 (m, 6H), 5.04 (m, 1H), 4.14-4.10 (m, 1H), 4.07-3.98 (m, 4H), 3.96-3.90 (m, 1H), 3.79-3.72 (m, 2H), 2.81-2.51 (m, 4H), 2.38-2.27 (m, 7H), 1.67-1.53 (m, 9H), 1.46-1.20 (m, 44H), 1.05 (s, 9H), 0.90-0.85 (m, 12H).

### Step 3: synthesis of compound 52

52-6 (350 mg, 0.31 mmol) and THF (3 mL) were added into a 25 mL flask, and the mixture was cooled in an ice bath to 0-5 °C. TBAF (245 mg, 0.94 mmol) was added dropwise with the temperature maintained at 0-5 °C, and the mixture was stirred at this temperature for 3 h. The extent of the reaction was detected by TLC (10% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 530 mg). The crude product was purified and separated using a Flash column (50 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; 100 mL of 30% EtOAc + 70% *n*-heptane; and 400 mL of 50% EtOAc + 50% *n*-heptane) to give a pure product (110 mg, 40% yield). ¹H NMR (400 MHz, CDCl₃): *δ* 4.18-4.10 (m, 2H), 4.06-4.03 (m, 6H), 3.78-3.70 (m, 8H), 3.32-3.29 (m, 1H), 2.81-2.54 (m, 6H), 2.44 (s, 4H), 2.37-2.26 (m, 6H), 1.70-1.52 (m, 13H), 1.47-1.24 (m, 54H), 1.02-0.99 (m, 2H), 0.88-0.84 (m, 12H).

### Synthesis of compound 53

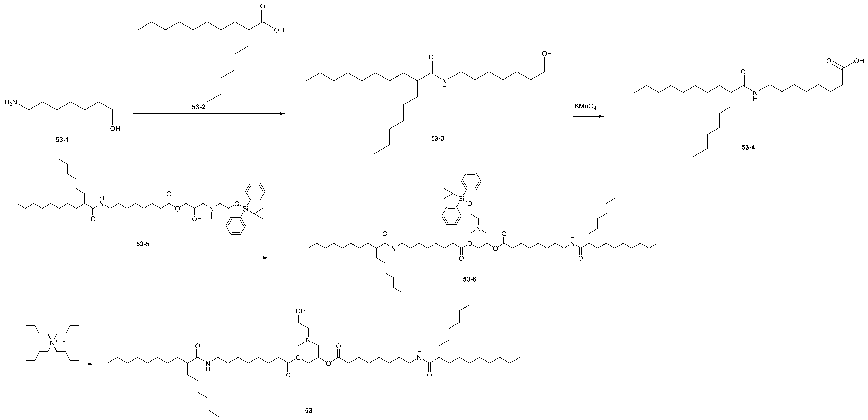

### Step 1: synthesis of compound 53-3

2-Hexyldecanoic acid (53-2, 2.93 g, 11.4 mmol), DCC (2.36 g, 11.4 mmol), DMAP (1.4 g, 11.4 mmol), and DCM (10 mL) were added into a 50 mL flask, and the mixture was stirred at room temperature for 30 min. 7-Aminoheptanol (53-1, 1.5 g, 11.4 mmol) was then added dropwise, and the mixture was stirred at room temperature overnight. The extent of the reaction was detected by TLC (30% EtOAc/*n*-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 3 g). The crude product was purified and separated using a Flash column (100 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 10% EtOAc + 90% *n*-heptane; 100 mL of 20% EtOAc + 80% *n*-heptane; and 700 mL of 50% EtOAc + 50% *n*-heptane) to give a pure product (1.8 g, 43% yield).

### Step 2: synthesis of compound 53-4

53-3 (2.3 g, 6 mmol), DCM (46 mL), acetic acid (14 mL), and tetrabutylammonium bromide (0.96 g, 3 mmol) were added into a 250 mL flask, and the mixture was cooled in an ice bath to 0-5 °C. A mixed solution of potassium permanganate (2.83 g, 17.9 mmol) and water (37 mL) was added dropwise with the temperature maintained at 0-5 °C, and after the dropwise addition, the mixture was stirred for 16 h. The extent of the reaction was detected by TLC (5% MeOH/DCM). After completion of the reaction, the reaction was quenched with a saturated aqueous sodium sulfite solution, and the mixture was extracted with DCM (50 mL × 2). The organic phases were combined, dried over anhydrous Na₂SO₄, filtered, and concentrated to give a crude product (about 3 g). The crude product was purified and separated using a Flash column (60 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; and 500 mL of 30% EtOAc + 70% n-heptane) to give a pure product (1.4 g, 59% yield).

### Step 3: synthesis of compound 53-6

53-4 (500 mg, 1.3 mmol), DCC (259.4 mg, 1.3 mmol), DMAP (153.6 mg, 1.3 mmol), and DCM (3 mL) were added into a 25 mL flask, and the mixture was stirred at room temperature for 30 min. 53-5 (913.1 mg, 1.3 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1 g). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% n-heptane; 100 mL of 8% EtOAc + 92% *n*-heptane; 100 mL of 15% EtOAc + 85% *n*-heptane; 100 mL of 20% EtOAc + 80% n-heptane; and 500 mL of 50% EtOAc + 50% *n*-heptane) to give a pure product (500 mg, 36% yield). ¹H NMR (400 MHz, CDCl₃): *δ* 7.67-7.63 (m, 4H), 7.44-7.36 (m, 6H), 5.61-5.59 (m, 1H), 4.13-4.04 (m, 2H), 3.73-3.70 (m, 2H), 3.53-3.34 (m, 4H), 2.99-2.92 (m, 1H), 2.77-2.54 (m, 3H), 2.33-2.19 (m, 6H), 1.97-1.91 (m, 2H), 1.71-1.13 (m, 80H), 1.05 (s, 9H), 0.89-0.84 (m, 12H).

### Step 3: synthesis of compound 53

53-6 (370 mg, 0.32 mmol) and THF (3 mL) were added into a 25 mL flask, and the mixture was cooled in an ice bath to 0-5 °C. TBAF (253 mg, 0.97 mmol) was added dropwise with the temperature maintained at 0-5 °C, and the mixture was stirred at this temperature for 3 h. The extent of the reaction was detected by TLC (10% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 230 mg). The crude product was purified and separated using a Flash column (20 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; 100 mL of 30% EtOAc + 70% *n*-heptane; and 200 mL of 50% EtOAc + 50% *n*-heptane) to give a pure product (90 mg, 31% yield). ¹H NMR (400 MHz, CDCl₃): *δ* 5.66-45.50 (m, 2H), 5.27-5.25 (m, 1H), 4.38-4.34 (m, 1H), 4.12-4.07 (m, 1H), 3.64-3.62 (m, 2H), 3.26-3.21 (m, 4H), 2.69 (m, 3H), 2.40-2.29 (m, 6H), 1.99-1.92 (m, 2H), 1.66-1.46 (m, 12H), 1.41-1.20(m, 56H), 0.89-0.85 (m, 12H).

### Synthesis of compound 54

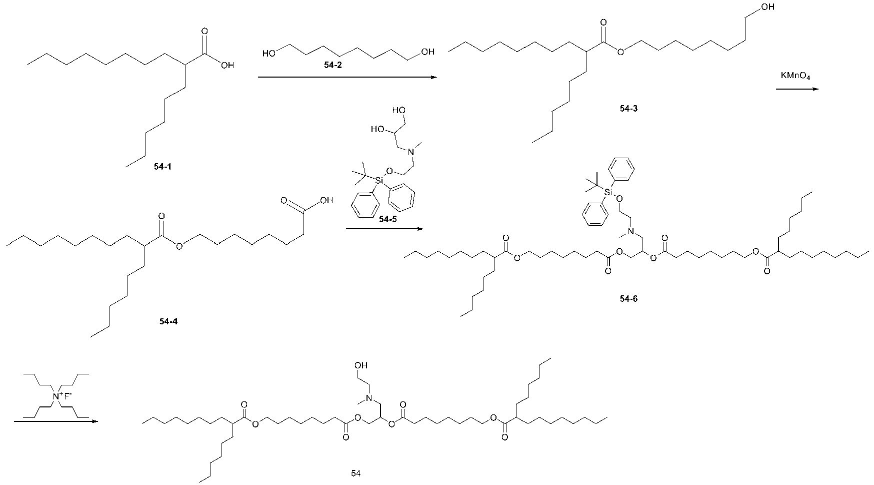

### Step 1: synthesis of compound 54-3

2-Hexyldecanoic acid (54-1, 10.5 g, 41 mmol), DCC (8.47 g, 41 mmol), DMAP (5 g, 41 mmol), and DCM (120 mL) were added into a 250 mL flask, and the mixture was stirred at room temperature for 30 min. 1,8-Octanediol (54-2, 6.0 g, 41 mmol) was then added dropwise, and the mixture was stirred at room temperature overnight. The extent of the reaction was detected by TLC (20% EtOAc/*n*-heptane). After completion of the reaction, the mixture was filtered, and the filtrate was washed with 100 mL of water and 100 mL of saturated brine, dried over anhydrous Na₂SO₄, and filtered. The organic phase was concentrated to give a crude product (about 9 g). The crude product was purified and separated using a Flash column (100 g of silica gel; the mobile phases were respectively: 100 mL of *n*-heptane; 200 mL of 1% EtOAc + 99% n-heptane; 200 mL of 2% EtOAc + 98% *n*-heptane; 200 mL of 8% EtOAc + 92% *n*-heptane; and 1500 mL of 15% EtOAc + 85% *n*-heptane) to give a pure product (7 g, 44% yield).

### Step 2: synthesis of compound 54-4

54-3 (1.0 g, 2.6 mmol), DCM (20 mL), acetic acid (6 mL), and tetrabutylammonium bromide (0.42 g, 1.3 mmol) were added into a 50 mL flask, and the mixture was cooled in an ice bath to 0-5 °C. A mixed solution of potassium permanganate (1.23 g, 7.8 mmol) and water (16 mL) was added dropwise with the temperature maintained at 0-5 °C, and after the dropwise addition, the mixture was stirred for 16 h. The extent of the reaction was detected by TLC (5% MeOH/DCM). After completion of the reaction, the reaction was quenched with a saturated aqueous sodium sulfite solution, and the mixture was extracted with DCM (50 mL × 2). The organic phases were combined, dried over anhydrous Na₂SO₄, filtered, and concentrated to give a crude product (about 1.2 g). The crude product was purified and separated using a Flash column (30 g of silica gel, loaded with 100 mL of *n*-heptane; the mobile phases were respectively: 100 mL of 1% EtOAc + 99% *n*-heptane; 100 mL of 2% EtOAc + 98% *n*-heptane; and 500 mL of 5% EtOAc + 95% *n*-heptane) to give a pure product (700 mg, 70% yield).

### Step 3: synthesis of compound 54-6

54-4 (1.0 g, 2.5 mmol), DCC (517.6 mg, 2.5 mmol), DMAP (153.2 mg, 1.25 mmol), and DCM (5 mL) were added into a 25 mL flask, and the mixture was stirred at room temperature for 30 min. 54-5 (486.2 mg, 1.25 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.8 g). The crude product was purified and separated using a Flash column (50 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 300 mL of 15% EtOAc + 85% *n*-heptane) to give a pure product (760 mg, 53% yield). ¹H NMR (400 MHz, CDCl₃): *δ* 7.68-7.65 (m, 4H), 7.44-7.36 (m, 6H), 5.13 (m, 1H), 4.35-4.31 (m, 1H), 4.10-4.03 (m, 5H), 3.71 (m, 2H), 2.60-2.56 (m, 3H), 2.34-2.24 (m, 8H), 1.64-1.54 (m, 13H), 1.46-1.19 (m, 62H), 1.04 (s, 9H), 0.89-0.85 (m, 12H).

### Step 4: synthesis of compound 54

54-6 (750 mg, 0.65 mmol) and THF (8 mL) were added into a 50 mL flask, and the mixture was cooled in an ice bath to 0-5 °C. TBAF (512.1 mg, 1.96 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 830 mg). The crude product was purified and separated using a Flash column (50 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; and 400 mL of 20% EtOAc + 80% n-heptane) to give a pure product (350 mg, 59% yield). ¹H NMR (400 MHz, CDCl₃): *δ* 5.25-5.19 (m, 1H), 4.35-4.31 (m, 1H), 4.12-4.03 (m, 5H), 3.59 (t, J=4Hz, 2H), 2.69-2.58 (m, 4H), 2.35-2.26 (m, 10H), 1.63-1.53 (m, 12H), 1.46-1.19 (m, 56H), 0.88-0.82 (m, 12H).

### Synthesis of compound 55

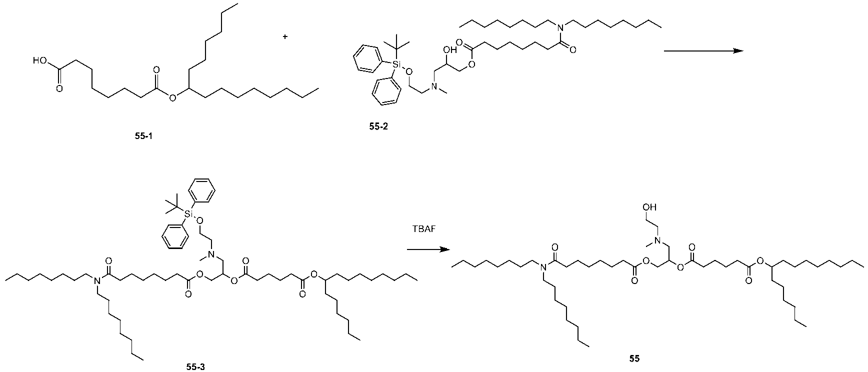

### Step 1: synthesis of compound 55-3

55-1 (232.4 mg, 0.65 mmol), DCC (134.5 mg, 0.65 mmol), DMAP (79.62 mg, 0.65 mmol), and DCM (3 mL) were added into a 25 mL flask, and the mixture was stirred at room temperature for 30 min. 55-2 (500 mg, 0.65 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.2 g). The crude product was purified and separated using a Flash column (60 g of silica gel; the mobile phases were respectively: 100 mL of *n*-heptane; 100 mL of 1% EtOAc + 99% *n*-heptane; 100 mL of 2% EtOAc + 98% *n*-heptane; and 500 mL of 8% EtOAc + 92% *n*-heptane) to give a pure product (550 mg, 76% yield). ¹H NMR (400 MHz, CDCl₃): *δ* 7.67-7.65 (m, 4H), 7.42-7.36 (m, 6H), 5.12 (m, 1H), 4.89-4.83 (m, 1H), 4.34-4.31 (m, 1H), 4.09-4.04 (m, 1H), 3.70-3.68 (m, 2H), 3.29-3.16 (m, 5H), 2.61-2.54 (m, 3H), 2.28-2.24 (m, 10H), 1.64-1.47 (m, 20H), 1.34-1.25 (m, 52H), 1.04 (s, 9H), 0.90-0.85 (m, 12H).

### Step 2: synthesis of compound 55

55-3 (550 mg, 0.5 mmol) and THF (3 mL) were added into a 25 mL flask, and the mixture was cooled in an ice bath to 0-5 °C. TBAF (390.2 mg, 1.5 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 530 mg). The crude product was purified and separated using a Flash column (50 g of silica gel, loaded with 100 mL of n-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 30% EtOAc + 70% *n*-heptane; and 400 mL of 50% EtOAc + 50% n-heptane) to give a pure product (250 mg, 58% yield). ¹H NMR (400 MHz, CDCl₃): *δ* 5.22-5.19 (m, 1H), 4.88-4.81 (m, 1H), 4.33-4.29 (m, 1H), 4.12-4.07 (m, 1H), 3.57 (t, *J*=4Hz, 2H), 3.28-3.16 (m, 4H), 2.66-2.56 (m, 4H), 2.35-2.24 (m, 10H), 1.65-1.44 (m, 15H), 1.36-1.24 (m, 48H), 0.89-0.84 (m, 12H).

### Synthesis of compound 56

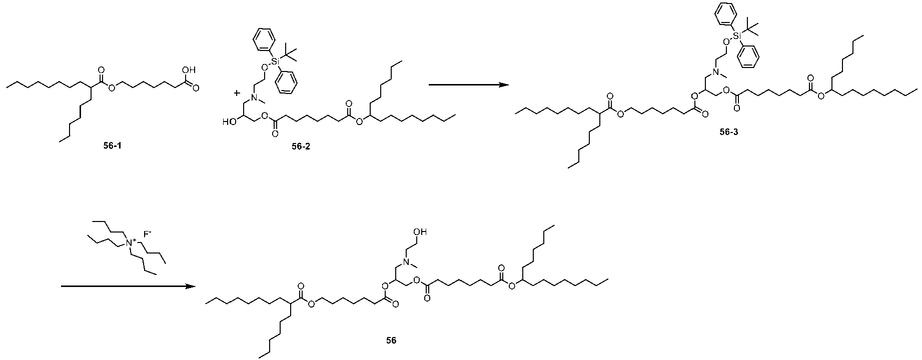

### Step 1: synthesis of compound 56-3

56-1 (200 mg, 0.52 mmol), DCC (107.3 mg, 0.52 mmol), DMAP (31.8 mg, 0.26 mmol), and DCM (2 mL) were added into a 25 mL flask, and the mixture was stirred at room temperature for 30 min. 56-2 (384.9 mg, 0.52 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1 g). The crude product was purified and separated using a Flash column (60 g of silica gel; the mobile phases were respectively: 100 mL of *n*-heptane; 100 mL of 2% EtOAc + 98% *n*-heptane; 100 mL of 8% EtOAc + 92% *n*-heptane; and 500 mL of 20% EtOAc + 80% *n*-heptane) to give a pure product (450 mg, 78% yield). ¹H NMR (400 MHz, CDCl₃): *δ* 7.67-7.65 (m, 4H), 7.44-7.26 (m, 6H), 5.13-5.12 (m, 1H), 4.89-4.83 (m, 1H), 4.35-4.31 (m, 1H), 4.09-4.03 (m, 3H), 3.72-3.69 (m, 2H), 2.65-2.55 (m, 3H), 2.34-2.22 (m, 10H), 1.65-1.19 (m, 72H), 1.04 (s, 9H), 0.89-0.85 (m, 12H).

### Step 2: synthesis of compound 56

56-3 (450 mg, 0.41 mmol) and THF (3 mL) were added into a 25 mL flask, and the mixture was cooled in an ice bath to 0-5 °C. TBAF (318.9 mg, 1.22 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 530 mg). The crude product was purified and separated using a Flash column (50 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; and 300 mL of 25% EtOAc + 75% *n*-heptane) to give a pure product (240 mg, 68% yield). ¹H NMR (400 MHz, CDCl₃): *δ* 5.23-5.18 (m, 1H), 4.88-4.82 (m, 1H), 4.35-4.31 (m, 1H), 4.12-4.03 (m, 1H), 3.58 (t, *J*=4Hz, 2H), 2.66-2.56 (m, 5H), 2.34-2.25 (m, 10H), 1.66-1.25 (m, 68H), 0.88-0.85 (m, 12H).

### Synthesis of compound 57

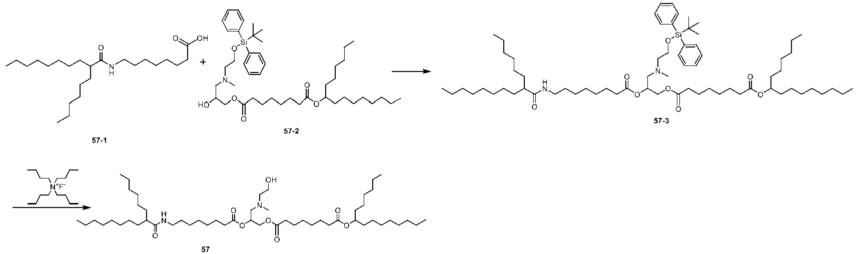

### Step 1: synthesis of compound 57-3

57-1 (500 mg, 1.26 mmol), DCC (259.4 mg, 1.26 mmol), DMAP (153.6 mg, 1.26 mmol), and DCM (5 mL) were added into a 25 mL flask, and the mixture was stirred at room temperature for 30 min. 57-2 (913.1 mg, 1.26 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1 g). The crude product was purified and separated using a Flash column (60 g of silica gel; the mobile phases were respectively: 100 mL of *n*-heptane; 100 mL of 2% EtOAc + 98% *n*-heptane; 100 mL of 8% EtOAc + 92% *n*-heptane; 100 mL of 20% EtOAc + 80% *n*-heptane; and 400 mL of 50% EtOAc + 50% *n*-heptane) to give a pure product (600 mg, 43% yield). ¹H NMR (400 MHz, CDCl₃): *δ* 7.68-7.65 (m, 4H), 7.42-7.35 (m, 6H), 5.13 (m, 1H), 4.87-4.84 (m, 1H), 4.35-4.31 (m, 1H), 4.09-4.05 (m, 1H), 3.72-3.69 (m, 2H), 3.26-3.21 (m, 2H), 2.61-2.55 (m, 3H), 2.29-2.24 (m, 10H), 1.67-1.46 (m, 15H), 1.39-1.19 (m, 56H), 1.03 (s, 9H), 0.89-0.85 (m, 12H).

### Step 2: synthesis of compound 57

57-3 (550 mg, 0.5 mmol) and THF (3 mL) were added into a 25 mL flask, and the mixture was cooled in an ice bath to 0-5 °C. TBAF (390.2 mg, 1.5 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (50 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 20% EtOAc + 80% *n*-heptane; and 300 mL of 50% EtOAc + 50% *n*-heptane) to give a pure product (200 mg, 46% yield). ¹H NMR (400 MHz, CDCl₃): *δ* 5.60-5.56 (m, 1H), 5.22-5.18 (m, 1H), 4.88-4.81 (m, 1H), 4.35-4.30 (m, 1H), 3.57 (t, *J*=4Hz, 2H), 3.26-3.21 (m, 2H), 2.64-2.56 (m, 5H), 2.32-2.25 (m, 10H), 1.64-1.19 (m, 72H), 0.88-0.84 (m, 12H).

### Synthesis of compound 58

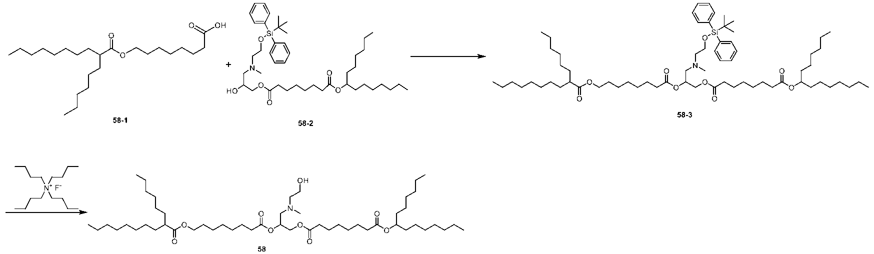

### Step 1: synthesis of compound 58-3

58-1 (300 mg, 0.75 mmol), DCC (155.3 mg, 0.75 mmol), DMAP (91.9 mg, 0.75 mmol), and DCM (3 mL) were added into a 25 mL flask, and the mixture was stirred at room temperature for 30 min. 58-2 (557 mg, 0.75 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 700 mg). The crude product was purified and separated using a Flash column (50 g of silica gel; the mobile phases were respectively: 100 mL of *n*-heptane; 100 mL of 2% EtOAc + 98% *n*-heptane; 100 mL of 8% EtOAc + 92% *n*-heptane; and 500 mL of 20% EtOAc + 80% *n*-heptane) to give a pure product (500 mg, 59% yield). ¹H NMR (400 MHz, CDCl₃): *δ* 7.69-7.66 (m, 4H), 7.45-7.37 (m, 6H), 4.16-4.12 (m, 1H), 4.07-3.97 (m, 3H), 3.88-3.71 (m, 4H), 2.75-2.57 (m, 3H), 2.47-2.46 (m, 2H), 2.36-2.28 (m, 7H), 1.65-1.55 (m, 7H), 1.46-1.20 (m, 34H), 1.05 (s, 9H), 0.90-0.85 (m, 12H).

### Step 2: synthesis of compound 58

58-3 (520 mg, 0.46 mmol) and THF (3 mL) were added into a 25 mL flask, and the mixture was cooled in an ice bath to 0-5 °C. TBAF (363.9 mg, 1.39 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (50 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; and 300 mL of 20% EtOAc + 80% *n*-heptane) to give a pure product (230 mg, 56% yield). ¹H NMR (400 MHz, CDCl₃): *δ* 5.23-5.18 (m, 1H), 4.88-4.82 (m, 1H), 4.34-4.31 (m, 1H), 4.12-4.03 (m, 3H), 3.57 (t, *J*=4Hz, 2H), 2.68-2.56 (m, 5H), 2.33-2.25 (m, 10H), 1.65-1.25 (m, 72H), 0.88-0.84 (m, 12H).

### Synthesis of compound 59

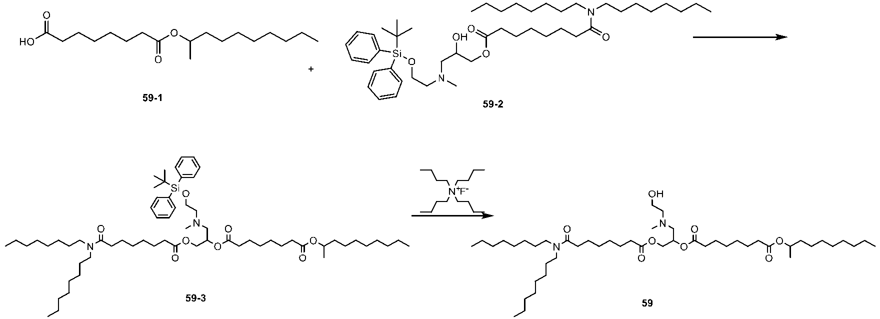

### Step 1: synthesis of compound 59-3

59-1 (204.9 mg, 0.65 mmol), DCC (134.5 mg, 0.65 mmol), DMAP (79.62 mg, 0.65 mmol), and DCM (3 mL) were added into a 25 mL flask, and the mixture was stirred at room temperature for 30 min. 59-2 (500 mg, 0.65 mmol) was then added dropwise, and the mixture was stirred at room temperature overnight. The extent of the reaction was detected by TLC (5% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1 g). The crude product was purified and separated using a Flash column (60 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% n-heptane; and 500 mL of 20% EtOAc + 80% *n*-heptane) to give a pure product (440 mg, 63% yield). ¹H NMR (400 MHz, CDCl₃): *δ* 7.67-7.65 (m, 4H), 7.44-7.36 (m, 6H), 4.16-4.12 (m, 1H), 5.12 (m, 1H), 4.91-4.86 (m, 1H), 4.34-4.30 (m, 1H), 4.09-4.04 (m, 1H), 3.71 (m, 2H), 3.29-3.16 (m, 4H), 2.60-2.55 (m, 4H), 2.29-2.23 (m, 10H), 1.66-1.42 (m, 17H), 1.37-1.18 (m, 50H), 1.04 (s, 9H), 0.90-0.85 (m, 9H).

### Step 2: synthesis of compound 59

59-3 (440 mg, 0.41 mmol) and THF (3 mL) were added into a 25 mL flask, and the mixture was cooled in an ice bath to 0-5 °C. TBAF (324.5 mg, 1.24 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 530 mg). The crude product was purified and separated using a Flash column (50 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; 100 mL of 30% EtOAc + 70% *n*-heptane; and 400 mL of 50% EtOAc + 50% *n*-heptane) to give a pure product (190 mg, 56% yield). ¹H NMR (400 MHz, CDCl₃): *δ* 5.23-5.18 (m, 1H), 4.90-4.85 (m, 1H), 4.34-4.30 (m, 1H), 4.12-4.07 (m, 3H), 3.59 (t, J=4Hz, 2H), 3.28-3.16 (m, 4H), 2.65-2.59 (m, 5H), 2.34-2.23 (m, 10H), 1.65-1.17 (m, 62H), 0.88-0.84 (m, 9H).

### Synthesis of compound 60

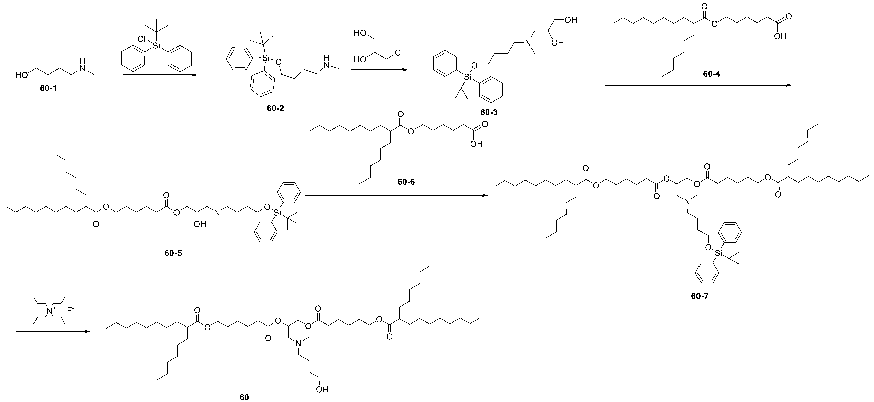

### Step 1: synthesis of compound 60-2

4-(Methylamino)butan-1-ol (60-1, 2.0 g, 19.4 mmol) and DCM (10 mL) were added into a 100 mL flask, and the solution was cooled in an ice-salt bath to 0-5 °C. A solution of TBDPSCl (5.33 g, 19.4 mmol) in DCM (5 mL) was slowly added dropwise to the cooled solution, and after the dropwise addition, the mixture was stirred at room temperature for 2 h. The extent of the reaction was detected by TLC (10% MeOH/DCM). After completion of the reaction, the reaction solution was concentrated to dryness to give a crude product (about 8 g). The crude product was purified and separated using a Flash column (70 g of silica gel, loaded with 100 mL of n-heptane; the mobile phases were respectively: 200 mL of *n*-heptane; 200 mL of 0.1% EtOAc + 99.9% *n*-heptane; 200 mL of 0.3% EtOAc + 99.7% *n*-heptane; 200 mL of 1% EtOAc + 99% *n*-heptane; 200 mL of 1% EtOAc + 99% n-heptane; 200 mL of 2% MeOH + 98% DCM; and 300 mL of 10% MeOH + 90% DCM) to give a pure product (5.5 g, 83% yield).

### Step 2: synthesis of compound 60-3

60-2 (5.0 g, 14.6 mmol), potassium carbonate (5.1 g, 36.6 mmol) and 3-chloro-1,2-propanediol (1.94 g, 17.6 mmol) dissolved in isopropanol (15 mL) were added into a 100 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 5.3 g). The crude product was purified and separated using a Flash column (60 g of silica gel; 100 mL of 0.1% MeOH + 99.9% DCM; 100 mL of 0.2% MeOH + 99.8% DCM; 100 mL of 0.3% MeOH + 99.7% DCM; and 300 mL of 0.5% MeOH + 99.5% DCM) to give a pure product (3.2 g, 53% yield).

### Step 3: synthesis of compound 60-5

60-4 (350 mg, 0.88 mmol), DCC (181.2 mg, 0.44 mmol), DMAP (182.5 mg, 0.44 mmol), and DCM (5 mL) were added into a 25 mL flask, and the mixture was stirred at room temperature for 30 min. 60-3 (486.2 mg, 1.25 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1 g). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 300 mL of 30% EtOAc + 70% *n*-heptane) to give a pure product (290 mg, 56% yield).

### Step 4: synthesis of compound 60-7

60-6 (145 mg, 0.36 mmol), DCC (75.2 mg, 0.36 mmol), DMAP (44.6 mg, 0.36 mmol), and DCM (5 mL) were added into a 25 mL flask, and the mixture was stirred at room temperature for 30 min. 60-5 (290 mg, 0.36 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 25 mL of water and 25 mL of saturated brine, dried over anhydrous Na₂SO₄, and filtered. The organic phase was concentrated to give a crude product (about 600 mg). The crude product was purified and separated using a Flash column (30 g of silica gel; the mobile phases were respectively: 100 mL of n-heptane; 100 mL of 1% EtOAc + 99% n-heptane; 100 mL of 2% EtOAc + 98% n-heptane; 100 mL of 8% EtOAc + 92% n-heptane; and 100 mL of 30% EtOAc + 70% n-heptane) to give a pure product (60 mg, 14% yield).

### Step 5: synthesis of compound 60

60-7 (550 mg, 0.47 mmol) and THF (3 mL) were added into a 25 mL flask, and the mixture was cooled in an ice bath to 0-5 °C. TBAF (366.6 mg, 1.4 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 830 mg). The crude product was purified and separated using a Flash column (50 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; and 400 mL of 30% EtOAc + 70% *n*-heptane) to give a pure product (200 mg, 46% yield). ¹H NMR (400 MHz, CDCl₃): *δ* 5.29-5.25 (m, 1H), 4.35-4.31 (m, 1H), 4.08-4.03 (m, 5H), 3.58-3.55 (m, 2H), 2.70-2.65 (m, 1H), 2.52-2.26 (m, 12H), 1.69-1.53 (m, 16H), 1.44-1.25 (m, 52H), 0.89-0.85 (m, 12H).

### Synthesis of compound 63

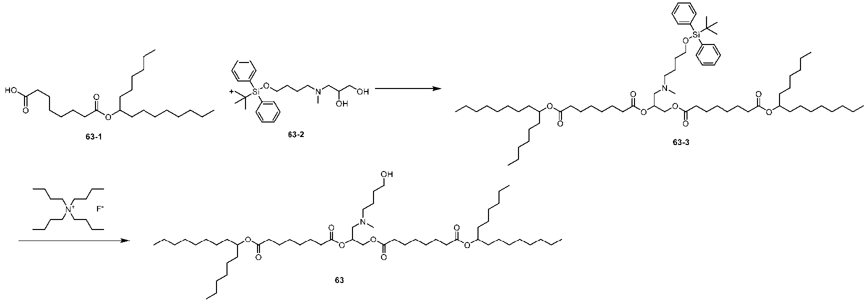

### Step 1: synthesis of compound 63-3

63-1 (200 mg, 0.52 mmol), DCC (107.3 mg, 0.52 mmol), DMAP (31.8 mg, 0.26 mmol), and DCM (5 mL) were added into a 25 mL flask, and the mixture was stirred at room temperature for 30 min. 63-2 (107.8 mg, 0.26 mmol) was then added, and the mixture was stirred at room temperature overnight. The extent of the reaction was detected by TLC (5% MeOH/DCM). After completion of the reaction, the mixture was filtered, and the filtrate was washed with 25 mL of water and 25 mL of saturated brine, dried over anhydrous Na₂SO₄, and filtered. The organic phase was concentrated to give a crude product (about 300 mg). The crude product was purified and separated using a Flash column (50 g of silica gel; the mobile phases were respectively: 100 mL of n-heptane; 100 mL of 2% EtOAc + 98% n-heptane; 100 mL of 8% EtOAc + 92% n-heptane; and 200 mL of 20% EtOAc + 80% n-heptane) to give a pure product (130 mg, 43% yield).

### Step 2: synthesis of compound 63

63-3 (130 mg, 0.11 mmol) and THF (3 mL) were added into a 25 mL flask, and the mixture was cooled in an ice bath to 0-5 °C. TBAF (88.8 mg, 0.33 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (50 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; and 200 mL of 20% EtOAc + 80% *n*-heptane) to give a pure product (60 mg, 60% yield). ¹H NMR (400 MHz, CDCl₃): *δ* 5.29-5.25 (m, 1H), 4.88-4.82 (m, 2H), 4.34-4.30 (m, 1H), 4.08-4.03 (m, 1H), 3.58-3.55 (m, 2H), 2.71-2.66 (m, 1H), 2.54-2.21 (m, 14H), 1.65-1.47 (m, 20H), 1.36-1.25 (m, 52H), 0.89-0.85 (m, 12H).

### Synthesis of compound 64

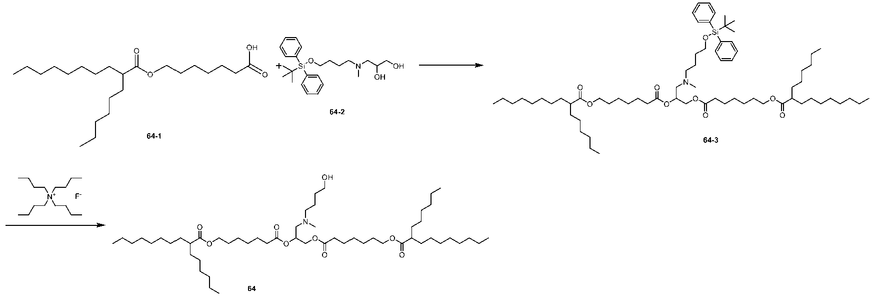

### Step 1: synthesis of compound 64-3

64-1 (400 mg, 1.04 mmol), DCC (214.6 mg, 0.52 mmol), DMAP (63.5 mg, 0.52 mmol), and DCM (5 mL) were added into a 25 mL flask, and the mixture was stirred at room temperature for 30 min. 64-2 (215.6 mg, 0.52 mmol) was then added, and the mixture was stirred at room temperature overnight. The extent of the reaction was detected by TLC (5% MeOH/DCM). After completion of the reaction, the mixture was filtered, and the filtrate was washed with 25 mL of water and 25 mL of saturated brine, dried over anhydrous Na₂SO₄, and filtered. The organic phase was concentrated to give a crude product (about 500 mg). The crude product was purified and separated using a Flash column (50 g of silica gel; the mobile phases were respectively: 100 mL of n-heptane; 100 mL of 2% EtOAc + 98% n-heptane; 100 mL of 8% EtOAc + 92% n-heptane; and 200 mL of 20% EtOAc + 80% n-heptane) to give a pure product (220 mg, 37% yield).

### Step 2: synthesis of compound 64

64-3 (220 mg, 0.19 mmol) and THF (3 mL) were added into a 25 mL flask, and the mixture was cooled in an ice bath to 0-5 °C. TBAF (150.2 mg, 0.57 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 200 mg). The crude product was purified and separated using a Flash column (50 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; and 200 mL of 20% EtOAc + 80% *n*-heptane) to give a pure product (80 mg, 46% yield). ¹H NMR (400 MHz, CDCl₃): *δ* 5.29-5.25 (m, 1H), 4.35-4.31 (m, 1H), 4.08-4.03 (m, 5H), 3.58-3.55 (m, 2H), 2.72-2.66 (m, 1H), 2.54-2.26 (m, 12H), 1.65-1.25 (m, 72H), 0.88-0.85 (m, 12H).

### Synthesis of compound 65

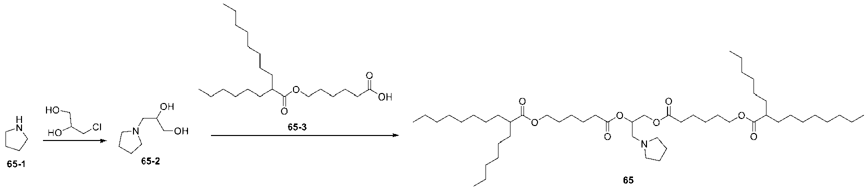

### Step 1: synthesis of compound 65-2

Pyrrolidine (65-1, 5.0 g, 70.3 mmol), potassium carbonate (24.3 g, 175.8 mmol) and 3-chloro-1,2-propanediol (7.77 g, 70.3 mmol) dissolved in isopropanol (50 mL) were added into a 250 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 1.5 g, 15% yield).

### Step 2: synthesis of compound 65

65-3 (1.0 g, 2.51 mmol), DCC (517.6 mg, 2.51 mmol), DMAP (153.2 mg, 1.25 mmol), and DCM (3 mL) were added into a 25 mL flask, and the mixture was stirred at room temperature for 30 min. 65-2 (182.1 mg, 1.25 mmol) was then added dropwise, and the mixture was stirred at room temperature overnight. The extent of the reaction was detected by TLC (20% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 600 mg). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 400 mL of 30% EtOAc + 70% n-heptane) to give a pure product (380 mg, 33% yield). ¹H NMR (400 MHz, CDCl₃): *δ* 5.24-5.19 (m, 1H), 4.40-4.37 (m, 1H), 4.12-4.04 (m, 5H), 2.65-2.55 (m, 6H), 2.34-2.28 (m, 6H), 1.77-1.53 (m, 17H), 1.46-1.19 (m, 51H), 0.89-0.85 (m, 12H).

### Synthesis of compound 66

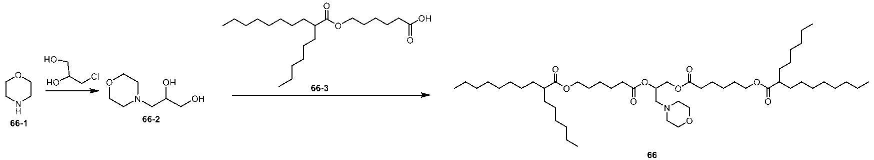

### Step 1: synthesis of compound 66-2

Morpholine (66-1, 5.0 g, 57.4 mmol), potassium carbonate (19.8 g, 143.5 mmol) and 3-chloro-1,2-propanediol (6.34 g, 57.4 mmol) dissolved in isopropanol (50 mL) were added into a 250 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 2.2 g, 24% yield).

### Step 2: synthesis of compound 66

66-3 (500 mg, 1.35 mmol), DCC (278.4 mg, 1.35 mmol), DMAP (82.4 mg, 0.67 mmol), and DCM (2 mL) were added into a 25 mL flask, and the mixture was stirred at room temperature for 30 min. 66-2 (108.75 mg, 0.67 mmol) was then added dropwise, and the mixture was stirred at room temperature overnight. The extent of the reaction was detected by TLC (20% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 600 mg). The crude product was purified and separated using a Flash column (30 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 200 mL of 30% EtOAc + 70% n-heptane) to give a pure product (300 mg, 51% yield). ¹H NMR (400 MHz, CDCl₃): *δ* 5.23 (m, 1H), 4.39-4.35 (m, 1H), 4.12-4.04 (m, 5H), 3.66 (m, 4H), 2.49 (m, 6H), 2.34-2.26 (m, 6H), 1.70-1.53 (m, 14H), 1.46-1.19 (m, 54H), 0.89-0.85 (m, 12H).

### Synthesis of compound 67

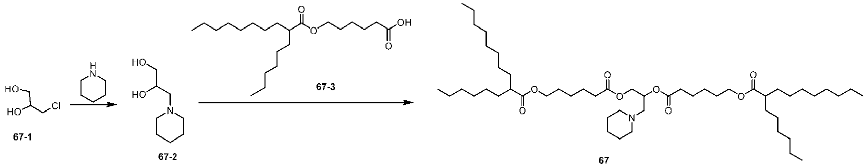

### Step 1: synthesis of compound 67-2

Piperidine (3.0 g, 35.2 mmol), potassium carbonate (12.2 g, 88.1 mmol) and 3-chloro-1,2-propanediol (67-1, 3.89 g, 35.2 mmol) dissolved in isopropanol (20 mL) were added into a 100 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 3.2 g, 57% yield).

### Step 2: synthesis of compound 67

67-3 (512 mg, 1.38 mmol), DCC (285.1 mg, 1.38 mmol), DMAP (84.4 mg, 0.69 mmol), and DCM (3 mL) were added into a 25 mL flask, and the mixture was stirred at room temperature for 30 min. 67-2 (110 mg, 0.69 mmol) was then added dropwise, and the mixture was stirred at room temperature overnight. The extent of the reaction was detected by TLC (20% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 700 mg). The crude product was purified and separated using a Flash column (20 g of silica gel; the mobile phases were respectively: 100 mL of n-heptane; 100 mL of 1% EtOAc + 99% n-heptane; 100 mL of 2% EtOAc + 98% n-heptane; 100 mL of 8% EtOAc + 92% n-heptane; and 200 mL of 20% EtOAc + 80% n-heptane) to give a pure product (250 mg, 42% yield). ¹H NMR (400 MHz, CDCl₃): *δ* 5.22 (m, 1H), 4.37-4.33 (m, 1H), 4.12-4.04 (m, 5H), 2.44-2.26 (m, 12H), 1.70-1.19 (m, 76H), 0.89-0.85 (m, 12H).

### Synthesis of compound 68

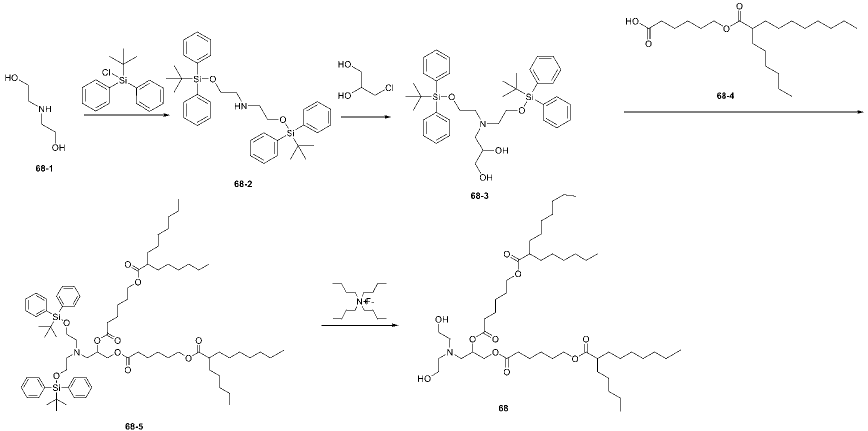

### Step 1: synthesis of compound 68-2

Diethanolamine (68-1, 2.0 g, 19 mmol) and DCM (20 mL) were added into a 100 mL flask, and the solution was cooled in an ice-salt bath to 0-5 °C. A solution of TBDPSCl (10.5 g, 38 mmol) in DCM (5 mL) was slowly added dropwise to the cooled solution, and after the dropwise addition, the mixture was stirred at room temperature for 2 h. The extent of the reaction was detected by TLC (20% MeOH/DCM). After completion of the reaction, the reaction solution was concentrated to dryness to give a crude product (about 20 g). The crude product was purified and separated using a Flash column (200 g of silica gel, loaded with 100 mL of *n-*heptane; the mobile phases were respectively: 200 mL of *n*-heptane; 200 mL of 0.1% EtOAc + 99.9% *n*-heptane; 200 mL of 0.3% EtOAc + 99.7% *n*-heptane; 200 mL of 1% EtOAc + 99% *n-*heptane; 200 mL of 1% EtOAc + 99% n-heptane; 200 mL of 2% MeOH + 98% DCM; and 300 mL of 10% MeOH + 90% DCM) to give a pure product (6 g, 54% yield).

### Step 2: synthesis of compound 68-3

68-2 (3.0 g, 5.2 mmol), potassium carbonate (1.8 g, 12.9 mmol) and 3-chloro-1,2-propanediol (0.57 g, 5.2 mmol) dissolved in isopropanol (10 mL) were added into a 50 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 4 g). The crude product was purified and separated using a Flash column (50 g of silica gel; 100 mL of 0.1% MeOH + 99.9% DCM; 100 mL of 0.2% MeOH + 99.8% DCM; 100 mL of 0.3% MeOH + 99.7% DCM; and 300 mL of 0.5% MeOH + 99.5% DCM) to give a pure product (2.5 g, 74% yield).

### Step 3: synthesis of compound 68-5

68-4 (564.9 mg, 1.5 mmol), DCC (314.5 mg, 1.5 mmol), DMAP (93.1 mg, 0.76 mmol), and DCM (5 mL) were added into a 25 mL flask, and the mixture was stirred at room temperature for 30 min. 68-3 (500 mg, 0.76 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.2 g). The crude product was purified and separated using a Flash column (12 g of silica gel; the mobile phases were respectively: 100 mL of *n*-heptane; 100 mL of 1% EtOAc + 99% *n*-heptane; 100 mL of 2% EtOAc + 98% *n*-heptane; and 100 mL of 8% EtOAc + 92% *n*-heptane) to give a pure product (680 mg, 67% yield).

### Step 4: synthesis of compound 68

68-5 (680 mg, 0.52 mmol) and THF (3 mL) were added into a 25 mL flask, and the mixture was cooled in an ice bath to 0-5 °C. TBAF (404.4 mg, 1.6 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (12 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 30% EtOAc + 70% *n*-heptane; and 1000 mL of 50% EtOAc + 50% *n*-heptane) to give a pure product (200 mg, 48% yield). ¹H NMR (400 MHz, CDCl₃): *δ* 5.29-5.25 (m, 1H), 4.35-4.31 (m, 1H), 4.08-4.03 (m, 5H), 3.58-3.55 (m, 2H), 2.70-2.56 (m, 1H), 2.52-2.26 (m, 12H), 1.69-1.53 (m, 16H), 1.44-1.22 (m, 50H), 0.89-0.85 (m, 12H).

### Synthesis of compound 70

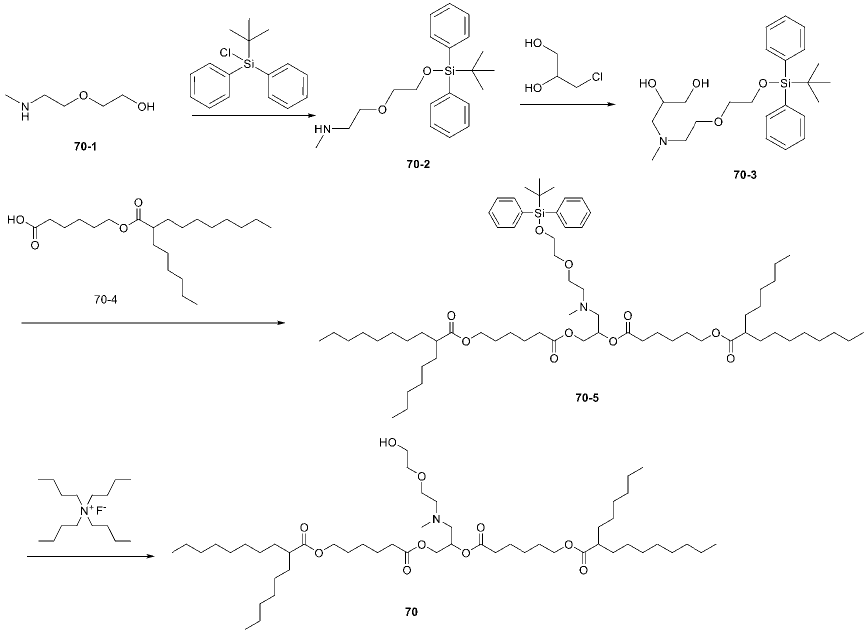

### Step 1: synthesis of compound 70-2

2-[2-(Methylamino)ethoxy]ethan-1-ol (70-1, 500 mg, 4.2 mmol) and DCM (3 mL) were added into a 25 mL flask, and the solution was cooled in an ice-salt bath to 0-5 °C. A solution of TBDPSCl (1.15 g, 4.2 mmol) in DCM (2 mL) was slowly added dropwise to the cooled solution, and after the dropwise addition, the mixture was stirred at room temperature for 2 h. The extent of the reaction was detected by TLC (20% MeOH/DCM). After completion of the reaction, the reaction solution was concentrated to dryness to give a crude product (about 2 g). The crude product was purified and separated using a Flash column (14 g of silica gel, loaded with 100 mL of *n*-heptane; the mobile phases were respectively: 100 mL of *n*-heptane; 100 mL of 0.1% EtOAc + 99.9% n-heptane; 100 mL of 0.3% EtOAc + 99.7% *n*-heptane; 100 mL of 1% EtOAc + 99% n-heptane; 100 mL of 1% EtOAc + 99% n-heptane; 100 mL of 2% MeOH + 98% DCM; and 300 mL of 10% MeOH + 90% DCM) to give a pure product (800 mg, 53% yield).

### Step 2: synthesis of compound 70-3

70-2 (690 mg, 1.9 mmol), potassium carbonate (666.7 mg, 4.8 mmol) and 3-chloro-1,2-propanediol (213.3 mg, 1.9 mmol) dissolved in isopropanol (5 mL) were added into a 25 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 1 g). The crude product was purified and separated using a Flash column (12 g of silica gel; 100 mL of 0.1% MeOH + 99.9% DCM; 100 mL of 0.2% MeOH + 99.8% DCM; 100 mL of 0.3% MeOH + 99.7% DCM; and 300 mL of 0.5% MeOH + 99.5% DCM) to give a pure product (500 mg, 60% yield).

### Step 3: synthesis of compound 70-5

70-4 (858.5 mg, 2.4 mmol), DCC (478 mg, 2.4 mmol), DMAP (141.5 mg, 1.2 mmol), and DCM (5 mL) were added into a 25 mL flask, and the mixture was stirred at room temperature for 30 min. 70-3 (500 mg, 1.2 mmol) was then added dropwise, and the mixture was stirred at room temperature overnight. The extent of the reaction was detected by TLC (20% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.5 g). The crude product was purified and separated using a Flash column (12 g of silica gel; the mobile phases were respectively: 100 mL of *n*-heptane; 100 mL of 1% EtOAc + 99% *n*-heptane; 100 mL of 2% EtOAc + 98% *n*-heptane; and 100 mL of 8% EtOAc + 92% *n*-heptane) to give a pure product (650 mg, 49% yield).

### Step 4: synthesis of compound 70

70-5 (650 mg, 0.57 mmol) and THF (3 mL) were added into a 25 mL flask, and the mixture was cooled in an ice bath to 0-5 °C. TBAF (448.5 mg, 1.7 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 700 mg). The crude product was purified and separated using a Flash column (12 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; and 100 mL of 30% EtOAc + 70% *n*-heptane) to give a pure product (250 mg, 49% yield). ¹H NMR (400 MHz, CDCl₃): *δ* 5.25-5.20 (m, 1H), 4.40-4.36 (m, 1H), 4.12-4.04 (m, 5H), 3.70-3.68 (m, 2H), 3.60-3.55 (m, 4H), 2.68-2.60 (m, 4H), 2.35-2.26 (m, 9H), 1.69-1.53 (m, 13H), 1.46-1.34 (m, 9H), 1.30-1.19 (m,44H), 0.88-0.85 (m, 12H).

### Synthesis of compound 76

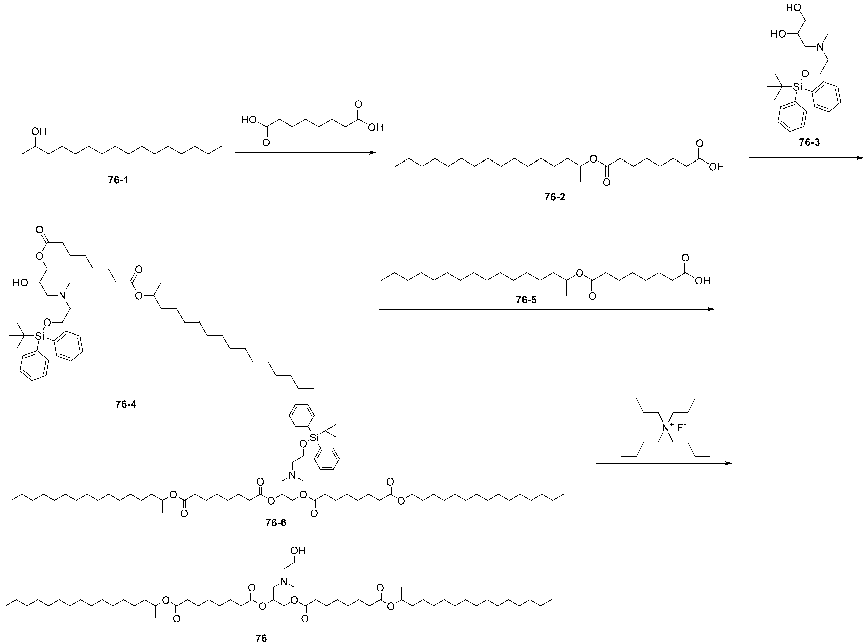

### Step 1: synthesis of compound 76-2

Suberic acid (3.6 g, 20.6 mmol), DCC (4.3 g, 20.6 mmol), DMAP (503.9 mg, 4.1 mmol), and DCM (10 mL) were added into a 25 mL flask, and the mixture was stirred at room temperature for 30 min. 2-Hexadecanol (76-1, 1 g, 4.1 mmol) was then added dropwise, and the mixture was stirred at room temperature overnight. The extent of the reaction was detected by TLC (30% EtOAc/*n*-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 2.5 g). The crude product was purified and separated using a Flash column (25 g of silica gel; the mobile phases were respectively: 100 mL of *n*-heptane; 100 mL of 1% EtOAc + 99% *n*-heptane; 100 mL of 2% EtOAc + 98% n-heptane; 100 mL of 8% EtOAc + 92% n-heptane; and 100 mL of 20% EtOAc + 80% n-heptane) to give a pure product (800 mg, 49% yield).

### Step 2: synthesis of compound 76-4

76-2 (300 mg, 0.56 mmol), DCC (116.5 mg, 0.56 mmol), DMAP (69 mg, 0.56 mmol), and DCM (2 mL) were added into a 25 mL flask, and the mixture was stirred at room temperature for 30 min. 76-3 (218.8 mg, 0.56 mmol) was then added dropwise, and the mixture was stirred at room temperature overnight. The extent of the reaction was detected by TLC (30% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 500 mg). The crude product was purified and separated using a Flash column (12 g of silica gel; the mobile phases were respectively: 100 mL of *n*-heptane; 100 mL of 1% EtOAc + 99% *n*-heptane; 100 mL of 2% EtOAc + 98% n-heptane; and 100 mL of 8% EtOAc + 92% n-heptane) to give a pure product (200 mg, 46% yield).

### Step 3: synthesis of compound 76-6

76-5 (138.4 mg, 0.26 mmol), DCC (53.7 mg, 0.26 mmol), DMAP (31.8 mg, 0.26 mmol), and DCM (3 mL) were added into a 25 mL flask, and the mixture was stirred at room temperature for 30 min. 76-4 (500 mg, 1.2 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 350 mg). The crude product was purified and separated using a Flash column (12 g of silica gel; the mobile phases were respectively: 100 mL of *n*-heptane; 100 mL of 1% EtOAc + 99% *n*-heptane; 100 mL of 2% EtOAc + 98% *n*-heptane; and 100 mL of 8% EtOAc + 92% *n*-heptane) to give a pure product (180 mg, 60% yield). ¹H NMR (400 MHz, CDCl₃): *δ* 7.68-7.64 (m, 4H), 7.44-7.36 (m, 6H), 5.14 (m, 1H), 4.93-4.85 (m, 2H), 4.35-4.31 (m, 1H), 4.09-4.05 (m, 1H), 3.72 (m, 2H), 2.62-2.58 (m, 4H), 2.31-2.23 (m, 11H), 1.63-1.52(m, 11H), 1.49-1.41 (m, 4H), 1,35-1.25 (m, 62H), 1.21-1.18 (m, 6H), 1.04 (s, 9H), 0.89-0.86 (m, 6H).

### Step 4: synthesis of compound 76

76-6 (180 mg, 0.16 mmol) and THF (3 mL) were added into a 25 mL flask, and the mixture was cooled in an ice bath to 0-5 °C. TBAF (122.9 mg, 0.47 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 200 mg). The crude product was purified and separated using a Flash column (4 g of silica gel, loaded with 100 mL of *n*-heptane; the mobile phases were respectively: 100 mL of 1% EtOAc + 99% *n-*heptane; 100 mL of 2% EtOAc + 98% *n*-heptane; 100 mL of 10% EtOAc + 90% *n*-heptane; and 400 mL of 30% EtOAc + 70% n-heptane) to give a pure product (80 mg, 56% yield). ¹H NMR (400 MHz, CDCl₃): *δ* 5.24-5.19 (m, 1H), 4.92-4.84 (m, 2H), 4.35-4.31 (m, 1H), 4.12-4.08 (m, 1H), 3.60-3.57 (m, 2H), 2.65-2.58 (m, 4H), 2.34-2.23 (m, 11H), 1.65-1.52(m, 11H), 1.48-1.18 (m, 71H), 0.89-0.86 (m, 6H).

### Synthesis of compound 78

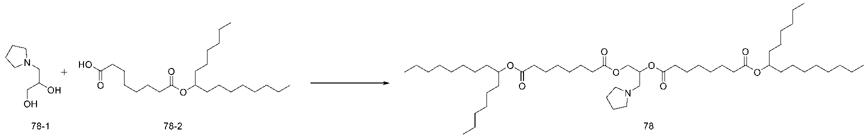

### Step 1: synthesis of compound 78

78-2 (529.8 mg, 1.38 mmol), DCC (284.2 mg, 1.38 mmol), DMAP (84.1 mg, 0.69 mmol), and DCM (3 mL) were added into a 25 mL flask, and the mixture was stirred at room temperature for 30 min. 78-1 (100 mg, 0.69 mmol) was then added dropwise, and the mixture was stirred at room temperature overnight. The extent of the reaction was detected by TLC (20% EtOAc/n-heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 800 mg). The crude product was purified and separated using a Flash column (10 g of silica gel; the mobile phases were respectively: 100 mL of *n*-heptane; 100 mL of 1% EtOAc + 99% *n*-heptane; 100 mL of 2% EtOAc + 98% *n*-heptane; 100 mL of 8% EtOAc + 92% *n*-heptane; and 200 mL of 20% EtOAc + 80% *n*-heptane) to give a pure product (200 mg, 33% yield). ¹H NMR (400 MHz, CDCl₃): *δ* 5.23-5.18 (m, 1H), 4.89-4.83 (m, 2H), 4.39-4.36 (m, 1H), 4.12-4.07 (m, 1H), 2.66-2.55 (m, 6H), 2.33-2.25 (m, 8H), 1.77-1.74 (m, 2H), 1.65-1.47 (m, 17H), 1.37-1.25 (m, 55H), 0.89-0.85 (m, 12H).

### Synthesis of compound 79

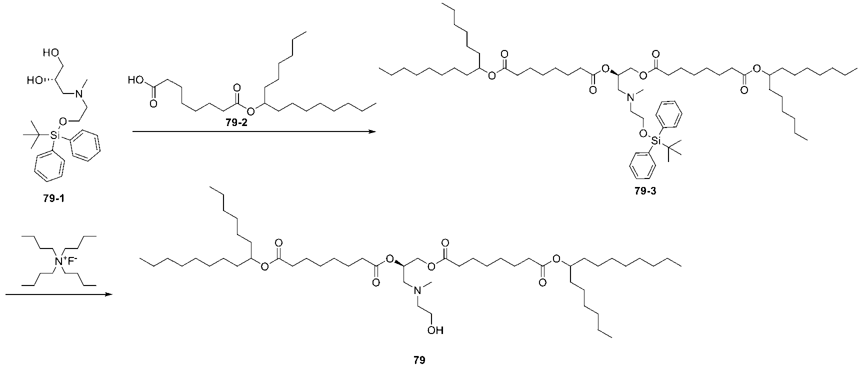

### Step 1: synthesis of compound 79-3

79-2 (793.8 mg, 2.1 mmol), DCC (425.9 mg, 2.1 mmol), DMAP (126.1 mg, 1.05 mmol), and DCM (5 mL) were added into a 25 mL flask, and the mixture was stirred at room temperature for 30 min. 79-1 (400 mg, 1.05 mmol) was then added, and the mixture was stirred at room temperature overnight. The extent of the reaction was detected by TLC (30% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.2 g). The crude product was purified and separated using a Flash column (12 g of silica gel; the mobile phases were respectively: 100 mL of *n*-heptane; 100 mL of 2% EtOAc + 98% *n*-heptane; 100 mL of 8% EtOAc + 92% *n*-heptane; and 200 mL of 20% EtOAc + 80% *n*-heptane) to give a pure product (880 mg, 76% yield). ¹H NMR (400 MHz, CDCl₃): *δ* 7.67-7.65 (m, 4H), 7.42-7.36 (m, 6H), 5.13 (m, 1H), 4.89-4.83 (m, 2H), 4.35-4.31 (m, 1H), 4.09-4.05 (m, 1H), 3.71 (m, 2H), 2.61-2.56 (m, 4H), 2.28-2.24 (m, 12H), 1.63-1.48 (m, 20H), 1.34-1.19 (m, 56H), 1.04 (s, 9H), 0.89-0.86 (m, 12H).

### Step 2: synthesis of compound 79

79-3 (880 mg, 0.79 mmol) and THF (3 mL) were added into a 25 mL flask, and the mixture was cooled in an ice bath to 0-5 °C. TBAF (623.7 mg, 2.4 mmol) was added dropwise with the temperature maintained at 0-5 °C, and the mixture was stirred at this temperature for 4 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 800 mg). The crude product was purified and separated using a Flash column (12 g of silica gel, loaded with 100 mL of n-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; and 300 mL of 30% EtOAc + 70% *n*-heptane) to give a pure product (500 mg, 71% yield). ¹H NMR (400 MHz, CDCl₃): *δ* 5.24-5.18 (m, 1H), 4.88-4.82 (m, 2H), 4.35-4.31 (m, 1H), 4.12-4.08 (m, 1H), 3.59-3.57 (m, 2H), 2.65-2.57 (m, 4H), 2.34-2.25 (m, 11H), 1.65-1.58 (m, 8H), 1.52.1.47 (m, 8H), 1.37-1.25 (m, 51H), 0.89-0.85 (m, 12H).

### Synthesis of compound 80

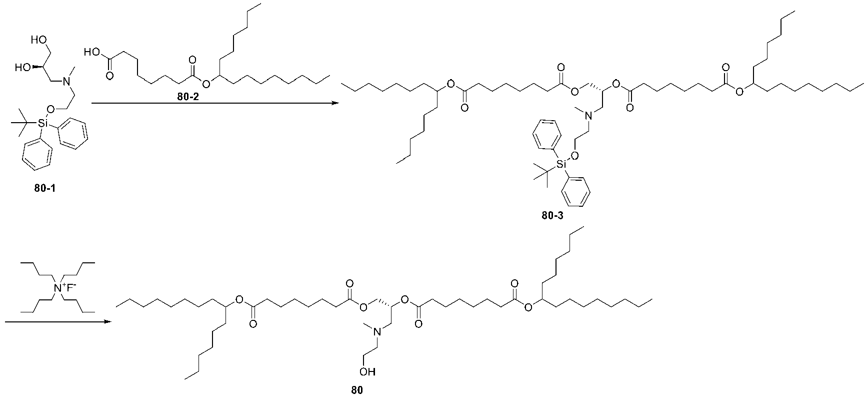

### Step 1: synthesis of compound 80-3

80-2 (793.8 mg, 2.1 mmol), DCC (425.9 mg, 2.1 mmol), DMAP (126.1 mg, 1.05 mmol), and DCM (5 mL) were added into a 25 mL flask, and the mixture was stirred at room temperature for 30 min. 80-1 (400 mg, 1.05 mmol) was then added, and the mixture was stirred at room temperature overnight. The extent of the reaction was detected by TLC (30% MeOH/DCM). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.3 g). The crude product was purified and separated using a Flash column (25 g of silica gel; the mobile phases were respectively: 100 mL of *n*-heptane; 100 mL of 2% EtOAc + 98% *n*-heptane; 100 mL of 8% EtOAc + 92% *n*-heptane; and 200 mL of 20% EtOAc + 80% *n*-heptane) to give a pure product (770 mg, 61% yield).

### Step 2: synthesis of compound 80

80-3 (770 mg, 0.7 mmol) and THF (3 mL) were added into a 25 mL flask, and the mixture was cooled in an ice bath to 0-5 °C. TBAF (545.7 mg, 2.1 mmol) was added dropwise with the temperature maintained at 0-5 °C, and the mixture was stirred at this temperature for 4 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 700 mg). The crude product was purified and separated using a Flash column (12 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 30% EtOAc + 70% *n*-heptane; and 300 mL of 50% EtOAc + 50% *n*-heptane) to give a pure product (300 mg, 49% yield). ¹H NMR (400 MHz, CDCl₃): *δ* 5.63-5.59 (m, 1H), 5.38-5.29 (m, 4H), 4.59-4.49 (m, 2H), 4.16-4.06 (m, 1H), 3.82-3.75 (m, 1H), 3.53 (s, 9H), 2.49-2.41 (m, 2H), 2.05-1.86 (m, 11H), 1.68-1.60 (m, 2H), 1.43-1.12 (m, 52H), 0.89-0.85 (m, 6H).

### Synthesis of compound 81

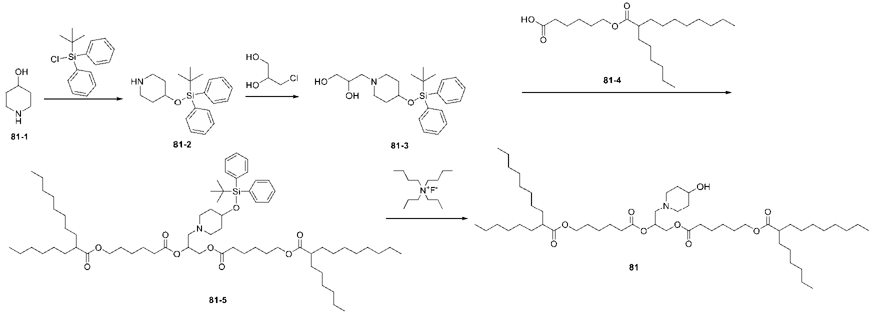

### Step 1: synthesis of compound 81-2

4-Hydroxypiperidine (81-1, 2.0 g, 19.8 mmol) and DCM (20 mL) were added into a 100 mL flask, and the solution was cooled in an ice-salt bath to 0-5 °C. A solution of TBDPSCl (5.4 g, 19.8 mmol) in DCM (10 mL) was slowly added dropwise to the cooled solution, and after the dropwise addition, the mixture was stirred at room temperature for 2 h. The extent of the reaction was detected by TLC (20% MeOH/DCM). After completion of the reaction, the reaction solution was concentrated to dryness to give a crude product (about 7 g). The crude product was purified and separated using a Flash column (150 g of silica gel, loaded with 100 mL of n-heptane; the mobile phases were respectively: 100 mL of *n*-heptane; 200 mL of 1% EtOAc + 99% *n*-heptane; 200 mL of 2% EtOAc + 98% *n*-heptane; and 400 mL of 8% EtOAc + 92% *n*-heptane) to give a pure product (4.2 g, 63% yield).

### Step 2: synthesis of compound 81-3

81-2 (1.5 g, 4.4 mmol), potassium carbonate (1.2 g, 8.8 mmol) and 3-chloro-1,2-propanediol (0.49 g, 4.4 mmol) dissolved in isopropanol (5 mL) were added into a 25 mL flask, and the mixture was heated to 80 °C and stirred overnight. The extent of the reaction was detected by TLC (10% MeOH/DCM). After completion of the reaction, the mixture was filtered, and the organic phase was concentrated to give a crude product (about 2 g). The crude product was purified and separated using a Flash column (20 g of silica gel; 100 mL of 0.1% MeOH + 99.9% DCM; 100 mL of 0.2% MeOH + 99.8% DCM; 100 mL of 0.3% MeOH + 99.7% DCM; 100 mL of 0.5% MeOH + 99.5% DCM; and 300 mL of 10% MeOH + 90% DCM) to give a pure product (900 mg, 49% yield).

### Step 3: synthesis of compound 81-5

81-4 (716.7 mg, 1.9 mmol), DCC (399.1 mg, 1.9 mmol), DMAP (118.1 mg, 0.97 mmol), and DCM (3 mL) were added into a 25 mL flask, and the mixture was stirred at room temperature for 30 min. 81-3 (500 mg, 0.76 mmol) was then added dropwise, and the mixture was stirred at room temperature overnight. The extent of the reaction was detected by TLC (20% EtOAc/*n-*heptane). After completion of the reaction, the mixture was filtered, and the filtrate was washed with 5 mL of water and 5 mL of saturated brine, dried over anhydrous Na₂SO₄, and filtered. The organic phase was concentrated to give a crude product (about 1.2 g). The crude product was purified and separated using a Flash column (12 g of silica gel; the mobile phases were respectively: 100 mL of *n*-heptane; 100 mL of 1% EtOAc + 99% *n*-heptane; 100 mL of 2% EtOAc + 98% *n*-heptane; and 100 mL of 8% EtOAc + 92% *n*-heptane) to give a pure product (640 mg, 59% yield). ¹H NMR (400 MHz, CDCl₃): *δ* 7.65-7.63 (m, 4H), 7.43-7.34 (m, 6H), 5.17 (m, 1H), 4.07-4.04 (m, 6H), 3.73 (m, 1H), 2.69 (m, 2H), 2.44-2.41 (m, 2H), 2.32-2.28 (m, 7H), 2.22-2.16 (m, 2H), 1.68-1.55 (m, 20H), 1.45-1.36 (m, 11H), 1.31-1.21 (m, 50H), 1.05 (s, 9H), 0.89-0.85 (m, 12H).

### Step 4: synthesis of compound 81

81-5 (520 mg, 0.46 mmol) and THF (3 mL) were added into a 25 mL flask, and the mixture was cooled in an ice bath to 0-5 °C. TBAF (364.6 mg, 1.4 mmol) was added dropwise with the temperature maintained at 0-5 °C, and the mixture was stirred at this temperature for 3 h. The extent of the reaction was detected by TLC (5% MeOH/DCM). After completion of the reaction, the reaction was quenched with a saturated aqueous ammonium chloride solution, and the mixture was extracted with EtOAc (5 mL × 2). The organic phases were combined, dried over anhydrous Na₂SO₄, filtered, and concentrated to give a crude product (about 500 mg). The crude product was purified and separated using a Flash column (14 g of silica gel, loaded with 100 mL of *n*-heptane; the mobile phases were respectively: 100 mL of 1% EtOAc + 99% *n*-heptane; 100 mL of 2% EtOAc + 98% *n*-heptane; 100 mL of 10% EtOAc + 90% *n*-heptane; 100 mL of 30% EtOAc + 70% *n*-heptane; and 100 mL of 50% EtOAc + 50% *n*-heptane) to give a pure product (40 mg, 10% yield). ¹H NMR (400 MHz, CDCl₃): *δ* 5.21 (m, 1H), 4.37-4.33 (m, 1H), 4.12-4.04 (m, 5H), 3.67 (m, 1H), 2.78 (m, 2H), 2.48 (m, 2H), 2.34-2.18 (m, 9H), 1.85 (m, 2H), 1.68-1.20 (m, 78H), 0.89-0.85 (m, 12H).

### Example 2: Preparation of Lipid Nanoparticle TMF

### Preparation of lipid nanoparticle TMF1 (containing 46.3% compound ALC-0315):

1. Compound ALC-0315 (cationic lipid), DSPC (helper lipid), cholesterol (helper lipid), and ALC-0159 (helper lipid) were dissolved in ethanol to give an oil phase (ethanol phase) stock solution, in which the molar ratios of the four lipids were 46.3%, 9.4%, 42.7%, and 1.6%, respectively.
2. A luciferase-expressing mRNA stock solution was diluted to 0.3 mg/mL with a citrate buffer (pH 4) to give an aqueous phase.
3. The oil phase (ethanol phase) containing the mixture of four lipids and the aqueous phase containing mRNA were rapidly mixed in a volume ratio of 1:3 to prepare TMF1 with a weight ratio of total lipids to mRNA of 25.7:1. The sample was then concentrated with an ultrafiltration tube having a molecular weight cutoff of 100 kd, then washed with a PBS buffer (pH 7.4), and filtered, and finally the filtrate was buffer-exchanged with a 120 mg/mL sucrose PBS buffer to give a final sample.

### Preparation of lipid nanoparticle TMF2 (containing 46.3% compound 1):

1. Compound 1 (cationic lipid/GOLD lipid), DSPC (helper lipid), cholesterol (helper lipid), and ALC-0159 (helper lipid) were dissolved in ethanol to give an oil phase (ethanol phase) stock solution, in which the molar ratios of the four lipids were 46.3%, 9.4%, 42.7%, and 1.6%, respectively.
2. A luciferase-expressing mRNA stock solution was diluted to 0.3 mg/mL with a citrate buffer (pH 4) to give an aqueous phase.
3. The oil phase (ethanol phase) containing the mixture of four lipids and the aqueous phase containing mRNA were rapidly mixed in a volume ratio of 1:3 to prepare TMF2 with a weight ratio of total lipids to mRNA of 26.8:1. The sample was then concentrated with an ultrafiltration tube having a molecular weight cutoff of 100 kd, then washed with a PBS buffer (pH 7.4), and filtered, and finally the filtrate was buffer-exchanged with a 120 mg/mL sucrose PBS buffer to give a final sample.

Lipid nanoparticles TMF3 to TMF13 were prepared with reference to the preparation method for TMF2, wherein the lipid composition was replaced according to the GOLD lipids and helper lipids listed in Table 2.

**Table 2. Lipid composition of lipid nanoparticles TMF3 to TMF13**

| Lipid nanoparticle No. | Cationic lipid | Helper lipid 1 | Helper lipid 2 | Helper lipid 3 | Number of component types |
|---|---|---|---|---|---|
| TMF3 | 46.3% compound 2 | 9.4% DSPC | 42.7% cholesterol | 1.6% ALC-0159 | Four |
| TMF4 | 46.3% compound 3 | 9.4% DSPC | 42.7% cholesterol | 1.6% ALC-0159 | Four |
| TMF5 | 46.3% compound 4 | 9.4% DSPC | 42.7% cholesterol | 1.6% ALC-0159 | Four |
| TMF6 | 46.3% compound 5 | 9.4% DSPC | 42.7% cholesterol | 1.6% ALC-0159 | Four |
| TMF7 | 46.3% compound 6 | 9.4% DSPC | 42.7% cholesterol | 1.6% ALC-0159 | Four |
| TMF8 | 46.3% compound 7 | 9.4% DSPC | 42.7% cholesterol | 1.6% ALC-0159 | Four |
| TMF9 | 46.3% compound 8 | 9.4% DSPC | 42.7% cholesterol | 1.6% ALC-0159 | Four |
| TMF10 | 46.3% compound 1 | 9.4% DOPE | 42.7% cholesterol | 1.6% ALC-0159 | Four |
| TMF11 | 46.3% compound 1 | 9.4% DPPC | 42.7% cholesterol | 1.6% ALC-0159 | Four |
| TMF12 | 46.3% compound 1 | 9.4% DOPC | 42.7% cholesterol | 1.6% ALC-0159 | Four |
| TMF13 | 46.3% compound 1 | 9.4% DSPC | 42.7% sitosterol | 1.6% ALC-0159 | Four |
| | | | | | |

Preparation of lipid nanoparticle TMF14 (three components; containing 46.3% compound 1 without DSPC):
1. Compound 1 (cationic lipid/GOLD lipid), cholesterol (helper lipid), and ALC-0159 (helper lipid) were dissolved in ethanol to give an oil phase (ethanol phase) stock solution, in which the molar ratios of the three lipids were 51.1%, 47.1%, and 1.8%, respectively.
2. A luciferase-expressing mRNA stock solution was diluted to 0.3 mg/mL with a citrate buffer (pH 4) to give an aqueous phase.
3. The oil phase (ethanol phase) containing the mixture of three lipids and the aqueous phase containing mRNA were rapidly mixed in a volume ratio of 1:3 to prepare TMF14 with a weight ratio of total lipids to mRNA of 23.8:1. The sample was then concentrated with an ultrafiltration tube having a molecular weight cutoff of 100 kd, then washed with a PBS buffer (pH 7.4), and filtered, and finally the filtrate was buffer-exchanged with a 120 mg/mL sucrose PBS buffer to give a final sample.

### Preparation of lipid nanoparticle TMF15 (two components: containing 66% compound 1 and 34% DOPE):

1. Compound 1 (cationic lipid/GOLD lipid) and DOPE (helper lipid) were dissolved in ethanol to give an oil phase (ethanol phase) stock solution, in which the molar ratios of the two lipids were 66% and 34%, respectively.
2. A luciferase-expressing mRNA stock solution was diluted to 0.09 mg/mL with a citrate buffer (pH 4) to give an aqueous phase.
3. The oil phase (ethanol phase) containing the mixture of two lipids and the aqueous phase containing mRNA were rapidly mixed in a volume ratio of 1:3 to prepare TMF15 with a molar ratio of total lipids to mRNA of 1.3:2. The sample was then concentrated with an ultrafiltration tube having a molecular weight cutoff of 100 kd, then washed with a PBS buffer (pH 7.4), and filtered, and finally the filtrate was buffer-exchanged with 120 mg of a sucrose PBS buffer to give a final sample.

### Preparation of lipid nanoparticle TMF16 (single component: containing 100% compound 1):

1. Compound 1 (cationic lipid/GOLD lipid) was dissolved in ethanol to give an oil phase (ethanol phase) stock solution, in which the molar ratio of the lipid was 100%.
2. A luciferase-expressing mRNA stock solution was diluted to 0.09 mg/mL with a citrate buffer (pH 4) to give an aqueous phase.
3. The oil phase (ethanol phase) containing the mixture of one lipid and the aqueous phase containing mRNA were rapidly mixed in a volume ratio of 1:3 to prepare TMF16 with a molar ratio of compound 1 to mRNA of 1.3:2. The sample was then concentrated with an ultrafiltration tube having a molecular weight cutoff of 100 kd, then washed with a PBS buffer (pH 7.4), and filtered, and finally the filtrate was buffer-exchanged with 120 mg of a sucrose PBS buffer to give a final sample.

Lipid nanoparticles TMF17 to TMF53 were prepared with reference to the preparation method for TMF2, wherein the lipid composition was replaced according to the cationic lipids and helper lipids listed in Table 3.

**Table 3. Lipid composition of lipid nanoparticles TMF17 to TMF54**

| Lipid nanoparticle No. | Cationic lipid | Helper lipid 1 | Helper lipid 2 | Helper lipid 3 | Number of component types |
|---|---|---|---|---|---|
| TMF17 | 46.3% DODAP | 9.4% DOPE | 42.7% cholesterol | 1.6% PEG2000-DMG | Four |
| TMF18 | 46.3% compound 17 | 9.4% DOPE | 42.7% cholesterol | 1.6% PEG2000-DMG | Four |
| TMF19 | 46.3% compound 19 | 9.4% DOPE | 42.7% cholesterol | 1.6% PEG2000-DMG | Four |
| TMF20 | 46.3% compound 22 | 9.4% DOPE | 42.7% cholesterol | 1.6% PEG2000-DMG | Four |
| TMF21 | 46.3% compound 25 | 9.4% DOPE | 42.7% cholesterol | 1.6% PEG2000-DMG | Four |
| TMF22 | 46.3% compound 29 | 9.4% DOPE | 42.7% cholesterol | 1.6% PEG2000-DMG | Four |
| TMF23 | 46.3% compound 31 | 9.4% DOPE | 42.7% cholesterol | 1.6% PEG2000-DMG | Four |
| TMF24 | 46.3% compound 33 | 9.4% DOPE | 42.7% cholesterol | 1.6% PEG2000-DMG | Four |
| TMF25 | 46.3% compound 34 | 9.4% DOPE | 42.7% cholesterol | 1.6% PEG2000-DMG | Four |
| TMF26 | 46.3% compound 35 | 9.4% DOPE | 42.7% cholesterol | 1.6% PEG2000-DMG | Four |
| TMF27 | 46.3% compound 36 | 9.4% DOPE | 42.7% cholesterol | 1.6% PEG2000-DMG | Four |
| TMF28 | 46.3% compound 37 | 9.4% DOPE | 42.7% cholesterol | 1.6% PEG2000-DMG | Four |
| TMF29 | 46.3% compound 38 | 9.4% DOPE | 42.7% cholesterol | 1.6% PEG2000-DMG | Four |
| TMF30 | 46.3% compound 43 | 9.4% DOPE | 42.7% cholesterol | 1.6% PEG2000-DMG | Four |
| TMF31 | 46.3% compound 44 | 9.4% DOPE | 42.7% cholesterol | 1.6% PEG2000-DMG | Four |
| TMF32 | 46.3% compound 45 | 9.4% DOPE | 42.7% cholesterol | 1.6% PEG2000-DMG | Four |
| TMF33 | 46.3% compound 46 | 9.4% DOPE | 42.7% cholesterol | 1.6% PEG2000-DMG | Four |
| TMF34 | 46.3% compound 48 | 9.4% DOPE | 42.7% cholesterol | 1.6% PEG2000-DMG | Four |
| TMF35 | 46.3% compound 50 | 9.4% DOPE | 42.7% cholesterol | 1.6% PEG2000-DMG | Four |
| TMF36 | 46.3% compound 52 | 9.4% DOPE | 42.7% cholesterol | 1.6% PEG2000-DMG | Four |
| TMF37 | 46.3% compound 53 | 9.4% DOPE | 42.7% cholesterol | 1.6% PEG2000-DMG | Four |
| TMF38 | 46.3% compound 54 | 9.4% DOPE | 42.7% cholesterol | 1.6% PEG2000-DMG | Four |
| TMF39 | 46.3% compound 55 | 9.4% DOPE | 42.7% cholesterol | 1.6% PEG2000-DMG | Four |
| TMF40 | 46.3% compound 56 | 9.4% DOPE | 42.7% cholesterol | 1.6% PEG2000-DMG | Four |
| TMF41 | 46.3% compound 57 | 9.4% DOPE | 42.7% cholesterol | 1.6% PEG2000-DMG | Four |
| TMF42 | 46.3% compound 58 | 9.4% DOPE | 42.7% cholesterol | 1.6% PEG2000-DMG | Four |
| TMF43 | 46.3% compound 60 | 9.4% DOPE | 42.7% cholesterol | 1.6% PEG2000-DMG | Four |
| TMF44 | 46.3% compound 63 | 9.4% DOPE | 42.7% cholesterol | 1.6% PEG2000-DMG | Four |
| TMF45 | 46.3% compound 64 | 9.4% DOPE | 42.7% cholesterol | 1.6% PEG2000-DMG | Four |
| TMF46 | 46.3% compound 65 | 9.4% DOPE | 42.7% cholesterol | 1.6% PEG2000-DMG | Four |
| TMF47 | 46.3% compound 66 | 9.4% DOPE | 42.7% cholesterol | 1.6% PEG2000-DMG | Four |
| TMF48 | 46.3% compound 67 | 9.4% DOPE | 42.7% cholesterol | 1.6% PEG2000-DMG | Four |
| TMF49 | 46.3% compound 68 | 9.4% DOPE | 42.7% cholesterol | 1.6% PEG2000-DMG | Four |
| TMF50 | 46.3% compound 76 | 9.4% DOPE | 42.7% cholesterol | 1.6% PEG2000-DMG | Four |
| TMF51 | 46.3% compound 78 | 9.4% DOPE | 42.7% cholesterol | 1.6% PEG2000-DMG | Four |
| TMF52 | 46.3% compound 79 | 9.4% DOPE | 42.7% cholesterol | 1.6% PEG2000-DMG | Four |
| TMF53 | 46.3% compound 80 | 9.4% DOPE | 42.7% cholesterol | 1.6% PEG2000-DMG | Four |

### Example 3: Detection of Lipid Nanoparticles

The size and polydispersity index (PDI) of the lipid nanoparticles were determined using NanoBrook 90plus PLAS (Brookhaven Instruments, US) by dynamic light scattering (DLS) technology at a side scatter angle of 90°. The determination results are shown in Table 4.

**Table 4. Characterization data for lipid nanoparticles prepared from representative cationic lipid compounds**

| Lipid nanoparticle No. | Cationic lipid | Helper lipid | Number of component types | Particle size (nm) | PDI (polydispersity index) |
|---|---|---|---|---|---|
| TMF1 | 46.3% ALC-0315 | 9.4% DSPC | Four | 106.32±1.99 | 0.220±0.020 |
| TMF2 | 46.3% 1 | 9.4% DSPC | Four | 213.10±2.13 | 0.132±0.032 |
| TMF3 | 46.3% 2 | 9.4% DSPC | Four | 220.39±3.19 | 0.149±0.039 |
| TMF4 | 46.3% 3 | 9.4% DSPC | Four | 207.86±1.25 | 0.149±0.035 |
| TMF5 | 46.3% 4 | 9.4% DSPC | Four | 231.95±3.30 | 0.189±0.014 |
| TMF6 | 46.3% 5 | 9.4% DSPC | Four | 215.72±3.33 | 0.106±0.035 |
| TMF7 | 46.3% 6 | 9.4% DSPC | Four | 216.80±3.87 | 0.132±0.040 |
| TMF8 | 46.3% 7 | 9.4% DSPC | Four | 175.62±3.28 | 0.145±0.008 |
| TMF9 | 46.3% 8 | 9.4% DSPC | Four | 247.68±5.17 | 0.174±0.005 |
| TMF10 | 46.3% 1 | 9.4% DOPE | Four | 223.99±3.14 | 0.127±0.014 |
| TMF11 | 46.3% 1 | 9.4% DPPC | Four | 228.05±2.96 | 0.189±0.030 |
| TMF12 | 46.3% 1 | 9.4% DOPC | Four | 233.08±1.79 | 0.132±0.029 |
| TMF13 | 46.3% 1 | 9.4% DSPC & 42.7% sitosterol | Four | 232.61±2.91 | 0.165±0.038 |
| TMF14 | 46.3% 1 | Without DSPC | Three | 212.60±2.08 | 0.147±0.040 |
| TMF17 | 46.3%DOD AP | 9.4% DOPE | Four | 222.61±5.71 | 0.203±0.026 |
| TMF18 | 46.3% 17 | 9.4% DOPE | Four | 241.76±3.37 | 0.239±0.023 |
| TMF19 | 46.3% 19 | 9.4% DOPE | Four | 244.59±4.23 | 0.201±0.040 |
| TMF20 | 46.3% 22 | 9.4% DOPE | Four | 231.65±4.77 | 0.200±0.034 |
| TMF21 | 46.3% 25 | 9.4% DOPE | Four | 229.39±4.28 | 0.181±0.007 |
| TMF22 | 46.3% 29 | 9.4% DOPE | Four | 176.50±1.10 | 0.107±0.027 |
| TMF23 | 46.3% 31 | 9.4% DOPE | Four | 234.41±3.34 | 0.096±0.036 |
| TMF24 | 46.3% 33 | 9.4% DOPE | Four | 283.47±5.45 | 0.201±0.014 |
| TMF25 | 46.3% 34 | 9.4% DOPE | Four | 238.52±1.86 | 0.098±0.068 |
| TMF26 | 46.3% 35 | 9.4% DOPE | Four | 229.31±6.87 | 0.151±0.028 |
| TMF27 | 46.3% 36 | 9.4% DOPE | Four | 217.06±4.18 | 0.082±0.029 |
| TMF28 | 46.3% 37 | 9.4% DOPE | Four | 225.14±3.26 | 0.168±0.050 |
| TMF29 | 46.3% 38 | 9.4% DOPE | Four | 226.34±2.14 | 0.145±0.044 |
| TMF30 | 46.3% 43 | 9.4% DOPE | Four | 210.60±1.83 | 0.136±0.018 |
| TMF31 | 46.3% 44 | 9.4% DOPE | Four | 215.34±2.85 | 0.130±0.037 |
| TMF32 | 46.3% 45 | 9.4% DOPE | Four | 229.03±2.20 | 0.179±0.023 |
| TMF33 | 46.3% 46 | 9.4% DOPE | Four | 23 1.82±0.86 | 0.164±0.081 |
| TMF34 | 46.3% 48 | 9.4% DOPE | Four | 220.10±2.28 | 0.185±0.004 |
| TMF35 | 46.3% 50 | 9.4% DOPE | Four | 191.10±1.36 | 0.135±0.016 |
| TMF36 | 46.3% 52 | 9.4% DOPE | Four | 197.30±2.40 | 0.155±0.018 |
| TMF37 | 46.3% 53 | 9.4% DOPE | Four | 238.10±5.17 | 0.258±0.024 |
| TMF38 | 46.3% 54 | 9.4% DOPE | Four | 195.60±1.06 | 0.147±0.022 |
| TMF39 | 46.3% 55 | 9.4% DOPE | Four | 193.80±3.61 | 0.119±0.005 |
| TMF40 | 46.3% 56 | 9.4% DOPE | Four | 191.20±1.40 | 0.133±0.019 |
| TMF41 | 46.3% 57 | 9.4% DOPE | Four | 194.00±0.70 | 0.128±0.023 |
| TMF42 | 46.3% 58 | 9.4% DOPE | Four | 191.60±1.35 | 0.110±0.038 |
| TMF43 | 46.3% 60 | 9.4% DOPE | Four | 195.40±2.46 | 0.116±0.014 |
| TMF44 | 46.3% 63 | 9.4% DOPE | Four | 199.10±1.07 | 0.131±0.012 |
| TMF45 | 46.3% 64 | 9.4% DOPE | Four | 197.50±1.91 | 0.134±0.021 |
| TMF46 | 46.3% 65 | 9.4% DOPE | Four | 198.40±0.34 | 0.114±0.041 |
| TMF47 | 46.3% 66 | 9.4% DOPE | Four | 177.90±1.06 | 0.111±0.022 |
| TMF48 | 46.3% 67 | 9.4% DOPE | Four | 195.40±2.46 | 0.116±0.014 |
| TMF49 | 46.3% 68 | 9.4% DOPE | Four | 185.90±3.03 | 0.120±0.024 |
| TMF50 | 46.3% 76 | 9.4% DOPE | Four | 208.70±0.95 | 0.132±0.017 |
| TMF51 | 46.3% 78 | 9.4% DOPE | Four | 202.20±1.78 | 0.110±0.011 |
| TMF52 | 46.3% 79 | 9.4% DOPE | Four | 204.30±1.17 | 0.147±0.021 |
| TMF53 | 46.3% 80 | 9.4% DOPE | Four | 202.80±2.89 | 0.152±0.004 |

### Example 4: Detection of Organ Distribution of Delivery Systems

The liposomes prepared in Example 2 were each injected at a dose of 0.5 mg/kg through the inner canthus venous plexus of the fundus to female ICR mice aged 6-8 weeks. 4 h after the administration, 15 mg/mL D-luciferin potassium salt was intraperitoneally injected at a dose of 150 mg/kg. 10 min after injection of luciferase substrate, mice were placed under an *in vivo* imaging system (IVIS Lumina XRMS Series III, PerkinElmer) and observed for *in vivo* fluorescence intensity and distribution. The mice were then sacrificed, and organs (heart, liver, spleen, lung, and kidney) were isolated for *ex vivo* imaging to observe the fluorescence intensity and distribution in the different organs. The organ distribution of Luc-mRNA delivered by representative lipid compounds is shown in Table 5. As shown in Table 5, the ratio of fluorescence intensity in the spleen to that in the liver (spleen/liver) reached above 60 for lipid nanoparticles TMF10, TMF21, TMF19, TMF26 and TMF29 containing compound 1, compound 25, compound 19, compound 35 and compound 38, respectively, at 4 h after intravenous administration in mice, wherein the ratio for TMF29 was the highest, reaching 69.5. The fluorescence intensity in different organs represents the delivery efficiency of the corresponding delivery system in different organs. The lipid nanoparticles of the present application can successfully deliver nucleic acid molecules into the spleen and enable the expression of the nucleic acid molecules, and the delivery efficiency for the spleen is significantly higher than that for the liver and other tissues/organs.

**Table 5. Expression intensity of Luc-mRNA delivered by lipid nanoparticles prepared from representative cationic lipids**

| Lipid nanoparticle No. | Fluorescence intensity | | |
|---|---|---|---|
| | Spleen [p/s] | Liver [p/s] | Spleen/liver |
| TMF1 | 3.52E+07 | 1.99E+09 | 0.02 |
| TMF2 | 1.71E+07 | 4.58E+05 | 34.5 |
| TMF3 | 2.32E+05 | 3.97E+04 | 4.2 |
| TMF4 | 3.93E+07 | 1.34E+06 | 24.9 |
| TMF5 | 8.37E+06 | 2.15E+05 | 37.1 |
| TMF6 | 2.36E+05 | 3.05E+04 | 5.9 |
| TMF7 | 7.02E+05 | 2.66E+05 | 6.6 |
| TMF8 | 9.71E+03 | 1.18E+04 | 1.0 |
| TMF9 | 1.41E+06 | 1.01E+05 | 15.3 |
| TMF10 | 1.19E+08 | 1.52E+06 | 60.6 |
| TMF11 | 2.09E+07 | 5.82E+05 | 48.4 |
| TMF12 | 1.20E+08 | 4.13E+06 | 31.9 |
| TMF13 | 2.58E+07 | 4.35E+06 | 9.9 |
| TMF14 | 4.52E+07 | 1.50E+06 | 28.0 |
| TMF17 | 1.45E+06 | 1.69E+06 | 0.9 |
| TMF18 | 1.49E+08 | 3.43E+06 | 39.6 |
| TMF19 | 2.50E+08 | 3.10E+06 | 64.8 |
| TMF20 | 1.83E+08 | 8.69E+06 | 21.8 |
| TMF21 | 2.16E+08 | 3.40E+06 | 63.5 |
| TMF22 | 2.57E+08 | 4.77E+07 | 7.4 |
| TMF23 | 2.93E+08 | 1.39E+08 | 2.4 |
| TMF24 | 3.58E+07 | 9.20E+05 | 47.6 |
| TMF25 | 1.82E+08 | 9.74E+07 | 2.1 |
| TMF26 | 2.48E+08 | 3.69E+06 | 69.3 |
| TMF27 | 7.92E+07 | 3.65E+06 | 21.5 |
| TMF28 | 1.51E+08 | 3.41E+06 | 46.6 |
| TMF29 | 5.73E+08 | 8.48E+06 | 69.5 |
| TMF30 | 1.31E+09 | 3.56E+07 | 38.8 |
| TMF31 | 3.19E+08 | 1.59E+08 | 2.5 |
| TMF32 | 3.29E+07 | 1.07E+06 | 32.2 |
| TMF33 | 8.22E+07 | 1.67E+06 | 48.3 |
| TMF34 | 1.46E+09 | 6.30E+07 | 26.1 |
| TMF35 | 1.18E+09 | 6.87E+07 | 18.3 |
| TMF36 | 1.90E+09 | 6.63E+07 | 38.9 |
| TMF37 | 5.34E+07 | 3.38E+06 | 18.4 |
| TMF38 | 1.91E+09 | 1.25E+08 | 15.8 |
| TMF39 | 1.37E+09 | 3.98E+07 | 34.8 |
| TMF40 | 1.06E+09 | 4.20E+07 | 31.2 |
| TMF41 | 1.28E+08 | 1.05E+07 | 10.5 |
| TMF42 | 3.15E+09 | 8.89E+07 | 34.7 |
| TMF43 | 2.41E+08 | 1.35E+07 | 24.3 |
| TMF44 | 8.90E+08 | 5.90E+07 | 14.9 |
| TMF45 | 7.33E+08 | 3.54E+07 | 20.6 |
| TMF46 | 1.39E+08 | 3.94E+06 | 39.4 |
| TMF47 | 4.12E+04 | 2.48E+04 | 1.7 |
| TMF48 | 2.22E+07 | 1.73E+06 | 16.7 |
| TMF49 | 6.41E+08 | 1. 16E+07 | 59.1 |
| TMF50 | 8.96E+07 | 1.85E+06 | 47.3 |
| TMF51 | 2.22E+08 | 7.35E+06 | 30.6 |
| TMF52 | 2.21E+09 | 1.68E+08 | 26.4 |
| TMF53 | 1.34E+09 | 2.58E+07 | 52.4 |

The present application is described above in detail so that those skilled in the art can understand the mechanisms and content of the present application and implement it. However, the description does not limit the protection scope of the present application, and any equivalent changes or modifications made in line with the spirit of the present application shall fall within the protection scope of the present application.

### Industrial Applicability

The cationic lipid compound provided by the present application can be applied to a lipid nanoparticle, wherein the lipid nanoparticle is capable of targeting different tissues and organs for drug delivery; further, the lipid nanoparticle may further comprise at least one helper lipid. The cationic lipid compound or a lipid nanoparticle composition comprising the same is capable of specifically delivering a prophylactic/therapeutic agent (particularly a nucleic acid component) to a target organ.

## Claims

1. A compound of formula (I), 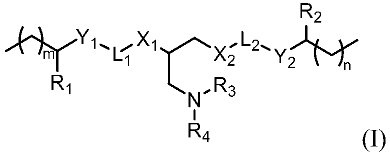
or a pharmaceutically acceptable salt, prodrug, or stereoisomer thereof, wherein
X₁ and X₂ are each independently -OC(=O)-, -C(=O)O-, -NHC(=O)-, or -C(=O)NH-;
Y₁ and Y₂ are each independently -OC(=O)-, -C(=O)O-, -NR₅C(=O)-, -C(=O)NR₅-bond, wherein R₅ is selected from hydrogen or linear or branched C1-C8 hydrocarbyl;
L₁ and L₂ are each independently a bond, or optionally substituted C1-C10 alkylene;
R₃ and R₄ are each independently methyl, ethyl, propyl, hydroxymethyl, hydroxyethyl, hydroxypropyl, hydroxybutyl, 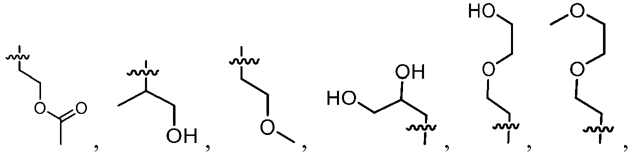 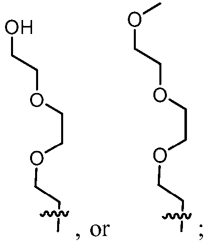 or R₃ and R₄, together with the nitrogen attached thereto, form a cyclic moiety, wherein R₃ or R₄ are selected from a group containing hydrogen or deuterium, and the cyclic moiety is 4- to 8-membered heterocycloalkyl selected from azetidin-1-yl, pyrrolidinyl, piperidin-1-yl, 4-hydroxypiperidin-1-yl, azepan-1-yl, morpholinyl, and 4-acetylpiperazin-1-yl; R₁ and R₂ are each independently H or deuterium or linear or branched C1-C8 hydrocarbyl; m is an integer of 1-13;
n is an integer of 1-13.

2. The compound or the pharmaceutically acceptable salt, prodrug, or stereoisomer thereof according to claim 1, wherein the compound is selected from compounds are as follows:
| No. | Structural formula |
|---|---|
| 1 | 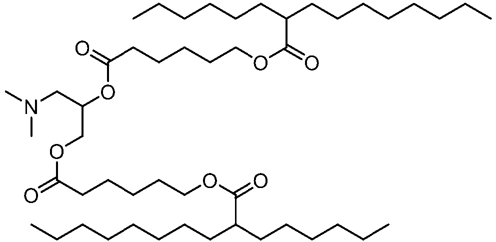 |
| 2 | 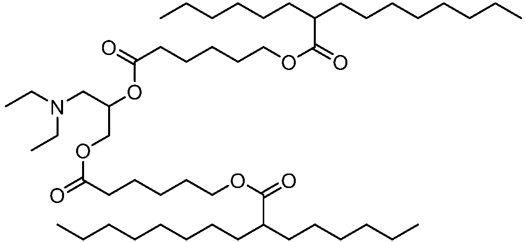 |
| 3 | 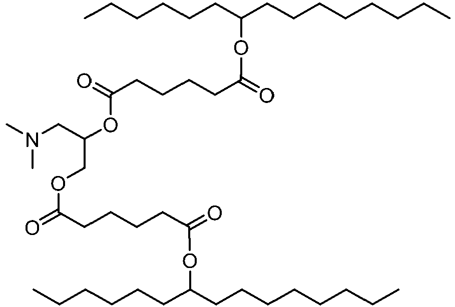 |
| 4 | 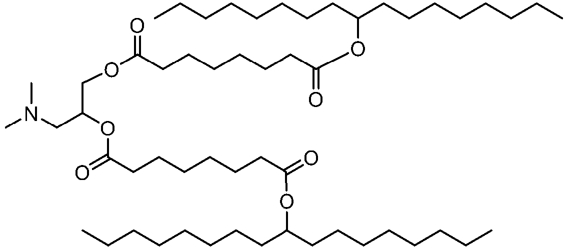 |
| 5 | 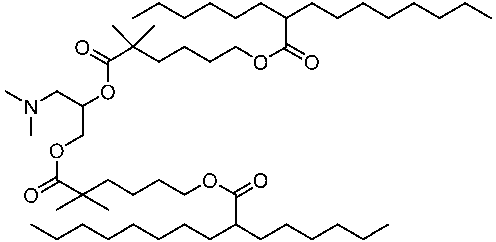 |
| 8 | 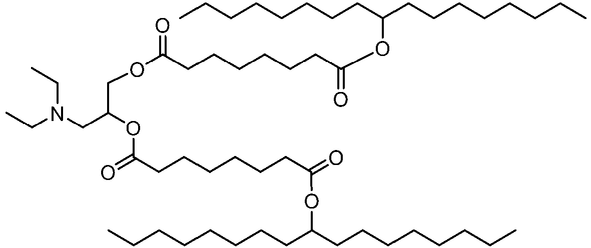 |
| 9 | 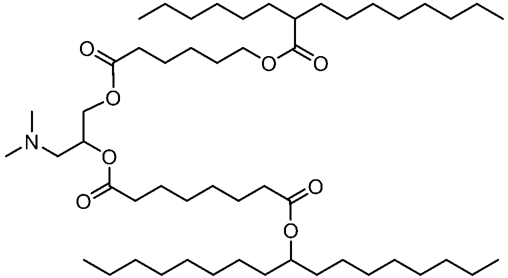 |
| 10 | 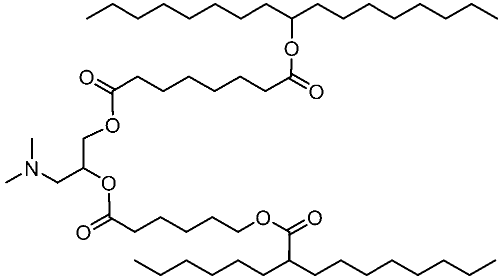 |
| 11 | 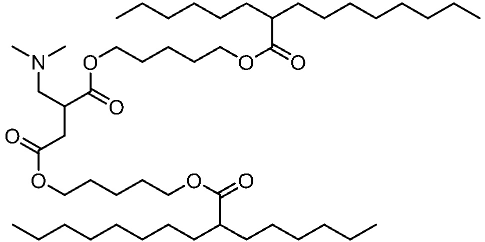 |
| 12 | 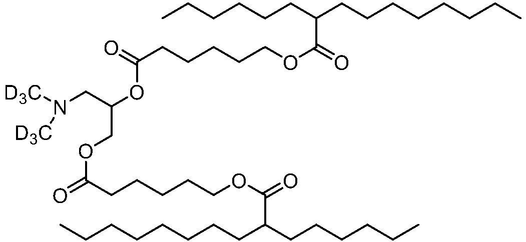 |
| 14 | 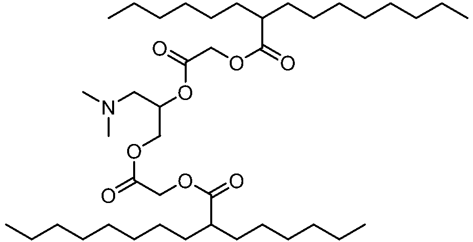 |
| 15 | 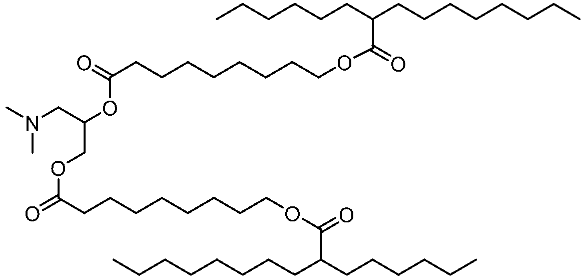 |
| 16 | 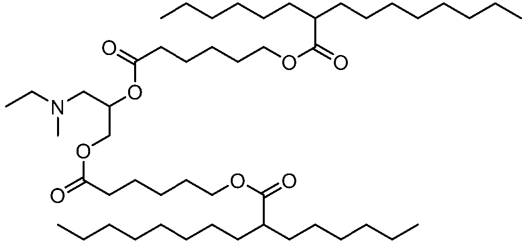 |
| 17 | 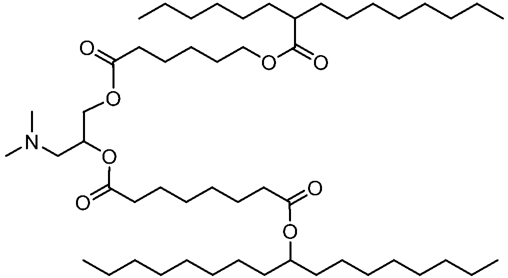 |
| 18 | 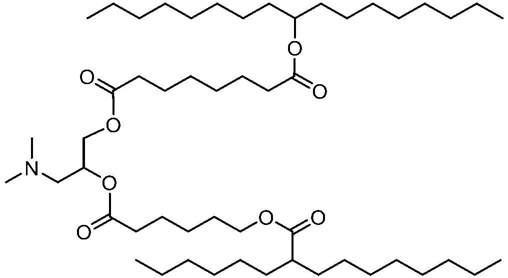 |
| 19 | 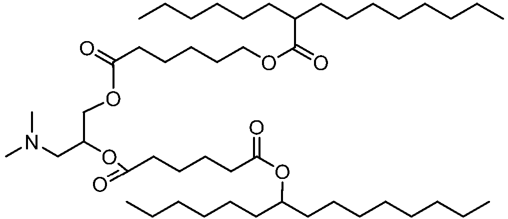 |
| 20 | 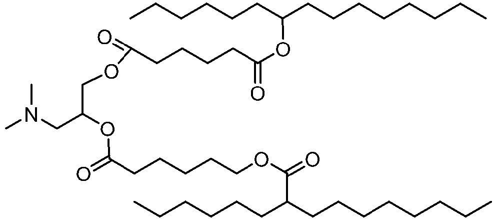 |
| 21 | 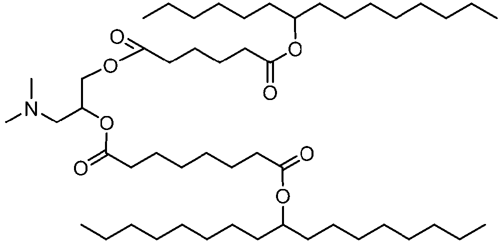 |
| 22 | 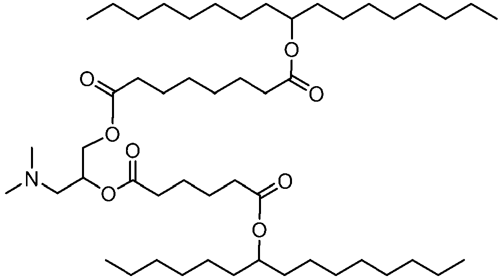 |
| 25 | 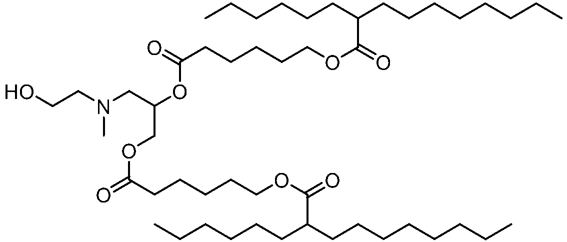 |
| 26 | 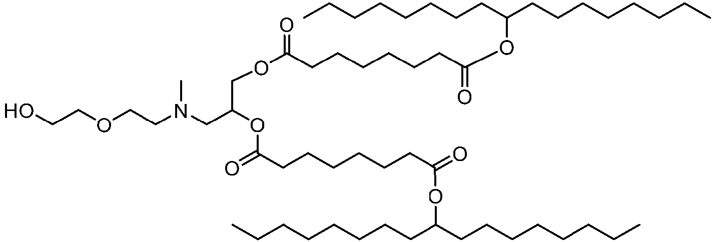 |
| 27 | 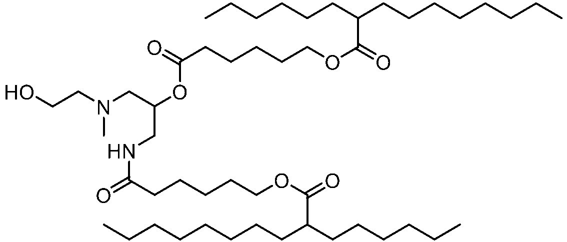 |
| 28 | 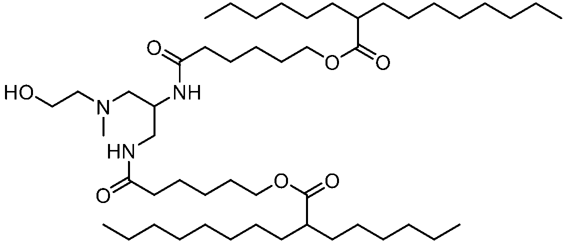 |
| 29 | 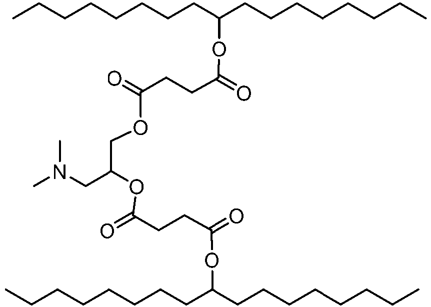 |
| 30 | 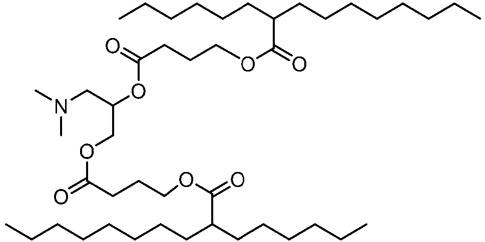 |
| 31 | 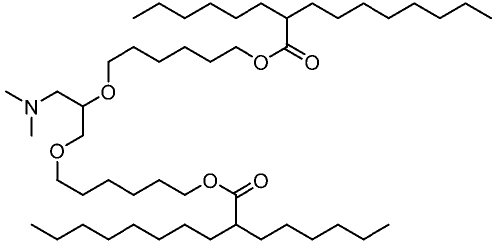 |
| 32 | 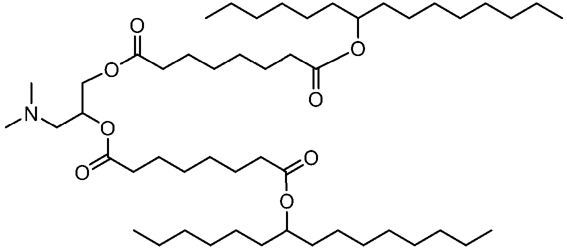 |
| 33 | 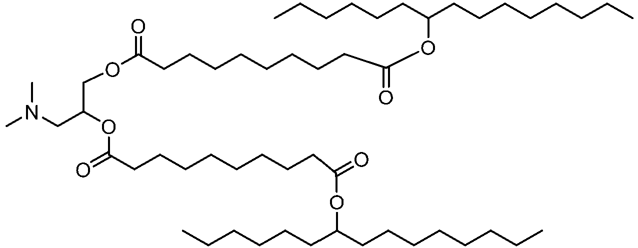 |
| 34 | 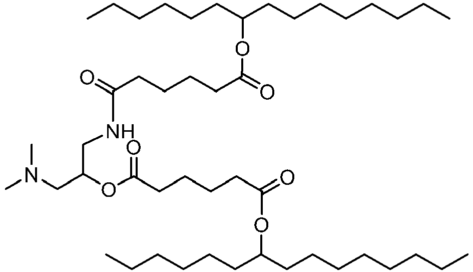 |
| 35 | 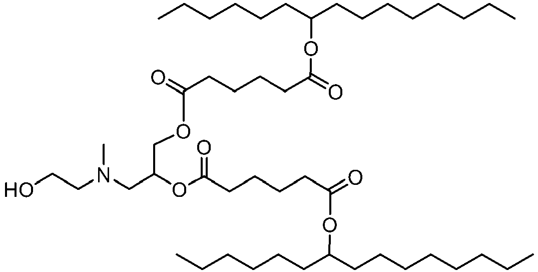 |
| 36 | 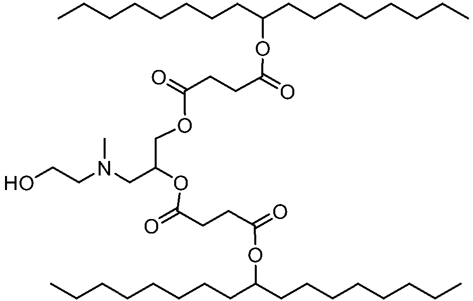 |
| 37 | 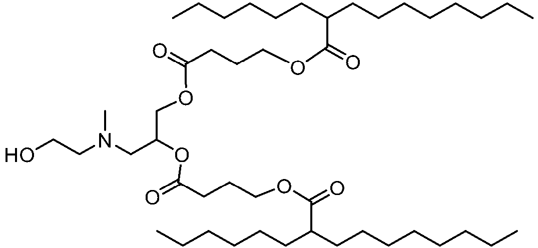 |
| 38 | 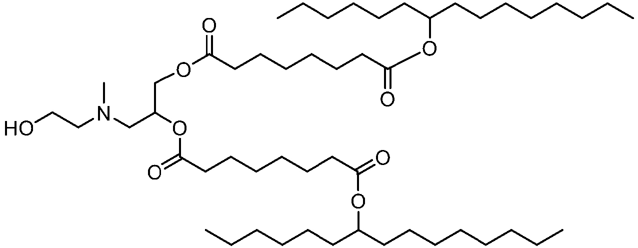 |
| 39 | 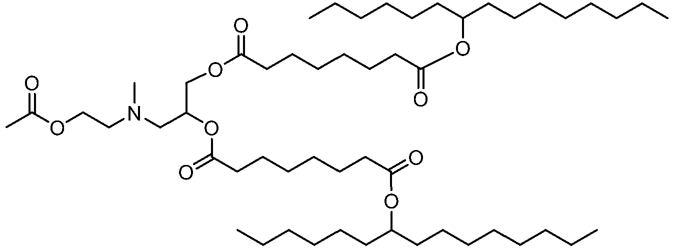 |
| 40 | 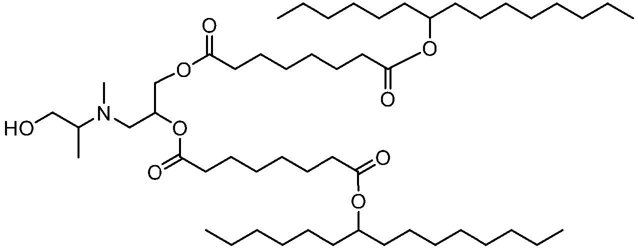 |
| 41 | 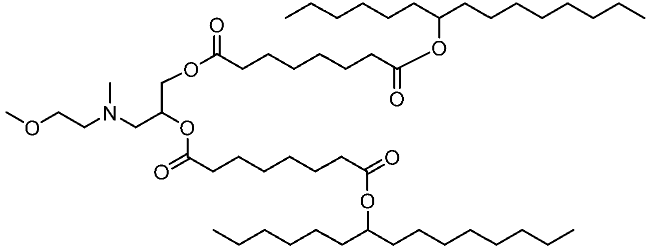 |
| 42 | 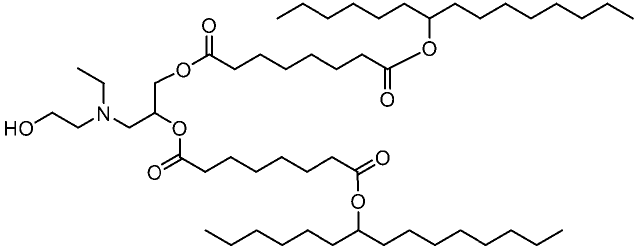 |
| 43 | 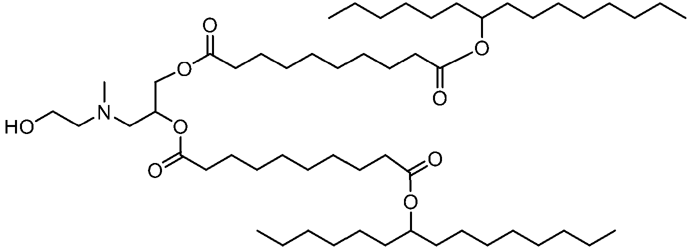 |
| 44 | 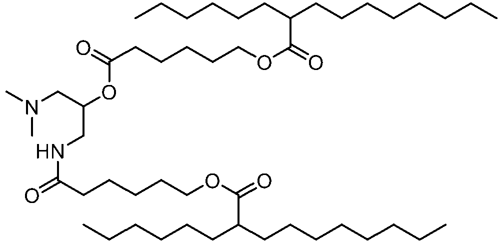 |
| 45 | 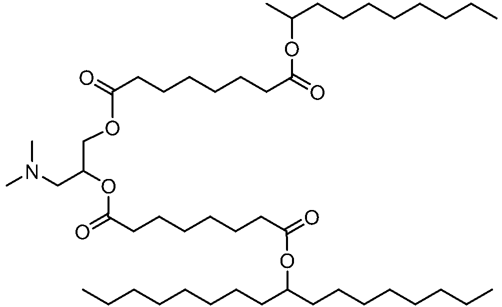 |
| 46 | 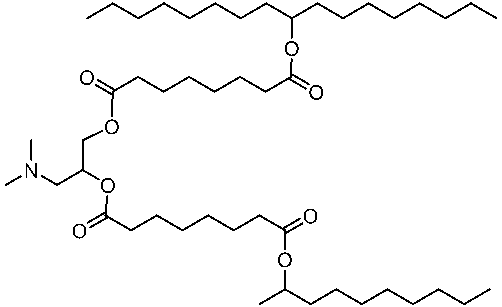 |
| 47 | 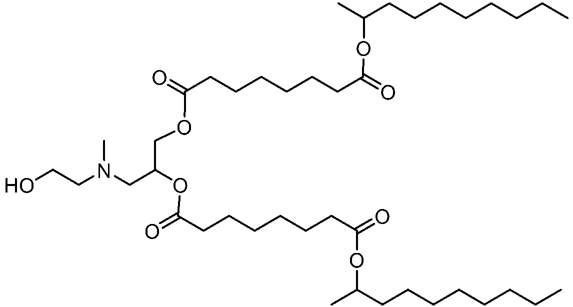 |
| 48 | 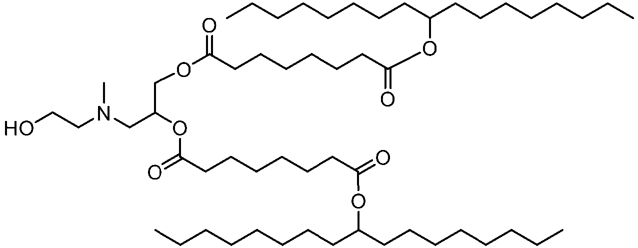 |
| 49 | 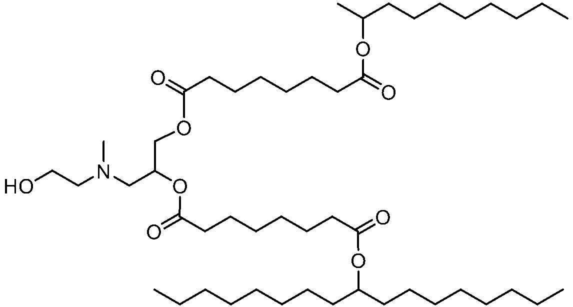 |
| 50 | 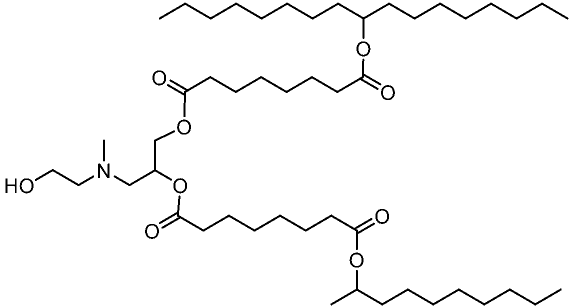 |
| 51 | 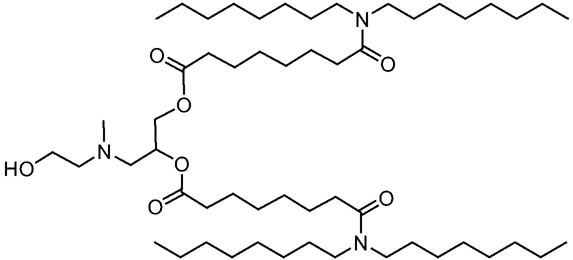 |
| 52 | 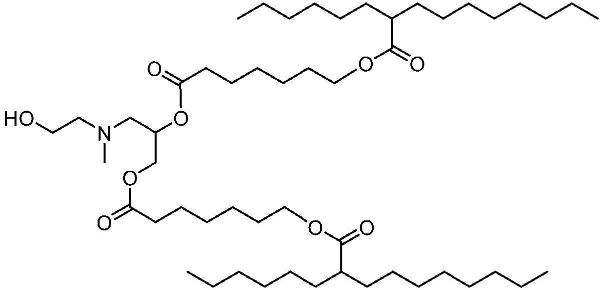 |
| 53 | 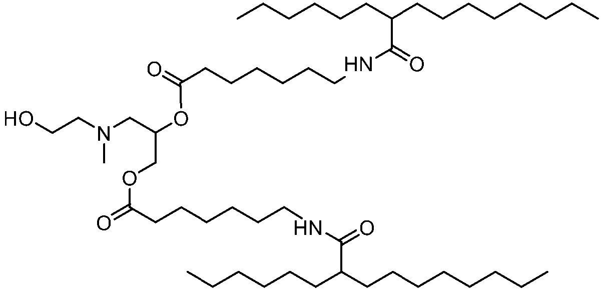 |
| 54 | 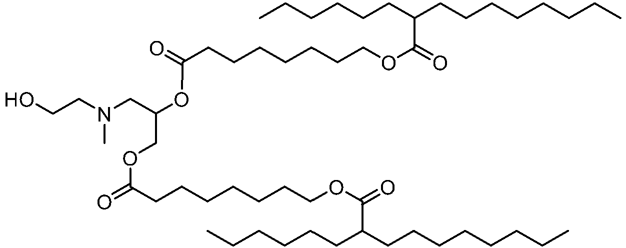 |
| 55 | 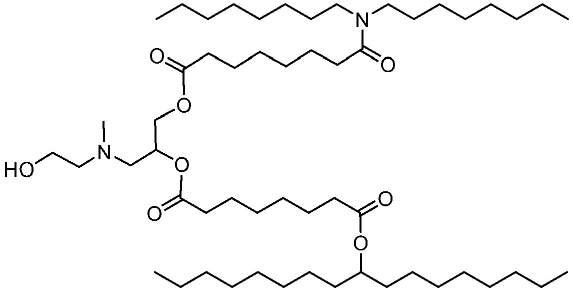 |
| 56 | 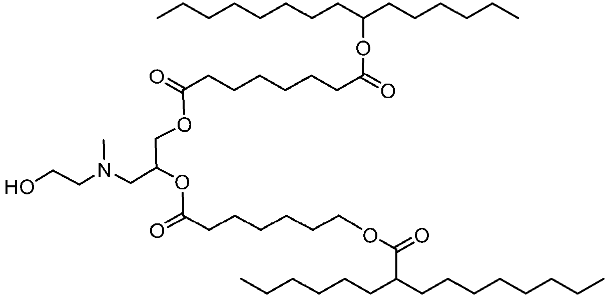 |
| 57 | 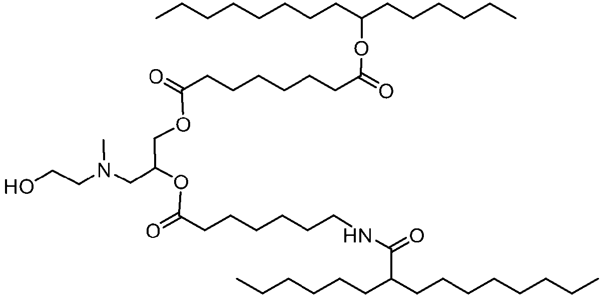 |
| 58 | 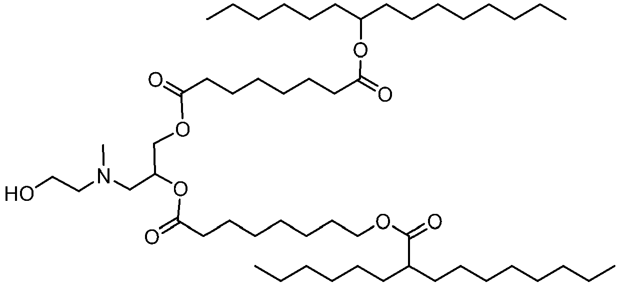 |
| 59 | 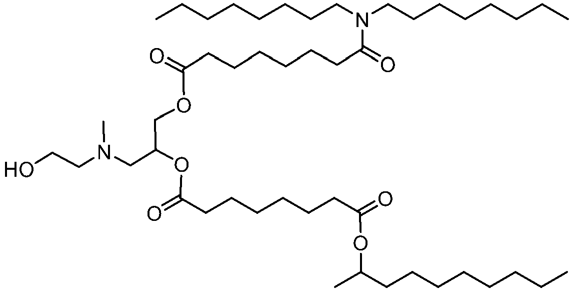 |
| 60 | 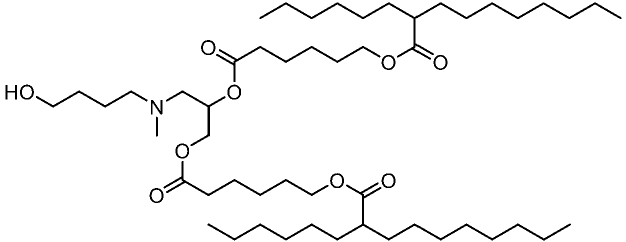 |
| 61 | 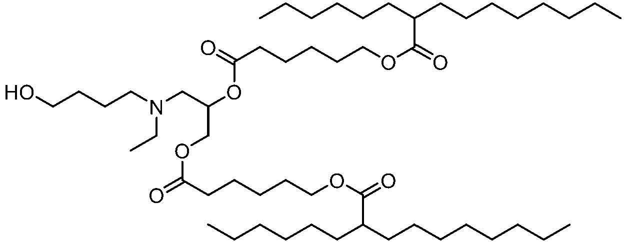 |
| 62 | 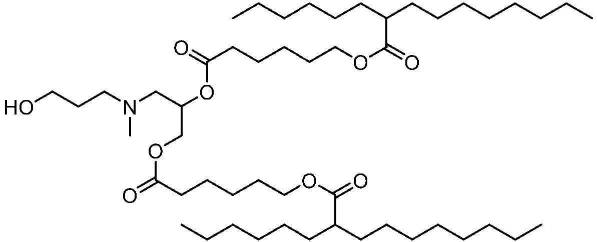 |
| 63 | 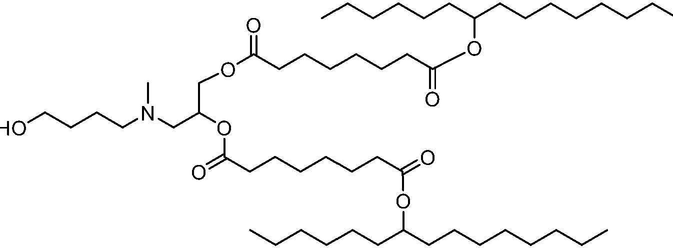 |
| 64 | 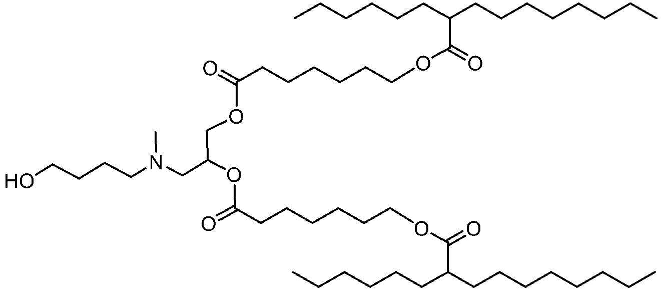 |
| 65 | 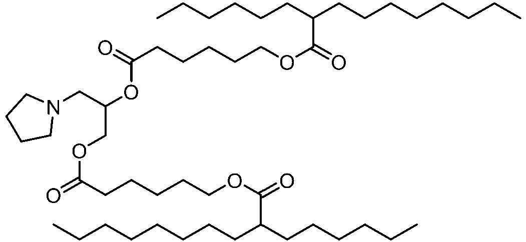 |
| 66 | 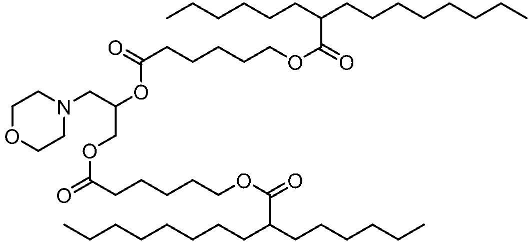 |
| 67 | 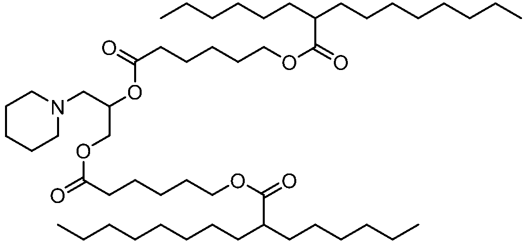 |
| 68 | 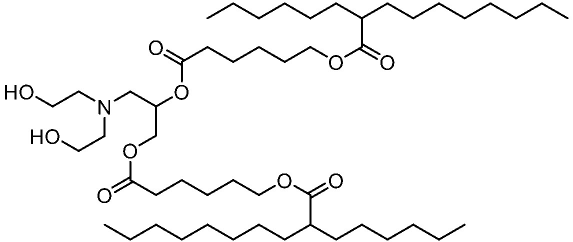 |
| 69 | 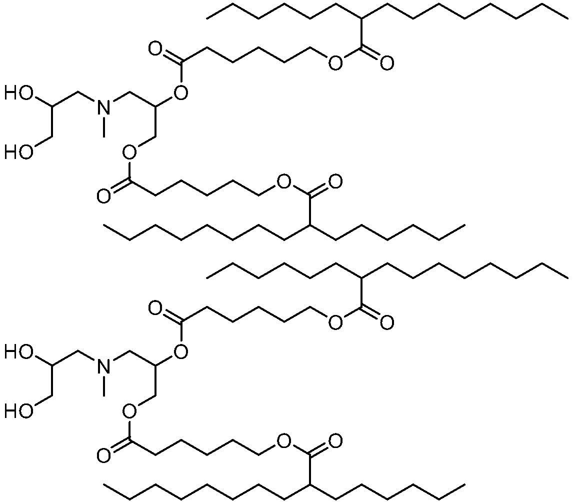 |
| 70 | 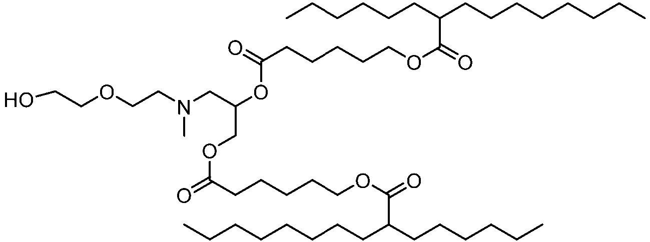 |
| 71 | 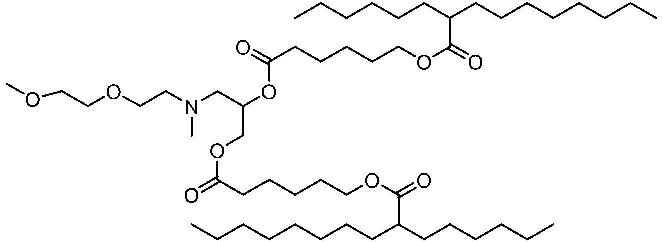 |
| 72 | 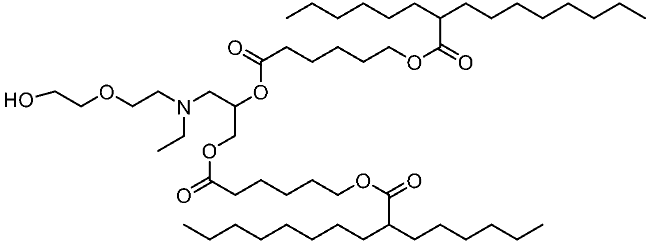 |
| 73 | 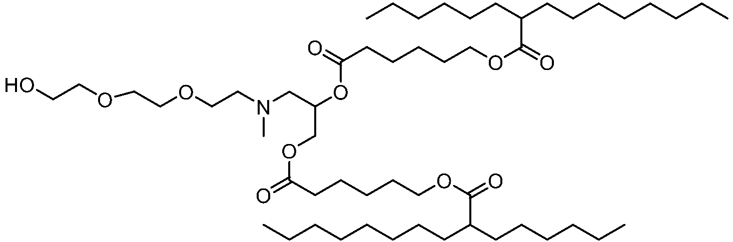 |
| 74 | 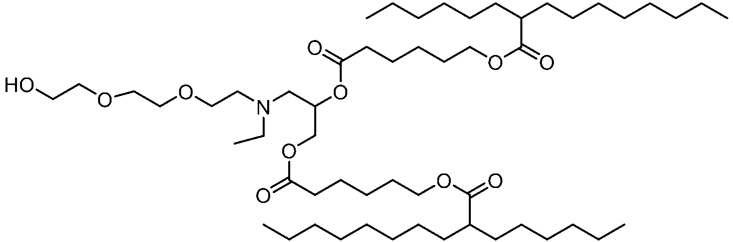 |
| 75 | 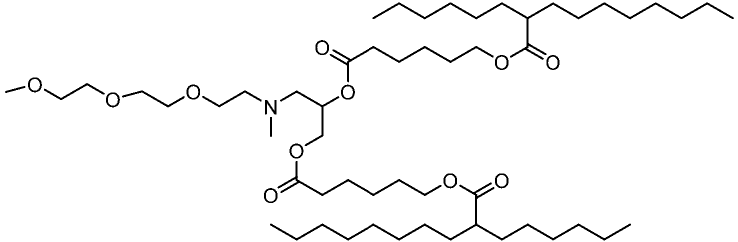 |
| 76 | 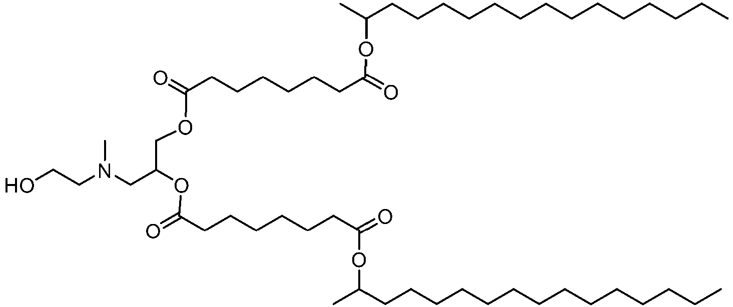 |
| 77 | 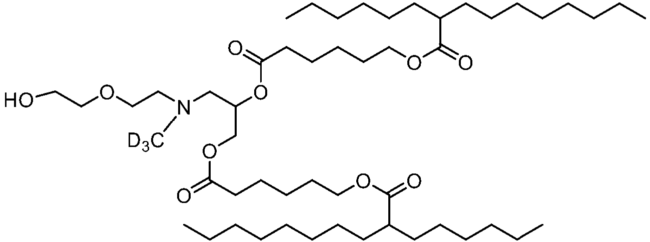 |
| 78 | 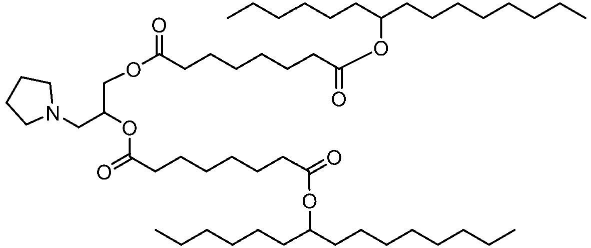 |
| 79 | 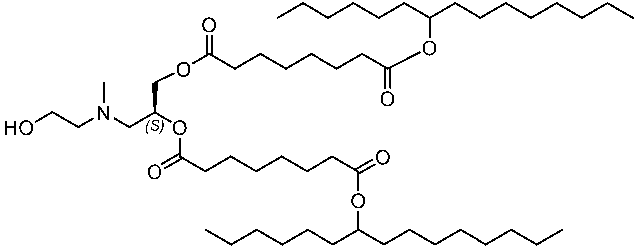 |
| 80 | 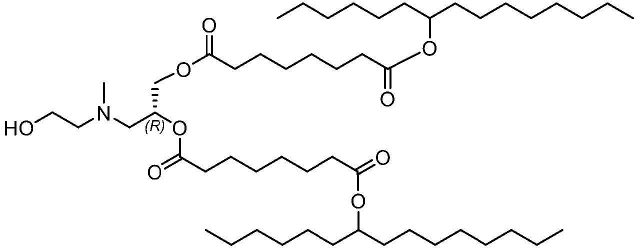 |
| 81 | 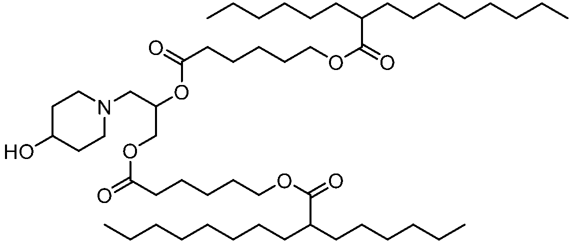 |
| 82 | 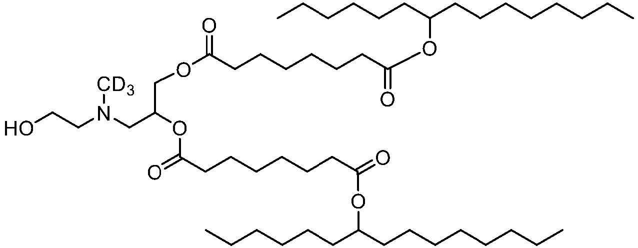 |

3. A lipid nanoparticle, comprising the compound or the pharmaceutically acceptable salt, prodrug, or stereoisomer thereof according to claim 1.

4. The lipid nanoparticle according to claim 3, further comprising a helper lipid, wherein the helper lipid is selected from one or more of a phospholipid, a steroid, a polymer-conjugated lipid, and a modifiable lipid.

5. The lipid nanoparticle according to claim 4, wherein the phospholipid is selected from any one of DOPE, DSPC, DPPC, DMPC, DOPC, POPC, and SM, or a combination thereof.

6. The lipid nanoparticle according to claim 4, wherein the steroid is selected from one or more of cholesterol, sitosterol, stigmasterol, and ergosterol, further preferably, the steroid is cholesterol and sitosterol.

7. The lipid nanoparticle according to claim 4, wherein the polymer-conjugated lipid is selected from a lipid conjugated with polyethylene glycol, polylactic acid, polyamide, cationic polymer, polysarcosine (pSar), poly lactic-co-glycolic acid (PLGA), polyamino acid, polypeptide or polypeptoid.

8. The lipid nanoparticle according to claim 7, wherein the polymer-conjugated lipid is a polyethylene glycol-conjugated lipid selected from one or more of ALC-0159, PEG1000-DMG, PEG5000-DMG, PEG2000-DMG, and PEG2000-DSPE.

9. The lipid nanoparticle according to claim 4, wherein the molar ratio of the compound or the pharmaceutically acceptable salt, prodrug or stereoisomer thereof according to claim 1 to the helper lipid is 1:(0.5-2), preferably 1:(0.6-1.5), further preferably 1:(0.8-1.2).

10. Use of the lipid nanoparticle according to any one of claims 3 to 9 in targeted delivery, wherein preferably, a target organ for the targeted delivery is the following organ: lung, heart, brain, spleen, lymph node, bone, skeletal muscle, stomach, small intestine, large intestine/colorectum, kidney, bladder, breast, testis, ovary, uterus, spleen, thymus, brainstem, cerebellum, spinal cord, eye, ear, tongue, or skin; preferably, the target organ is spleen.

11. A composition, comprising the lipid nanoparticle according to any one of claims 3 to 9 and a therapeutic/prophylactic agent, wherein preferably, the therapeutic/prophylactic agent is a nucleic acid; further preferably, the nucleic acid is selected from a single-stranded DNA, a double-stranded DNA, a single-stranded RNA, a double-stranded RNA, a short isomer, a plasmid DNA, a complementary DNA/cDNA, an antisense oligonucleotide/ASO, a small interference nucleic acid/siRNA, a small activating nucleic acid/saRNA, an asymmetric interfering nucleic acid/aiRNA, a micro nucleic acid/miRNA, a miRNA inhibitor (agomir or antagomir), a Dicer-substrate nucleic acid, a small hairpin nucleic acid (shRNA), a transfer RNA (tRNA), a messenger RNA/mRNA, a circular RNA/circRNA, a self-amplifying mRNA/samRNA, or an aptamer.

12. The composition according to claim 11, wherein the nucleic acid comprises a natural nucleotide, a nucleotide mimic, a functional analog, or a chemically modified nucleotide.

13. The composition according to any one of claims 11 and 12, wherein the composition has an average particle size of 90 nm to 600 nm, preferably 200 nm to 400 nm, further preferably 200 nm to 300 nm.

14. The composition according to any one of claims 11 and 12, wherein the composition has a polydispersity index (PDI) of 0.001 to 0.5, preferably 0.001 to 0.45, further preferably 0.001 to 0.4.

15. The composition according to any one of claims 11 and 12, wherein the mass ratio of the lipid nanoparticle to the therapeutic/prophylactic agent in the composition is 10:1 to 100:1, preferably 20:1 to 50:1, further preferably 20:1 to 30:1.

16. An agent, comprising the composition according to any one of claims 11 to 15 and a pharmaceutically acceptable auxiliary material.
